(19) Europäisches Patentamt
European Patent Office
Office européen des brevets

(11) **EP 2 407 102 A1**

(12) **EUROPEAN PATENT APPLICATION**

(43) Date of publication:
**18.01.2012 Bulletin 2012/03**

(51) Int Cl.:
*A61B 5/08* (2006.01)      *A61B 5/053* (2006.01)

(21) Application number: **11173346.5**

(22) Date of filing: **08.07.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **13.04.2011 JP 2011088773**
**13.04.2011 JP 2011088772**
**15.07.2010 JP 2010160949**

(71) Applicant: **Tanita Corporation**
**Tokyo 174-8630 (JP)**

(72) Inventor: **Masuo, Yoshihisa**
**Itabashi-ku, Tokyo 174-8630 (JP)**

(74) Representative: **Haley, Stephen**
**Gill Jennings & Every LLP**
**The Broadgate Tower**
**20 Primrose Street**
**London EC2A 2ES (GB)**

(54) **Respiration characteristic analysis apparatus and respiration characteristic analysis system**

(57)   A respiration characteristic analysis apparatus includes a bioelectrical impedance determiner adapted for determining bioelectrical impedance at a part including the right lung of a human subject and for determining bioelectrical impendance at a part including the left lung of the human subject; and a respiration depth calculator adapted for calculating a right lung respiration depth related to a respiration capability of the right lung of the human subject on the basis of change over time in the bioelectrical impedance at the part including the right lung determined by the bioelectrical impedance determiner, and for calculating a left lung respiration depth related to a respiration capability of the left lung of the human subject on the basis of change over time in the bioelectrical impedance at the part including the left lung determined by the bioelectrical impedance determiner.

Fig. 1

EP 2 407 102 A1

**Description**

FIELD OF THE INVENTION

The present invention relates to apparatuses and systems for analyzing characteristics of respiration (breathing) in human subjects.

RELATED ART

There have been known various apparatuses for measuring bioelectrical impedance and for estimating body conditions based on measured impedance. For example, US 2007/043302 A1 discloses a technique for estimating the breathing capacity of the lungs of a human subject on the basis of the impedance of the body trunk.
For healthy people, there is no difference in functions (difference in respirations capability) between the right lung and the left lung. On the other hand, for people with a disorder, e.g., a disorder (e.g., a disease) in the right or left lung, there may be significant differences in functions between the right lung and the left lung. However, in conventional techniques, it is difficult to determine accurately the respiration capability of each of the right lung and the left lung.
Accordingly, the present invention provides apparatuses and systems to determine accurately and easily the respiration capability of each of the right lung and the left lung of a human subject.

SUMMARY OF THE INVENTION

In accordance with the present invention, there is provided a respiration characteristic analysis apparatus including: a bioelectrical impedance determiner, adapted for determining a bioelectrical impedance at a part including the right lung of a human subject and for determining a bioelectrical impedance at a part including the left lung of the human subject; and a respiration depth calculator adapted for calculating a right lung respiration depth related to a respiration capability of the right lung of the human subject on the basis of change over time in the bioelectrical impedance at the part including the right lung determined by the bioelectrical impedance determiner, and for calculating a left lung respiration depth related to a respiration capability of the left lung of the human subject on the basis of change over time in the bioelectrical impedance at the part including the left lung determined by the bioelectrical impedance determiner.
According to the present invention, on the basis of change over time in the bioelectrical impedance at the part including the right lung, the respiration depth calculator calculates the right lung respiration depth related to the respiration capability of the right lung of the human subject, and on the basis of change over time in the bioelectrical impedance at the part including the left lung, the respiration depth calculator calculates the left lung respiration depth related to the respiration capability of the left lung of the human subject. Thus, respiration capability of each of the right lung and the left lung of a human subject can be measured accurately and easily. In addition, if the respiration characteristic analysis apparatus is used for breathing training of the human subject, an appropriate training menu can be decided on the basis of respiration capability of each of the right lung and the left lung.
The bioelectrical impedance determiner may be adapted for determining a first bioelectrical impedance at the right upper body trunk of the human subject including the upper lobe of the right lung of the human subject and excluding the abdomen of the human subject, and for determining a second bioelectrical impedance at the left upper body trunk of the human subject including the upper lobe of the left lung of the human subject and excluding the abdomen of the human subject. The respiration depth calculator may be adapted for calculating a first right lung respiration depth related to a respiration capability of the upper lobe of the right lung of the human subject on the basis of change over time in the first bioelectrical impedance, and for calculating a first left lung respiration depth related to a respiration capability of the upper lobe of the left lung of the human subject on the basis of change over time in the second bioelectrical impedance.
When a human performs respiratory actions that consist of inhalation and exhalation, the first bioelectrical impedance at the right upper body trunk and the second bioelectrical impedance at the left upper body trunk change. The bioelectrical impedance at the lungs increases at inhalations due to increase in the volume of air inside tissues in the lungs, and the bioelectrical impedance at the lungs decreases at exhalations due to decrease in the volume of air inside tissues in the lungs. Therefore, the first bioelectrical impedance at the right upper body trunk including the upper lobe of the right lung and excluding the abdomen and the second bioelectrical impedance at the left upper body trunk including the upper lobe of the left lung and excluding the abdomen increase at inhalations and decrease at exhalations. The greater the volume of air entering and leaving the right lung (the greater the ventilation volume of the right lung), the greater the amplitude of the first bioelectrical impedance. Since the amplitude of the first bioelectrical impedance corresponds to the ventilation volume of the upper lobe of the right lung, the first right lung respiration depth related to the respiration capability of the upper lobe of the right lung can be calculated on the basis of change over time in the first bioelectrical impedance. On the other hand, the greater the volume of air entering and leaving the left lung (the greater the ventilation volume of the left lung), the greater the amplitude of the second bioelectrical impedance. Since the amplitude of the second bioelectrical impedance corresponds to the ventilation volume of the upper lobe of the left lung, the first left lung respiration depth related to the respiration capability of the upper lobe of the left lung can be calculated on the basis of change over time in the second bioelectrical impedance. Therefore, the respiration capability of the upper lobe of the right lung and the respiration capability of the upper lobe of the left lung can be measured accurately and easily.

It can be envisioned to adhere current electrodes and voltage electrodes to the surface of body trunk outside the right lung in order to measure the bioelectrical impedance at the part including the right lung. It can also be envisioned to adhere current electrodes and voltage electrodes to the surface of body trunk outside the left lung in order to measure the bioelectrical impedance at the part including the lett lung. However, it is troublesome to adhere the current electrodes and voltage electrodes to the human subject. When the limb-lead eight-electrode method is used, current electrodes and voltage electrodes are deployed at both palms and both soles, and it is possible to measure the first bioelectrical impedance at the right upper body trunk ofthe human subject including the upper lobe of the right lung of the human subject and excluding the abdomen of the human subject and the second bioelectrical impendance at the left upper body trunk of the human subject including the upper lobe of the left lung of the human subject and excluding the abdomen of the human subject. It is unnecessary to adhere current electrodes and voltage electrodes to the body trunk of the human subject.

The respiration characteristic analysis apparatus may further include a first centering value generator adapted for generating a first centering value that is an average of the first bioelectrical impedances within a past unit time on the basis of change over time in the first bioelectrical impedance, and for generating a second centering value that is an average of the second bioelectrical impedances within a past unit time on the basis of change over time in the second bioelectrical impedance, the first centering value being a standard level of change over time in the first bioelectrical impedance, the second centering value being a standard level of change over time in the second bioelectrical impedance; a first difference calculator adapted for calculating a first difference between the first bioelectrical impedance and the first centering value; and a second difference calculator adapted for calculating a second difference between the second bioelectrical impedance and the second centering value. The respiration depth calculator may be adapted for calculating the first right lung respiration depth on the basis of the first difference, and for calculating the first left lung respiration depth on the basis of the second difference.

In this aspect, the respiration depth calculator calculates the first right lung respiration depth and the first left lung respiration depth, on the basis of the first difference and the second difference. The respiration capability of the upper lobe of the right lung and the respiration capability of the upper lobe of the left lung can be determined accurately.

The respiration depth calculator may be adapted for calculating, at every respiration of the human subject, the first right lung respiration depth that is a sum of absolute values of a local maximum and a local minimum of the first differences within a single respiration. The respiration depth calculator may be adapted for calculating, at every respiration of the human subject, the first left lung respiration depth that is a sum of absolute values of a local maximum and a local minimum of the second differences within a single respiration.

The bioelectrical impedance determiner may be adapted for determining the first bioelectrical impedance and the second bioelectrical impedance at each sampling time occurring at a predetermined cycle. The first centering value generator may be adapted for generating the first centering value on the basis of the first bioelectrical impedance at each of sampling times, the number of the sampling times being predetermined. The first centering value generator may be adapted for generating the second centering value on the basis of the second bioelectrical impedance at each of the sampling times, the number of the sampling times being predetermined.

In this case, it is possible to obtain a suitable first centering value and a suitable second centering value even if one or more instantaneous values of the first bioelectrical impedance and the second bioelectrical impedance are disturbed by body motion or for other reasons.

More specifically, the first centering value generator may be adapted for calculating a first moving average at each sampling time, the first moving average being a moving average of the first bioelectrical impedances at multiple sampling times within a centering period starting from a time point that is a predetermined time length before a current sampling time and ending at the current sampling time. The first centering value generator is adapted for generating the first centering value at the current sampling time on the basis of the first moving averages at multiple sampling times. The first centering value generator may be adapted for calculating a second moving average at each sampling time, the second moving average being a moving average of the second bioelectrical impedances at multiple sampling times within the centering period. The first centering value generator is adapted for generating the second centering value at the current sampling time on the basis of the second moving averages at multiple sampling times.

In this case, it is possible to obtain a suitable first centering value and a suitable second centering value even if one or more instantaneous values of the first bioelectrical impedance and the second bioelectrical impedance are disturbed by body motion or for other reasons.

The time length of the centering period may be variable and may be set depending on the respiration speed of the human subject at the current sampling time. The moving average may be calculated with or without the use of weighting factors. For example, the moving average may be calculated with the use of weighting factors depending on the frequency at each sampling time.

The bioelectrical impedance determiner may be adapted for determining a third bioelectrical impedance at the right middle body trunk of the human subject including the median and lower lobes of the right lung of the human subject and the abdomen of the human subject at each sampling time, and may be adapted for determining a fourth bioelectrical impedance at the left middle body trunk of the human subject including the median and lower lobes of the left lung of the human subject and the abdomen of the human subject at each sampling time. The respiration characteristic analysis apparatus may further include: a second centering value generator adapted for generating a third centering value that is an average of the third bioelectrical impedances within a past unit time on the basis of change over time in the third bioelectrical impedance, and for generating a fourth centering value that is an average of the fourth bioelectrical impedances within a past unit time on the basis of change over time in the fourth bioelectrical impedance, the third centering value being a standard level of change over time in the third bioelectrical impedance, the fourth centering value being a standard level of change over time in the fourth bioelectrical impedance; a third differences calculator adapted for calculating a third difference between the third bioelectrical impedance and the third centering value; a fourth difference calculator adapted for calculating a fourth difference between the fourth bioelectrical impedance and the fourth centering value; and a zero-cross time decider adapted for deciding first zero-cross times in which the first bioelectrical impedance is equal to the first centering value, and for deciding second zero-cross times in which the second bioelectrical impedance is equal to the second centering value. The second centering value generator may be adapted for generating a third centering value on the basis of the third bioelectrical impedances at the first zero-cross times decided by the zero-cross time decider, and for generating a fourth centering value on the basis of the fourth bioelectrical impedances at the second zero-cross times decided by the zero-cross time decider. The respiration depth calculator may be adapted for calculating a second right lung respiration depth related to a respiration capability of the median and lower lobes of the right lung of the human subject on the basis of the third difference, and for calculating a second left lung respiration depth related to a respiration capability of the median and lower lobes of the left lung of the human subject on the basis of the fourth difference.

When a human performs respiratory actions, the bioelectrical impedance at the middle body trunk changes. The greater the ventilation volume of the right lung, the greater the amplitude of the third bioelectrical impedance at the right middle body trunk including the median and lower lobes of the right lung and the abdomen. The greater the ventilation volume of the left lung, the greater the amplitude of the fourth bioelectrical impedance at the left middle body trunk including the median and lower lobes of the left lung and the abdomen. Accordingly, whereas the second right lung respiration depth related to the respiration capability of the median and lower lobes of the right lung of the human subject is calculated on the basis of change over time in the third bioelectrical impedance, the second left lung respiration depth related to the respiration capability of the median and lower lobes of the left lung of the human subject is calculated on the basis of change over time in the fourth bioelectrical impedance.

In abdominal respiration, by the action of the abdominal skeletal muscle, the visceral tissue raises the diaphragm at exhalations, so that the abdominal bioelectrical impedance increases, In other words, increase in the bioelectrical impedance of the abdominal region beneath the diaphragm cancels decrease in the bioelectrical impedance at the chest region above the diaphragm when the human subject performs exhalation in abdominal respiration. The same is not true for costal respiration. Consequently, when respiration of the human subject is abdominal respiration, change in the third bioelectrical impedance at the right middle body trunk including the median and lower lobes of the right lung and the abdomen is not sinusoidal and is different from that of the first bioelectrical impedance. Accordingly, in contrast to the calculation of the first centering value and the second centering value, if a moving average is calculated on the basis of measurement values of the third bioelectrical impedance at the sampling times of which the number is predetermined, the third centering value (standard level of change over time in the third bioelectrical impedance used for extracting information on respiration of the human subject) cannot be calculated accurately. The same is true for the fourth centering value. Accordingly, in this aspect, the second centering value generator may be adapted for generating the third centering value on the basis of the third bioelectrical impedance at each of zero-cross times decided by the first zero-cross time decider, and for generating the fourth centering value on the basis of the fourth bioelectrical impedance at each of zero-cross times decided by the second zero-cross time decider. In this case, the third centering value and the fourth centering value are accurately calculated on the basis of change over time in the third bioelectrical impedance and the fourth bioelectrical impendance even if respiration of the human subject is abdominal respiration.

More specifically, the second centering value generator may be adapted for deciding whether or not each sampling time is a first zero-cross time, and for generating the third centering value at the current sampling time on the basis of the third bioelectrical impedances including the third bioelectrical impedance at the current sampling time if the current sampling time is a first zero-cross time. The centering value generator may be adapted for deciding the third centering value generated at a last sampling time as the third centering value at the current sampling time if the current sampling time is not a first zero-cross time.

The second centering value generator may be adapted for deciding whether or not each sampling time is a second zero-cross time, and for generating the fourth centering value at the current sampling time on the basis of the fourth bioelectrical impedances including the fourth bioelectrical impedance at the current sampling time if the current sampling time is a second zero-cross time. The centering value generator may be adapted for deciding the fourth centering value generated at a last sampling time as the fourth centering value at the current sampling time if the current sampling time is not a second zero-cross time.

The respiration depth calculator may be adapted for calculating, at every respiration of the human subject, the second right lung respiration depth that is a sum of absolute values of a local maximum and a local minimum of the third differences within a single respiration. The respiration depth calculator may be adapted for calculating, at every respiration of the human subject, the second left lung respiration depth that is a sum of absolute values of a local maximum and a local minimum of the fourth differences within a single respiration.

The bioelectrical impedance determiner may be adapted for determining a third bioelectrical impedance at the right middle body trunk of the human subject including the median and lower lobes of the right lung of the human subject and the abdomen of the human subject, and for determining a fourth bioelectrical impedance at the left middle body trunk of the human subject including the median and lower lobes of the left lung of the human subject and the abdomen of the human subject, The respiration depth calculator may be adapted for calculating a second right lung respiration depth related to a respiration capability of the median and lower lobes of the right lung of the human subject on the basis of change over time in the third bioelectrical impedance, and may be adapted for calculating a second left lung respiration, depth related to a respiration capability of the median and lower lobes of the left lung of the human subject on the basis of change over time in the fourth bioelectrical impedance.

In this case, without determining the first right lung respiration depth and the first left lung respiration depth, the second right lung respiration depth related to the respiration capability of the median and lower lobes of the right lung of the human subject and the second left lung respiration depth related to the respiration capability of the median and lower lobes of the left lung of the human subject may be determined. The limb-lead eight-electrode method may be used, in which current electrodes and voltage electrodes are deployed at both palms and both soles to determine the third bioelectrical impedance at the right middle body trunk and the fourth bioelectrical impedance at the left middle body trunk. Then, it is unnecessary to adhere current electrodes and voltage electrodes to the body trunk of the human subject.

The bioelectrical impedance determiner may be adapted for determining a first bioelectrical impedance at the right upper body trunk of the human subject including the upper lobe of the right lung of the human subject and excluding the abdomen of the human subject, for determining a second bioelectrical impedance at the left upper body trunk of the human subject including the upper lobe of the left lung of the human subject and excluding the abdomen of the human subject, for determining a third bioelectrical impedance at the right middle body trunk of the human subject including the median and lower lobes of the right lung of the human subject and the abdomen of the human subject, and for determining a fourth bioelectrical impedance at the left middle body trunk of the human subject including the median and lower lobes of the left lung of the human subject and the abdomen of the human subject. The respiration depth calculator may be adapted for calculating the right lung respiration depth related to the respiration capability of the right lung of the human subject on the basis of change over time in each of the first bioelectrical impedance and the third bioelectrical impedance, and for calculating the left lung respiration depth related to the respiration capability of the left lung of the human subject on the basis of change over time in each of the second bioelectrical impedance and the fourth bioelectrical impedances,

Consequently, as the right lung respiration depth related to the respiration capability of the right lung of the human subject, both of the first right lung respiration depth and the second right lung respiration depth may be calculated. In addition, as the left lung respiration depth related to the respiration capability of the left lung of the human subject, both of the above-described first left lung respiration depth and the second left lung respiration depth may be calculated.

The respiration characteristic analysis apparatus may further include a display data generator adapted for generating first display data for displaying a first Lissajous figure showing change over time in the first bioelectrical impedance and change over time in the third bioelectrical impedance in an orthogonal coordinate system having two orthogonal coordinate axes in which a first axis is the first bioelectrical impedance and a second axis is the third bioelectrical impedance, and for generating second display data for displaying a second Lissajous figure showing change over time in the second bioelectrical impedance and change over time in the fourth bioelectrical impedance in an orthogonal coordinate system having two orthogonal coordinate axes in which a first axis is the second bioelectrical impedance and a second axis is the fourth bioelectrical impedance.

Two orthogonal coordinate axes may be, for example, an X axis and a Y axis. However, two orthogonal coordinate axes may be two axes obtained by inclining the X axis and the Y axis by 45 degrees. The Lissajous figure may show the status of only a single respiration as shown in Fig. 55 or 56, or may show the status of multiple respirations continually as shown in Fig. 57 or Fig. 58. The respiration characteristic analysis apparatus may include a display device for displaying the Lissajous figure, or an output part for outputting display data for displaying the Lissajous figure to an external display device.

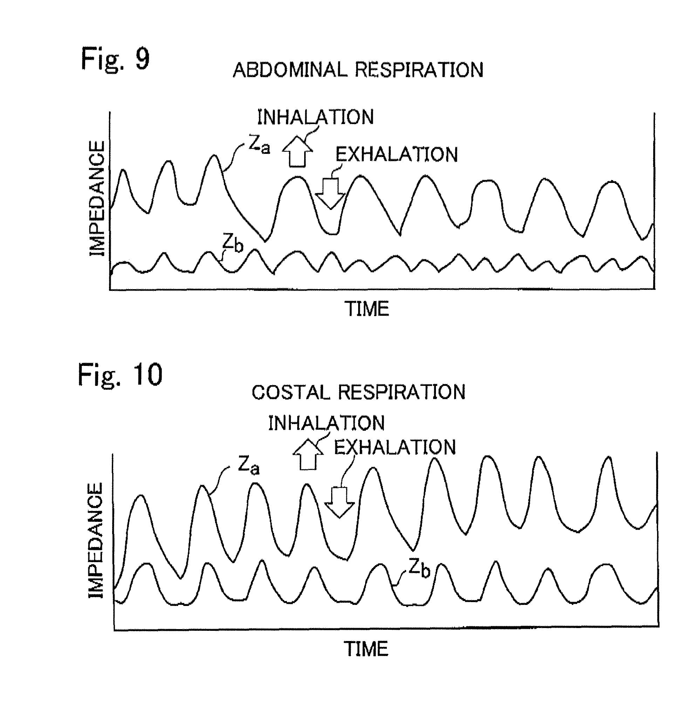

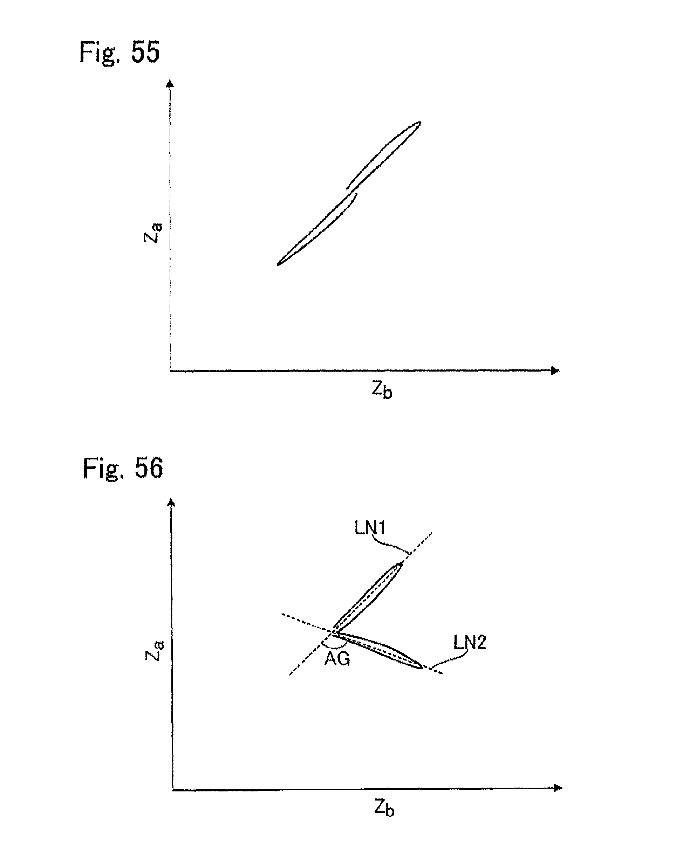

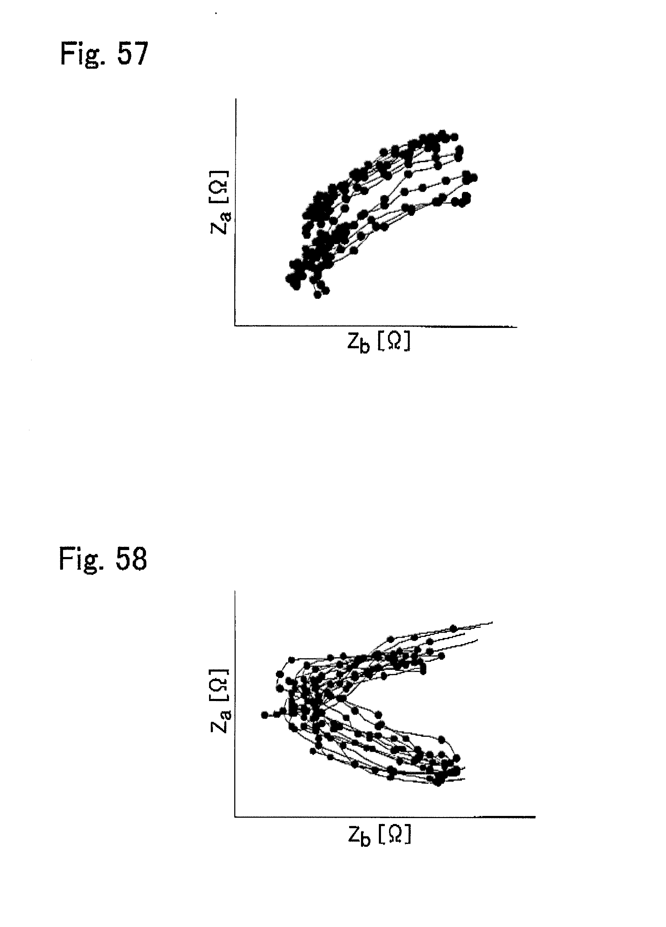

When respiration of the human subject is costal respiration, as shown in Fig. 10, both of the bioelectrical

impedance $Z_a$ at the upper body trunk and the bioelectrical impedance $Z_b$ at the middle body trunk increase at inhalations, whereas both of the bioelectrical impedance $Z_a$ at the upper body trunk and the bioelectrical impedance $Z_b$ at the middle body trunk decrease at exhalations. Therefore, when the ratio of costal respiration in respiration is extremely high, the track of the Lissajous figure is of an inclined straight shape as shown in Fig. 55 or Fig. 57. When costal respiration is shallow, the track of the Lissajous figure is small. When costal respiration is deep, the track of the Lissajous figure is large.

In contrast, in abdominal respiration, as shown in Fig. 9, both of the first bioelectrical impedance $Z_a$ and the second bioelectrical impedance $Z_b$ increase at inhalations, but the first bioelectrical impedance $Z_a$ decreases, whereas the second bioelectrical impedance $Z_b$ increases at exhalation, Therefore, when respiration includes abdominal respiration, the track of the Lissajous figure for the right or left lung is of a bent shape, as shown in Fig. 56 or Fig. 58.

The Lissajous figure shown in Fig. 56 indicates a case in which 50% of a single respiration is costal and 50% of the single respiration is abdominal. In this case, the track of the Lissajous figure is of a boomerang shape (an L-shape) that is symmetric with respect to a horizontal line. However, if the percentage of abdominal respiration is less than that of costal respiration, the upper upward-sloping portion of the track of the Lissajous figure corresponding to costal respiration is larger than that in Fig. 56, whereas the lower downward-sloping portion of the track of the Lissajous figure corresponding to abdominal respiration is smaller than that in Fig. 56. If the percentage of abdominal respiration is greater than that of costal respiration, the upper upward-sloping portion of the track of the Lissajous figure corresponding to costal respiration is smaller than that in Fig. 56, whereas the lower downward-sloping portion of the track of the Lissajous figure corresponding to abdominal respiration is larger than that in Fig. 56. Thus, depending on the percentage of abdominal respiration, the track of the Lissajous figure describes various tracks.

Theoretically, when abdominal respiration occupies 100% of respiration, the track of the Lissajous figure is of an inclined straight shape in which the inclination is opposite to that in costal respiration. However, respiration of human beings must include costal respiration, except for those in which the diaphragms do not work at all due to a disorder (e.g., a disease). This can be confirmed by observing that even if a human stops breathing, when the human expands and contracts the abdomen, the diaphragm moves up and down so as to expand and contract the lungs. Accordingly, even if the human subject performs abdominal respiration as much as possible, the track of the Lissajous figure is of a bent shape having a straight portion corresponding to costal respiration.

The bend angle AG formed between the straight portion (approximate straight line LN1) corresponding to costal respiration and the straight portion (approximate straight line LN2) corresponding to abdominal respiration shown in Fig. 56 is small when abdominal respiration is shallow. When abdominal respiration is deep, the bend angle AG is large. In addition, the shallower the abdominal respiration, the smaller the track of the Lissajous figure.

Thus, the track of the Lissajous figure varies depending on whether or not respiration is costal or abdominal. The size and the shape of the track of the Lissajous figure vary depending on the magnitude (depth) of each of costal respiration and abdominal respiration. By observing the Lissajous figure, the human subject or other person can understand whether current respiration of the human subject is costal or abdominal, or can understand whether respiration of the human subject is mainly dependent on costal respiration or abdominal, respiration. The human subject or another person can also understand the magnitude of each of costal respiration and abdominal respiration by the Lissajous figure. Accordingly, the respiration characteristic analysis apparatus can be used as a breathing training apparatus.

When the human subject trains for costal breathing, the human subject may pay attention to the Lissajous figure so that the track of the Lissajous figure becomes an inclined straight shape and the size of the track becomes large. When the human subject trains for abdominal breathing, the human subject may pay attention to the Lissajous figure so that the track of the Lissajous figure is of a bent shape, and the size and the bend angle AG become large. Thus, by observing the Lissajous figure and confirming the type and the magnitude of respiration at any time, the human subject can train for appropriate costal or abdominal breathing.

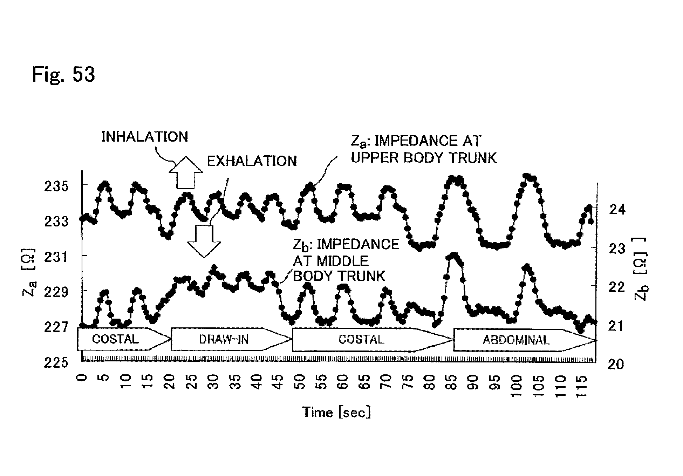

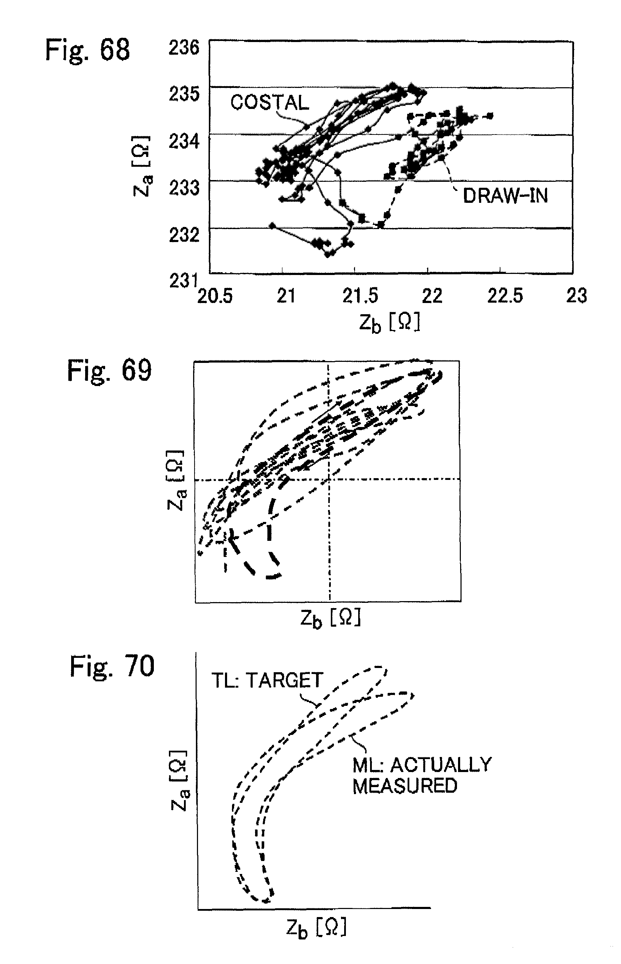

When respiration of the human subject is draw-in respiration, both of the bioelectrical impedance $Z_a$ at the upper body trunk and the bioelectrical impedance $Z_b$ at the middle body trunk increase at inhalations whereas both of the bioelectrical impedance $Z_a$ at the upper body trunk and the bioelectrical impedance $Z_b$ at the middle body trunk at exhalations. The manner of change is the same as that in costal respiration since human beings expand and contract the thoracic cage in both of costal respiration and draw-in respiration. However, in draw-in respiration, the abdomen is held in a constricted position continually so as to be stressed continually. Therefore, the standard level of the bioelectrical impedance $Z_b$ at the middle body in draw-in respiration is higher than that in costal respiration as shown in Fig. 53. For this reason, as in Fig. 68 which shows Lissajous figures in draw-in respiration and costal respiration, both tracks of the Lissajous figures are of a straight shape rising from bottom left to top right, but locations of both tracks are different in the axis indicating the bioelectrical impedance $Z_b$ at the middle body trunk (the X axis in Fig. 68).

Thus, by observing the Lissajous figure, the human subject or another person can understand whether or not respiration of the human subject is draw-in respiration. The shallower the draw-in respiration, the smaller the track of the Lissajous figure, so that the magnitude of the draw-in respiration can be understood from the Lissajous figure. By observing the Lissajous figure and confirming the type and the magnitude of respiration at any time, the human subject can train for appropriate draw-in breathing.

As has been described above, by virtue of displaying the first and second Lissajous figures for the right lung and the left lung, the human subject or other person can understand the type and magnitude of respiration of the human subject, and can understand whether or not the human subject is performing appropriately the target type of breathing with respect to both lungs. In addition, by comparing the first Lissajous figure with the second Lissajous figure, the difference between the respiration capabilities of the right lung and the left lung can be understood easily.

Since two Lissajous figures for the right lung and the left lung are displayed, it is possible to train for breathing of the right lung and the left lung, respectively. There is no significant difference between the respiration capabilities of the right lung and the left lung of a physically unimpaired person. However, if one of the right lung and the left lung has been diseased, there is a significant difference between the respiration capabilities of the right lung and the left lung. If one of the right lung and the left lung was previously diseased, there may be a difference between the respiration capabilities of the right lung and the left lung. A method for improving the respiration capability of only the left lung is one in which the human subject repeats respiration while a load is applied to the left lung by positioning the left arm behind the right shoulder and pushing the left elbow backward with the right hand. This method is suitable for, for example, a person whose respiration capability of the left lung is lower than the respiration capability of the right lung.

In respiratory inductance plethysmography, the variation ratio in the costal circumference $R_{rc}$ (%) and the variation ratio in the abdominal circumference $R_{abd}$ (%) in respiration may be determined on the basis of variation of inductance of each coil wound around human bodies. The coils are incorporated into bands that can be wound around the chest (at the level of the ensiform cartilage) and the abdomen (at the level of the navel). In the field of respiratory inductance plethysmography, the Konno-Mead Diagram is known, which is a Lissajous figure in which, for example, the X axis is the abdominal displacement $R_{abd}$ whereas the Y axis is the rib cage displacement $R_{rc}$.

However, in the respiratory inductance plethysmography, bands must be deployed around the chest and the abdomen of the human subject. In addition, if the human subject is conscious of measurements or feels nervous in measurements, the measurements of the rib cage displacement $R_{rc}$ and the abdominal displacement $R_{abd}$ are disturbed. When the human subject is asleep, the measurements in the respiratory inductance plethysmography may be of high reliability. However, when the human subject is awake, the measurements in the respiratory inductance plethysmography may be of lower reliability

In contrast, in determination with the use of bioelectrical impedances, for example, when the limb-lead eight-electrode method is used, current electrodes and voltage electrodes are deployed at both palms and both soles. Then, it is unnecessary to adhere current electrodes and voltage electrodes to the body trunk of the human subject, or to restrict the human body. In addition, for example, the bioelectrical impedance $Z_a$ at the upper body trunk is about 80 percent dependent on the air entering and leaving the lungs, and only 20 percent dependent on the respiratory muscle. Accordingly, even if the human subject is conscious of measurements or feels nervous in measurements, reliability of measurements may be enhanced in comparison with the respiratory inductance plethysmography. In addition, determination with the use of bioelectrical impedances is more reliable since it is more sensitive to actions related to respiration, e.g., the flow of air into and from the lungs, and the vertical movement of the diaphragm.

Therefore, the Lissajous figure obtained from the determination of bioelectrical impedances is more reflective of actions related to respiration, e.g., the flow of air into and from the lungs, and the vertical movement of the diaphragm in comparison with the Lissajous figure obtained by the respiratory inductance plethysmography. In addition, as described above, in the Lissajous figure obtained by the respiratory inductance plethysmography, for example, the X axis is the abdominal displacement $R_{abd}$, whereas the Y axis is the rib cage displacement $R_{rc}$. In this case, the track of the Lissajous figure for a single costal respiration and the track of the Lissajous figure for a single abdominal respiration are of a straight shape rising from bottom left to top right. The inclination angle of the upward-sloping track of the Lissajous figure with respect to the X axis is larger (nearer to 90 degrees) when the ratio of costal respiration in respiration is higher. Consequently, the shapes of the tracks of the Lissajous figures for costal respiration and abdominal respiration obtained by the respiratory inductance plethysmography are similar to each other although the inclination angles are different from each other, so that the type of respiration cannot be easily understood from the shape of the Lissajous figure.

The same is true for a Lissajous figure, which is similar to the Konno-Mead Diagram, using the costal circumference $R_{ib}$ and the abdominal circumference $A_b$ measured by Respitrace (Trademark, AMI Inc, Ardsley, New York, U.S.A.).

The display data generator may be adapted for generating the first display data for displaying the first Lissajous figure and the second display data for displaying the second Lissajous figure so that the first Lissajous figure and the second Lissajous figure are overlaid on a screen. In this case, since the first Lissajous figure for the right lung and the second Lissajous figure for the left lung are overlaid on a screen, it is possible to easily understand the difference between the respiration capabilities of the right lung and the left lung.

The display data generator may be adapted for generating the first display data for displaying the first Lissajous figure and the second display data for displaying the second Lissajous figure so that a displaying manner for the first Lissajous figure is different from a displaying manner for the second Lissajous figure. In this case, it is possible to easily distinguish the first and second Lissajous figures in view of the variation of the displaying manner (e.g., color or line type) although the first and second Lissajous figures are overlaid on the screen.

The respiration characteristic analysis apparatus may further include a track analyzer adapted for detecting differences between a track of the first Lissajous figure and a track of the second Lissajous figure. The display data generator may be adapted for generating the first display data for displaying the first Lissajous figure and the second display data for displaying the second Lissajous figure so that the differences are highlighted on a screen. In this case, it is possible to easily understand the difference between the respiration capabilities of the right lung and the left lung.

The respiration characteristic analysis apparatus may further include a local-maximum-and-minimum decider adapted for deciding a first local maximum that is a local maximum of change in the first bioelectrical impedance, for deciding a first local minimum that is a local minimum of change in the first bioelectrical impedance, for deciding a second local maximum that is a local maximum of change in the second bioelectrical impedance, for deciding a second local minimum that is a local minimum of change in the second bioelectrical impedance, for deciding a third local maximum that is a local maximum of change in the third bioelectrical impedance, for deciding a third local minimum that is a local minimum of change in the third bioelectrical impedance, for deciding a fourth local maximum that is a local maximum of change in the fourth bioelectrical impedance, and for deciding a fourth local minimum that is a local minimum of change in the fourth bioelectrical impedance. The display data generator may be adapted for generating the first display data for displaying the first Lissajous figure so that a range of the first Lissajous figure on a screen in which the first and second Lissajous figures are displayed in the first axis is adjusted on the basis of the first local maximum and the first local minimum, and a range of the first Lissajous figure on the screen in the second axis is adjusted on the basis of the third local maximum and the third local minimum. The display data generator may be adapted for generating the second display data for displaying the second Lissajous figure so that a range of the second Lissajous figure on the screen in the first axis is adjusted on the basis of the second local maximum and the second local minimum, and a range of the second Lissajous figure on the screen in the second axis is adjusted on the basis of the fourth local maximum and the fourth local minimum.

In this case, the first and second Lissajous figures for the right lung and the left lung can be displayed at a suitable size with respect to the screen by adjusting the range in the first and second axes, and can be centered with respect to the screen, so that visualization of the Lissajous figures can be facilitated.

The respiration characteristic analysis apparatus may further include a local-maximum-and-minimum decider adapted for deciding a first local maximum that is a local maximum of change in the first bioelectrical impedance, for deciding a first local minimum that is a local minimum of change in the first bioelectrical impedance, for deciding a second local maximum that is a local maximum of change in the second bioelectrical impedance, for deciding a second local minimum that is a local minimum of change in the second bioelectrical impedance, for deciding a third local maximum that is a local maximum of change in the third bioelectrical impedance, for deciding a third local minimum that is a local minimum of change in the third bioelectrical impedance, for deciding a fourth local maximum that is a local maximum of change in the fourth bioelectrical impedance, and for deciding a fourth local minimum that is a local minimum of change in the fourth bioelectrical impedance. When the display data generator generates the first display data for displaying the first Lissajous figure and the second display data for displaying the second Lissajous figure, the display data generator may be adapted for executing a first range adjustment process in which a range of the first Lissajous figure on a screen in which the first and second Lissajous figures are displayed in the first axis is adjusted on the basis of the first local maximum and the first local minimum, whereas a range of the second Lissajous figure on the screen in the first axis is adjusted on the basis of the second local maximum and the second local minimum, and may be adapted for executing a second range adjustment process in which a range of the first Lissajous figure on the screen in the second axis is adjusted on the basis of the third local maximum and the third local minimum, whereas a range of the second Lissajous figure on the screen in the second axis is adjusted on the basis of the fourth local maximum and the fourth local minimum. The display data generator may be adapted for executing the second range adjustment process less frequently than that for the first range adjustment process.

As in Fig. 68 which shows Lissajous figures in draw-in respiration and costal respiration, both tracks of the Lissajous figures are of a straight shape rising from bottom left to top right, but positions of both tracks are different in the axis indicating the second bioelectrical impedance $Z_b$ (the X axis in Fig. 68). If adjustment of the range of the Lissajous figures is repeated at small intervals, the Lissajous figures will be always displayed at the center of the screen, and it will be difficult to understand whether respiration of the human subject is draw-in respiration or costal respiration from observation of the Lissajous figure. In this aspect, the second range adjustment process is executed less frequently than that for the first location centering process. For example, the first range adjustment process may be executed at every respiration, whereas the second range adjustment process may be executed only once (for example, at an initial stage of the process). It will be easy to understand whether respiration of the human subject is draw-in respiration or costal respiration from observation of the Lissajous figures. This is because the locations of tracks of the Lissajous figures for draw-in respiration and costal respiration will become different in the second axis for a certain period, even though the shapes of the tracks are similar. In addition, it is possible to reduce power consumption at the respiration characteristic analysis apparatus by reducing the frequency of the second range adjustment process. Although the frequency is less, by executing the second range adjustment process, two Lissajous figures for the right lung and the left lung can be displayed at a suitable size with respect to the screen.

The respiration characteristic analysis apparatus may further include a display data generator adapted for generating display data for displaying a Lissajous figure showing change over time in the first bioelectrical impedance and change over time in the third bioelectrical impedance in an orthogonal coordinate system having two orthogonal coordinate axes in which a first axis is the first bioelectrical impedance and a second axis is the third bioelectrical impedance, or for generating display data for displaying a Lissajous figure showing change over time in the second bioelectrical impedance and change over time in the fourth bioelectrical impedance in an orthogonal coordinate system having two orthogonal coordinate axes in which a first axis is the second bioelectrical impedance and a second axis is the fourth bioelectrical impedance. Thus, it is possible to display either one of the Lissajous figures for the right lung and the left lung.

In this case, the display data generator may be adapted for generating the display data for displaying the Lissajous figure so that a displaying manner for a track of the Lissajous figure for a latest single respiration is different from a displaying manner for a track of the Lissajous figure for past respirations.

In a Lissajous figure that continually shows status of multiple respirations, for example, as shown in Fig. 57 or 58, if the manner for displaying the track within a single respiration is the same as that for the track within another single respiration, it is difficult to identify the track for the latest single respiration. However, in this aspect, it is possible to easily understand the track for the latest single respiration in view of the variation of the displaying manner (e.g., color or line type).

The display data generator may be adapted for generating the display data for displaying the Lissajous figure so that a displaying manner for tracks of the Lissajous figure is changed depending on an elapsed time.

For example, the display data generator may lighten the color as the elapsed time increases. In this case, the newer the track, the fainter the color of the track. It is easy to identify the tracks for newer respiration (e.g., the track for the latest respiration).

The display data generator may be adapted for further generating target display data for displaying a target Lissajous figure showing a target model of breathing having a type and a magnitude of respiration to be performed by the human subject for guiding the human subject to perform breathing.

In this case, in addition to the measured Lissajous figure showing the status of breathing of the human subject, a target Lissajous figure showing a target model of breathing to be performed by the human subject is displayed. Thus, the human subject can train for breathing comparing both of the Lissajous figures. The human subject may focus on making the track of the Lissajous figure showing the status of breathing of the human subject coincide with the track of the target Lissajous figure, so as to learn the target breathing. Thus, the human subject is effectively guided to perform appropriate breathing by the use of the Lissajous figure for breathing guidance.

The display data generator may be adapted for generating the display data for the measured Lissajous figure and the target display data so that the measured Lissajous figure showing the status of breathing of the human subject and the target Lissajous figure are overlaid on a screen. In this case, it is possible to easily understand the difference between the target respiration and the actual respiration. The display data generator may be adapted for generating the display data for the measured Lissajous figure and the target display data so that a displaying manner for a track of the measured Lissajous figure is different from a displaying manner for a track of the target Lissajous figure. In this case, it is possible to easily distinguish the Lissajous figures in view of the variation of the displaying manner (e.g., color or line type) although the measured Lissajous figure showing the status of breathing of the human subject and the target Lissajous figure are overlaid on the screen.

The respiration characteristic analysis apparatus may further include: an inclination angle calculator adapted for calculating an inclination angle of a track of the Lissajous figure; and a ventilation capability determiner adapted for comparing the inclination angle calculated by the inclination angle calculator with a predetermined reference inclination angle, so as to decide whether or not a lung ventilation capability of the human subject is good or bad.

In this case, it is possible to easily determine whether the lung ventilation capability is good or bad on the basis of the inclination angle of the track of the Lissajous figure. Depending on the posture of a human subject (standing, sitting, or supine), the inclination angle may be varied. Accordingly, multiple reference inclination angles may be defined depending on the posture.

The respiration characteristic analysis apparatus may further include: a memory adapted for storing training menus that are used for breathing training the human subject, the training menus being classified into rankings of respiration capability, the memory storing requirements for advancing through the rankings; a respiration capability determiner adapted for determining a respiration capability of the human subject on the basis of determination by the bioelectrical impedance determiner or calculation by the respiration depth calculator; and a training manager adapted for referring to the memory for identifying a ranking corresponding to the respiration capability determined by the respiration capability determine, and for executing a process for training the human subject for breathing using the training menus corresponding to the ranking. The training manager may be adapted for advancing the ranking to a next ranking if the requirement for advancing through the ranking is satisfied.

In this case, the human subject can effectively train for breathing in accordance with the training menus that match the respiration capability of the human subject. The training menus are prepared at each ranking of respiration capability, and if the requirement defined at each ranking is overcome, the human subject can advance to the next ranking. Accordingly, the training process has a game element by which the human subject is amused, and the human subject is motivated to train for breathing.

In addition, with such a structure, since the respiration capability determiner determines the respiration capability of the human subject on the basis of the bioelectrical impedances at the body trunk, it is unnecessary to deploy a mouthpiece-type respiration sensor in the mouth or to deploy a micro pressure sensor or a temperature sensor under the nose of the human subject. In particular, when the limb-lead eight-electrode method is used, current electrodes and voltage electrodes are deployed at both palms and both soles. Then, it is unnecessary to adhere current electrodes and voltage electrodes to the body trunk of the human subject, or to restrict the human body. The human subject may use either or both of the mouth and nose for respiration, so that training for breathing can be conducted easily. Because of the above reasons, breathing training can be conducted easily.

The bioelectrical impedance determiner may be adapted for determining a fifth bioelectrical impedance at the upper body trunk of the human subject including the upper lobes of the lungs of the human subject and excluding the abdomen of the human subject, and for determining a sixth bioelectrical impedance at the middle body trunk of the human subject including the median and lower lobes of lungs of the human subject and the abdomen of the human subject. The respiration capability determiner may be adapted for determining a type and a magnitude of respiration of the human subject as the respiration capability of the human subject on the basis of determination by the bioelectrical impedance determiner.

In this case, as the respiration capability of the human subject, the type and magnitude of respiration can be determined accurately. The type of respiration may include costal respiration and abdominal respiration, and furthermore a respiration in which inhalation and exhalation are repeated with the abdomen held in a constricted position (draw-in respiration).

The respiration characteristic analysis apparatus may further include: a third centering value generator adapted for generating a fifth centering value that is an average of the fifth bioelectrical impedances within a past unit time on the basis of change over time in the fifth bioelectrical impedance, and for generating a sixth centering value that is an average of the sixth bioelectrical impedances within a past unit time on the basis of change over time in the sixth bioelectrical impedance, the fifth centering value being a standard level of change over time in the fifth bioelectrical impedance, the sixth centering value being a standard level of change over time in the sixth bioelectrical impedance; a fifth difference calculator adapted for calculating a fifth difference between the fifth bioelectrical impedance and the fifth centering value; and a sixth difference calculator adapted for calculating a sixth difference between the sixth bioelectrical impedance and the sixth centering value. The respiration capability determiner may be adapted for determining a type and a magnitude of respiration of the human subject on the basis of the fifth difference and the sixth difference.

In this case, the respiration capability analyzer determines, as the respiration capability of the human subject, the type and magnitude of respiration accurately, on the basis of the fifth difference and the sixth difference.

The respiration characteristic analysis apparatus may further include a second zero-cross time decider adapted for deciding third zero-cross times in which the fifth bioelectrical impedance is equal to the fifth centering value, and for deciding fourth zero-cross times in which the sixth bioelectrical impedance is equal to the sixth centering value. The bioelectrical impedance determiner may be adapted for determining the fifth bioelectrical impedance and the sixth bioelectrical impedance at each sampling time occurring at a predetermined cycle. The third centering value generator may be adapted for generating the fifth centering value on the basis of the fifth bioelectrical impedance at each of sampling times, a number of the sampling times being predetermined. The third centering value generator may be adapted for generating the sixth centering value on the basis of the sixth bioelectrical impedance at each of third zero-cross times decided by the second zero-cross time decider, a number of the third zero-cross times being predetermined.

When a human performs respiratory actions that consist of inhalation and exhalation, the fifth bioelectrical impedance at the upper body trunk and the sixth bioelectrical impedance at the middle body trunk change. In all of abdominal respiration, costal respiration, and draw-in respiration, the lungs expand and contract, so that the bioelectrical impendance at the lungs increases at inhalations due to increase in the volume of air inside tissues in the lungs, and the bioelectrical impedance at the lungs decreases at exhalations due to decrease in the volume of air inside tissues in the lungs. Therefore, irrespective to the type of respiration of the human subject, the fifth bioelectrical impendance at the upper body trunk including the upper lobes of the lungs of the human subject and excluding the abdomen of the human subject increases at inhalations and decreases at exhalations.

In abdominal respiration, by the action of the abdominal skeletal muscle, the visceral tissue raises the diaphragm at exhalations, so that the abdominal bioelectrical impedance increases. In other words, increase in the bioelectrical impedance of the abdominal region beneath the diaphragm cancels decrease in the bioelectrical impedance at the chest region above the diaphragm when the human subject performs exhalation in abdominal respiration. The same is not true for costal respiration or draw-in respiration. Consequently, when respiration of the human subject is abdominal respiration, change in the sixth bioelectrical impedance at the middle body trunk including the median and lower lobes of lungs and the abdomen of the human subject is different from that in the fifth bioelectrical impedance.

Irrespective to the type of respiration of the human subject, the waveform of change in the fifth bioelectrical impedance in respiration is nearly sinusoidal. It is preferable to obtain a suitable fifth centering value (standard level of change over time in the fifth bioelectrical impedance used for extracting information on respiration of the human subject) even if one or more instantaneous values of the fifth bioelectrical impedance are disturbed by body motion or for other reasons. Accordingly, the third centering value generator may be adapted for generating the fifth centering value on the basis of fifth bioelectrical impedance at each of sampling times, the number of the sampling times being predetermined.

More specifically, the third centering value generator may be adapted for calculating a moving average at each sampling time, the moving average bering a moving average of the fifth bioelectrical impedances at multiple sampling times within a centering period starting from a time point that is a predetermined time length before a current sampling time and ending at the current sampling time. The third centering value generator may be adapted for generating the fifth centering value at the current sampling time on the basis of the moving averages at multiple sampling times.

The time length ofthe centering period may be variable and may be set depending on the respiration speed of the human subject at the current sampling time. The moving average may be calculated with or without the use of weighting factors. For example, the moving average may be calculated with the use of weighting factors depending on the frequency at each sampling time.

On the other hand, change in the sixth bioelectrical impedance in abdominal respiration is not sinusoidal and is different from that of the fifth bioelectrical impedance. Accordingly, in contrast to the calculation of the fifth centering value, if a moving average is calculated on the basis of measurement values of the sixth bioelectrical impedance at the sampling times of which the number is predetermined, the sixth centering value cannot be calculated

Accordingly, in this aspect, the third centering value generator may be adapted for generating the sixth centering value on the basis of the sixth bioelectrical impedance at each of third zero-cross times decided by the second zero-cross time decider. Specifically, the third centering value generator may be adapted for deciding whether or not each sampling time is a third zero-cross time, and for generating the sixth centering value at the current sampling time on the basis of the sixth bioelectrical impedances including the sixth bioelectrical impedance at the current sampling time if the current sampling time is a third zero-cross time. The third centering value generator may be adapted for deciding the sixth centering value generated at a last sampling time as the sixth centering value at the current sampling time if the current sampling time is not a third zero-cross time. In this case, the sixth centering value that is the standard level of the sixth bioelectrical impedance can be calculated accurately.

The respiration characteristic analysis apparatus may further include a display data generator adapted for generating display data for displaying a Lissajous figure showing change over time in the fifth bioelectrical impendance and change over time in the sixth bioelectrical impendance in an orthogonal coordinate system having two orthogonal coordinate axes in which a first axis is the fifth bioelectrical impedance and a second axis is the sixth bioelectrical impendance.

Two orthogonal coordinate axes may be, for example, the X axis and the Y axis. However, two orthogonal coordinate axes may be two axes obtained by inclining the X axis and the Y axis by 45 degrees. The Lissajous figure may show only the status of a single respiration as shown in Fig. 55 or 56, or may show the status of multiple respirations continually as shown in Fig. 57 or Fig. 58. The respiration characteristic analysis apparatus may include a display device for displaying the Lissajous figure, or an output part for outputting display data for displaying the Lissajous figure to an external display device. The respiration characteristic analysis apparatus may be, for example, a game machine, a personal computer, or a portable electrical device (e.g., a cell phone).

By observing the Lissajous figure, the human subject or another person can understand whether current respiration of the human subject is costal or abdominal, or can understand whether respiration of the human subject is mainly dependent on costal respiration or abdominal respiration. The human subject or another person can also understand the magnitude of each of costal respiration and abdominal respiration from the Lissajous figure. Accordingly, this respiration characteristic analysis apparatus can be used as a breathing training apparatus. When the human subject trains for costal breathing, the human subject may pay attention to the Lissajous figure so that the track of the Lissajous figure becomes an inclined straight shape and the size of the track becomes large. When the human subject trains for abdominal breathing, the human subject may pay attention to the Lissajous figure so that the track of the Lissajous figure is of a bent shape, and the size and the bend angle AG become large. Thus, by observing the Lissajous figure and confirming the type and the magnitude of respiration at any time, the human subject can train for appropriate costal or abdominal breathing. By observing the Lissajous figure, the human subject or another person can understand whether or not respiration of the human subject is draw-in respiration. The shallower draw-in respiration, the smaller the track of the Lissajous figure, so that the magnitude of draw-in respiration can be understood from the Lissajous figure. By observing the Lissajous figure and confirming the type and the magnitude of respiration at any time, the human subject can train for appropriate draw-in breathing.

As has been described above, by virtue of displaying the Lissajous figures, the human subject or another person can understand the type and magnitude of respiration of the human subject, and can understand whether or not the human subject is performing appropriately the target type of breathing while the human subject trains for breathing. The human subject or another person can objectively understand the type and magnitude of respiration or can understand whether or not the human subject is performing appropriately the target type of breathing by virtue of the Lissajous figure.

The respiration characteristic analysis apparatus may further include: a display data generator adapted for generating display data for displaying a Lissajous figure showing change over time in the fifth bioelectrical impedance and change over time in the sixth bioelectrical impedance in an orthogonal coordinate system having two orthogonal coordinate axes in which a first axis is the fifth bioelectrical impedance and a second axis is the sixth bioelectrical impedance; and a third centering value generator adapted for generating a fifth centering value that is an average of the fifth bioelectrical impedances within a past unit time on the basis of change over time in the fifth bioelectrical impedance, and for generating a sixth centering value that is an average of the sixth bioelectrical impedances within a past unit time on the basis of change over time in the sixth bioelectrical impedances, the fifth centering value being a standard level of change over time in the fifth bioelectrical impedance, the sixth centering value being a standard level of change over time in the sixth bioelectrical impedances. The display data generator may be adapted for generating the display data for displaying the Lissajous figure so that a position on the Lissajous figure defined by the fifth centering value and the sixth centering value is located at a center of a screen in which the Lissajous figure is displayed. In this case, since the location of the Lissajous figure is centered with respect to the screen, visualization of the Lissajous figure can be facilitated.

The respiration characteristic analysis apparatus may further include a display data generator adapted for generating display data for displaying a Lissajous figure showing change over time in the fifth bioelectrical impedance and change over time in the sixth bioelectrical impedance in an orthogonal coordinate system having two orthogonal coordinate axes in which a first axis is the fifth bioelectrical impedance and a second axis is the sixth bioelectrical impedance; and a third centering value generator adapted for generating a fifth centering value that is an average of the fifth bioelectrical impedances within a past unit time on the basis of change over time in the fifth bioelectrical impedance, and for generating a sixth centering value that is an average of the sixth bioelectrical impedances within a past unit time on the basis of change over time in the sixth bioelectrical impedance, the fifth centering value being a standard level of change over time in the fifth bioelectrical impedance, the sixth centering value being a standard level of change over time in the sixth bioelectrical impedance. When the display data generator generates the display data for displaying the Lissajous figure, the display data generator may be adapted for executing a first location centering process in which the Lissajous figure is centered in the first axis with respect to a screen in which the Lissajous figure is displayed on the basis of the fifth centering value, and may be adapted for executing a second location centering process in which the Lissajous figure is centered in the second axis with respect to the screen on the basis of the sixth centering value. The display data generator may be adapted for executing the second location centering process less frequently than that for the first location centering process.

As in Fig. 68, which shows Lissajous figures in draw-in respiration and costal respiration, both tracks of the Lissajous figures are of a straight shape rising from bottom left to top right, but locations of both tracks are different in the axis indicating the second bioelectrical impedance $Z_b$ (the X axis in Fig. 68). If centering of the displayed location of the Lissajous figure is repeated at small intervals, the Lissajous figure will always be displayed at the center of the screen and it will be difficult to understand whether respiration of the human subject is draw-in respiration or costal respiration from observation of the Lissajous figure. In this aspect, the second location centering process is executed less frequently than that for the first location centering process. For example, the first location centering process may be executed at every respiration, whereas the second location centering process may be executed only once (for example, at an initial stage of the process). It will be easy to understand whether respiration of the human subject is draw-in respiration or costal respiration from observation of the Lissajous figure. This is because the locations of tracks of the Lissajous figures for draw-in respiration and costal respiration will become different in the second axis for a certain period, even though the shapes of the tracks are similar. In addition, it is possible to reduce power consumption at the respiration characteristic analysis apparatus by reducing the frequency of the second location centering process. Although the frequency is less, by executing the second location centering process, the Lissajous figure can be displayed on the center of the screen.

The respiration characteristic analysis apparatus may further include a local-maximum-and-minimum decider adapted for deciding a fifth local maximum that is a local maximum of change in the fifth bioelectrical impedance, for deciding a fifth local minimum that is a local minimum of change in the fifth bioelectrical impedance, for deciding a sixth local maximum that is a local maximum of change in the sixth bioelectrical impedance, and for deciding a sixth local minimum that is a local minimum of change in the sixth bioelectrical impedance. The display data generator may be adapted for generating the display data for displaying the Lissajous figure so that a range of the Lissajous figure on a screen in which the Lissajous figure is displayed in the first and second axes is adjusted on the basis of the fifth local maximum, the fifth local minimum, the sixth local maximum, and the sixth local minimum.

In this case, the Lissajous figure can be displayed at a suitable size with respect to the screen by adjusting the range in the first and second axes, and can be centered with respect to the screen, so that visualization of the Lissajous figure can be facilitated.

The respiration characteristic analysis apparatus may further include a local-maximum-and-mnimum decider adapted for deciding a fifth local maximum that is a local maximum of change in the fifth bioelectrical impedance, for deciding a fifth local minimum that is a local minimum of change in the fifth bioelectrical impedance, for deciding a sixth local maximum that is a local maximum of change in the sixth bioelectrical impedance, and for deciding a sixth local minimum that is a local minimum of change in the sixth bioelectrical impedance. When the display data generator generates the display data for displaying the Lissajous figure, the display data generator may be adapted for executing a first range adjustment process in which a range of the Lissajous figure on a screen in which the Lissajous figure is displayed in the first axis is adjusted on the basis of the fifth local maximum and the fifth local minimum, and may be adapted for executing a second range adjustment process in which a range of the Lissajous figure on the screen in the second axis is adjusted on the basis of the sixth local maximum and the sixth local minimum. The display data generator may be adapted for executing the second range adjustment process less frequently than that for the first range adjustment process.

In this case, it will be easy to understand whether respiration of the human subject is draw-in respiration or costal respiration from observation of the Lissajous figure. In addition, it is possible to reduce power consumption at the respiration determination apparatus by reducing the frequency of the second range adjustment process. Although the frequency is less, by executing the second range adjustment process, the Lissajous figure can be displayed at a suitable size with respect to the screen.

The display data generator may be adapted for generating the display data for displaying the Lissajous figure so that a displaying manner for a track of the Lissajous figure for a latest single respiration is different from a displaying manner for a track of the Lissajous figure for past respirations.

In a Lissajous figure that continually shows status of multiple respirations, for example, as shown in Fig. 57 or 58, if the manner for displaying the track within a single respiration is the same as that for the track within another single respiration, it is difficult to identify the track for the latest single respiration. However, in this aspect, it is possible to easily understand the track for the latest single respiration in view of the variation of the displaying manner (e.g., color or line type).

The display data generator may be adapted for generating the display data for displaying the Lissajous figure so that a displaying manner for tracks of the Lissajous figure is changed depending on an elapsed time.

For example, the display data generator may lighten the color as the elapsed time increases. In this case, the newer the track, the fainter the color of the track. It is easy to identify the tracks for newer respirations (e.g., the track for the latest respiration).

The display data generator may be adapted for further generating target display data for displaying a target Lissajous figure showing a target model of breathing having a type and a magnitude of respiration to be performed by the human subject for guiding the human subject to perform breathing.

In this case, in addition to the measured Lissajous figure showing the status of breathing of the human subject, a target Lissajous figure showing a target model of breathing to be performed by the human subject is displayed. Thus, the human subject can train for breathing comparing both of the Lissajous figures. The human subject may focus on making the track of the Lissajous figure showing the status of breathing of the human subject coincide with the track of the target Lissajous figure, so as to learn the target breathing. Thus, the human subject is effectively guided to perform appropriate breathing by the use of the Lissajous figure for breathing guidance.

The display data generator may be adapted for generating the display data for the measured Lissajous figure and the target display data so that the measured Lissajous figure showing the status of breathing of the human subject and the target Lissajous figure are overlaid on a screen. In this case, it is possible to easily understand the difference between the target respiration and the actual respiration. The display data generator may be adapted for generating the display data for the measured Lissajous figure and the target display data so that a displaying manner for a track of the measured Lissajous figure is different from a displaying manner for a track of the target Lissajous figure. In this case, it is possible to easily distinguish the Lissajous figures in view of the variation of the displaying manner (e.g., color or line type) even when the measured Lissajous figure showing the status of breathing of the human subject and the target Lissajous figure are overlaid on the screen.

The respiration characteristic analysis apparatus may further include: an inclination angle calculator adapted for calculating an inclination angle of a track of the Lissajous figure; and a ventilation capability determiner adapted for comparing the inclination angle calculated by the inclination angle calculator with a predetermined reference inclination angle, so as to decide whether or not a lung ventilation capability of the human subject is good or bad.

In this case, it is possible to easily determine whether the lung ventilation capability is good or bad on the basis of the inclination angle of the track of the Lissajous figure. Depending on the posture of a human subject (standing, sitting, or supine), the inclination angle may be varied. Accordingly, multiple reference inclination angles may be defined depending on the posture.

The respiration characteristic analysis apparatus may further include: a respiration depth calculator adapted for calculating a respiration depth of the human subject at every respiration of the human subject; and a graph generator adapted for generating display data for indicating a graph showing change over time of respiration depth calculated by the respiration depth calculator, in such a manner that the graph is nonlinearly compressed in a direction of time axis and older time intervals are more compressed than later time intervals, so that a time resolution for later time intervals is higher than that for older time intervals.

The time for breathing training may frequently be long, e.g., ten minutes or more. In order to display the entire graph from the start of measurement to the current time, it is preferable that the graph be compressed in the direction of the time axis. If the entire graph is uniformly compressed, the time resolution will be reduced uniformly in the graph. This results in it being difficult to recognize details of the magnitude of the latest respirations. In this aspect, the graph is nonlinearly compressed in the direction of the time axis and earlier time intervals are more compressed than later time intervals, so that the time resolution for later time intervals is higher than that for earlier time intervals.

In another aspect of the present invention, a respiration characteristic analysis apparatus includes: an input part for inputting to the respiration characteristic analysis apparatus a bioelectrical impedance at a part including the right lung of a human subject and for determining a bioelectrical impedance at a part including the left lung of the human subject, the bioelectrical impedances being determined at a bioelectrical impedance determination apparatus; and a respiration depth calculator adapted for calculating a right lung respiration depth related to a respiration capability of the right lung of the human subject on the basis of change over time in the bioelectrical impedance at the part including the right lung, and for calculating a left lung respiration depth related to a respiration capability of the left lung of the human subject on the basis of change over time in the bioelectrical impedance at the part including the left lung.

This respiration characteristic analysis apparatus also accurately and easily determines respiration capability of each of the right lung and the left lung of the human subject. This respiration characteristic analysis apparatus may be, for example, a game machine, a personal computer, or a portable electrical device (e.g., a cell phone).

The respiration characteristic analysis apparatus may further include: a memory adapted for storing training menus that are used for training the human subject for breathing, the training menus being classified into rankings of respiration capability, the memory storing requirements for advancing through the rankings; a respiration capability determiner adapted for determining a respiration capability of the human subject on the basis of change over time in each of the bioelectrical impedances input by the input part; and a training manager adapted for referring to the memory for identifying a ranking corresponding to the respiration capability determined by the respiration capability determiner, and for executing a process for training the human subject for breathing using the training menus corresponding to the ranking. The training manager may be adapted for advancing the ranking to a next ranking if the requirement for advancing through the ranking is satisfied.

In this respiration characteristic analysis apparatus, it is unnecessary to deploy a respiration sensor in the mouth or under the nose of the human subject. The human subject may use either or both of the mouth and nose for respiration, so that training for breathing can be conducted easily. Because of the above reasons, training for breathing can be conducted easily The human subject can effectively train for breathing in accordance with the training menus that match the respiration capability of the human subject. The training menus are prepared at each ranking of respiration capability, and if the requirement defined at each ranking is overcome, the human subject can advance to the next ranking. Accordingly, the training process has a game element by which the human subject is amused, and the human subject is motivated to train in breathing.

According to another aspect of the present invention, there is provided a respiration characteristic analysis system including: a bioelectrical impedance determiner adapted for determining a bioelectrical impedance at a part including the right lung of a human subject and for determining a bioelectrical impedance at a part including the left lung of the human subject; and a respiration depth calculator adapted for calculating a right lung respiration depth related to a respiration capability of the right lung of the human subject on the basis of change over time in the bioelectrical impedance at the part including the right lung determined by the bioelectrical impedance determiner, and for calculating a left lung respiration depth related to a respiration capability of the left lung of the human subject on the basis of change over time in the bioelectrical impedance at the part including the left lung determined by the bioelectrical impedance determiner.

This respiration characteristic analysis system also accurately and easily determines respiration capability of each of the right lung and the left lung of the human subject. This respiration characteristic analysis system may include, for example, a game machine, a personal computer, or a portable electrical device.

The respiration characteristic analysis system may further include: a memory adapted for storing training menus that are used for training the human subject for breathing, the training menus being classified into rankings of respiration capability, the memory storing requirements for advancing through the rankings; a respiration capability determiner adapted for determining a respiration capability of the human subject on the basis of the bioelectrical impedances determined by the bioelectrical impedance determiner; and a training manager adapted for referring to the memory for identifying a ranking corresponding to the respiration capability determined by the respiration capability determiner, and for executing a process for training the human subject for breathing using the training menus corresponding to the ranking. The training manager may be adapted for advancing the ranking to a next ranking if the requirement for advancing through the ranking is satisfied.

In this respiration characteristic analysis system, it is unnecessary to deploy a respiration sensor in the mouth or under the nose of the human subject. The human subject may use either or both of the mouth and nose for respiration, so that training in breathing can be conducted easily. Because of the above reasons, training in breathing can be conducted easily. The human subject can effectively train in breathing in accordance with the training menus that match the respiration capability of the human subject. The training menus are prepared at each ranking of respiration capability, and if the requirement defined at each ranking is satisfied, the human subject can advance to the next ranking. Accordingly, the training process has a game element by which the human subject is amused, and the human subject is motivated to train for breathing.

BRIEF DESCRIPTION OF THE DRAWINGS

With reference to the accompanying drawings, various embodiments of the present invention will be described hereinafter. In the drawings:

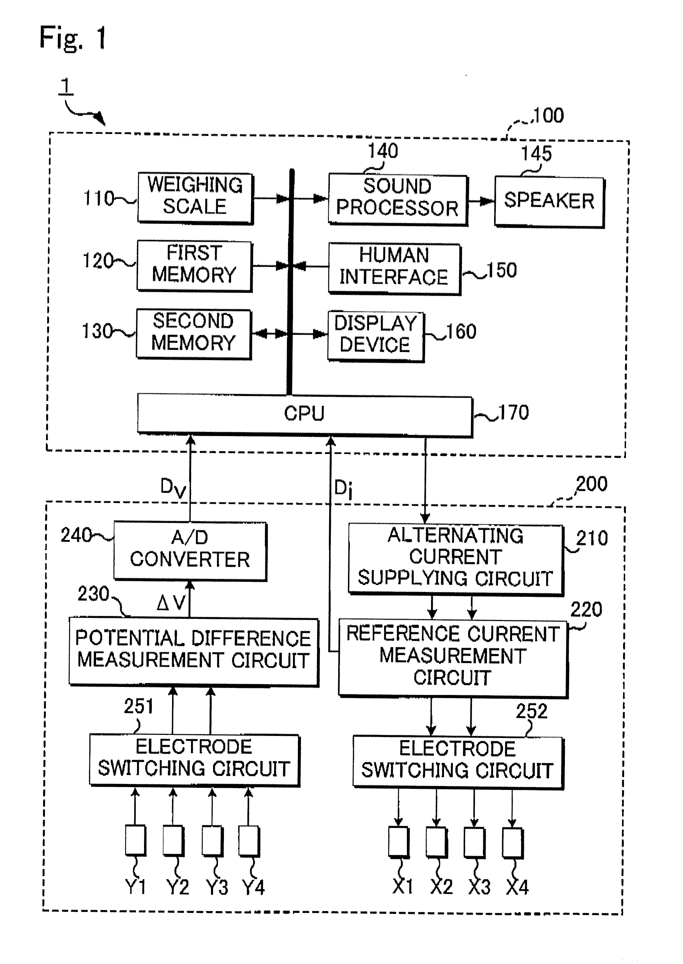

Fig. 1 is a block diagram showing an electrical structure of a body condition determination apparatus of a first embodiment according to the present invention;

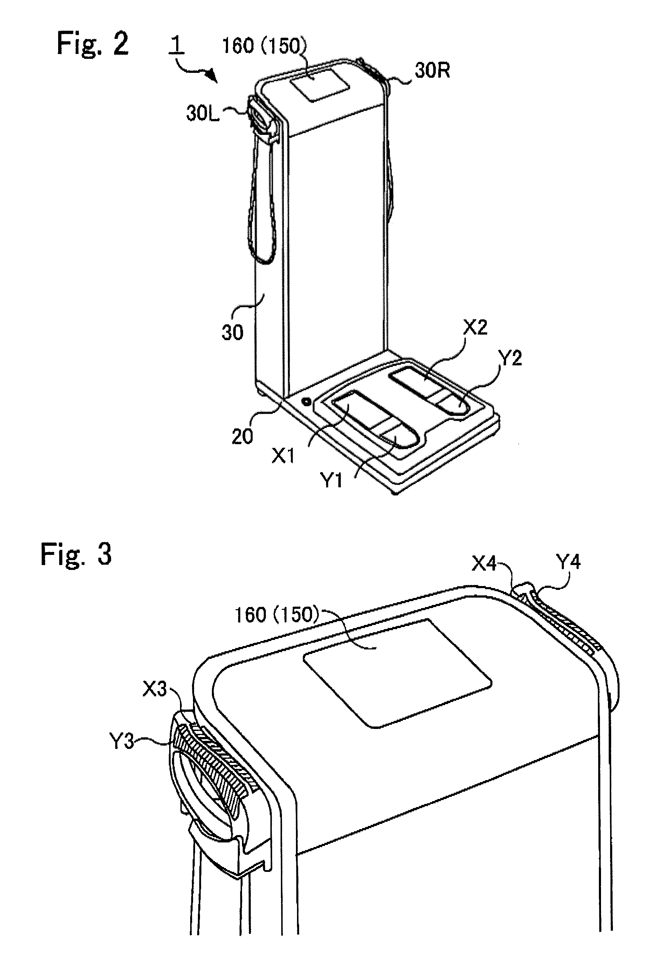

Fig. 2 is a perspective view showing the body condition determination apparatus;
Fig. 3 is an enlarged view of a part of Fig. 2;

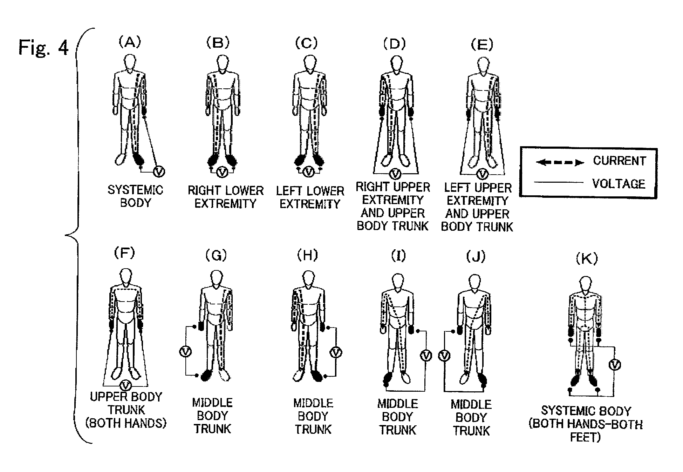

Fig. 4 is a diagram for explaining body regions determined by using selected current electrodes and selected voltage electrodes;

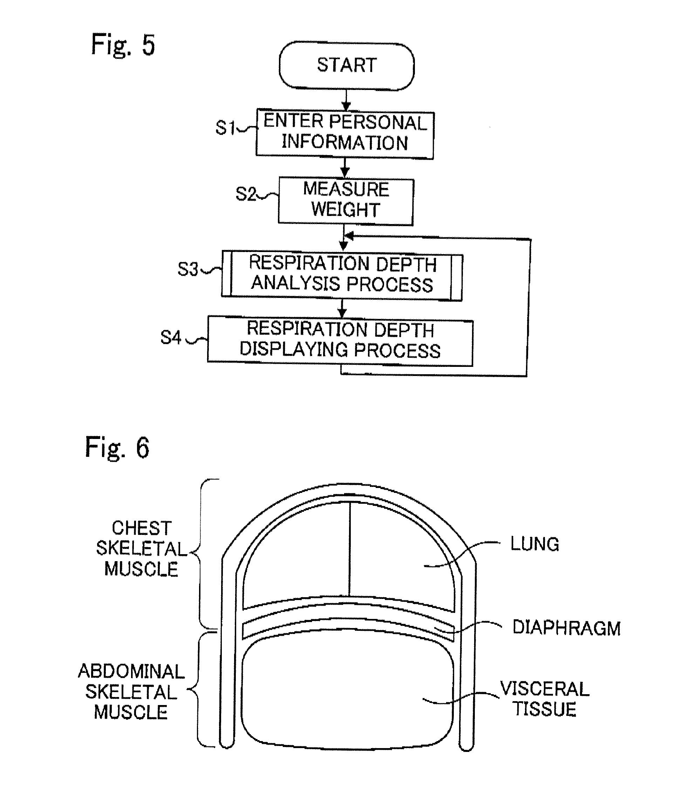

Fig. 5 is a flow chart showing an operation of the body condition determination apparatus;
Fig. 6 is a schematic view showing tissues in the body trunk of humans;

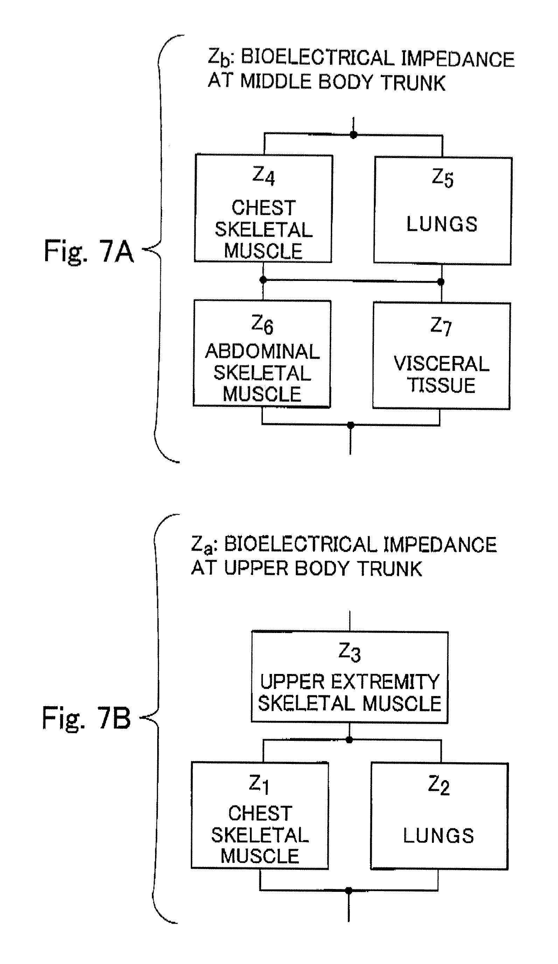

Fig. 7A shows an equivalent circuit model of the middle body trunk of humans;
Fig. 7B shows an equivalent circuit model of the upper body trunk of humans;

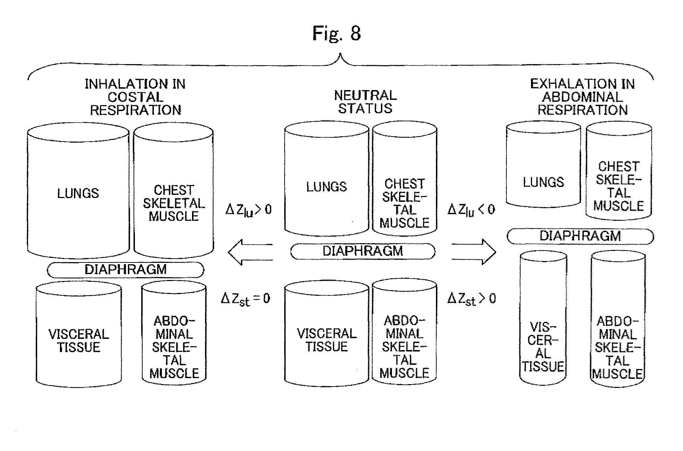

Fig. 8 is a diagram showing relationships between respiration and change in bioelectrical impedance;
Fig. 9 is a graph showing change over time of each of bioelectrical impedances at the middle body trunk and the upper body trunk in abdominal respiration;
Fig. 10 is a graph showing change over time of each of bioelectrical impedances at the middle body trunk and the upper body trunk in costal respiration;

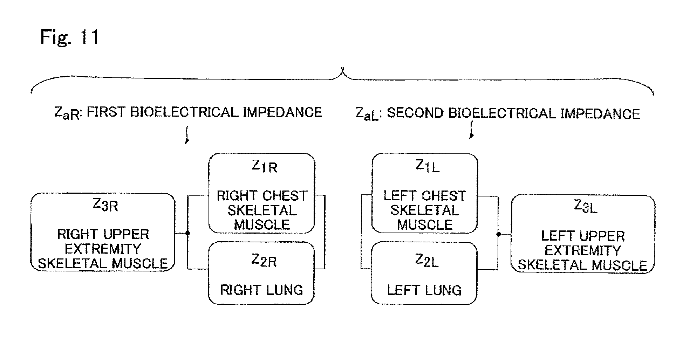

Fig. 11 shows equivalent circuit models of the upper body trunk of humans;

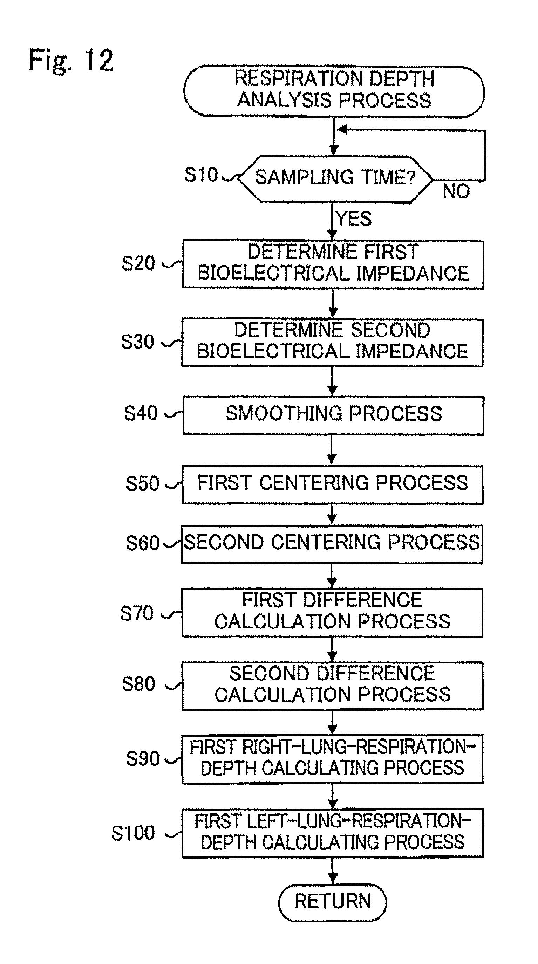

Fig. 12 is a flow chart showing an example of a respiration depth analysis process executed in the body condition determination apparatus;

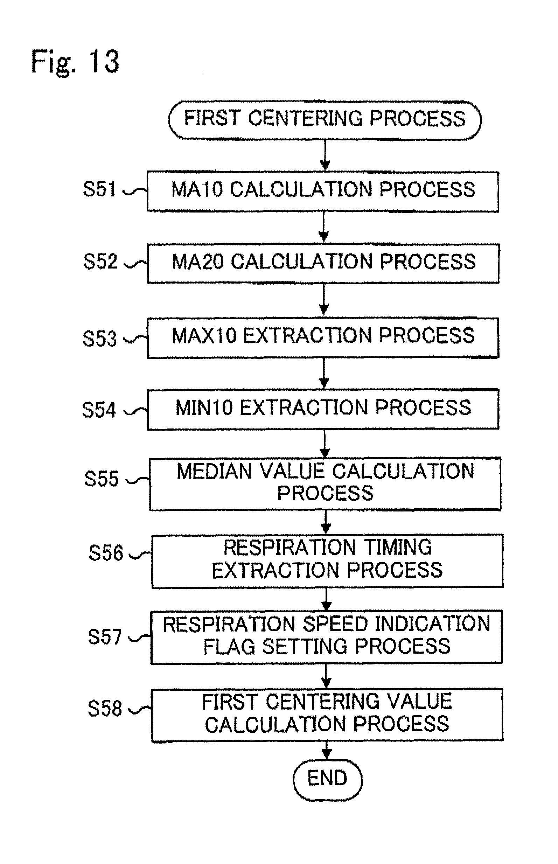

Fig. 13 is a flow chart showing an example of a first centering process executed in the body condition determination apparatus;

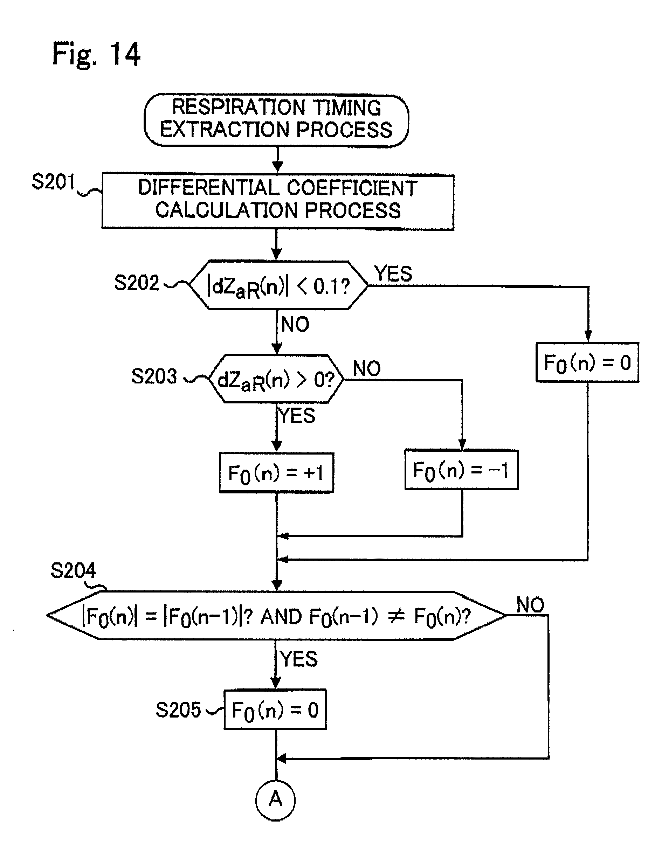

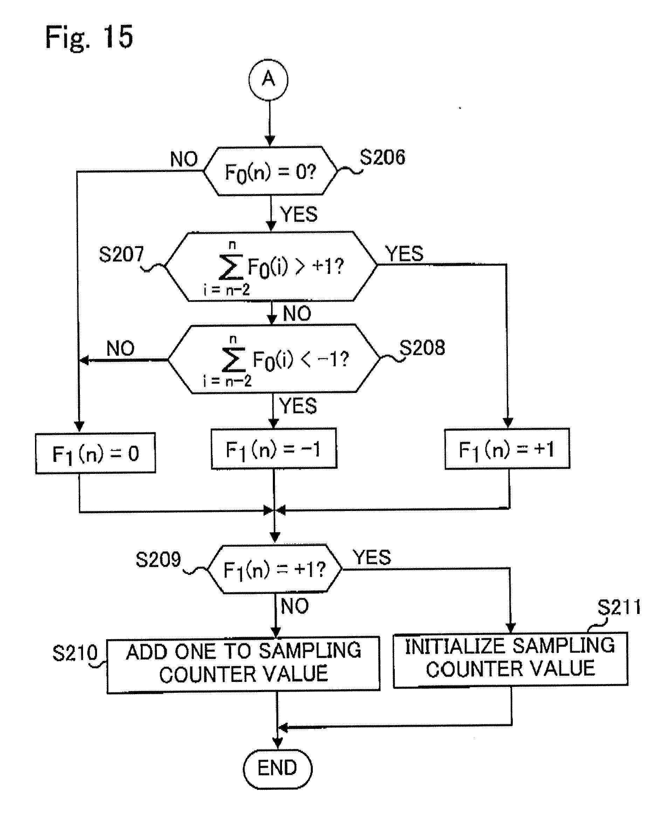

Figs. 14 and 15 form a flow chart showing an example of a respiration timing extraction process executed in the body condition determination apparatus;

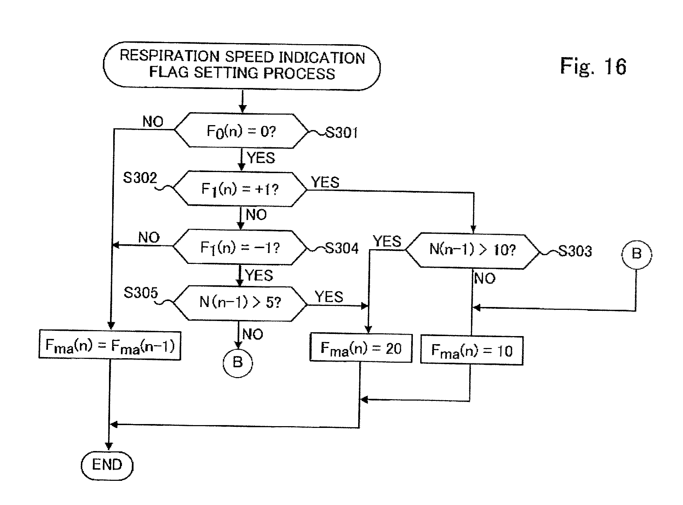

Fig. 16 is a flow chart showing an example of a respiration speed indication flag setting process executed in the body condition determination apparatus;

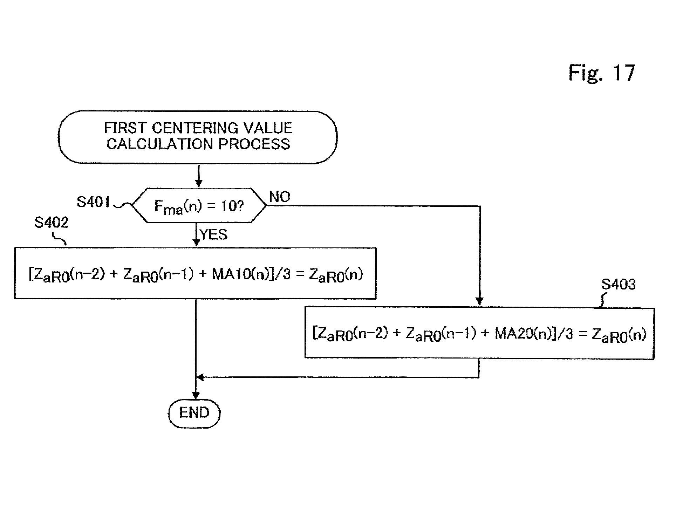

Fig. 17 is a flow chart showing an example of a first centering value calculating process executed in the body condition determination apparatus;

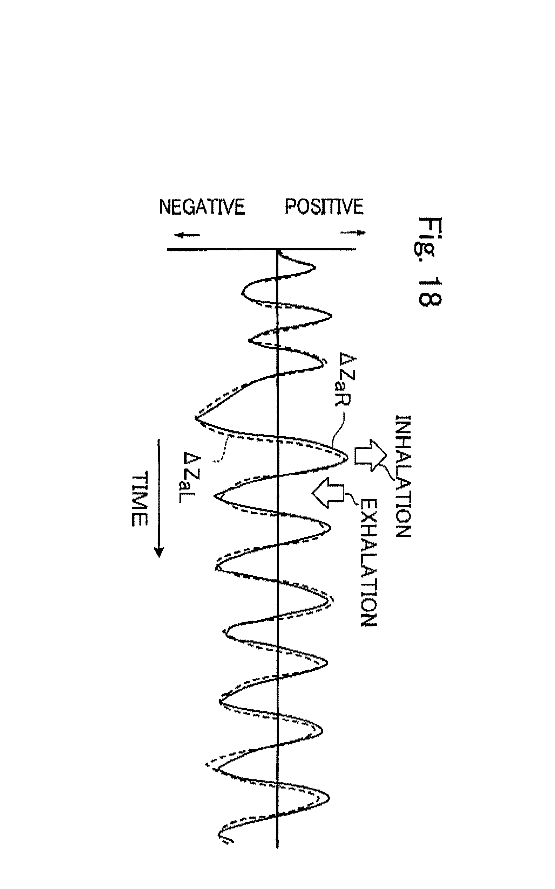

Fig. 18 is a graph showing change over time of each of a first difference and a second difference in respiration of a human subject;

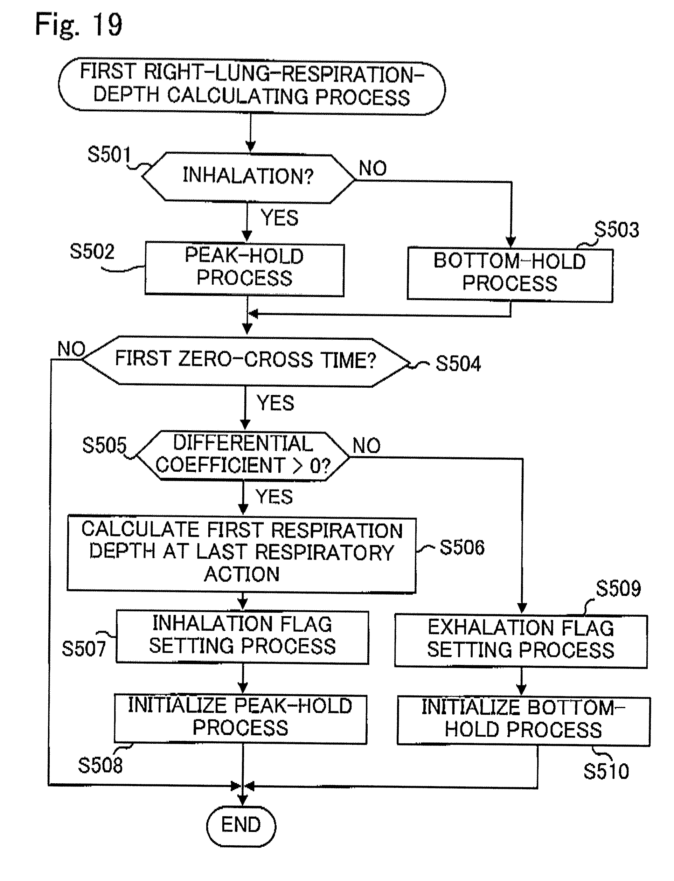

Fig. 19 is a flow chart showing an example of a first right-lung-respiration-depth calculating process executed in the body condition determination apparatus;

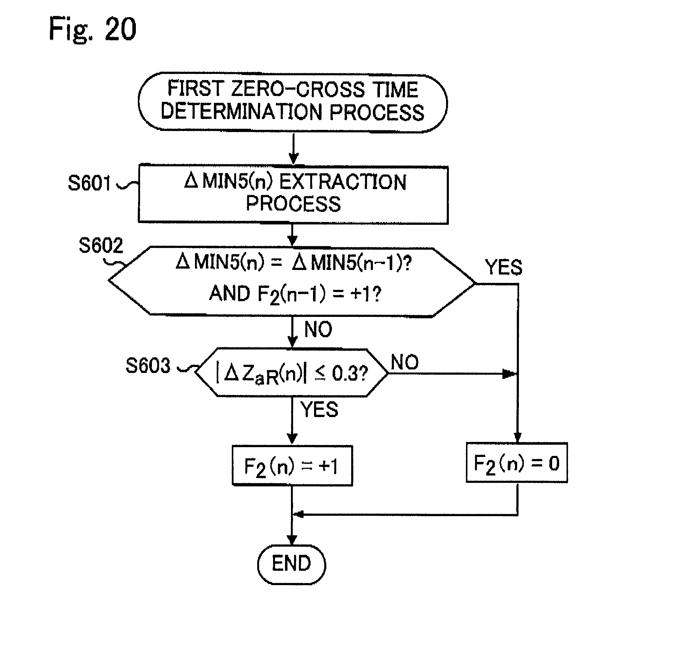

Fig. 20 is a flow chart showing an example of a first zero-cross time determination process executed in the body condition determination apparatus;

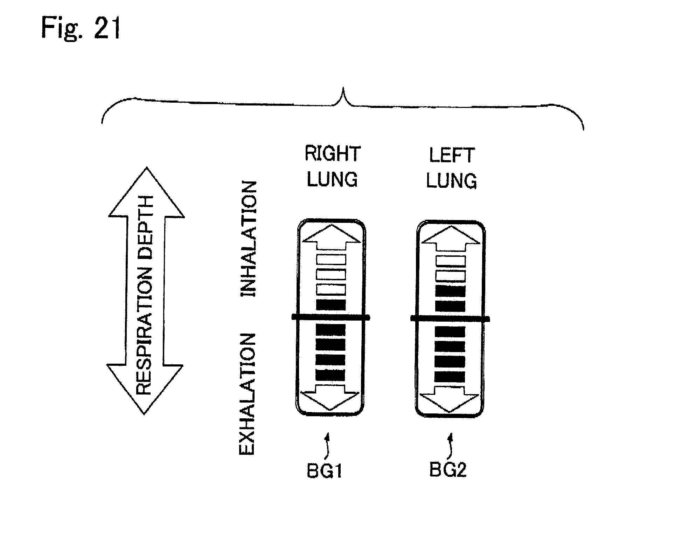

Fig. 21 is a view showing an image shown on a display device of the body condition determination apparatus;

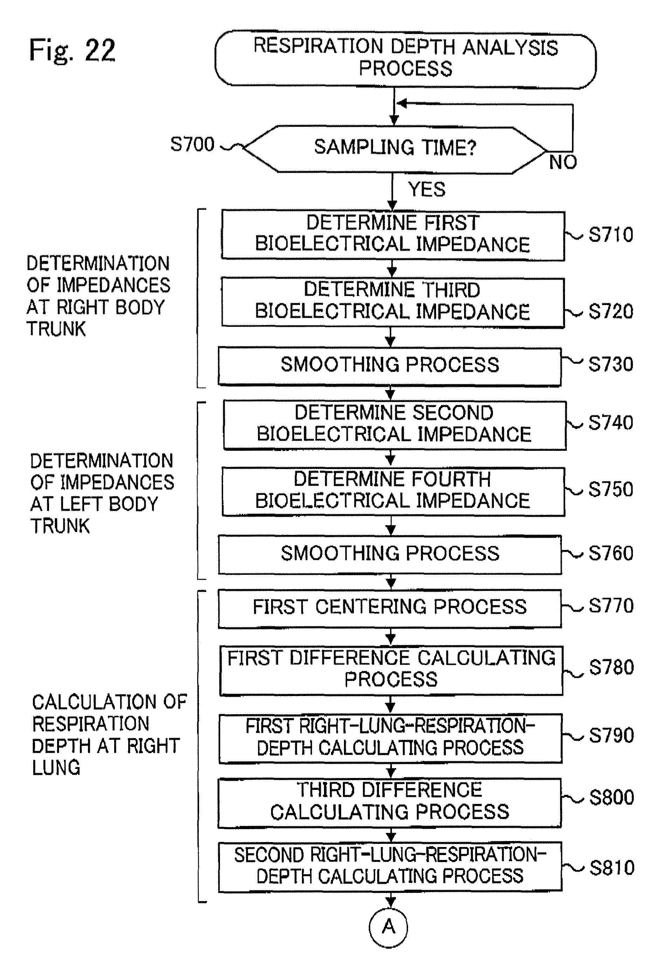

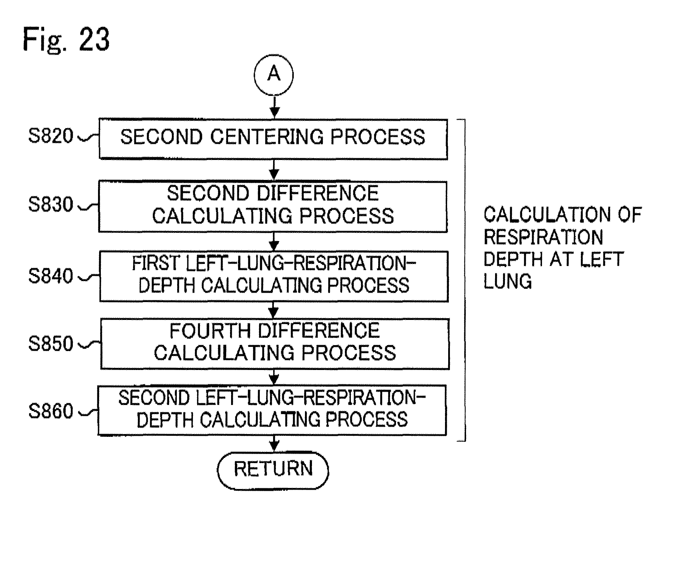

Figs. 22 and 23 form a flow chart showing an example of a respiration depth analysis process executed in a body condition determination apparatus of the second embodiment according to the present invention;

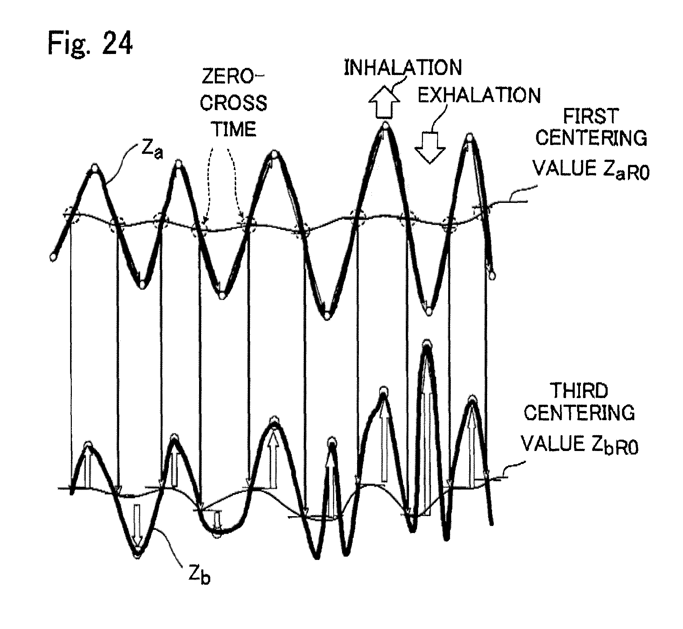

Fig. 24 is a graph for explaining a scheme for generating a third centering value;

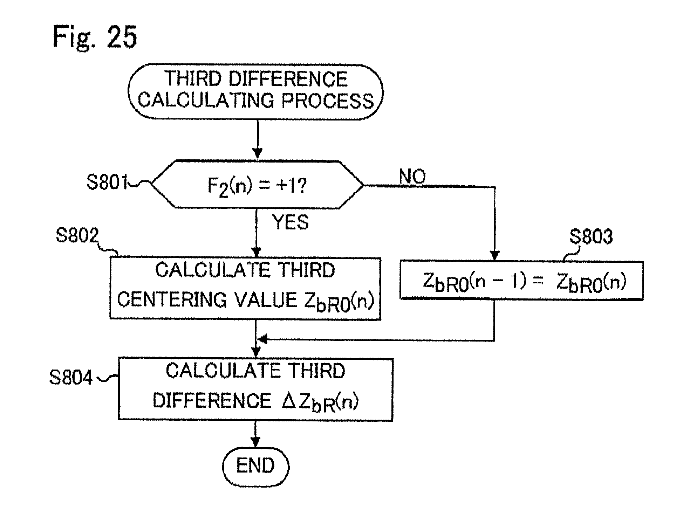

Fig. 25 is a flow chart showing an example of a third difference calculating process executed in the body condition determination apparatus of the second embodiment according to the present invention;

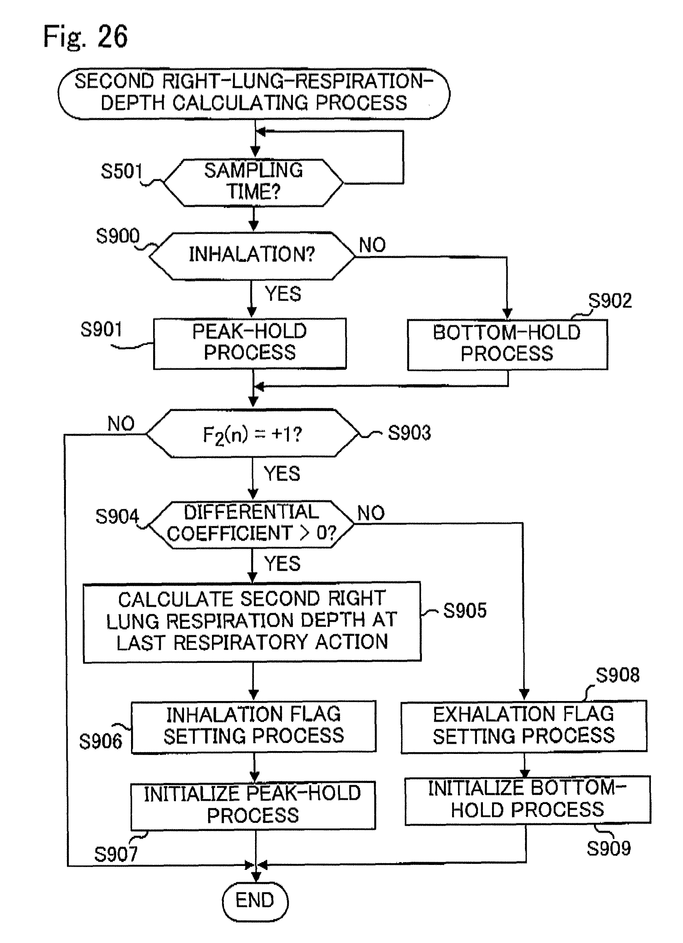

Fig. 26 is a flow chart showing an example of a second right-lung-respiration-depth calculating process executed in the body condition determination apparatus;

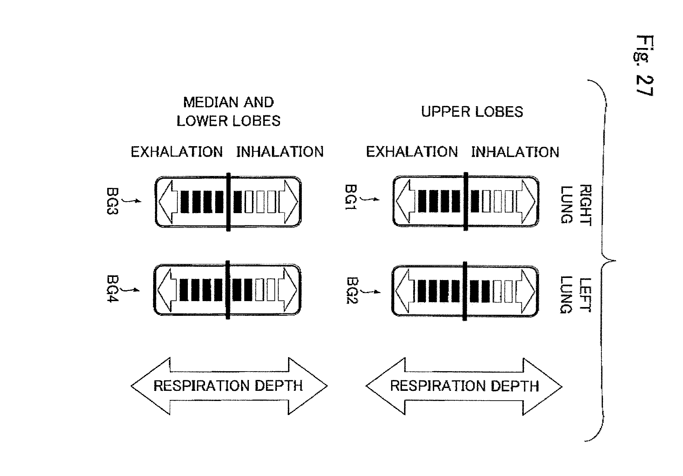

Fig. 27 is a view showing an image shown on a display device of the body condition determination apparatus;

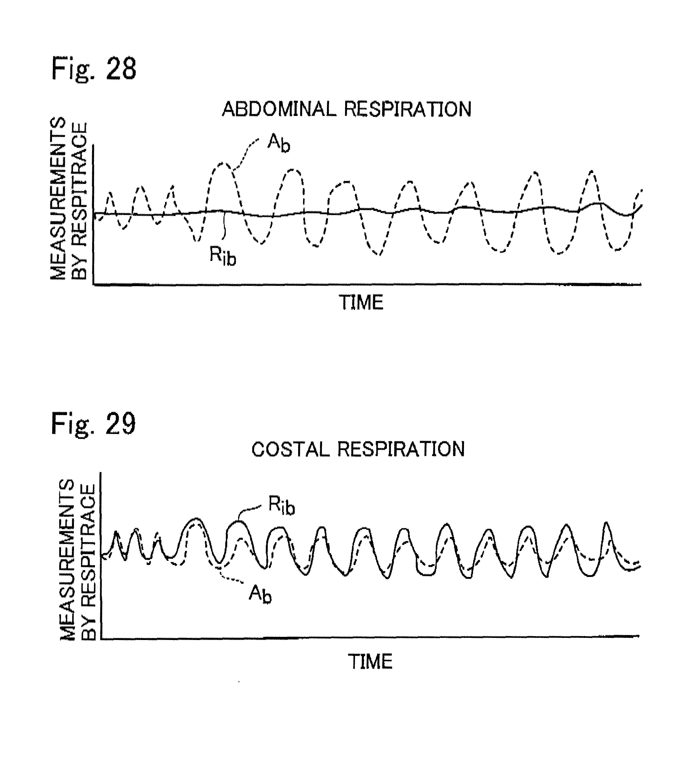

Fig. 28 is a graph showing change over time of each of costal and abdominal circumferences in abdominal respiration;
Fig. 29 is a graph showing change over time of each of costal and abdominal circumferences in costal respiration;

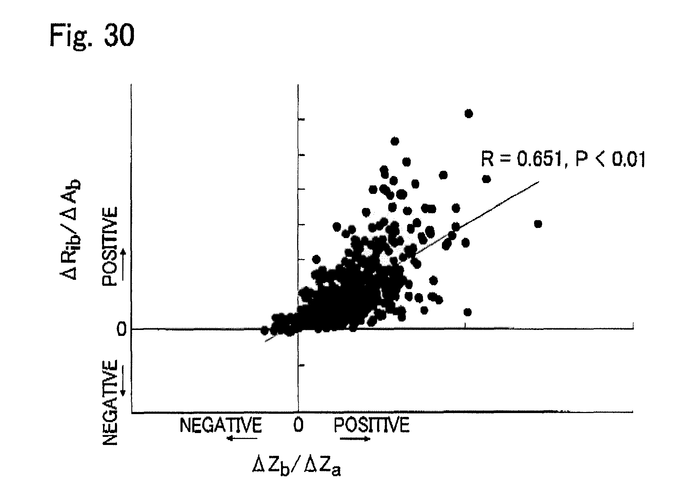

Fig. 30 is a graph showing relationships between a ratio of change in costal circumference to change in abdominal circumference and a ratio of change in bioelectrical impedance at the middle body trunk to change in bioelectrical impedance at the upper body trunk;

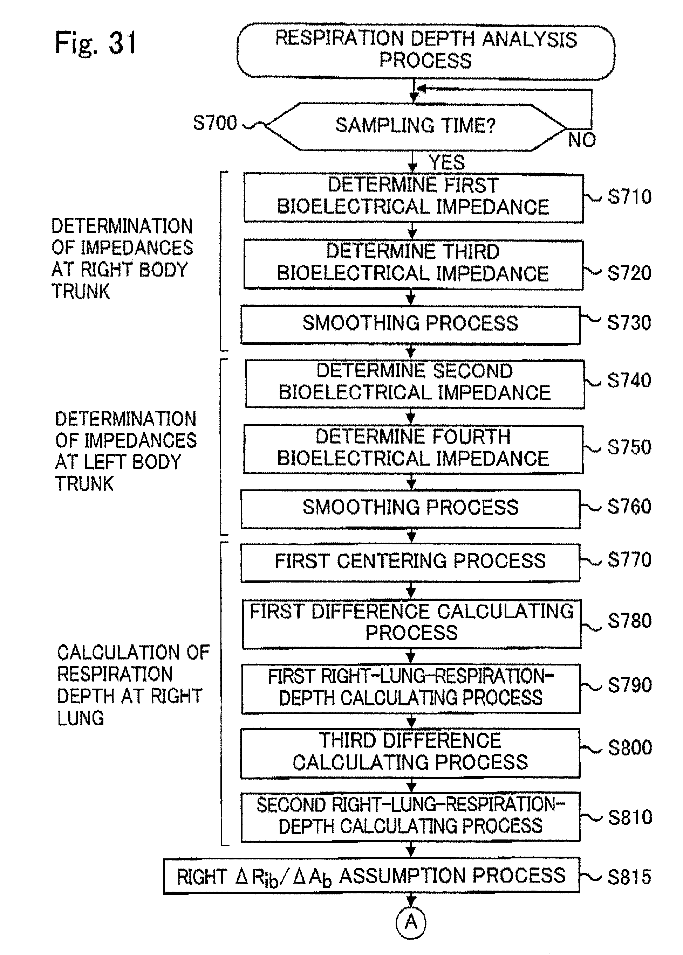

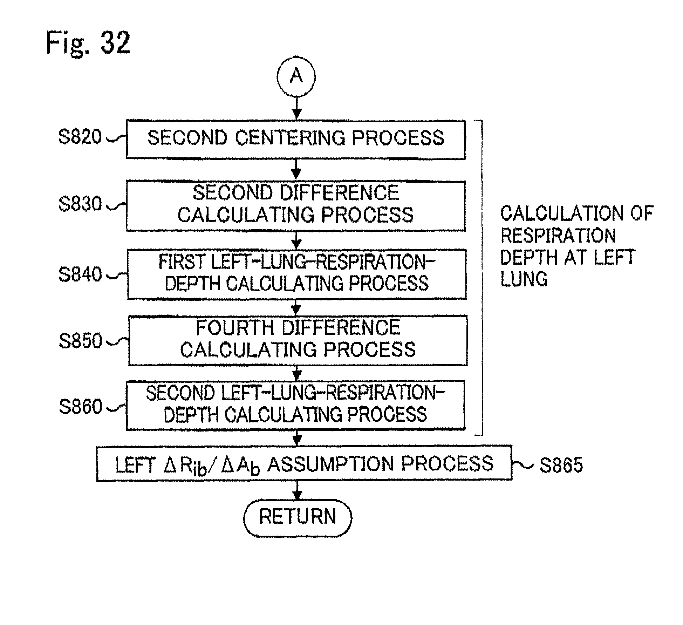

Figs. 31 and 32 form a flow chart showing an example of a respiration depth analysis process executed in the body condition determination apparatus of a third embodiment;

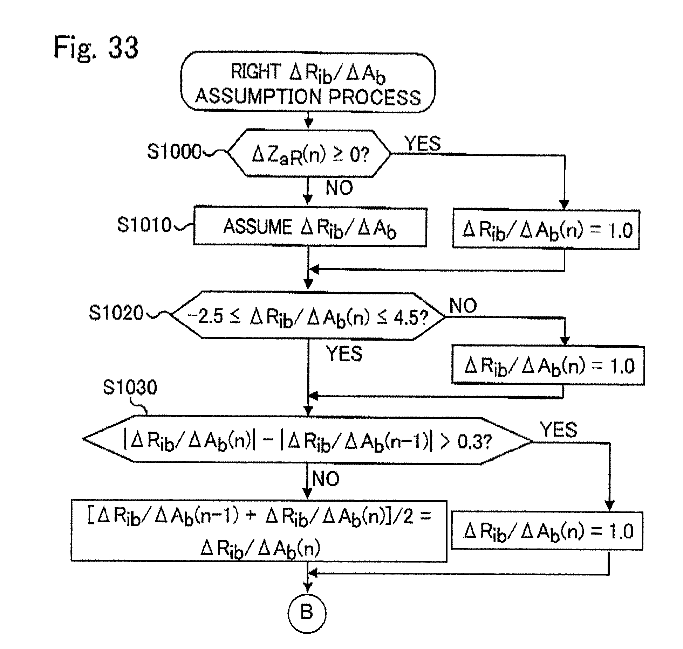

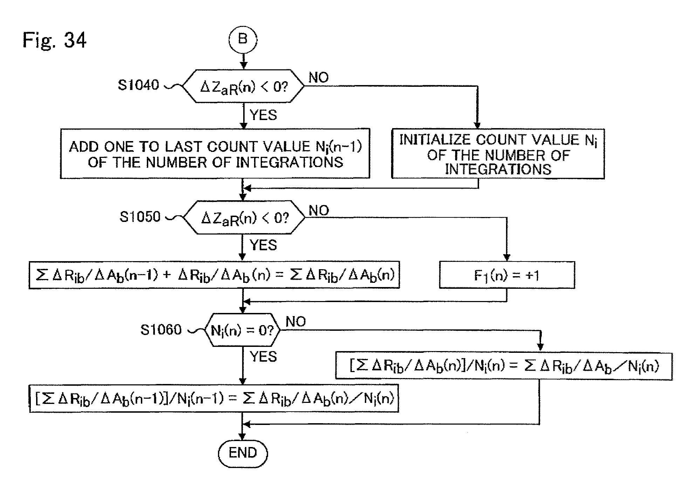

Figs. 33 and 34 form a flow chart showing an example of a $\Delta R_{ib}/\Delta A_b$ assumption process executed in the body condition determination apparatus;

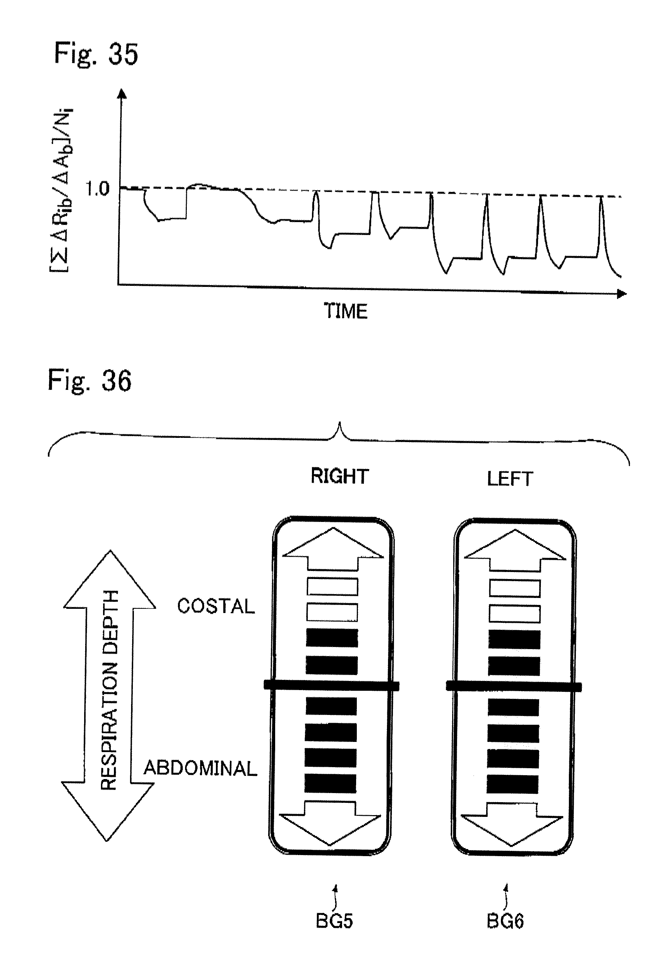

Fig. 35 is a graph showing the algorithmic results of the $\Delta R_{ib}/\Delta A_b$ assumption process;

Fig. 36 is a view showing an image shown on a display device of the body condition determination apparatus;

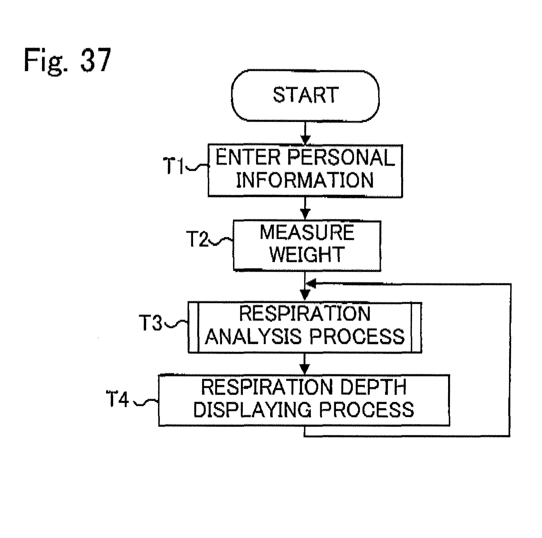

Fig. 37 is a flow chart showing an operation of a body condition determination apparatus of a fourth embodiment according to the present invention;

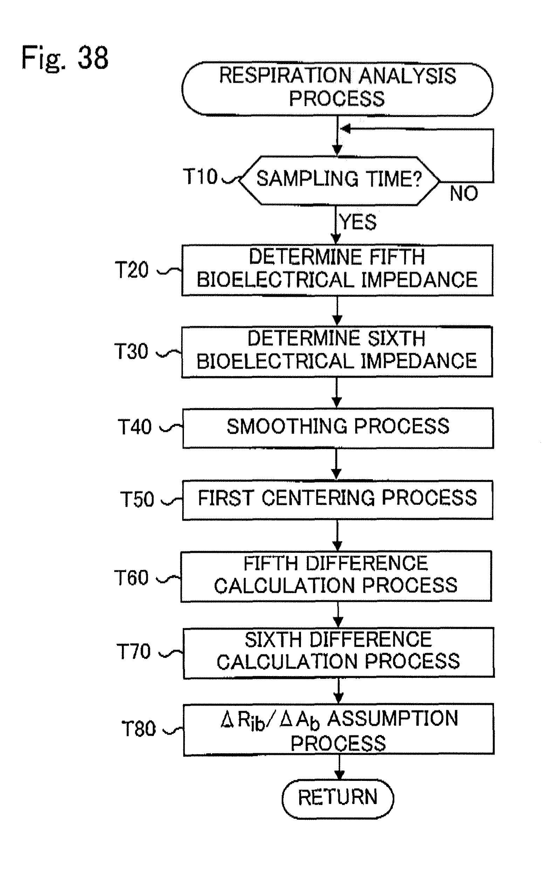

Fig. 38 is a flow chart showing an example of a respiration depth analysis process executed in the body condition determination apparatus of the fourth embodiment;

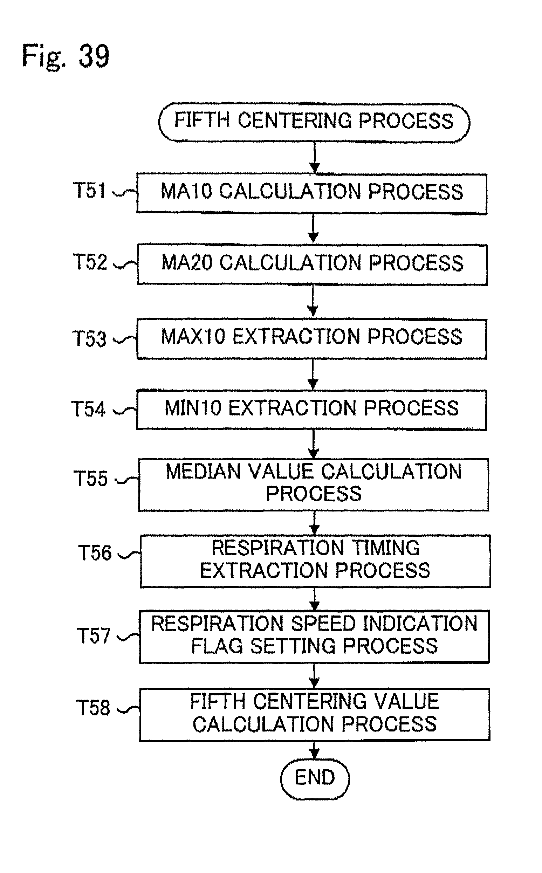

Fig. 39 is a flow chart showing an example of a fifth centering process executed in the body condition determination apparatus of the fourth embodiment;

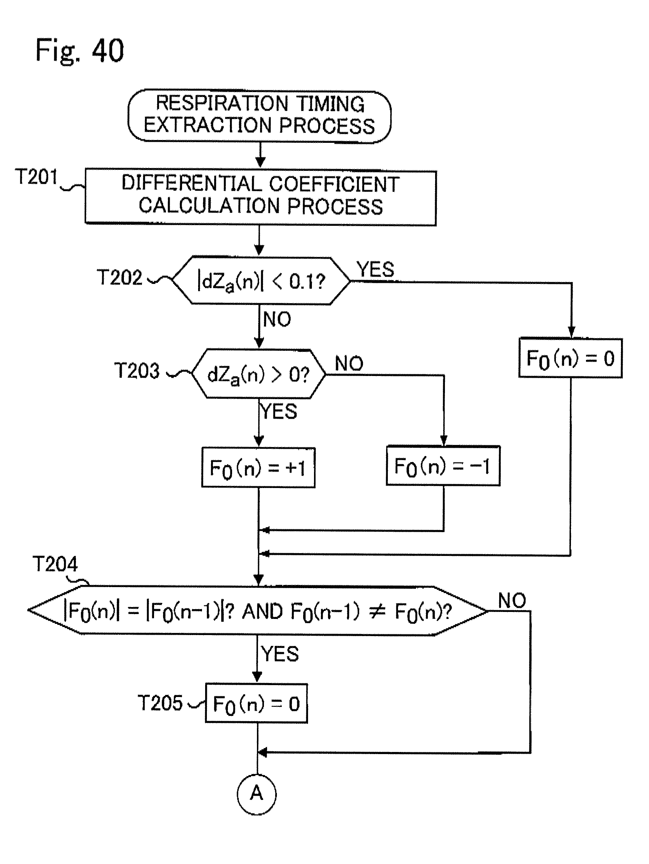

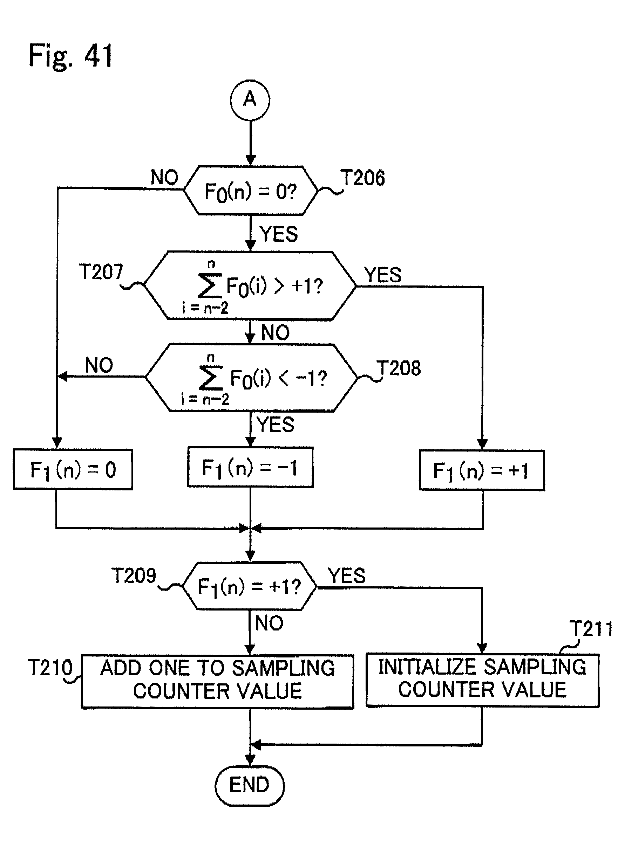

Figs. 40 and 41 form a flow chart showing an example of a respiration timing extraction process executed in the body condition determination apparatus of the fourth embodiment;

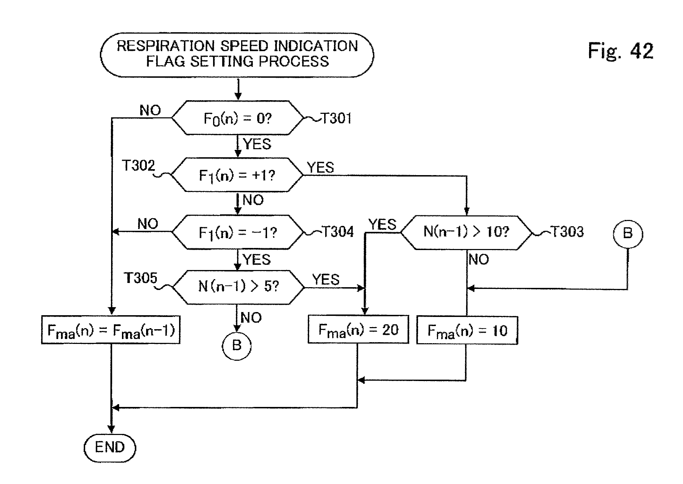

Fig. 42 is a flow chart showing an example of a respiration speed indication flag setting process executed in the body condition determination apparatus of the fourth embodiment;

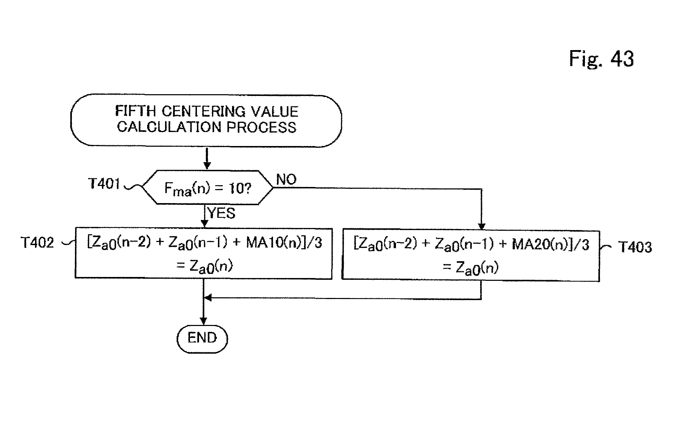

Fig. 43 is a flow chart showing an example of a fifth centering value calculating process executed in the body condition determination apparatus of the fourth embodiment;

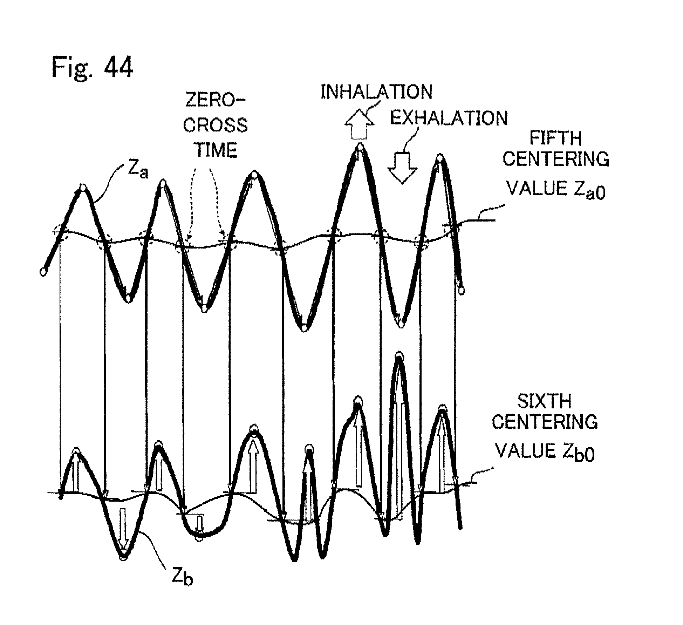

Fig. 44 is a graph for explaining a scheme for generating a sixth centering value;

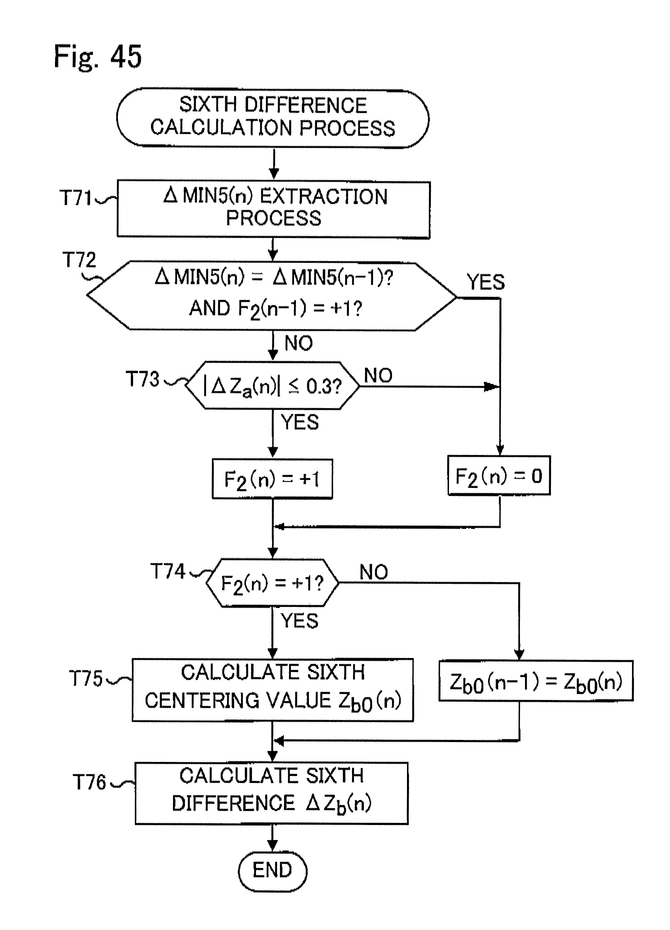

Fig. 45 is a flow chart showing an example of a sixth difference calculating process executed in the body condition determination apparatus of the fourth embodiment;

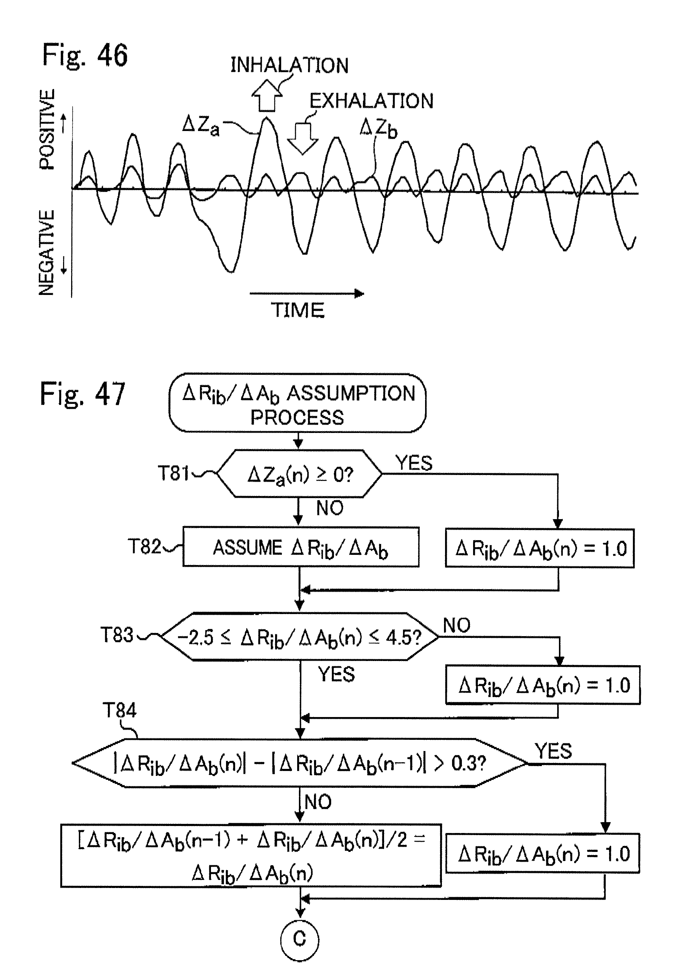

Fig. 46 is a graph showing change over time of each of a fifth difference and a sixth difference in abdominal respiration;

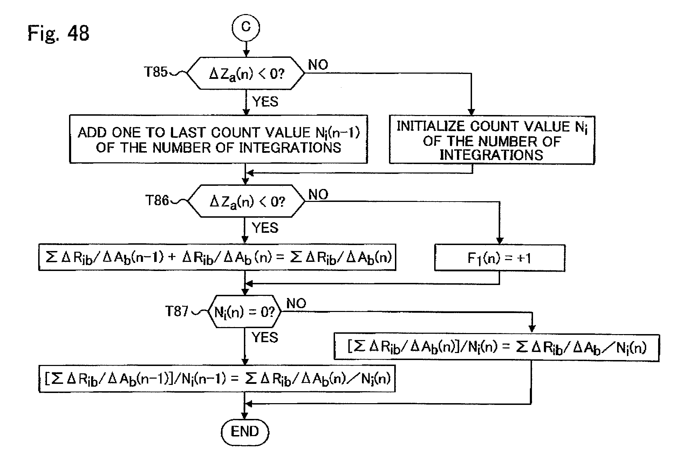

Figs. 47 and 48 form a flow chart showing an example of a $\Delta R_{ib}/\Delta A_b$ assumption process executed in the body condition determination apparatus;

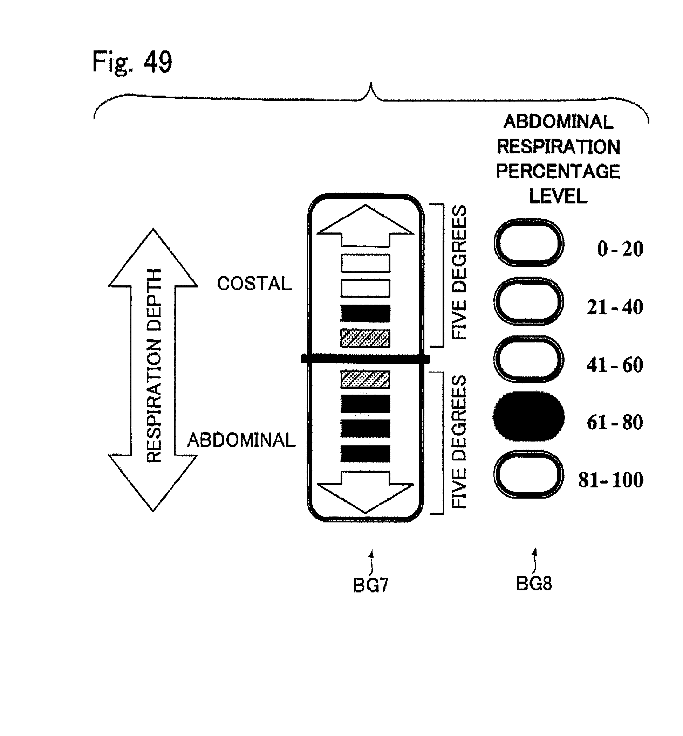

Fig. 49 is a view showing an image shown on a display device of the body condition determination apparatus of the fourth embodiment;

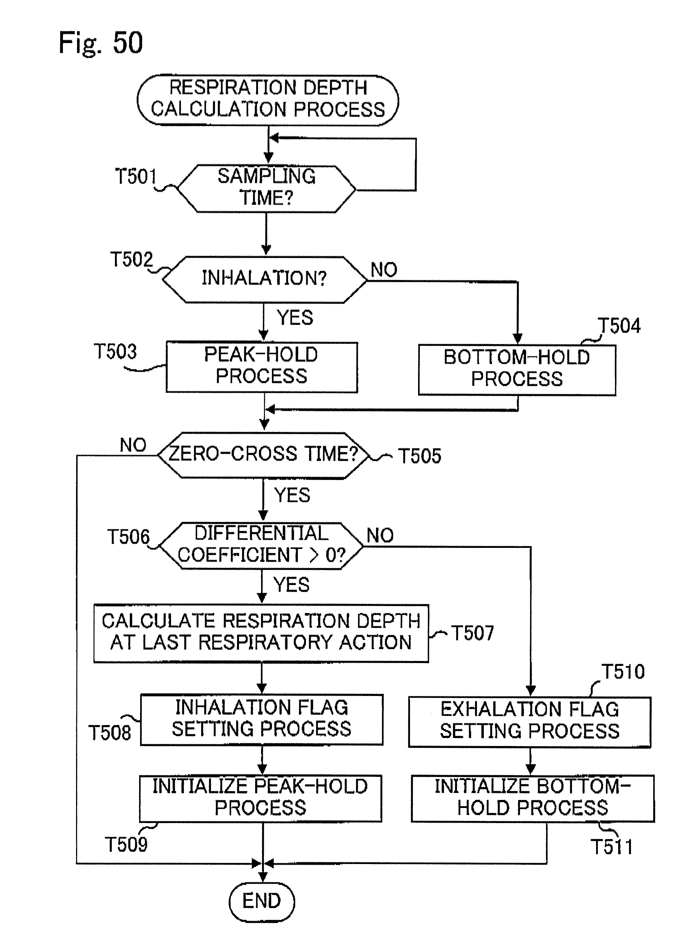

Fig. 50 is a flow chart showing an example of a respiration depth calculating process executed in the body condition determination apparatus of the fourth embodiment;

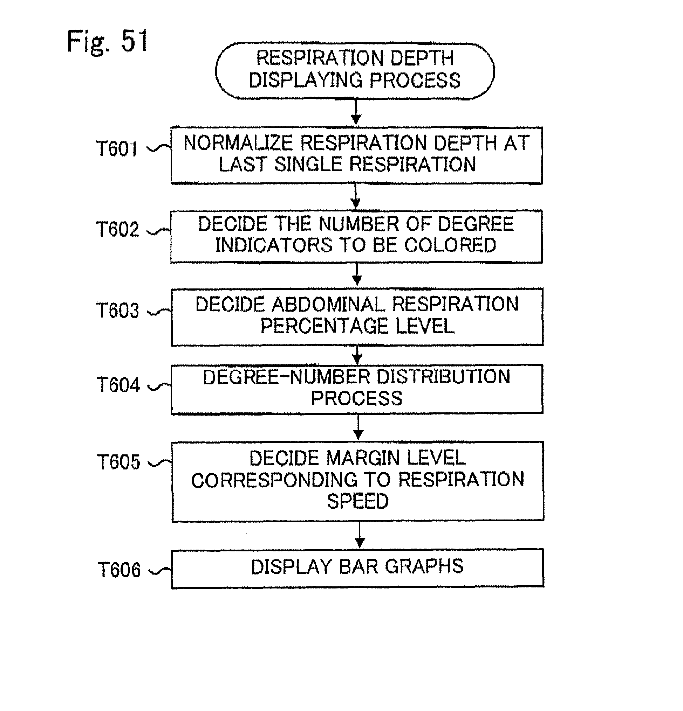

Fig. 51 is a flow chart showing an example of a respiration depth displaying process executed in the body condition determination apparatus of the fourth embodiment;

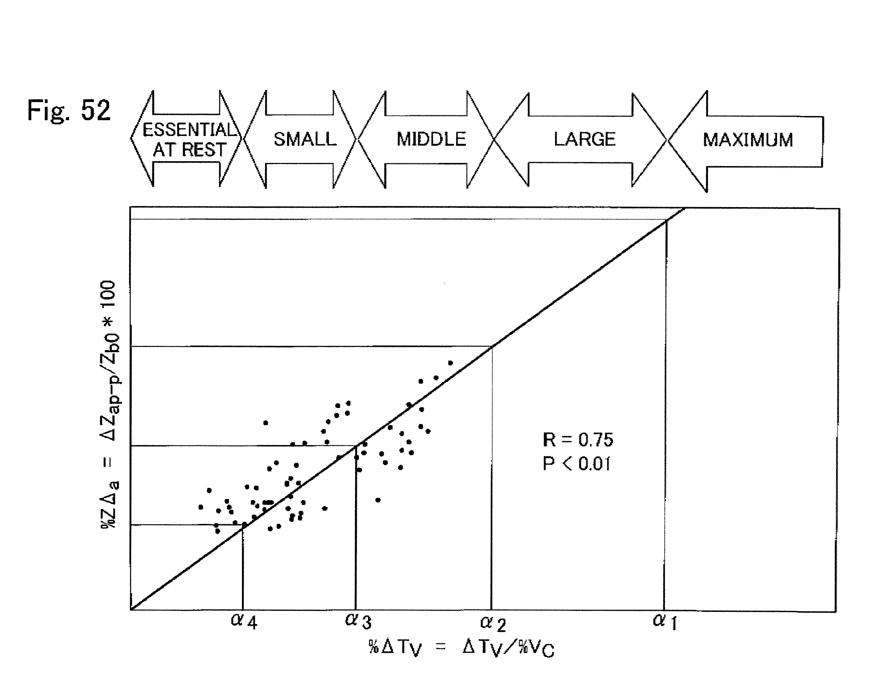

Fig. 52 is a diagram showing relationships between a respiration depth and a one-time ventilation volume;

Fig. 53 is a graph showing change over time of each of bioelectrical impedances at the upper body trunk and the middle body trunk, which is related to the fifth embodiment according to the present invention;

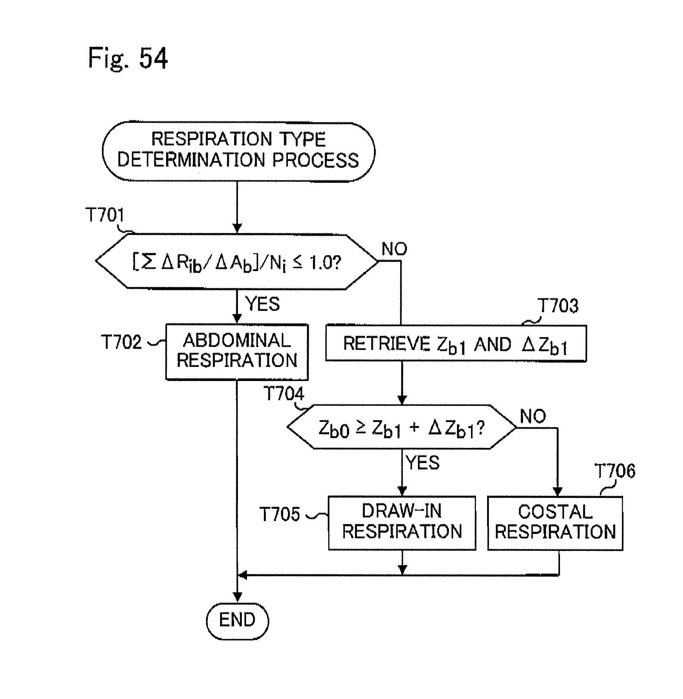

Fig. 54 is a flow chart showing an example of a respiration type determination process executed in the body condition determination apparatus;

Fig. 55 is a diagram showing a Lissajous figure obtained through a single costal respiratory action, which is related to the sixth embodiment according to the present invention;

Fig. 56 is a diagram showing a Lissajous figure obtained through a single abdominal respiratory action, which is related to the sixth embodiment according to the present invention;

Fig. 57 is a diagram showing a Lissajous figure obtained through multiple costal respiratory actions;

Fig. 58 is a diagram showing a Lissajous figure obtained through multiple abdominal respiratory actions;

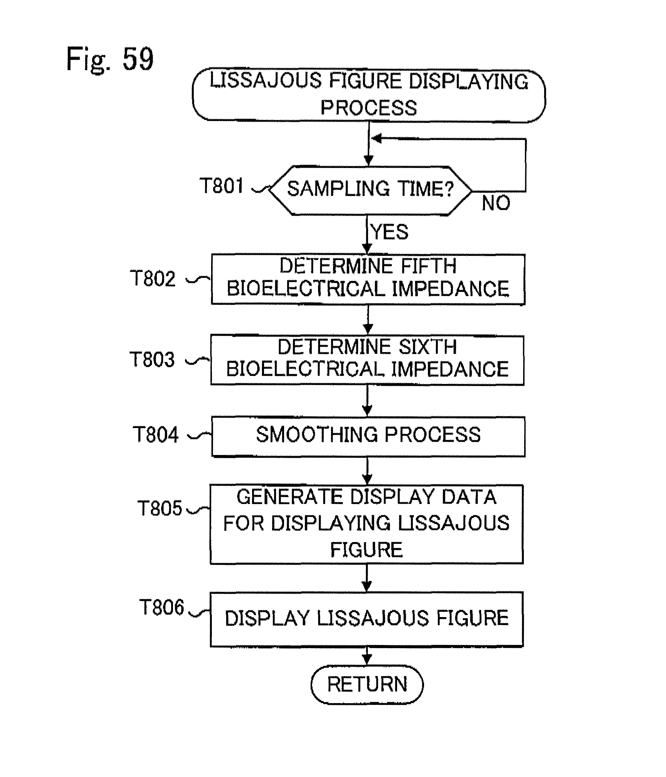

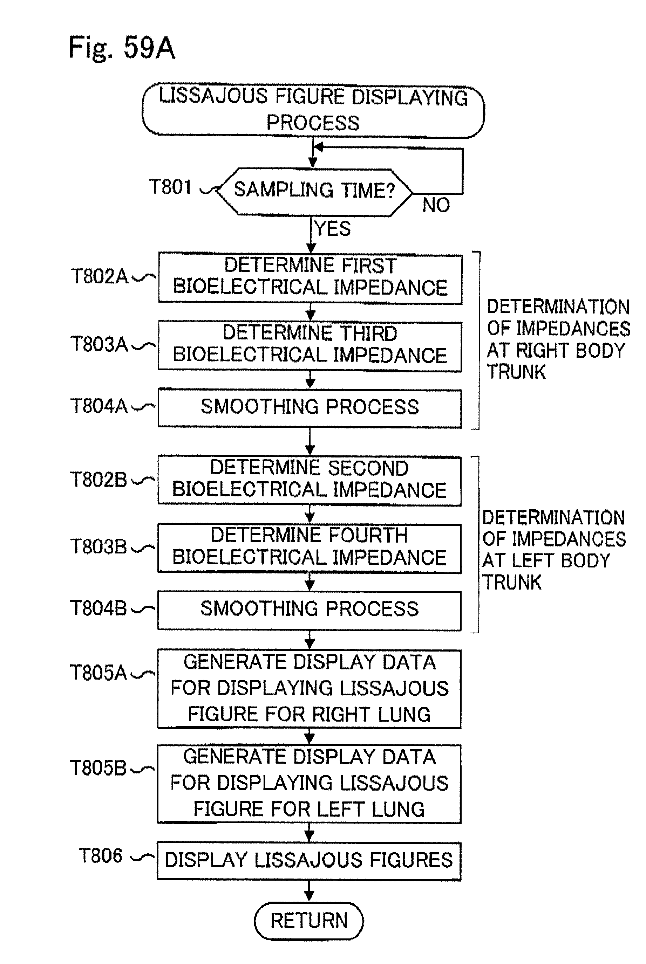

Fig. 59 is a flow chart showing an example of a Lissajous figure displaying process executed in the body condition determination apparatus of the sixth embodiment;

Fig. 59A is a flow chart showing a variation of the Lissajous figure displaying process

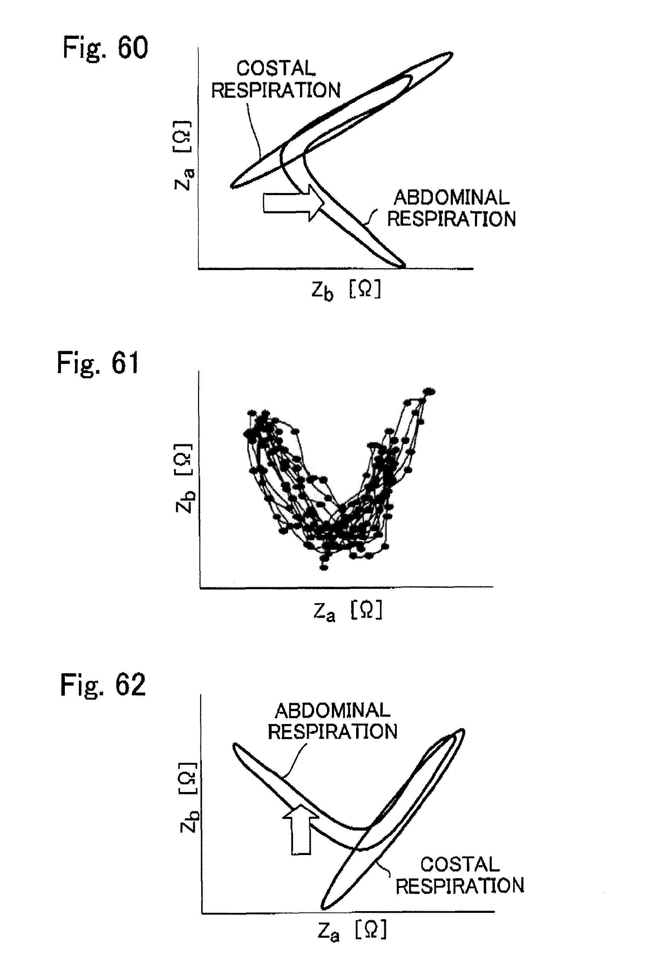

Fig. 60 is a diagram showing change in the Lissajous figure when a human subject switches from costal respiration to abdominal respiration;

Fig. 61 is a diagram showing a Lissajous figure in which the X axis and the Y axis are switched;

Fig. 62 is a diagram showing change in the Lissajous figure when a human subject switches from costal respiration to abdominal respiration, in which the X axis and the Y axis are switched;

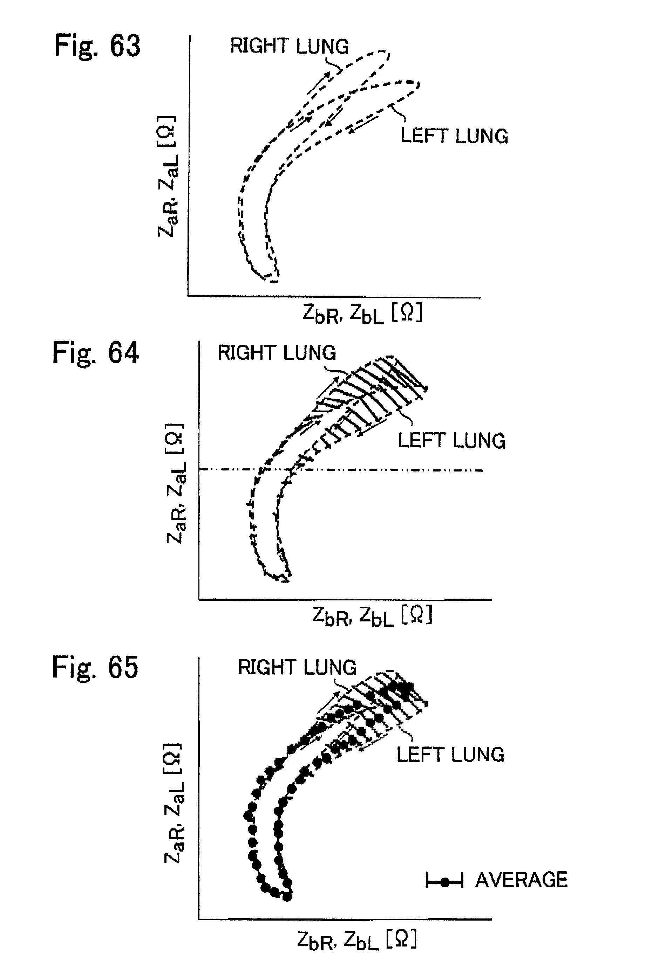

Fig. 63 is a diagram in which two Lissajous figures for the right lung and the left lung obtained through a single respiratory action overlap each other;

Fig. 64 is a diagram in which differences between two Lissajous figures for the right lung and the left lung are highlighted;

Fig. 65 is a diagram showing a Lissajous figure in which averages of samples for the right lung and the left lung are plotted;

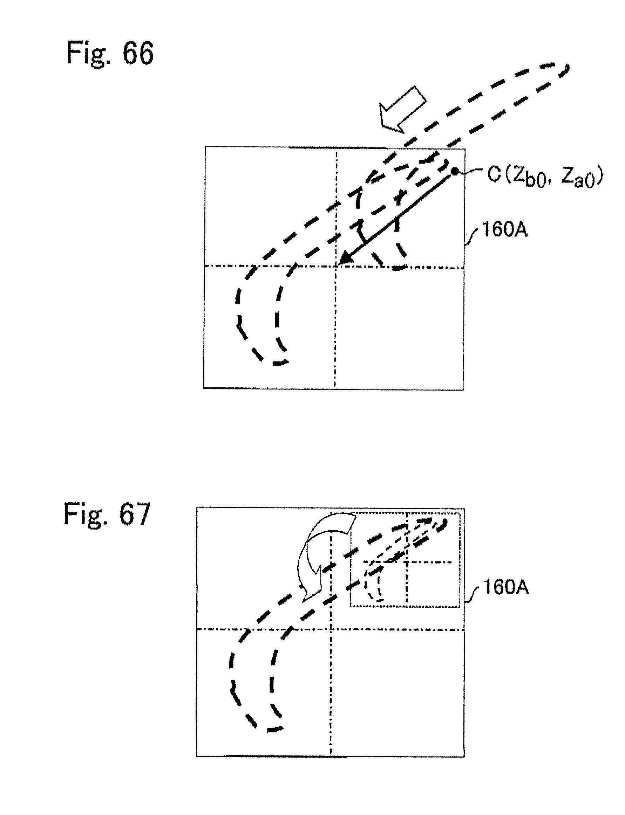

Fig. 66 is a diagram for explaining a scheme for centering the displayed location of a Lissajous figure;

Fig. 67 is a diagram for explaining another scheme for centering the displayed location of a Lissajous figure;

Fig. 68 is a diagram in which two Lissajous figures for draw-in respiration and costal respiration are shown;

Fig. 69 is a diagram showing a Lissajous figure in which the track for the latest respiration and the track for earlier respirations are shown in different depth of color;

Fig. 70 is a diagram for explaining an assistance display using Lissajous figures;

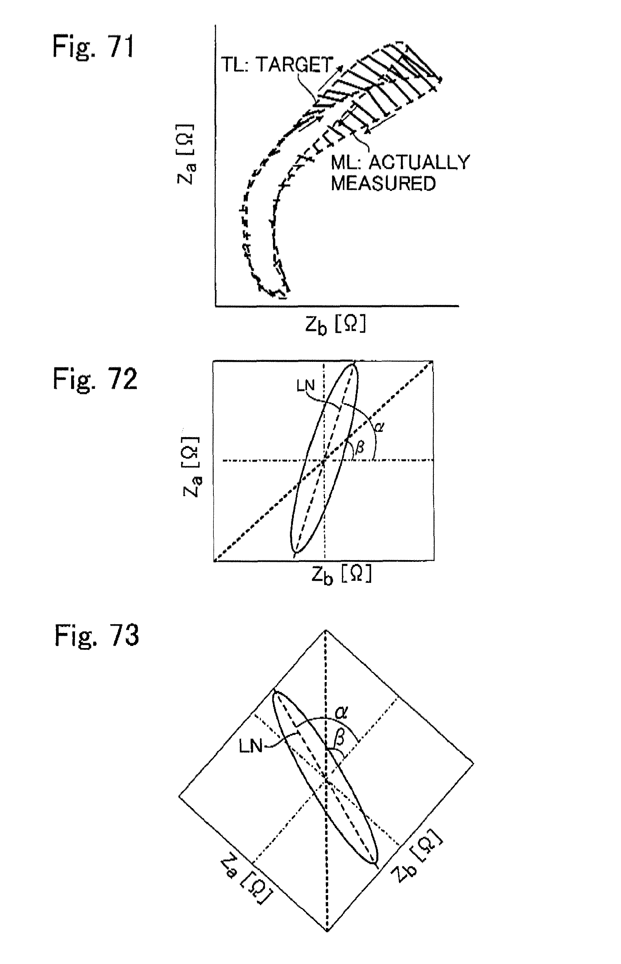

Fig. 71 is a diagram for explaining another assistance display using Lissajous figures;

Fig. 72 is a diagram for explaining a scheme for determining whether the respiration capability is good or bad;

Fig. 73 is a diagram for explaining another scheme for determining whether the respiration capability is good or bad;

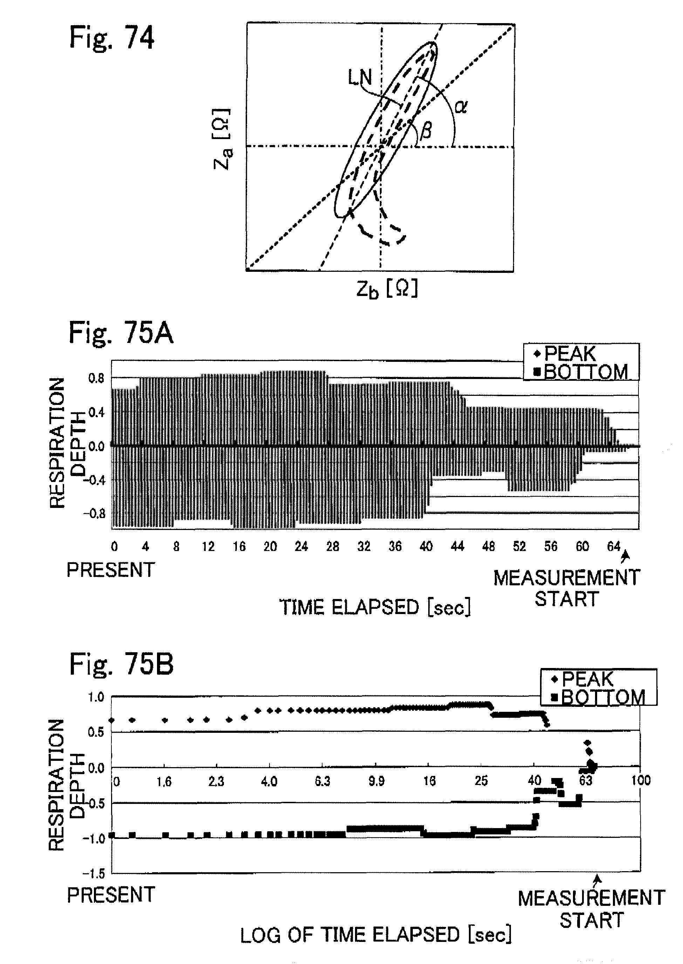

Fig. 74 is a diagram for explaining another scheme for determining whether the respiration capability is good or bad;

Fig. 75A is a graph showing an example of change over time of respiration depth;

Fig. 75B is a graph showing an example of change over time of respiration depth;

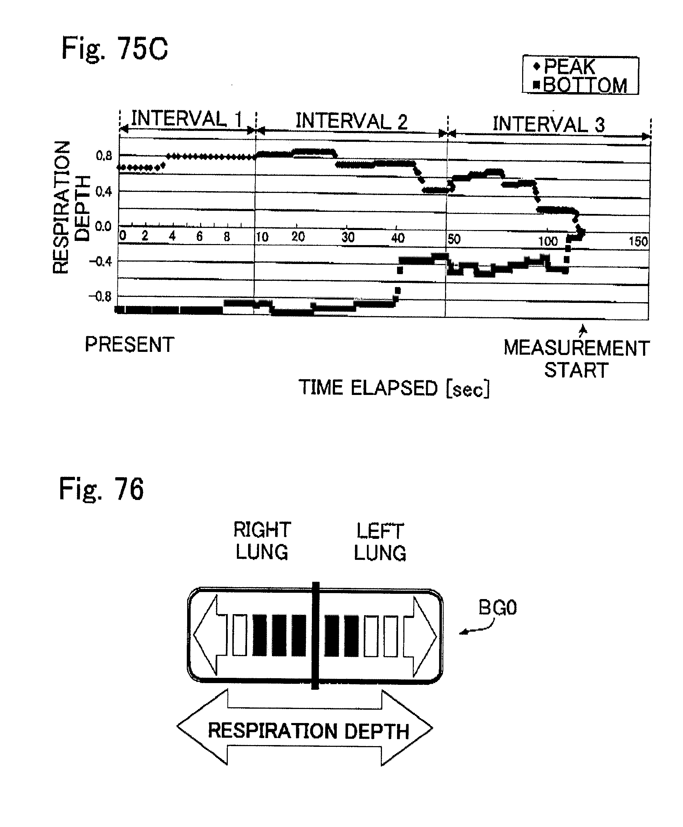

Fig. 75C is a graph showing an example of change over time of respiration depth;

Fig. 76 is a view showing an image shown on a display device of the body condition determination apparatus of a third variation according to the present invention;

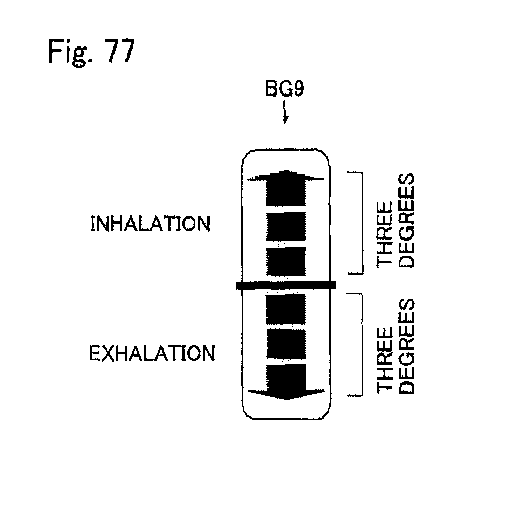

Fig. 77 is a view showing an image of assistance information shown on a display device of the body condition determination apparatus;

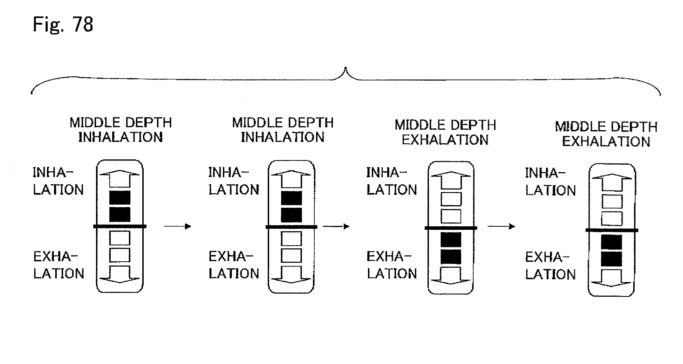

Fig. 78 is a view showing change over time of the assistance information shown on the display device;

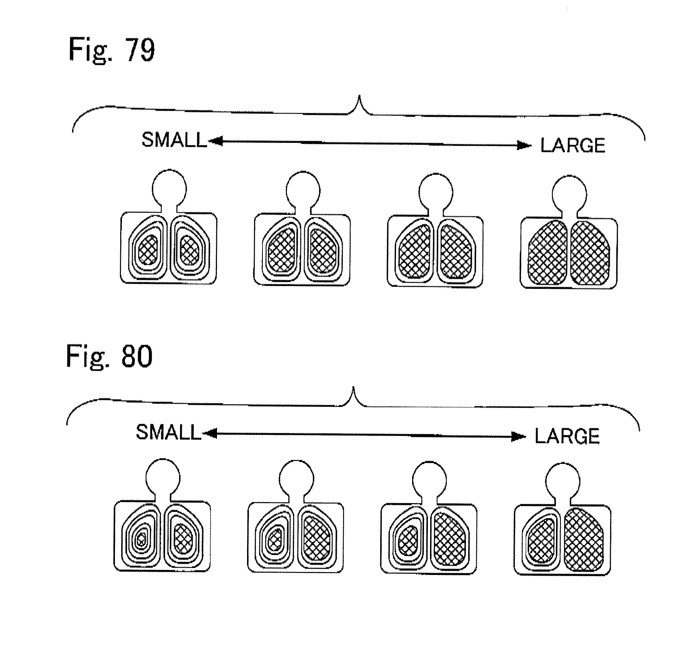

Fig. 79 is a view showing a displaying scheme for reporting a respiration magnitude, which is related to a sixth variation of the present invention;

Fig. 80 is a view showing a displaying scheme for reporting a respiration magnitude, which is related to a seventh variation of the present invention;

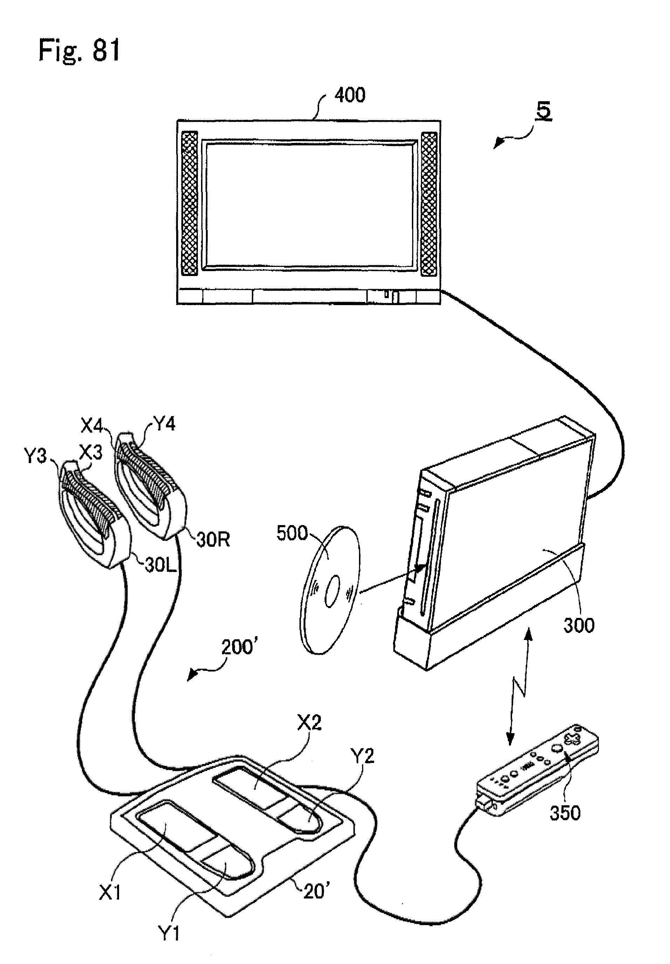

Fig. 81 is an overall view showing a body condition determination system of an embodiment according to the eleventh variation of the present invention having a household game machine;

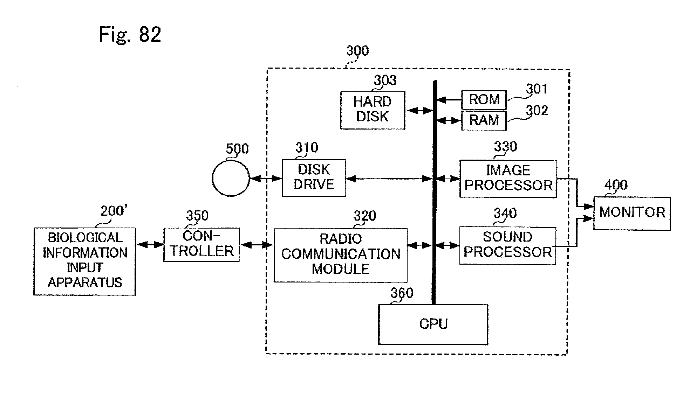

Fig. 82 is a block diagram showing a structure of the game machine;

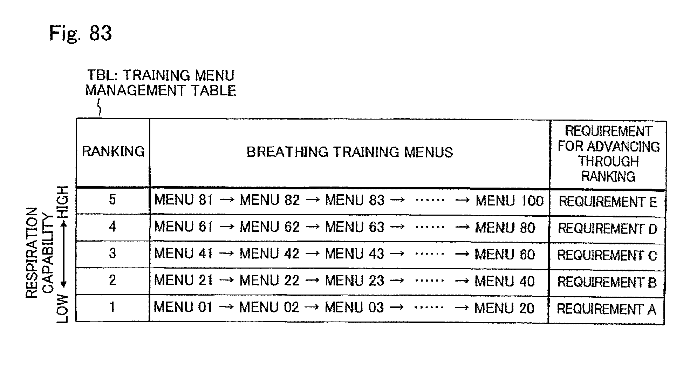

Fig. 83 is a diagram showing a data format of a training menu management table;

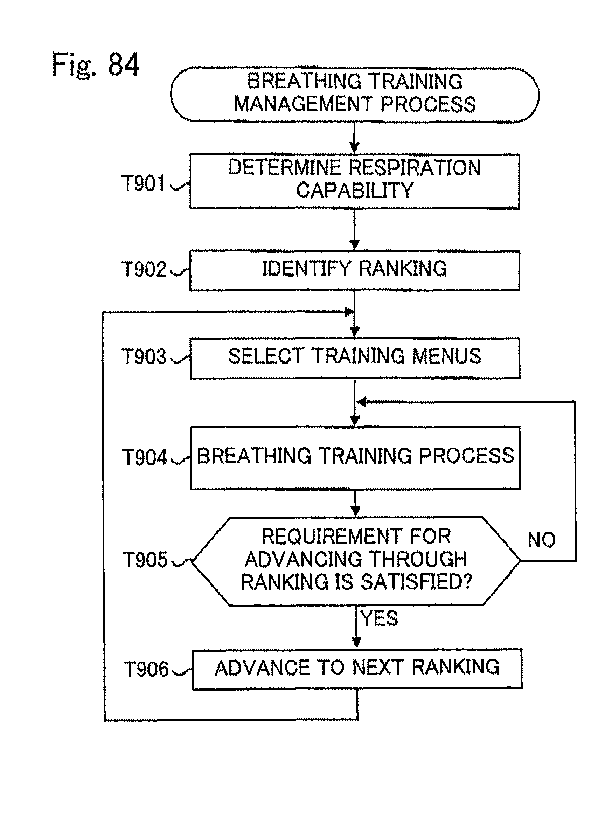

Fig. 84 is a flow chart showing an example of a breathing training management process executed in the body condition determination apparatus;

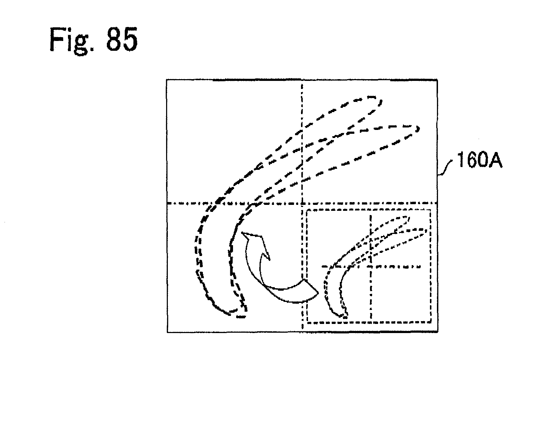

Fig. 85 is a diagram for explaining a scheme for centering the displayed locations of two Lissajous figures for the right lung and the left lung;

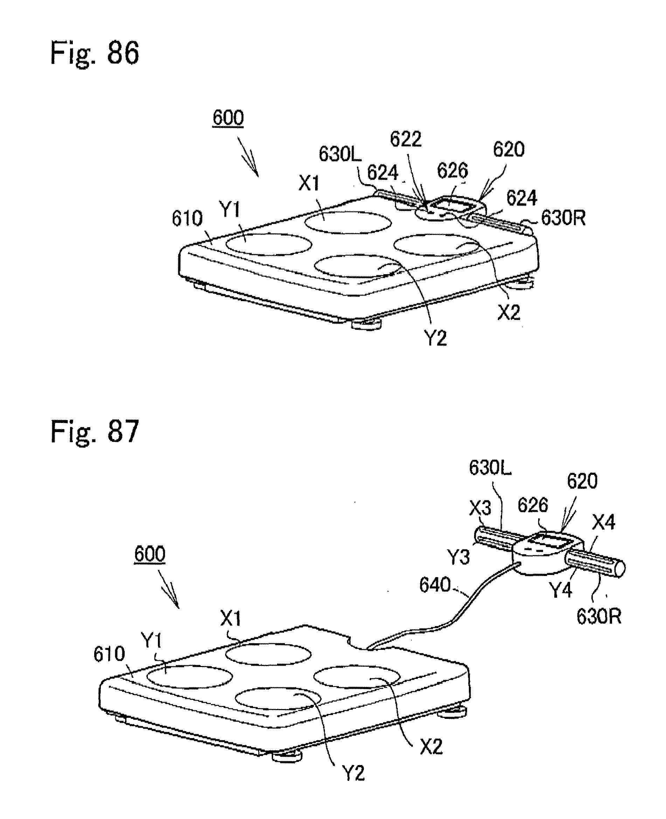

Fig. 86 is a perspective view showing a body condition determination apparatus of a variation; and

Fig. 87 is a perspective view showing the body condition determination apparatus of Fig. 86 in a different position.

DETAILED DESCRIPTION OF PREFERRED EMBODIMENTS

1. FIRST EMBODIMENT

1.1. STRUCTURE

Fig. 1 is a block diagram showing an electrical structure of a body condition determination apparatus 1 of a first embodiment according to the present invention. The body condition determination apparatus 1 determines conditions of human subjects, and functions as a respiration characteristic analysis apparatus that analyzes respiration capability of each of the right lung and the left lung of the human subjects.

The body condition determination apparatus 1 includes a management part 100 for measuring the body weights of human subjects and for managing overall operations of the body condition determination apparatus 1, and a bioelectrical impedance determination part 200 for determining bioelectrical impedances at various body regions of human subjects.

The management part 100 includes a weighing scale 110, a first memory 120, a second memory 130, a sound processor 140, a speaker 145, a human interface 150, and a display device 160. These elements are connected with a processor that is typically a CPU (Central Processing Unit) 170 via a bus. The CPU 170 serves as a main controller for controlling the entire apparatus. During operation of the CPU 170, the CPU 170 receives clock signals from a clock signal generation circuit (not shown). When a power switch (not shown) is turned on, a power source circuit supplies these elements with power.

The weighing scale 110 measures weights of human subjects and supplies weight data to the CPU 170 via the bus.

The first memory 120 is a nonvolatile memory, for example, a ROM (Read Only Memory). The first memory 120 stores a control program for controlling the entire apparatus. In accordance with the control program, the CPU 170 executes a predetermined arithmetic process.

The second memory 130 is a volatile memory, for example, a DRAM (Dynamic Random Access Memory). The second memory 130 serves as a work area for the CPU 170. During the execution of the predetermined arithmetic process by the CPU 170, the second memory 130 stores various data.

Under control by the CPU 170, the sound processor 140 conducts digital-to-analog conversion on sound data, amplifies the resulting sound signals to the speaker 145, and supplies the amplified sound signals to the speaker 145. The speaker 145 converts the amplified sound signals into sound vibrations and emits sound. Accordingly, the speaker 145 can provide human subjects with advisory or informative sounds, for example, guidance in manners of breathing.

The human interface 150 includes input devices. The human subject or another person may manipulate the input devices in order to input personal information on the human subject, for example, the height, age, and sex into the body condition determination apparatus 1.

The display device 160 shows the measurement results, such as the weight or the type of respiration. The display device 160 also shows instructions (of rhythm and pattern of exhalation and inhalation) to exhale and inhale in order to lead the human subject to perform abdominal breathing. The display device 160 shows messages for leading the human subject to input various information into the human interface 150. The display device 160 (reporter) may be, for example, a liquid crystal display device.

The bioelectrical impedance determination part 200 is used for determining bioelectrical impedances of the human subject (human body). The bioelectrical impedance determination part 200 includes an alternating current supplying circuits 210, a reference current measurement circuit 220, a potential difference measurement circuit 230, an A/D converter 240, and electrode switching circuits 251 and 252.

The alternating current supplying circuit 210 generates a reference current $I_{ref}$ having a frequency determined in the control program. The reference current measurement circuit 220 supplies the reference current $I_{ref}$ to the human subject, measures the actual value of the reference current $I_{ref}$ flowing through the human subject, and supplies electric current data $D_i$ indicative of the actual value of the reference current $I_{ref}$. The electrode switching circuit 252 selects, among four alternating current electrodes X1 through X4, two or four electrodes through which the current flows. The current electrodes should be brought into contact with the human subject.

The potential difference measurement circuit 230 measures the potential difference between two voltage electrodes selected from among four voltage electrodes Y1 through Y4 by the other electrode switching circuit 251, and generates a potential difference signal $\Delta V$ indicative of the potential difference. The A/D converter 240 converts the analog potential difference signal $\Delta V$ into a digital signal, that is, voltage data $D_v$, and supplies the voltage data $D_v$ to the CPU 170. The CPU 170 calculates the bioelectrical impedance Z on the basis of the voltage data $D_v$ and the current data $D_i$. That is, the bioelectrical impedance Z is the potential difference divided by the current. Thus, the CPU 170 and the bioelectrical impedance determination part 200 serve as a bioelectrical impedance determiner for determining bioelectrical impedance at at least a body region of the human subject.

The first memory 120 may store various data in advance. For example, the first memory 120 may store correlation equations or tables for deriving the body fat percentage and the amount of muscle mass on the basis of bioelectrical impedances at various body regions.

The CPU 170 calculates the weight and bioelectrical impedances at various body regions (e.g., bioelectrical impedances at the upper limbs, bioelectrical impedances at the lower limbs, and the bioelectrical impedance at the body trunk), and controls various operations including signal inputting, signal outputting, measurements, and calculations. The CPU 170 can calculate the ratio of visceral fat to subcutaneous fat, the visceral fat amount, the subcutaneous fat ratio, the subcutaneous fat amount, the systemic body fat ratio, and body regional fat ratios (e.g., body fat ratios at the upper limbs, the lower limbs, and the body trunk).

Fig. 2 shows the appearance of the body condition determination apparatus 1. The body condition determination apparatus 1 has an L-shape including a platform 20 on which the human subject stands and a rectangular-columnar housing 30 extending upwardly from the platform 20. The platform 20 is provided with a left-foot current electrode X1 and a left-foot voltage electrode Y1 on which the left foot of the human subject will be placed, and a right-foot current electrode X2 and a right-foot voltage electrode Y2 on which the right foot of the human subject will be placed.

At the top of the housing 30, the aforementioned display device 160 is located. The display device 160 is a touch panel and serves as the human interface 150.

A left electrode handle 30L is located at the left side surface of the housing 30 whereas a right electrode handle 30R is located at the right side surface of the housing 30.

Fig. 3 is an enlarged view showing the upper part of the housing 30. As shown in Fig. 3, the left electrode handle 30L includes a left-hand current electrode X3 and a left-hand voltage electrode Y3, whereas the right electrode handle 30R includes a right-hand current electrode X4 and a right-hand voltage electrode Y4. For measurements of bioelectrical impedances, the human subject stands on the platform 20 and grips the electrode handles 30L and 30R with the left and right hands, respectively, with the arms extending downward.

Under control by the CPU 170, the electrode switching circuit 251 selects two voltage electrodes from among the four voltage electrodes Y1 through Y4, whereas the electrode switching circuit 252 selects two current electrodes from among the four current electrodes X1 through X4, so that impedances Z at various body regions can be determined.

More specifically, as shown in part (A) of Fig. 4, when the reference current $I_{ref}$ is supplied to flow between the left-foot current electrode X1 and the left-hand current electrode X3 and when the potential difference between the left-foot voltage electrode Y1 and the left-hand voltage electrode Y3 is measured, the systemic body bioelectrical impedance can be determined. Although not illustrated, when the reference current $I_{ref}$ is supplied to flow between the right-foot current electrode X2 and the right-hand current electrode X4 and when the potential difference between the right-foot voltage electrode Y2 and the right-hand voltage electrode Y4 is measured, the systemic body bioelectrical impedance can also be determined.

As shown in part (K) of Fig. 4, when the current is supplied to all of the four current electrodes so that a current flows between both hands and a current flows between both feet, and when the potential difference between a hand and a foot is measured, the systemic body bioelectrical impedance can also be determined.

As shown in part (B) of Fig. 4, when the reference current $I_{ref}$ is supplied to flow between the right-foot current electrode X2 and the right-hand current electrode X4 and when the potential difference between the right-foot voltage electrode Y2 and the left-foot voltage electrode Y1 is measured, the bioelectrical impedance at the right lower extremity can be determined.

As shown in part (C) of Fig. 4, when the reference current $I_{ref}$ is supplied to flow between the left-foot current electrode X1 and the left-hand current electrode X3 and when the potential difference between the left-foot voltage electrode Y1 and the right-foot voltage electrode Y2 is measured, the bioelectrical impedance at the left lower extremity can be determined.

As shown in part (D) of Fig. 4, when the reference current $I_{ref}$ is supplied to flow between the right-hand current electrode X4 and the right-foot current electrode X2 and when the potential difference between the right-hand voltage electrode Y4 and the left-hand voltage electrode Y3 is measured, the bioelectrical impedance at the right upper extremity and the upper body trunk can be determined. Although not illustrated, when the reference current $I_{ref}$ is supplied to flow between the left-hand current electrode X3 and the right-hand current electrode X4 and when the potential difference between the right-hand voltage electrode Y4 and the right-foot voltage electrode Y2 is measured, the bioelectrical impedance at the right upper extremity and the upper body trunk can also be determined.

As shown in part (E) of Fig. 4, when the reference current $I_{ref}$ is supplied to flow between the left-hand current electrode X3 and the left-foot current electrode X1 and when the potential difference between the right-hand voltage electrode Y4 and the left-hand voltage electrode Y3 is measured, the bioelectrical impedance at the left upper extremity and the upper body trunk can be determined. Although not illustrated, when the reference current $I_{ref}$ is supplied to flow between the left-hand current electrode X3 and the right-hand current electrode X4 and when the potential difference between the left-hand voltage electrode Y3 and the left-foot voltage electrodes Y1 is measured, the bioelectrical impedance at the left upper extremity and the upper body trunk can also be determined.

As shown in part (F) of Fig. 4, when the reference current $I_{ref}$ is supplied to flow between the right-hand current electrode X4 and the left-hand current electrode X3 and when the potential difference between the right-hand voltage electrode Y4 and the left-hand voltage electrode Y3 is measured, the bioelectrical impedance at both upper extremities and the upper body trunk can be determined.

As will be understood from each of parts (D), (E), and (F) of Fig. 4, the determined bioelectrical impedance is influenced by the bioelectrical impedance at the upper body trunk. If right and left upper extremities do not move during the measurement of bioelectrical impedance, the bioelectrical impedances thereat do not change. Accordingly, change over time of the bioelectrical impedance at the right upper extremity and the upper body trunk can be considered as change over time of the bioelectrical impedance at the upper body trunk. Similarly, change over time of the bioelectrical impedance at the left upper extremity and the upper body trunk can be considered as change over time of the bioelectrical impedance at the upper body trunk. Change over time of the bioelectrical impedance at both upper extremities and the upper body trunk can be considered as change over time of the bioelectrical impedance at the upper body trunk.

As shown in part (G) of Fig. 4, when the reference current $I_{ref}$ is supplied to flow between the left-hand current electrode X3 and the left-foot current electrode X1 and when the potential difference between the right-hand voltage electrode Y4 and the right-foot voltage electrode Y2 is measured, the bioelectrical impedance at the middle body trunk can be determined.

As shown in part (H) of Fig. 4, when the reference current $I_{ref}$ is supplied to flow between the right-hand current electrode X4 and the right-foot current electrode X2 and when the potential difference between the left-hand voltage electrode Y3 and the left-foot voltage electrode Y1 is measured, the bioelectrical impedance at the middle body trunk can be determined.

As shown in part (I) of Fig. 4, when the reference current $I_{ref}$ is supplied to flow between the right-hand current electrode X4 and the left-foot current electrode X1 and when the potential difference between the left-hand voltage electrode Y3 and the right-foot voltage electrode Y2 is measured, the bioelectrical impedance at the middle body trunk can be determined.

As shown in part (J) of Fig. 4, when the reference current $I_{ref}$ is supplied to flow between the right-foot current electrode X2 and the left-hand current electrode X3 and when the potential difference between the right-hand voltage electrode Y4 and the left-foot voltage electrode Y1 is measured, the bioelectrical impedance at the middle body trunk can be determined.

The manner for determining the bioelectrical impedance at the upper or middle body trunk is not limited to the above-described manner. For example, bioelectrical impedances at various regions, e.g., upper and lower extremities, and the systemic body are determined by suitably selecting the current electrodes through which the reference current $I_{ref}$ is supplied and by suitably selecting the voltage electrodes for measuring the potential difference. Then, the bioelectrical impedance at the upper or middle body trunk can be obtained by addition or subtraction of the bioelectrical impedances.

In addition, if electrodes are brought into contact with the ear-lobes instead of extremities, the bioelectrical impedance at the body trunk can be obtained. Alternatively, if electrodes are brought into contact with the body trunk directly, the bioelectrical impedance at the upper or middle body trunk can be obtained.

### 1.2. OPERATION

Fig. 5 is a flow chart showing an operation of the body condition determination apparatus 1. Once the power switch (not shown) on the human interface 150 is turned on, the power source circuit (not shown) provides power to electrical elements. Then, the CPU 170 causes the display device 160 to show a screen for facilitating the user to enter personal or individual body information (including the height, age, and sex) at step S1.

After the human interface 150 inputs the personal information into the CPU 170, the CPU 170 causes the weighing scale 110 to measure the weight, and obtains the weight from the weighing scale 110 at step S2.

The CPU 170 causes the bioelectrical impedance determination part 200 to measure the voltages and the currents, each of which is influenced by bioelectrical impedances at desired regions (for example, the extremities and the body trunk), and determines the impedances at the desired body region. Thus, the CPU 170 and the bioelectrical impedance determination part 200 serve as a bioelectrical impedance determiner for determining bioelectrical impedance at the desired body regions. The CPU 170 executes a respiration depth analysis process at step S3. As will be described later in detail, in the respiration depth analysis process, the CPU 170 serves as an analyzer that obtains the right lung respiration depth of the human subject related to the respiration capability of the right lung of the human subject, and the left lung respiration depth of the human subject related to the respiration capability of the left lung of the human subject.

After step S3, the CPU 170 executes a respiration depth displaying process (step S4). As will be described later in detail, in the respiration depth displaying process, the CPU 170 causes the display device 160 to show the right lung respiration depth and the left lung respiration depth at every respiration. Although not shown, the operation may be ended by the user's instruction.

### 1.3. PRINCIPLES OF RESPIRATION DEPTH ANALYSIS

The principles of respiration depth analysis will be described. Fig. 6 is a schematic view showing tissues in the body trunk of humans. As shown in Fig. 6, tissues in the body trunk are separated by the diaphragm into the upper and lower parts. The upper part includes the lungs and the chest skeletal muscle including the internal and external intercostal muscles. On the other hand, the lower part includes the visceral tissue and the abdominal skeletal muscle including the internal and external abdominal oblique muscles, the transverse abdominal muscle, and the abdominal rectus muscle.

In both abdominal respiration and costal respiration, the diaphragm moves up to compress the lungs at exhalation and moves down to expand the lungs at inhalation. The characteristic of abdominal respiration that does not appear in costal respiration is that the visceral tissue expands and contracts in the perpendicular direction so as to move the diaphragm up and down due to ventrodorsal contraction and expansion of the abdominal respiratory muscles including the abdominal rectus muscle, the internal and external abdominal oblique muscles, and the transverse abdominal muscle.

With reference to the equivalent circuit models in Figs. 7A and 7B, factors influencing the bioelectrical impedance $Z_a$ at the upper body trunk and the bioelectrical impedance $Z_b$ at the middle body trunk will be described. Let us assume that the bioelectrical impedance at the chest skeletal muscle is $Z_1$ and $Z_4$, that at the lungs is $Z_2$ and $Z_5$, that at the upper extremity skeletal muscle is $Z_3$, that at the abdominal skeletal muscle is $Z_6$, and that at the visceral tissue is $Z_7$. As will be understood from Fig. 7B, the bioelectrical impedance $Z_a$ at the upper body trunk is influenced by impedances $Z_1$ and $Z_2$ in parallel with each other and by impedance $Z_3$. The synthetic impedance of $Z_1$ and $Z_2$ corresponds to the bioelectrical impedance at the upper lobes of the lungs.

As will be understood from Fig. 7A, the bioelectrical impedance $Z_b$ at the upper middle trunk is influenced by impedances $Z_4$ and $Z_5$ in parallel with each other and by impedances $Z_6$ and $Z_7$ in parallel with each other. The synthetic impedance of $Z_4$ and $Z_5$ corresponds to the bioelectrical impedance at the median and lower lobes of the lungs. The bioelectrical impedance $Z_7$ at the visceral tissue includes the bioelectrical impedance of the diaphragm.

With reference to Fig. 8, the relationship between respiration and change of bioelectrical impedance will be described.

Change in the bioelectrical impedance $Z_a$ at the upper body trunk in respiration is mainly caused by air entering and leaving the lungs, which has a high electrical insulation property and causes change in the electrical characteristics (i.e., the electrical conductivity or the inverse of the volume resistivity) at the upper body trunk. During exhalations (expirations), the bioelectrical impedance $Z_2$ at the lungs decreases due to reduction in the volume of air inside tissues in the lungs ($\Delta Z_{lu} < 0$). During inhalations (inspirations), the bioelectrical impedance $Z_2$ at the lungs increases due to increase in the volume of air inside tissues in the lungs ($\Delta Z_{lu} > 0$).

In costal respiration, which expands and contracts the thoracic cage, the chest skeletal muscle that contributes to respiration (e.g., internal and external intercostal muscles) expands and contracts in a similar way to the expansion and contraction of the lungs. Therefore, in costal respiration, when the lungs expand so that the bioelectrical impedance $Z_2$ at the lungs increases, the chest skeletal muscle also expands, and the bioelectrical impedance $Z_1$ at the chest skeletal muscle also increases. Similarly, when the lungs contract so that the bioelectrical impedance $Z_2$ at the lungs decreases, the chest skeletal muscle also contracts and the bioelectrical impedance $Z_1$ at the chest skeletal muscle also decreases.

However, in abdominal respiration in which the thoracic cage does not change in volume significantly, although the bioelectrical impedance $Z_2$ at the lungs changes significantly due to respiration, the bioelectrical impedance $Z_1$ at the chest skeletal muscle does not change significantly. The bioelectrical impedance $Z_a$ at the upper body trunk includes the bioelectrical impedance $Z_3$ at the upper extremity skeletal muscle, but the upper extremity skeletal muscle does not directly contribute to respiration. In this embodiment, the human subject stands on the platform 20 shown in Fig. 2 and grips the electrode handles 30L and 30R with the left and right hands, respectively, with the arms extending downward, so that the upper extremity skeletal muscle does not move during the measurement of bioelectrical impedance, and the bioelectrical impedance $Z_3$ thereat does not change significantly. As shown in Figs. 9 and 10, in both of costal respiration and abdominal respiration, the bioelectrical impedance $Z_a$ at the upper body trunk increases at inhalations and decreases at exhalations.

The bioelectrical impedance $Z_a$ at the upper body trunk is influenced by the bioelectrical impedance at the right upper body trunk and the bioelectrical impedance at the left upper body trunk. Hereinafter, the bioelectrical impedance at the right upper body trunk and the right upper extremity will be referred to as the first bioelectrical impedance $Z_{aR}$, whereas the bioelectrical impedance at the left upper body trunk and the left upper extremity will be referred to as the second bioelectrical impedance $Z_{aL}$. Factors influencing the first bioelectrical impedance $Z_{aR}$ at the right upper body trunk and the right upper extremity and the second bioelectrical impedance $Z_{aL}$ at the left upper body trunk and the left upper extremity can be expressed in the equivalent circuit models in Fig. 11.

As will be understood from Fig. 11, the first bioelectrical impedance $Z_{aR}$ is influenced by bioelectrical impedance $Z_{1R}$ at the right chest skeletal muscle and bioelectrical impedance $Z_{2R}$ at the right lung, which are in parallel with each other, and by bioelectrical impedance $Z_{3R}$ at the right upper extremity skeletal muscle. The synthetic impedance of $Z_{1R}$ and $Z_{2R}$ corresponds to the bioelectrical impedance at the upper lobe of the right lung. Change over time of bioelectrical impedance $Z_{3R}$ may be ignorable since the upper extremity skeletal muscle does not move during the measurement of bioelectrical impedance. Accordingly, the first bioelectrical impedance $Z_{aR}$ is the bioelectrical impedance at the right upper body trunk including the upper lobe of the right lung of the human subject and excluding the abdomen of the human subject.

The first bioelectrical impedance $Z_{aR}$ changes in respiration by air entering and leaving the right lung. The manner of change is similar to that of the above-described bioelectrical impedance $Z_a$ at the upper body trunk. When the volume of air entering and leaving the right lung (the ventilation volume at the right lung) is larger, the amplitude of the first bioelectrical impedance $Z_{aR}$ is larger. The amplitude of the first bioelectrical impedance $Z_{aR}$ corresponds to the respiration capability of the upper lobe of the right lung. Accordingly, on the basis of measurement values of the first bioelectrical impedance $Z_{aR}$, a first right lung respiration depth BR1 related to the respiration capability of the upper lobe of the right lung can be calculated, as will be described later in detail.

As will be understood from Fig. 11, the second bioelectrical impedance $Z_{aL}$ is influenced by bioelectrical impedance $Z_{1L}$ at the left chest skeletal muscle and bioelectrical impedance $Z_{2L}$ at the left lung, which are in parallel with each other, and by bioelectrical impedance $Z_{3L}$ at the left upper extremity skeletal muscle. The synthetic impedance of $Z_{1L}$ and $Z_{2L}$ corresponds to the bioelectrical impedance at the upper lobe of the left lung. Change over time of bioelectrical impedance $Z_{3L}$ may be ignorable since the upper extremity skeletal muscle does not move during the measurement of bioelectrical impedance. Accordingly, the second bioelectrical impedance $Z_{aL}$ is the bioelectrical impedance at the left upper body trunk including the upper lobe of the left lung of the human subject and excluding the abdomen of the human subject.

The second bioelectrical impedance $Z_{aL}$ changes in respiration by air entering and leaving the left lung. The manner of change is similar to that of the above-described bioelectrical impedance $Z_a$ at the upper body trunk. When the volume of air entering and leaving the left lung (the ventilation volume at the left lung) is larger, the amplitude of the first bioelectrical impedance $Z_{aR}$ is larger. The amplitude of the second bioelectrical impedance $Z_{aL}$ corresponds to the respiration capability of the upper lobe of the left lung. Accordingly, on the basis of measurement values of the second bioelectrical impedance $Z_{aL}$, the first left lung respiration depth BL1 related to the respiration capability of the upper lobe of the left lung can be calculated, as will be described later in detail.

Change in the bioelectrical impedance $Z_b$ at the middle body trunk in respiration relates to movement of the diaphragm. As described above, in both abdominal respiration and costal respiration, the diaphragm moves up to compress the lungs at exhalation and moves down to expand the lungs at inhalation. The characteristic of abdominal respiration is that the visceral tissue expands and contracts in the perpendicular direction for moving the diaphragm up and down due to ventrodorsal contraction and expansion of the abdominal respiratory muscles (abdominal skeletal muscles).

More specifically, in abdominal respiration, during exhalations, the human subject contracts the abdominal muscle ventrodorsally so as to move up the diaphragm together. As a result, the visceral tissue and the abdominal skeletal muscle expand in the perpendicular direction, thereby increasing the impedance $Z_6$ at the abdominal skeletal muscle and the impedance $Z_7$ at the visceral tissue ($\Delta Z_{st} > 0$). During exhalations, the bioelectrical impedance at the lungs decreases due to reduction in the volume of air inside tissues in the lungs as described above ($\Delta Z_{lu} < 0$). Therefore, in abdominal respiration, when the bioelectrical impedance at the chest region above the diaphragm decreases, the bioelectrical impedance at the abdominal region beneath the diaphragm increases. In other words, increase in the bioelectrical impedance of the abdominal region beneath the diaphragm cancels decrease in the bioelectrical impedance at the chest region above the diaphragm when the human subject performs exhalation in abdominal respiration. As will be understood from the above description, the movement of the abdominal region (including abdominal skeletal muscle and the visceral tissue) beneath the diaphragm varies depending on the type of respiration (costal and abdominal respirations).

As shown in Fig. 9, when respiration of the human subject is abdominal respiration, the bioelectrical impedance $Z_b$ at the middle body trunk increases at inhalations, and also increases at exhalations since the bioelectrical impedance of the abdominal region beneath the diaphragm cancels decrease in the bioelectrical impedance at the chest region above the diaphragm. As shown in Fig. 10, when respiration of the human subject is costal respiration, the bioelectrical impedance $Z_b$ at the middle body trunk increases at inhalations and decreases at exhalation, in a manner similar to the above-described change of the bioelectrical impedance $Z_a$ at the upper body trunk.

## 1.4. RESPIRATION DEPTH ANALYSIS PROCESS

Next, a respiration depth analysis process executed by the CPU 170 will be described. Fig. 12 is a flow chart showing an example of the respiration depth analysis process. In this embodiment, the CPU 170 executes the respiration depth analysis process ten times within a single respiration, i.e., single respiratory action (consisting of a single inhalation and a single exhalation) at a normal speed. Since a normal single respiration takes four seconds, the CPU 170 executes the respiration depth analysis process every 0.4 seconds. In the hollowing, the start time for each respiration depth analysis process that occurs every 0.4 seconds will be referred to as a sampling time. However, this is only an example, and the cycle of the respiration depth analysis process may be decided in a different manner.

As shown in Fig. 12, the CPU 170 first decides whether or not the current time reaches a sampling time (step S10), and the process proceeds to step S20 if the decision at step S10 is affirmative. Description of subsequent steps will be continued on the assumption that the current time reaches the n-th sampling time where n is a natural number that is equal to or greater than one. Prior to detailed description of subsequent steps, brief description will be made for each of subsequent steps.

As shown in Fig. 12, at step S20, the CPU 170 (bioelectrical impedance determiner) determines the first bioelectrical impedance $Z_{aR}$ at the right upper body truck and the right upper extremity. Next, at step S30, the CPU 170 (bioelectrical impedance determiner) determines the second bioelectrical impedance $Z_{aL}$ at the left upper body trunk and the left upper extremity. Next, at step S40, the CPU 170 executes a smoothing process for each of the first bioelectrical impedance $Z_{aR}$ determined at step S30 and the second bioelectrical impedance $Z_{aL}$ determined at step S30. Next, at step S50, the CPU 170 (first centering value generator) generates the first centering value $Z_{aR0}$ that is a standard level of change over time in the first bioelectrical impedance $Z_{aR}$. The first centering value $Z_{aR0}$ is a standard level of change over time in the first bioelectrical impedance $Z_{aR}$ used for extracting information on respiration of the human subject, and is the average of the first bioelectrical impedances $Z_{aR}$ within a unit time. Next, at step S60, the CPU 170 (first centering value generator) generates the second centering value $Z_{aL0}$ that is a standard level of change over time in the second bioelectrical impedance $Z_{aL}$. The second centering value $Z_{aL0}$ is a standard level of change over time in the second bioelectrical impedance $Z_{aL}$ used for extracting information on respiration of the human subject, and is the average of the second bioelectrical impedances $Z_{aL}$ within a unit time.

Next, at step S70, the CPU 170 (first difference calculator) calculates the first difference $\Delta Z_{aR}$ that is the difference between the instantaneous measurement value of the first bioelectrical impedance $Z_{aR}$ and the first centering value $Z_{aR0}$. Next, at step S80, the CPU 170 (second difference calculator) calculates the second difference $\Delta Z_{aL}$ that is the difference between the instantaneous measurement value of the second bioelectrical impedance $Z_{aL}$ and the second centering value $Z_{aL0}$. Next, at step S90, the CPU 170 (respiration depth calculator) executes a first right-lung-respiration-depth calculating process for calculating a first right lung respiration depth BR1 related to the respiration capability of the upper lobe of the right lung. Next, at step S100, the CPU 170 (respiration depth calculator) executes a first left-lung-respiration-depth calculating process for calculating the first left lung respiration depth BL1 related to the respiration capability of the upper lobe of the left lung. In the following, those steps will be described in detail.

As shown in Fig. 12, at step S20, the CPU 170 determines the first bioelectrical impedance $Z_{aR}$ at the right upper body trunk. For example, the CPU 170 controls the electrode switching circuit 252 to select the left-hand current electrode X3 and the right-hand current electrode X4, and controls the electrode switching circuit 251 to select the right-foot voltage electrode Y2 and the right-hand voltage electrode Y4. Then, the CPU 170 determines the first bioelectrical impedance $Z_{aR}$ at the right upper extremity and the upper body trunk on the basis of the current data $D_i$ indicating the reference current $I_{ref}$ flowing between the right and left hands and the voltage data $D_v$ indicating the potential difference between the right-foot voltage electrode Y2 and the right-hand voltage electrode Y4. Hereinafter, the actual measured value of the first bioelectrical impedance at the n-th sampling time (n is a natural number that is equal to or greater than one) will be referred to as $Z_{aR}(n)'$.

Next, the CPU 170 determines the second bioelectrical impedance $Z_{aL}$ at the left upper body trunk (step S30). For example, the CPU 170 controls the electrode switching circuit 252 to select the left-hand current electrode X3 and the right-hand current electrode X4, and controls the electrode switching circuit 251 to select the left-foot voltage electrode Y1 and the left-hand voltage electrode Y3. Then, the CPU 170 determines the second bioelectrical impedance $Z_{aL}$ at the left upper body trunk and the left upper extremity on the basis of the current data $D_i$ indicating the reference current $I_{ref}$ flowing between the left and right hands and the voltage data $D_v$ indicating the potential difference between the lee-foot voltage electrode Y1 and the left-hand voltage electrode Y3. Hereinafter, the actual measured value of the second bioelectrical impedance at the n-th sampling time (n is a natural number that is equal to or greater than one) will be referred to as $Z_{aL}(n)'$.

Next, at step S40, the CPU 170 executes a smoothing process for each of the first bioelectrical impedance $Z_{aR}(n)'$ determined at step S20 and the second bioelectrical impedance $Z_{aL}(n)'$ determined at step S30. First, the smoothing process for the first bioelectrical impedance $Z_{aR}(n)'$ will be described in detail. The CPU 170 calculates the moving average of the actual measured value $Z_{aR}(n-2)'$ of the first bioelectrical impedance at the n-2th sampling time, the actual measured value $Z_{aR}(n-1)'$ of the first bioelectrical impedance at the n-1th sampling time, and the actual measured value $Z_{aR}(n)'$ of the first bioelectrical impedance at the n-th sampling time. Then, the CPU 170 decides the calculated moving average as the measurement value of the first bioelectrical impedance at the n-th sampling time (this is called the smoothing process). The measurement value of the first bioelectrical impedance resulting from the smoothing process will be referred to as $Z_{aR}(n)$.

Next, the smoothing process for the second bioelectrical impedance $Z_{aL}(n)'$ will be described in detail. The CPU 170 calculates the moving average of the actual measured value $Z_{aL}(n-2)'$ of the second bioelectrical impedance at the n-2th sampling time, the actual measured value $Z_{aL}(n-1)'$ of the second bioelectrical impedance at the n-1th sampling time, and the actual measured value $Z_{aL}(n)'$ of the second bioelectrical impedance at the n-th sampling time. Then, the CPU 170 decides the calculated moving average as the measurement value of the second bioelectrical impedance at the n-th sampling time (this is called the smoothing process). The measurement value of the second bioelectrical impedance resulting from the smoothing process will be referred to as $Z_{aL}(n)$.

Next, the CPU 170 (first centering value generator) executes a first centering process for generating a first centering value $Z_{aR0}$ that is a standard level of change over time in the first bioelectrical impedance $Z_{aR}$ (step S50). Hereinafter, the first centering value at the n-th sampling time will be referred to as $Z_{aR0}(n)$. In this embodiment, the CPU 170 generates or calculates the first centering value $Z_{aR0}(n)$ on the basis of the measurement values of the first bioelectrical impedance $Z_{aR}$ at multiple sampling times within a centering period starting from a time point that is a predetermined time length before the n-th sampling time and ending at the n-th sampling time. The time length of the centering period is variable and is set depending on the respiration speed of the human subject at the n-th sampling time. The first centering process will be described in detail.

Fig. 13 is a flow chart showing an example of the first centering process. As shown in Fig. 13, the CPU 170 first executes a MA10 calculating process for calculating the moving average (that is, MA10) of the measurement values of the first bioelectrical impedance $Z_{aR}$ at the last ten sampling times (step S51). More specifically, the CPU 170 calculates the moving average of the measurement values ($Z_{aR}(n-9)$ through $Z_{aR}(n)$) of the first bioelectrical impedance $Z_{aR}$ at the n-9th through n-th (ten) sampling times. The resulting moving average is referred to as the moving average MA10(n) at the n-th sampling time. The calculation is expressed as:

$$[Z_{aR}(n-9) + Z_{aR}(n-8) + ... + Z_{aR}(n)]/10 = MA10(n).$$

Next, the CPU 170 executes a MA20 calculating process for calculating the moving average (that is, MA20) of the measurement values of the first bioelectrical impedance $Z_{aR}$ at the last 20 sampling times (step S52). More specifically, the CPU 170 calculates the moving average of the measurement values ($Z_{aR}(n-19)$ through $Z_{aR}(n)$) of the first bioelectrical impedance $Z_{aR}$ at the n-19th through n-th (twenty) sampling times. The resulting moving average is referred to as the moving average MA20(n) at the n-th sampling time. The calculation is expressed as:

$$[Z_{aR}(n-19) + Z_{aR}(n-18) + ... + Z_{aR}(n)]/20 = MA20(n).$$

Next, the CPU 170 executes a MAX10 extraction process for extracting the maximum (referred to as MAX10) among the measurement values of the first bioelectrical impedance $Z_{aR}$ at the last ten sampling times (step S53). More specifically, the CPU 170 selects the maximum among the measurement values of the first bioelectrical impedance $Z_{aR}$ at the n-9th through n-th (ten) sampling times, and determines it to be the maximum MAX10(n) at the n-th sampling time.

Next, the CPU 170 executes a MIN10 extraction process for extracting the minimum (referred to as MIN10) among the measurement values of the first bioelectrical impedance $Z_{aR}$ at the last ten sampling times (step S54). More specifically, the CPU 170 selects the minimum among the measurement values of the first bioelectrical impedance $Z_{aR}$ at the n-9th through n-th (ten) sampling times, and determines it to be the minimum MIN10(n) at the n-th sampling time.

Next, at step S55, the CPU 170 executes a median value calculating process for calculating the moving average of mean values AV10 at the last 20 sampling times, in which AV10 is the mean value of the maximum MAX10 and the minimum MIN10 at a sampling time. For example, AV10(n) is the mean value of the maximum MAX10(n) and the minimum MIN10(n) at the n-th sampling time. More specifically, the CPU 170 calculates the moving average of the mean values AV10(n-19) through AV10(n) at the n-19th through n-th (twenty) sampling times, and decides the resulting moving average as a median value CNT20(n) at the n-th sampling time. The calculation is expressed as:

$$[AV10(n-19) + AV10(n-18) + ... + AV10(n)]/20 = CNT20(n).$$

Although not described in detail, the median value CNT20(n) is calculated in order to exclude artifacts (measurement errors that are unsuitable for processing) caused by body motion or other reasons.

Next, the CPU 170 executes a respiration timing extraction process for deciding respiration timing of the human subject at the n-th sampling time (step S56). In the following, with reference to Figs. 14 and 15, respiration timing extraction process will be described in detail. Figs. 14 and 15 form a flow chart showing an example of the respiration timing extraction process. As shown in Fig. 14, the CPU 170 first executes a differential coefficient calculating process for calculating the differential coefficient $dZ_{aR}(n)$ of the first bioelectrical impedance $Z_{aR}$ at the n-th sampling time (step S201). More specifically, the CPU 170 executes an arithmetic process in accordance with formula (3) below for calculating the differential coefficient $dZ_{aR}(n)$.

$$[Z_{aR}(n) - Z_{aR}(n-2)]/1.2 = dZ_{aR}(n) \quad ... \quad (3)$$

Next, the CPU 170 decides whether or not the absolute value of the differential coefficient $dZ_{aR}(n)$ calculated at step S201 is less than 0.1 (step S202). If the decision at step S202 is affirmative, the CPU 170 sets the tendency indication flag $F_0(n)$ to zero, and the process proceeds to step S204. The tendency indication flag $F_0(n)$ indicates tendency (increase, decrease or no trend) of the differential coefficient $dZ_{aR}(n)$. The fact that the tendency indication flag $F_0(n)$ is zero indicates that the measurement value $Z_{aR}(n)$ of the first bioelectrical impedance $Z_{aR}$ at the n-th sampling time has no trend, that is to say, it is at a local maximum (peak value) or at a local minimum (bottom value).

If the decision at step S202 is negative, the CPU 170 decides whether or not the differential coefficient $dZ_{aR}(n)$ is greater than zero (step S203). If the decision at step S203 is affirmative, the CPU 170 sets the tendency indication flag $F_0(n)$ to plus one, and the process proceeds to step S204. The fact that the tendency indication flag $F_0(n)$ is plus one indicates that the measurement value $Z_{aR}(n)$ of the first bioelectrical impedance $Z_{aR}$ at the n-th sampling time has a positive (increasing) trend. If the decision at step S203 is negative, the CPU 170 sets the tendency indication flag $F_0(n)$ to minus one, and the process proceeds to step S204. The fact that the tendency indication flag $F_0(n)$ is minus one indicates that the measurement value $Z_{aR}(n)$ of the first bioelectrical impedance $Z_{aR}$ at the n-th sampling time has a negative (decreasing) trend.

At step S204, the CPU 170 decides whether or not the absolute value of the tendency indication flag $F_0(n)$ at the n-th sampling time is equal to the absolute value of the tendency indication flag $F_0(n-1)$ at the n-1th sampling time.

In addition, the CPU 170 decides whether or not the value of $F_0(n-1)$ is unequal to $F_0(n)$. If the composite decision at step S204 is affirmative, the CPU 170 sets the tendency indication flag $F_0(n)$ to zero, and the process proceeds to step S206 (see Fig. 15). If the decision at step S204 is negative, the CPU 170 holds $F_0(n)$ set before step S204, and the process proceeds to step S206.

With reference to Fig. 15, description of the respiration timing extraction process will be continued. The CPU 170 decides whether or not the tendency indication flag $F_0(n)$ at the n-th sampling time is zero (step S206). If the decision at step S206 is negative, the CPU 170 sets a peak-or-bottom indication flag $F_1(n)$ to zero, and the process proceeds to step S209. The fact that the peak-or-bottom indication flag $F_1(n)$ is zero indicates that the measurement values $Z_a(n)$ of the first bioelectrical impedance at the n-th sampling time is not the peak value or the bottom value.

If the decision at step S206 is affirmative, the CPU 170 decides whether or not the sum of the tendency indication flags $F_0$ at the last three sampling times is greater than plus one (step S207). More specifically, the CPU 170 calculates the sum of the tendency indication flags $F_0(n-2)$ through $F_0(n)$ at the n-2th through n-th sampling times, and makes the decision.

If the decision at step S207 is affirmative, the CPU 170 sets the peak-or-bottom indication flag $F_1(n)$ to plus one, and the process proceeds to step S209. The fact that the peak-or-bottom indication flag $F_1(n)$ is plus one indicates that the measurement value $Z_{aR}(n)$ of the first bioelectrical impedance at the n-th sampling time is at a local maximum (peak value).

If the decision at step S207 is negative, the CPU 170 decides whether or not the sum of the tendency indication flags $F_0$ at the last three sampling times is less than minus one (step S208). In other words, the CPU 170 decides whether or not the sum of the tendency indication flags $F_0(n-2)$ through $F_0(n)$ at the n-2th through the n-th sampling times is less than minus one.

If the decision at step S208 is affirmative, the CPU 170 sets the peak-or-bottom indication flag $F_1(n)$ to minus one, and the process proceeds to step S209. The fact that the peak-or-bottom indication flag $F_1(n)$ is minus one indicates the measurement value $Z_{aR}(n)$ of the first bioelectrical impedance at the n-th sampling time is at a local minimum (bottom value). If the decision at step S208 is negative, the CPU 170 sets the peak-or-bottom indication flag $F_1(n)$ to zero, and the process proceeds to step S209.

At step S209, the CPU 170 decides whether or not the peak-or-bottom indication flag $F_1(n)$ is plus one. If the decision at step S209 is negative, the CPU 170 adds one to a sampling counter value $N(n-1)$ at the n-1th sampling time (step S210). If the decision at step S209 is affirmative, the CPU 170 initializes the sampling counter value N (step S211). As will be understood from Figs. 9 and 10, irrespective of whether respiration of the human subject is costal respiration or abdominal respiration, the waveform of change in the first bioelectrical impedance $Z_{aR}$ in respiration is nearly sinusoidal. The sampling counter value N is initialized to be zero whenever the measurement value of the first bioelectrical impedance $Z_{aR}$ reaches a peak value. The sampling counter value N is incremented at each new sampling time, so that the number of sampling times is counted by the sampling counter until the next peak value of the measurement value of the first bioelectrical impedance $Z_{aR}$. Thus, the respiration timing extraction process at step S56 in Fig. 13 is completed.

Returning to Fig. 13, description will be continued. After the above-described respiration timing extraction process is completed, the CPU 170 sets a respiration speed indication flag that indicates whether respiration of the human subject is fast or slow (step S57). In the following, with reference to Fig. 18, the respiration speed indication flag setting process executed by the CPU 170 at step S57 will be described in detail. Fig. 16 is a flow chart showing an example of the respiration speed indication flag setting process. As shown in Fig. 16, the CPU 170 first decides whether or not the tendency indication flag $F_0(n)$ is zero (step S301). If the decision at step S301 is negative, the CPU 170 considers that the respiration speed indication flag $F_{ma}(n)$ at the n-th sampling time as being equal to the respiration speed indication flag $F_{ma}(n-1)$ at the n-1th sampling time, and the process ends.

If the decision at step S301 is affirmative, the CPU 170 decides whether or not the peak-or-bottom indication flag $F_1(n)$ is plus one (step S302). If the decision at step S302 is affirmative, the CPU 170 decides whether or not the sampling counter value $N(n-1)$ at the n-1th sampling time is greater than ten (step S303). If the respiration speed of the human subject is slower, the time length between the time point at which the measurement value of the first bioelectrical impedance $Z_{aR}$ is at a peak value and the time point at which the measurement value of the first bioelectrical impedance $Z_{aR}$ arrives at the next peak value is longer, so that the sampling counter value N directly before the next peak value is larger. In this embodiment, when the CPU 170 decides that the measurement value of the first bioelectrical impedance $Z_{aR}$ at n-th sampling time has reached a peak value, the CPU 170 decides whether or not the sampling counter value at the n-1th sampling time is greater than ten. If the CPU 170 has decided that the sampling counter value is greater than ten, the CPU 170 determines that the respiration of the human subject is slow. Specifically, if the decision at step S303 is affirmative, the CPU 170 sets the respiration speed indication flag $F_{ma}(n)$ to 20, and the process ends. The fact that the respiration speed indication flag $F_{ma}(n)$ is 20 indicates that the respiration of the human subject is slow. If the decision at step S303 is negative, the CPU 170 sets the respiration speed indication flag $F_{ma}(n)$ to ten, and the process ends. The fact that the respiration speed indication flag $F_{ma}(n)$ is ten indicates that respiration of the human subject is fast.

If the decision at step S302 is negative, the CPU 170 decides whether or not the peak-or-bottom indication flag

$F_1(n)$ is minus one (step S304). If the decision at step S304 is negative, the CPU 170 determines that the respiration speed indication flag $F_{ma}(n)$ at the n-th sampling time is equal to respiration speed indication flag $F_{ma}(n-1)$ at the n-1th sampling time, and the process ends. If the decision at step S304 is affirmative, the CPU 170 decides whether or not the sampling counter value N(n-1) at the n-1th sampling time is greater than five (step S305). In this embodiment, when the CPU 170 decides that the sampling counter value N(n-1) directly before its arrival at the bottom value is greater than five, the CPU 170 determines that respiration of the human subject is slow. When the CPU 170 decides that the sampling counter value N(n-1) directly before its arrival at the bottom value is less than five, the CPU 170 determines that respiration of the human subject is fast. Specifically, if the decision at step S305 is affirmative, the CPU 170 sets the respiration speed indication flag $F_{ma}(n)$ to 20, and the process ends. If the decision at step S305 is negative, the CPU 170 sets the respiration speed indication flag $F_{ma}(n)$ to ten, and the process ends. The respiration speed indication flag setting process at step S57 in Fig. 13 is thus completed.

Returning to Fig. 13, description will be continued. Once the above-described respiration speed indication flag setting process is completed, the CPU 170 calculates the first centering value $Z_{aR0}(n)$ at the n-th sampling time (step S58). In the following, with reference to Fig. 17, the first centering value calculating process executed by the CPU 170 at step S58 will be described in detail. Fig. 17 is a flow chart showing an example of the first centering value calculating process. As shown in Fig. 17, the CPU 170 first decides whether or not the respiration speed indication flag $F_{ma}(n)$ is ten (step S401). In other words, the CPU 170 decides whether or not respiration of the human subject at the n- th sampling time is fast.

In this embodiment, on the basis of the measurement values of the first bioelectrical impedance $Z_{aR}$ at multiple sampling times within a centering period that is variable and is set depending on the respiration speed of the human subject, the first centering value $Z_{aR0}(n)$ at the n-th sampling time is calculated or generated. If the decision at step S401 is affirmative (i.e., the respiration speed of the human subject at the n-th sampling time is fast), a time length (e.g., about four seconds) taken for a faster single respiration (single respiratory action) is set as the centering period. Specifically, if respiration of the human subject is fast, a period starting from the n-9th sampling time and ending at the n-th sampling time is set as the centering period, and the first centering value $Z_{aR0}(n)$ is generated or calculated on the basis of the moving average MA10(n) of the measurement values of the first bioelectrical impedance at the n-9th through n-th sampling times. In other words, if the decision at step S401 is affirmative, the CPU 170 generates or calculates the first centering value $Z_{aR0}(n)$ at the n-th sampling time on the basis of the moving average MA10(n) calculated at step S51 in Fig. 13 (step S402). More specifically, the CPU 170 calculates the moving average of the first centering value $Z_{aR0}$ (n-2) at the n-2th sampling time, the first centering value $Z_{aR0}(n-1)$ at the n-1th sampling time, and the moving average MA10(n), and decides the resulting moving average as the first centering value $Z_{aR0}(n)$ at the n-th sampling time. The calculation is expressed as:

$$[Z_{aR0}(n-2) + Z_{aR0}(n-1) + MA10(n)]/3 = Z_{aR0}(n).$$

If the decision at step S401 is negative (i.e., the respiration speed of the human subject at the n-th sampling time is slow), a time length (e.g., about eight seconds) taken for a slower single respiration (single respiratory action) is set as the centering period. Specifically, if respiration of the human subject is slow, a period starting from the n-19th sampling time and ending at the n-th sampling time is set as the centering period, and the first centering value $Z_{aR0}(n)$ is generated or calculated on the basis of the moving average MA20(n) of the measurement values of the first bioelectrical impedance at the n-19th through n-th sampling times. In other words, if the decision at step S401 is negative, the CPU 170 generates or calculates the first centering value $Z_{aR0}(n)$ at the n-th sampling time on the basis of the moving average MA20(n) calculated at step S52 in Fig. 13 (step S403). More specifically, the CPU 170 calculates the moving average of the first centering value $Z_{aR0}(n-2)$ at the n-2th sampling time, the first centering value $Z_{aR0}(n-1)$ at the n-1th sampling time, and the moving average MA20(n), and decides the resulting moving average as the first centering value $Z_{aR0}(n)$ at the n-th sampling time. The calculation is expressed

$$[Z_{aR0}(n-2) + Z_{aR0}(n-1) + MA20(n)]/3 = Z_{aR0}(n).$$

The first centering process at step S50 in Fig. 12 is thus completed.

As described above, irrespective of whether respiration of the human subject is costal respiration or abdominal respiration, the waveform of change in the bioelectrical impedance $Z_a$ at the upper body trunk in respiration is nearly sinusoidal, and thus the waveform of change in the first bioelectrical impedance $Z_{aR}$ at the right upper body trunk in respiration is also nearly sinusoidal. The CPU 170 (first centering value generator) generates or calculates the first centering value $Z_{aR0}$ on the basis of measurement values of the first bioelectrical impedance $Z_{aR}$ at the sampling times of which the number is predetermined in order to obtain a suitable first centering value $Z_{aR0}$ even if one or more instantaneous values of the first bioelectrical impedance $Z_{aR}$ are disturbed (even if artifacts occur) by body motion or for other reasons. More specifically, at each sampling time, the CPU 170 generates or calculates a moving average MA10(n) or MA20(n) of measurement values of the first bioelectrical impedance at multiple sampling times within a centering period starting from a time point that is a predetermined time length before the n-th sampling time and ending at the n-th sampling time, and calculates the first centering value $Z_{aR0}$ at the sampling time. Accordingly, even if one or more instantaneous values of the first bioelectrical impedance $Z_{aR}$ are disturbed, a suitable first centering value $Z_{aR0}$ can be generated. In addition, the time length of a centering period is variable and is set depending on the respiration speed of the human subject at the sampling time.

As shown in Fig. 12, after step S50, the CPU 170 (first centering value generator) executes a second centering process for generating a second centering value $Z_{aL0}$ that is a standard level of change over time in the second bioelectrical impedance $Z_{aL}$ (step S60). Hereinafter, the second centering value at the n-th sampling time will be referred to as $Z_{aL0}$(n). In a manner similar to calculation of the aforementioned first centering value $Z_{aR0}$(n), the CPU 170 generates or calculates the second centering value $Z_{aL0}$(n) at the n-th sampling time on the basis of measurement values of the second bioelectrical impedance $Z_{aL}$ at multiple sampling times within a centering period starting from a time point that is a predetermined time length before the n-th sampling time and ending at the n-th sampling time. Details of generating the second centering value $Z_{aL0}$(n) is similar to those of generating the first centering value $Z_{aR0}$(n), and will not be described in more detail.

As shown in Fig. 12, after the second centering process of step S60 is completed, the CPU 170 executes a first difference calculating process for calculating a first difference $\Delta Z_{aR}$(n) that is the difference between the instantaneous measurement value $Z_{aR}$(n) of the first bioelectrical impedance and the first centering value $Z_{aR0}$(n) (step S70). More specifically, the CPU 170 calculates the difference between the measurement value $Z_{aR}$(n) of the first bioelectrical impedance calculated at step S40 and the first centering value $Z_{aR0}$(n) calculated at step S50, and decides the difference as the first difference $\Delta Z_{aR}$(n) at the n-th sampling time.

Next, the CPU 170 executes a second difference calculating process for calculating a second difference $\Delta Z_{aL}$(n) that is the difference between the instantaneous measurement value $Z_{aL}$(n) of the second bioelectrical impedance and the second centering value $Z_{aL0}$(n) (step S80). More specifically, the CPU 170 calculates the difference between the measurement value $Z_{aL}$(n) of the second bioelectrical impedance calculated at step S40 and the second centering value $Z_{aL0}$(n) calculated at step S60, and decides the difference as the second difference $\Delta Z_{aL}$(n) at the n-th sampling time.

For example, if the human subject is a physically unimpaired person, there is no difference of functions (difference of respiration capability) between the right lung and the left lung. As a result, as shown in Fig. 18, the waveform of change over time in the first difference $\Delta Z_{aR}$ in respiration of the human subject is similar to that of change over time in the second difference $\Delta Z_{aL}$. In Fig. 18, the waveform of change over time in the first difference $\Delta Z_{aR}$ is described in such a manner that the first centering value $Z_{aR0}$ that is the standard level of measurement values of the first bioelectrical impedance $Z_{aR}$ is placed at zero on the Y axis, whereas the waveform of change over time in the second difference $\Delta Z_{aL}$ is described in such a manner that the second centering value $Z_{aL0}$ that is the standard level of measurement values of the second bioelectrical impedance $Z_{aL}$ is placed at zero on the Y axis. As shown in Fig. 18, at inhalations, the first difference $\Delta Z_{aR}$ and the second difference $\Delta Z_{aL}$ become positive. At exhalations, the first difference $\Delta Z_{aR}$ and the second difference $\Delta Z_{aL}$ become negative. The greater the ventilation volume of the right lung, the greater the amplitude of the first difference $\Delta Z_{aR}$. More exactly, the greater the volume of air inhaled into the right lung, the greater the absolute value of the peak value (positive maximum) of the first difference $\Delta Z_{aR}$, whereas the greater the volume of air exhaled from the right lung, the greater the absolute value of the bottom value (negative maximum) of the first difference $\Delta Z_{aR}$. Similarly, the greater the ventilation volume of the left lung, the greater the amplitude of the second difference $\Delta Z_{aL}$. More exactly, the greater the volume of air inhaled into the left lung, the greater the absolute value of the peak value of the second difference $\Delta Z_{aL}$, whereas the greater the volume of air exhaled from the left lung, the greater the absolute value of the bottom value of the second difference $\Delta Z_{aL}$.

As shown in Fig. 12, after step S80, the CPU 170 (respiration depth calculator) executes first right-lung-respiration-depth calculating process (step S90) for calculating a first right lung respiration depth BR1 related to the respiration capability of the upper lobe of the right lung.

In the following, with reference to Fig. 19, the first right-lung-respiration-depth calculating process executed at step S90 by the CPU 170 will be described in detail. Fig. 19 is a flow chart showing an example of the first right-lung-respiration-depth calculating process. As shown in Fig. 19, the CPU 170 first decides whether or not the respiratory action of the human subject is inhalation (step S501). Specifically, the CPU 170 decides that the respiratory action of the human subject is inhalation if the first difference $\Delta Z_{aR}$(n) at the n-th sampling time is positive, and that the respiratory action of the human subject is exhalation if the first difference $\Delta Z_{aR}$(n) at the n-th sampling time is negative. If the decision at step S501 is inhalation, the CPU 170 sets an inhalation-or-exhalation flag $F_3$ to plus one. If the decision at step S501 is exhalation, the CPU 170 sets the inhalation-or-exhalation flag $F_3$ to zero. In the initial status, the inhalation-or-exhalation

flag $F_3$ is plus one. At step S501, the CPU 170 may decide whether or not the respiratory action is inhalation on the basis of the second difference $\Delta Z_{aL}(n)$ instead of the first difference $\Delta Z_{aR}(n)$.

If the decision at step S501 is affirmative (inhalation), the CPU 170 executes a peak-hold process (step S502). More specifically, the CPU 170 holds the maximum among the first differences $\Delta Z_{aR}$ at multiple sampling times as a peak value $\Delta Z_{aR}(MAX)$. If the decision at step S501 is negative (exhalation), the CPU 170 executes a bottom-hold process (step S503). More specifically, the CPU 170 holds the minimum among the first differences $\Delta Z_{aR}$ at multiple sampling times as a bottom value $\Delta Z_{aR}(MIN)$.

Next, at step S504, the CPU 170 decides whether or not the n-th sampling time is a first zero-cross time at which the instantaneous measurement value $Z_{aR}(n)$ of the first bioelectrical impedance $Z_{aR}$ is equal to the first centering value $Z_{aR0}(n)$. More specifically, the CPU 170 executes a first zero-cross time determination process, and decides whether or not the sampling time is a first zero-cross time on the basis of the result of the process. The first zero-cross time determination process will be described in detail.

Fig. 20 is a flow chart showing an example of the first zero-cross time determination process executed by the CPU 170 at step S504. As shown in Fig. 20, the CPU 170 first extracts the minimum among the first difference $\Delta Z_{aR}$ at the last five sampling times (step S601). More exactly, the CPU 170 selects the minimum among the absolute value $|\Delta Z_{aR}|$ $|\Delta Z_{aR}(n-4)|$ through $|\Delta Z_{aR}(n)|$) of the first difference $\Delta Z_{aR}$ at the n-4th through the n-th sampling times, and decides the minimum as a first zero-cross-time reference value $\Delta MIN5(n)$ at the n-th sampling time.

Next, at step S602, the CPU 170 (zero-cross time decider) decides whether or not the first zero-cross-time reference value at the n-1th sampling time is equal to the current first zero-cross-time reference value decided at step S601. In addition, the CPU 170 decides whether or not the n-1th sampling time is a first zero-cross time. More specifically, the CPU 170 decides whether or not the first zero-cross-time reference value $\Delta MIN5(n-1)$ at the n-1th sampling time is equal to the current $\Delta MIN5(n)$. In addition, the CPU 170 decides whether or not a first zero-cross-time reference flag $F_2$ (n-1) at the n-1th sampling time is set to plus one. When the first zero-cross-time reference flag $F_2(n-1)$ is set to plus one, the n-1th sampling time is a first zero-cross time. The initial value (default value) of the first zero-cross-time reference flag $F_2$, i.e., the first zero-cross-time reference flag $F_2(1)$ at the first sampling time, is zero.

If the decision at step S602 is affirmative, the CPU 170 sets the first zero-cross-time reference flag $F_2(n)$ to zero, which means that the n-th sampling time is not a first zero-cross time, and the process ends. If the decision at step S602 is negative, the CPU 170 decides whether or not the absolute value of the first difference $\Delta Z_{aR}(n)$ at the n-th sampling time is equal to or less than 0.3 (step S603). If the decision at step S603 is negative, the CPU 170 sets the first zero-cross-time reference flag $F_2(n)$ to zero, which means that the n-th sampling time is not a first zero-cross time, and the process ends. If the decision at step S603 is affirmative, the CPU 170 sets the first zero-cross-time reference flag $F_2(n)$ to plus one, which means that the n-th sampling time is a first zero-cross time, and the process ends. The first zero-cross time determination process is thus completed.

Returning to Fig. 19, description will be continued. If at step S504 the CPU 170 decides that the n-th sampling time is not a first zero-cross time (i.e., the first zero-cross-time reference flag $F_2(n)$ is zero), the first right-lung-respiration-depth calculating process at the sampling time ends. If the CPU 170 decides that the n-th sampling time is a first zero-cross time (i.e., the first zero-cross-time reference flag $F_2(n)$ is plus one), the process proceeds to step S505.

At step S505, the CPU 170 decides whether or not the first differential coefficient $dZ_{aR}(n)$ of the first bioelectrical impedance $Z_{aR}$ at the n-th sampling time is positive (greater than zero). In other words, the CPU 170 decides whether or not the respiratory action is changing from exhalation to inhalation at the n-th sampling time.

If the decision at step S505 is affirmative, on the assumption that the respiratory action is changing from exhalation to inhalation at the n-th sampling time, the CPU 170 calculates the sum of the absolute values of the peak value $\Delta Z_{aR}(MAX)$ and the bottom value $\Delta Z_{aR}(MIN)$ held at that time, and decides the resulting sum as the first right lung respiration depth BR1 at the last respiratory action (step S506). The first right lung respiration depth BR1 calculated at step S506 relates to the respiration capability of the upper lobe of the right lung at the last respiratory action. The higher the respiration capability, the greater the first right lung respiration depth BR1. Then, the CPU 170 executes an inhalation flag setting process (step S507). More specifically, the CPU 170 sets the inhalation-or-exhalation flag $F_3$ to plus one. Then, the CPU 170 initializes the peak-hold process (step S508). More specifically, the CPU 170 sets the peak value $\Delta Z_a(MAX)$ set at step S502 to the initial value zero, and ends the first right-lung-respiration-depth calculating process at the sampling time.

If the decision at step S505 is negative, on the assumption that the respiratory action is changing from inhalation to exhalation at the n-th sampling time, the CPU 170 executes an exhalation flag setting process (step S509). More specifically, the CPU 170 sets the inhalation-or-exhalation flag $F_3$ to zero. Then, the CPU 170 initializes the bottom-hold process (step S510). More specifically, the CPU 170 sets the bottom value $\Delta Z_{aR}(MIN)$ set at step S503 to the initial value zero, and ends the first right-lung-respiration-depth calculating process at the sampling time. The first right-lung-respiration-depth calculating process at step S90 in Fig. 12 is thus completed.

As shown in Fig. 12, after step S90, the CPU 170 (respiration depth calculator) executes a first left-lung-respiration-depth calculating process for calculating the first left lung respiration depth BL1 related to the respiration capability of the upper lobe of the left lung (step S100). The first left-lung-respiration-depth calculating process is similar to the above-described first right-lung-respiration-depth calculating process, and will not be described in detail. In summary, if the n-th sampling time is a second zero-cross time at which the instantaneous measurement value $Z_{aL}(n)$ of the second bioelectrical impedance $Z_{aL}$ is equal to the second centering value $Z_{aL0}(n)$, and if respiratory action is changing from inhalation to exhalation at the n-th sampling time, the first left lung respiration depth BL1 is calculated as the sum of the absolute values of the peak value $\Delta Z_{aL}(MAX)$ and the bottom value $\Delta Z_{aL}(MIN)$ held at that time. The resulting first left lung respiration depth BL1 relates to the respiration capability of the upper lobe of the right lung at the last respiratory action. The higher the respiration capability, the greater the first left lung respiration depth BL1.

## 1.5. RESPIRATION DEPTH DISPLAYING PROCESS

Next, a respiration depth displaying process executed by the CPU 170 will be described. In this embodiment, the CPU 170 executes the respiration depth displaying process for causing the display device 160 to show the first right lung respiration depth BR1 and the first left lung respiration depth BL1 at every respiration of the human subject. As shown in Fig. 21, the CPU 170 controls the display device 160 to display the first right lung respiration depth BR1 in a first bar graph BG1 and the first left lung respiration depth BL1 in a second bar graph BG2 at every respiration of the human subject. More specifically, on the basis of the first right lung respiration depth BR1 at the last single respiration determined by the respiration depth analysis process, the CPU 170 decides the number of degree indicators to be colored in the first bar graph BG1. On the basis of the first left lung respiration depth BL1 at the last single respiration determined by the respiration depth analysis process, the CPU 170 decides the number of degree indicators to be colored in the second bar graph BG2. Then, on the basis of the peak value $\Delta Z_{aR}(MAX)$ and the bottom value $\Delta Z_{aR}(MIN)$ that constitutes the first right lung respiration depth BR1, the CPU 170 distributes the number of degree indicators to be colored in the first bar graph BG1 to inhalation and exhalation. In addition, on the basis of the peak value $\Delta Z_{aL}(MAX)$ and the bottom value $\Delta Z_{aL}(MIN)$ that constitute the first left lung respiration depth BL1, the CPU 170 distributes the number of degree indicators to be colored in the second bar graph BG2 to inhalation and exhalation.

The higher the peak value $\Delta Z_{aR}(MAX)$, the greater the number of degree indicators to be colored at the inhalation side of the first bar graph BG1. The higher the absolute value of the bottom value $\Delta Z_{aR}(MIN)$, the greater the number of degree indicators to be colored at the exhalation side of the first bar graph BG1. The higher the peak value $\Delta Z_{aL}(MAX)$, the greater the number of degree indicators to be colored at the inhalation side of the second bar graph BG2. The higher the absolute value of the bottom value $\Delta Z_{aL}(MIN)$, the greater the number of degree indicators to be colored at the exhalation side of the second bar graph BG2.

As has been described above, in this embodiment, the CPU 170 controls the display device 160 to show the first right lung respiration depth BR1 and the first left lung respiration depth BL1 at every respiration of the human subject. The human subject or other person can easily understand the difference between the respiration capabilities of the right lung and the left lung of the human subject. If the human subject is a physically unimpaired person, there is no significant difference in functions between the right lung and the left lung, and thus there is no significant difference between the number of degree indicators colored in the first bar graph BG1 and that in the second bar graph BG2. If the human subject has a disorder (e.g., a disease) in the right lung or the left lung, there is a significant difference in functions between the right lung and the left lung. The difference results in a significant difference between the first right lung respiration depth BR1 and the first left lung respiration depth BL1, and thus there is a significant difference between the number of degree indicators colored in the first bar graph BG1 and that in the second bar graph BG2. By observing the first bar graph BG1 and the second bar graph BG2, the human subject or another person can easily understand the difference between the respiration capabilities of the right lung and the left lung of the human subject, and can easily identify the area of pathology. The indication in the first bar graph BG1 and the second bar graph BG2 may be used for biofeedback information for reporting functional recovery of lungs after surgery.

## 2. SECOND EMBODIMENT

Next, the second embodiment according to the present invention will be described. The structure of the body condition determination apparatus in the second embodiment is the same as that in the first embodiment. The operation in the second embodiment is similar to that in the first embodiment, so that only differences will be described in detail. In this embodiment, the CPU 170 controls the display device 160 to show not only the first right lung respiration depth BR1 and the first left lung respiration depth BL1, but also a second right lung respiration depth BR2 related to the respiration capability of the median and lower lobes of the right lung and a second left lung respiration depth BL2 related to the respiration capability of the median and lower lobes of the left lung.

The aforementioned bioelectrical impedance $Z_b$ at the middle body trunk is influenced by the bioelectrical impedance at the right middle body trunk including the median and lower lobes of the right lung and the abdomen and the bioelectrical impedance at the left middle body trunk including the median and lower lobes of the left lung. Hereinafter, the bioelectrical impedance at the right middle body trunk will be referred to as the third bioelectrical impedance $Z_{bR}$, whereas the bioelectrical impedance at the left middle body trunk will be referred to as the fourth bioelectrical impedance $Z_{bL}$. The third bioelectrical impedance $Z_{bR}$ may be calculated from the current data $D_i$ indicating the reference current $I_{ref}$ flowing between the right hand and the right foot and the voltage data $D_v$ indicating the potential difference between the left hand and the left foot. The fourth bioelectrical impedance $Z_{bL}$ may be calculated from the current data $D_i$ indicating the reference current $I_{ref}$ flowing between the left hand and the left foot and the voltage data $D_v$ indicating the potential difference between the right hand and the right foot.

The third bioelectrical impedance $Z_{bR}$ changes in respiration by air entering and leaving the right lung. The manner of change is similar to that of the above-described bioelectrical impedance $Z_b$ at the middle body trunk. When the volume of air entering and leaving the right lung (the ventilation volume at the right lung) is greater, the amplitude of the third bioelectrical impedance $Z_{bR}$ is greater. The amplitude of the third bioelectrical impedance $Z_{bR}$ corresponds to the respiration capability of the median and lower lobes of the right lung. Accordingly, on the basis of measurement values of the third bioelectrical impedance $Z_{bR}$, a second right lung respiration depth BR2 related to the respiration capability of the median and lower lobes of the right lung can be calculated, as will be described later in detail.

The fourth bioelectrical impedance $Z_{bL}$ changes in respiration by air entering and leaving the left lung. The manner of change is similar to that of the above-described bioelectrical impedance $Z_b$ at the middle body trunk. When the volume of air entering and leaving the left lung (the ventilation volume at the left lung) is greater, the amplitude of the fourth bioelectrical impedance $Z_{bL}$ is greater. The amplitude of the fourth bioelectrical impedance $Z_{bL}$ corresponds to the respiration capability of the median and lower lobes of the left lung. Accordingly, on the basis of measurement values of the fourth bioelectrical impedance $Z_{bL}$, a second left lung respiration depth BL2 related to the respiration capability of the median and lower lobes of the left lung can be calculated, as will be described later in detail.

Next, the respiration depth analysis process in this embodiment will be described in detail. Figs. 22 and 23 form a flow chart showing an example of the respiration depth analysis process. As shown in Fig. 22, the CPU 170 first decides whether or not the current time reaches a sampling time (step S700), and if the decision at step S700 is affirmative, the process proceeds to step S710. Description of subsequent steps will be continued on the assumption that the current time reaches the n-th sampling time. In the following, steps that have been already described in conjunction the first embodiment will not be described in detail.

As shown in Fig. 22, at step S710, the CPU 170 determines the first bioelectrical impedance $Z_{aR}$ at the right upper body trunk. The manner of this determination is the same as in the first embodiment, and will not be described in detail. Next, the CPU 170 (bioelectrical impedance determiner) determines the third bioelectrical impedance $Z_{bR}$ at the right middle body trunk (step S720). For example, the CPU 170 controls the electrode switching circuit 252 to select the right-foot current electrode X2 and the right-hand current electrode X4, and controls the electrode switching circuit 251 to select the left-foot voltage electrode Y1 and the left-hand voltage electrode Y3. Then, the CPU 170 determines the third bioelectrical impedance $Z_{bR}$ at the right middle body trunk on the basis of the current data $D_i$ indicating the reference current $I_{ref}$ flowing between the right hand and the right foot and the voltage data $D_v$ indicating the potential difference between the left-foot voltage electrode Y1 and the left-hand voltage electrode Y3. Hereinafter, the actual measured value of the third bioelectrical impedance at the n-th sampling time (n is a natural number that is equal to or greater than one) will be referred to as $Z_{bR}(n)'$.

Next, at step S730, the CPU 170 executes a smoothing process for each of the first bioelectrical impedance $Z_{aR}(n)'$ determined at step S710 and the third bioelectrical impedance $Z_{bR}(n)'$ determined at step S720. The smoothing process is similar to that in the first embodiment, and will not be described in detail. The measurement value of the third bioelectrical impedance resulting from the smoothing process at the n-th sampling time will be referred to as $Z_{bR}(n)$.

Next, the CPU 170 determines the second bioelectrical impedance $Z_{aL}$ at the left upper body trunk (step S740). The manner of this determination is the same as in the first embodiment, and will not be described in detail. Next, the CPU 170 (bioelectrical impedance determiner) determines the fourth bioelectrical impedance $Z_{bL}$ at the left middle body trunk (step S750). For example, the CPU 170 controls the electrode switching circuit 252 to select the left-foot current electrode X1 and the left-hand current electrode X3, and controls the electrode switching circuit 251 to select the right-foot voltage electrode Y2 and the right-hand voltage electrode Y4. Then, the CPU 170 determines the fourth bioelectrical impedance $Z_{bL}$ at the left middle body trunk on the basis of the current data $D_i$ indicating the reference current $I_{ref}$ flowing between the left hand and the left foot and the voltage data $D_v$ indicating the potential difference between the right-foot voltage electrode Y2 and the right-hand voltage electrode Y4. Hereinafter, the actual measured value of the fourth bioelectrical impedance at the n-th sampling time (n is a natural number that is equal to or greater than one) will be referred to as $Z_{bL}(n)'$.

Next, at step S760, the CPU 170 executes a smoothing process for each of the second bioelectrical impedance $Z_{aL}(n)'$ determined at step S740 and the fourth bioelectrical impedance $Z_{bL}(n)'$ determined at step S750. The smoothing process is similar to that in the first embodiment, and will not be described in detail. The measurement value of the fourth bioelectrical impedance resulting from the smoothing process at the n-th sampling time will be referred to as $Z_{bL}(n)$.

As shown in Fig. 22, at step S770, the CPU 170 executes the first centering process. Next, at step S780, the CPU 170 executes the first difference calculating process. Next, at step S790, the CPU 170 executes the first right-lung-respiration-depth calculating process. The processes of steps S770 through S790 are the same as those in the above-described first embodiment (steps S50, S70, and S90 in Fig. 12), and will not be described in detail.

After step S790, the CPU 170 (second centering value generator) generates or calculates a third centering value $Z_{bR0}$ that is a standard level of change over time in the third bioelectrical impedance $Z_{bR}$. Furthermore, the CPU 170 (third difference calculator) calculates a third difference $\Delta Z_{bR}$ that is the difference between the instantaneous measurement value of the third bioelectrical impedance $Z_{bR}$ and the third centering value $Z_{bR0}$ (step S800). The third centering value $Z_{bR0}$ is a standard level of change over time in the third bioelectrical impedance $Z_{bR}$ used for extracting information on respiration of the human subject, and is the average of the third bioelectrical impedances $Z_{bR}$ within a unit time.

As described above, when respiration of the human subject is abdominal respiration, change in the bioelectrical impedance $Z_b$ at the middle body trunk is not sinusoidal and is completely different from that of the bioelectrical impedance $Z_a$ at the upper body trunk. Accordingly, in contrast to the calculation of the first centering value $Z_{aR0}$, if a moving average is calculated on the basis of measurement values of the third bioelectrical impedance $Z_{bR}$ at the sampling times of which the number is predetermined, the standard level (third centering value $Z_{bR}0$) of change over time in the third bioelectrical impedance $Z_{bR}$ cannot be calculated accurately.

Accordingly, in this embodiment, as shown in Fig. 24, first zero-cross times are decided in which the instantaneous measurement value of the first bioelectrical impedance $Z_{aR}$ is equal to the first centering value $Z_{aR0}$. In addition, the third centering value $Z_{bR0}$ is generated or calculated on the basis of measurement values of the third bioelectrical impedance $Z_{bR}$ at the first zero-cross times, so that the standard level (third centering value $Z_{bR0}$) of change over time in the third bioelectrical impedance $Z_b$ can be calculated accurately even if respiration of the human subject is abdominal respiration. Fig. 24 is a graph for explaining a scheme for generating the third centering value. In the following, with reference to Fig. 25, the third difference calculating process executed at step S800 by the CPU 170 will be described in detail.

Fig. 25 is a flow chart showing an example of the third difference calculating process. As shown in Fig. 25, the CPU 170 (the zero-cross time decider) first decides whether or not the aforementioned first zero-cross-time reference flag $F_2(n)$ is plus one (step S801). If the decision at step S801 is affirmative (i.e., the n-th sampling time is a first zero-cross time), the CPU 170 (second centering value generator) calculates the third centering value $Z_{bR0}(n)$ at the n-th sampling time (step S802). More specifically, the CPU 170 calculates the average of the third centering value $Z_{bR0}(n-2)$ at the n-2th sampling time, the third centering value $Z_{bR0}(n-1)$ at the n-1th sampling time, and the measurement value $Z_{bR}(n)$ of the third bioelectrical impedance at the n-th sampling time, and decides the resulting average as the third centering value $Z_{bR0}(n)$ at the n-th sampling time. The calculation is expressed as:

$$[Z_{bR0}(n\text{-}2) + Z_{bR0}(n\text{-}1) + Z_{bR}(n)]/3 = Z_{bR0}(n).$$

If the decision at step S801 is negative (i.e., the n-th sampling time is not a first zero-cross time), the CPU 170 decides the third centering value $Z_{bR0}(n-1)$ at the n-1th sampling time as the third centering value $Z_{bR0}(n)$ at the n-th sampling time. This is expressed as:

$$Z_{bR0}(n\text{-}1) = Z_{bR0}(n).$$

Next, the CPU 170 calculates the third difference $\Delta Z_{bR}(n)$ at the n-th sampling time that is the difference between the instantaneous measurement value $Z_{bR}(n)$ of the third bioelectrical impedance and the third centering value $Z_{bR0}(n)$ (step S804). The third difference calculating process at step S800 in Fig. 22 is thus completed.

Returning to Fig. 22, description will be continued. As shown in Fig. 22, after step S800, the CPU 170 (respiration depth calculator) executes a second right-lung-respiration-depth calculating process for calculating a second right lung respiration depth BR2 related to the respiration capability of the median and lower lobes of the right lung (step S810). In the following, with reference to Fig. 26, the second right-lung-respiration-depth calculating process executed at step S810 by the CPU 170 will be described in detail. Fig. 26 is a flow chart showing an example of the second right-lung-respiration-depth calculating process. As shown in Fig. 26, the CPU 170 first decides whether or not the respiratory action of the human subject is inhalation (step S900). Step S900 is similar to step S501 in the aforementioned first right-lung-respiration-depth calculating process shown in Fig. 19, and will not be described in detail.

If the decision at step S900 is affirmative (inhalation), the CPU 170 executes a peak-hold process (step S901). More specifically, the CPU 170 holds the maximum among the third differences $\Delta Z_{bR}$ at multiple sampling times as a

peak value $\Delta Z_{bR}(MAX)$. If the decision at step S900 is negative (exhalation), the CPU 170 executes a bottom-hold process (step S902). More specifically, the CPU 170 holds the minimum among the third differences $\Delta Z_{bR}$ at multiple sampling times as a bottom value $\Delta Z_{bR}(MIN)$.

Next, the CPU 170 decides whether or not the aforementioned first zero-cross-time reference flag $F_2(n)$ is plus one (step S903). If the decision at step S903 is negative (i.e., the n-th sampling time is not a first zero-cross time), the second right-lung-respiration-depth calculating process at the sampling time ends. If the decision at step S903 is affirmative (i.e., the n-th sampling time is a first zero-cross time), and the process proceeds to step S904. At step S904, the CPU 170 decides whether or not the differential coefficient $dZ_{aR}$ of the first bioelectrical impedance $Z_{aR}$ at the n-th sampling time is positive (greater than zero) as similar to step S505 in Fig. 10. In other words, the CPU 170 decides whether or not the respiratory action is changing from exhalation to inhalation at the n-th sampling time.

If the decision at step S904 is affirmative, on the assumption that the respiratory action is changing from exhalation to inhalation at the n-th sampling time, the CPU 170 calculates the sum of the absolute values of the held local maximum (peak value) $\Delta Z_{bR}(MAX)$ and the held local minimum (bottom value) $\Delta Z_{bR}(MIN)$, and decides the resulting sum as the second right lung respiration depth BR2 at the last respiratory action (step S905). The second right lung respiration depth BR2 calculated at step S905 relates to the respiration capability of the median and lower lobes of the right lung at the last respiratory action. The higher the respiration capability, the greater the second right lung respiration depth BR2. Then, the CPU 170 executes an inhalation flag setting process (step S906). More specifically, the CPU 170 sets the inhalation-or-exhalation flag $F_3$ to plus one. Then, the CPU 170 initializes the peak-hold process (step S907). More specifically, the CPU 170 sets the peak value $\Delta Z_{bR}(MAX)$ to the initial value zero, and ends the second right-lung-respiration-depth calculating process at the sampling time.

If the decision at step S904 is negative, on the assumption that the respiratory action is changing from inhalation to exhalation at the n-th sampling time, the CPU 170 executes an exhalation flag setting process (step S908). More specifically, the CPU 170 sets the inhalation-or-exhalation flag $F_3$ to zero. Then, the CPU 170 initializes the bottom-hold process (step S909). More specifically, the CPU 170 sets the bottom value $\Delta Z_{bR}(MIN)$ to the initial value zero, and ends the second right-lung-respiration-depth calculating process at the sampling time. The second right-lung-respiration-depth calculating process at step S810 in Fig. 22 ends and the process proceeds to step S820 (see Fig. 23).

With reference to Fig. 23, description of the respiration depth analysis process will be continued. As shown in Fig. 23, at step S820, the CPU 170 executes the second centering process. Next, at step S830, the CPU 170 executes the second difference calculating process. Next, at step S840, the CPU 170 executes the first left-lung-respiration-depth calculating process. The processes of steps S820 through S840 are the same as those in the above-described first embodiment (steps S60, S80, and S100 in Fig. 12), and will not be described in detail.

After step S840, the CPU 170 (second centering value generator) generates fourth centering value $Z_{bL0}$ that is a standard level of change over time in the fourth bioelectrical impedance $Z_{bL}$. Furthermore, the CPU 170 (fourth difference calculator) calculates a fourth difference $\Delta Z_{bL}$ that is the difference between the instantaneous measurement value of the fourth bioelectrical impedance $Z_{bL}$ and the fourth centering value $Z_{bL0}$ (step S850). The fourth centering value $Z_{bL0}$ is a standard level of change over time in the fourth bioelectrical impedance $Z_{bL}$ used for extracting information on respiration of the human subject, and is the average of the fourth bioelectrical impedances $Z_{bL}$ within a unit time.

In this embodiment, second zero-cross times are decided in which the instantaneous measurement value of the second bioelectrical impedance $Z_{aL}$ is equal to the second centering value $Z_{aL0}$. In addition, the fourth centering value $Z_{bL0}$ is generated or calculated on the basis of measurement values of the fourth bioelectrical impedance $Z_{bL}$ at the second zero-cross times. Details of generating the fourth centering value $Z_{bL0}(n)$ is similar to those of generating the aforementioned third centering value $Z_{bR0}(n)$, and will not be described in more detail. Then, the CPU 170 decides the difference between the measurement values $Z_{bL}(n)$ of the fourth bioelectrical impedances and the fourth centering value $Z_{bL0}(n)$ as the fourth difference $\Delta Z_{bL}(n)$ at the n-th sampling time.

As shown in Fig. 23, after step S850, the CPU 170 (respiration depth calculator) executes a second left-lung-respiration-depth calculating process for calculating a second left lung respiration depth BL2 related to the respiration capability of the median and lower lobes of the left lung (step S860). The second left-lung-respiration-depth calculating process is similar to the above-described second right-lung-respiration-depth calculating process, and will not be described in detail. In summary, if the n-th sampling time is a second zero-cross time at which the instantaneous measurement value $Z_{aL}(n)$ of the second bioelectrical impedance $Z_{aL}$ is equal to the second centering value $Z_{aL0}(n)$ and if respiratory action is changing from inhalation to exhalation at the n-th sampling time, the second left lung respiration depth BL2 is calculated as the sum of the absolute values of the local maximum (peak value) $\Delta Z_{bL}(MAX)$ and the local minimum (bottom value) $\Delta Z_{bL}(MIN)$ held at that time. The resulting second left lung respiration depth BL2 relates to the respiration capability of the median and lower lobes of the left lung at the last respiratory action. The higher the respiration capability, the greater the second left lung respiration depth BL2. The respiration depth analysis process at the n-th sampling time is thus completed.

Next, a respiration depth displaying process executed by the CPU 170 will be described. In this embodiment, the CPU 170 executes a respiration depth displaying process at every respiration of the human subject for displaying (reporting) the second right lung respiration depth BR2 and the second left lung respiration depth BL2 in addition to the first right lung respiration depth BR1 and the first left lung respiration depth BL1. As shown in Fig. 27, the CPU 170 controls the display device 160 to display the first right lung respiration depth BR1 in a first bar graph BG1, the first left lung respiration depth BL1 in a second bar graph BG2, the second right lung respiration depth BR2 in a third bar graph BG3, and the second left lung respiration depth BL2 in a fourth bar graph BG4. The manner of indication in the first bar graph BG 1 and the second bar graph BG2 is the same as that in the above-described first embodiment, and will not be described in detail. In the following, the manner of indication in the third bar graph BG3 and the fourth bar graph BG4 will be described.

On the basis of the second right lung respiration depth BR2 at the last single respiration determined by the above-described respiration depth analysis process, the CPU 170 decides the number of degree indicators to be colored in the third bar graph BG3. On the basis of the second left lung respiration depth BL2 at the last single respiration determined by the above-described respiration depth analysis process, the CPU 170 decides the number of degree indicators to be colored in the fourth bar graph BG4. Then, on the basis of the peak value $\Delta Z_{bR}(MAX)$ and the bottom value $\Delta Z_{bR}(MIN)$ that constitute the second right lung respiration depth BR2, the CPU 170 distributes the number of degree indicators to be colored in the third bar graph BG3 to inhalation and exhalation. In addition, on the basis of the peak value $\Delta Z_{bL}(MAX)$ and the bottom value $\Delta Z_{bL}(MIN)$ that constitute the second left lung respiration depth BL2, the CPU 170 distributes the number of degree indicators to be colored in the fourth bar graph BG4 to inhalation and exhalation.

As has been described above, in this embodiment, the CPU 170 controls the display device 160 to show the first right lung respiration depth BR1, the second right lung respiration depth BR2, the first left lung respiration depth BL1, and the second left lung respiration depth BL2 at every respiration of the human subject, the human subject or other person can easily understand the difference between the respiration capabilities of the upper lobes of the right lung and the left lung of the human subject, and the difference between the respiration capabilities of the median and lower lobes of the right lung and the left lung of the human subject. By observing the first through fourth bar graphs BG1 through BG4, the human subject or another person can easily understand these differences.

In the above-described second embodiment, in addition to the first right lung respiration depth BR1 and the first left lung respiration depth BL1, the second right lung respiration depth BR2 and the second left lung respiration depth BL2 are determined. The present invention is not limited to the embodiment. For example, only the second right lung respiration depth BR2 and the second left lung respiration depth BL2 may be determined (it is not necessary to determine the first right lung respiration depth BR1 and the first left lung respiration depth BL1). However, in this case, the right lung respiration depth and the left lung respiration depth are determined only on the basis of the measurement values of third bioelectrical impedance $Z_{bR}$ and the fourth bioelectrical impedance $Z_{bL}$. Change over time in each of the third bioelectrical impedance $Z_{bR}$ and the fourth bioelectrical impedance $Z_{bL}$ may be distorted during exhalations of abdominal respiration. Accordingly, it can be contemplated to distinguish exhalation and inhalation, and to use exhalation information in which distortions at exhalations are removed as information on the respiration depth. Any scheme for distinguishing exhalation and inhalation can be used. For example, it is possible to distinguish exhalation and inhalation by using respiration assistance information for guiding human subjects in performing appropriate breathing. When the respiration assistance information is not used, if distortion caused by exhalations of abdominal respiration does not occur, other distortion can be extracted on the assumption that the waveform is sinusoidal, and it is possible to distinguish exhalation and inhalation on the basis of the magnitude of the distortion.

3. THIRD EMBODIMENT

Next, the third embodiment according to the present invention will be described. In the third embodiment, at every respiration of the human subject, the CPU 170 controls the display device 160 to show not only the right lung respiration depth (the first right lung respiration depth BR1 and the first left lung respiration depth BL1) related to the respiration capability of the right lung, and the left lung respiration depth (the second right lung respiration depth BR2 and the second left lung respiration depth BL2) related to the respiration capability of the left lung, but also the magnitude of each of abdominal respiration and costal respiration at the right body trunk of the human subject and the magnitude of each of abdominal respiration and costal respiration at the left body trunk.

Next, with reference to Figs. 28 and 29, relationships among respiration of the human subject, the costal circumference $R_{ib}$ of human subjects, and abdominal circumference $A_b$ of human subjects will be described. Let us assume that respiration of the human subject is abdominal respiration. Fig. 28 is a graph showing change over time of each of costal and abdominal circumferences in abdominal respiration of a human subject measured by Respitrace (Trademark, AMI Inc, Ardsley, New York, U.S.A.). As will be understood from Fig. 28, when respiration of the human subject is abdominal respiration, the abdominal circumference $A_b$ significantly changes due to respirations, whereas the costal circumference $R_{ib}$ does not significantly change. Let us define that variation in the costal circumference $R_{ib}$ of a human subject is $\Delta R_{ib}$. More specifically, the difference between the measurement value of $R_{ib}$ and the standard level, i.e., standard value of measurement values of $R_{ib}$ is $\Delta R_{ib}$. Let us define that variation in the abdominal circumference

$A_b$ of the human subject is $\Delta A_b$. More specifically, the difference between the measurement value of $A_b$ and the standard level, i.e., standard value of measurement values of $A_b$ is $\Delta A_b$. In abdominal respiration, the ratio $\Delta R_{ib}/\Delta A_b$ is equal to or less than one. Respitrace outputs the absolute value (zero-to-peak value) of the peak value or the bottom value of differences between the measurement values and the standard value, or outputs the sum (peak-to-peak value) of the absolute values of the peak value and the bottom value.

Let us assume that respiration of the human subject is costal respiration. Fig. 29 is a graph showing change over time of each of costal and abdominal circumferences in costal respiration of the human subject measured by Respitrace. When respiration of the human subject is costal respiration, variation in the costal circumference $R_{ib}$ due to respirations is significantly large and greater than variation in the abdominal circumference $A_b$. Therefore, the above-described ratio $\Delta R_{ib}/\Delta A_b$ is greater than one. Thus, the ratio $\Delta R_{ib}/\Delta A_b$ can be considered as indicative information that is used for identifying whether respiration of the human subject is abdominal or costal.

The present inventor found that there was a close correlative relationship among the ratio $\Delta R_{ib}/\Delta A_b$ of the variation in the costal circumference $\Delta R_{ib}$ and the variation in the abdominal circumference $\Delta A_b$, a difference $\Delta Z_a$, and a difference $\Delta Z_b$. The difference $\Delta Z_a$ is a difference between an instantaneous measurement value of the bioelectrical impedance $Z_a$ at the upper body trunk and a centering value $Z_{a0}$ of measurement values of the bioelectrical impedance $Z_a$, whereas the difference $\Delta Z_b$ is a difference between an instantaneous measurement value of the bioelectrical impedance $Z_b$ at the middle body trunk and a centering value $Z_{b0}$ of measurement values of the bioelectrical impedance $Z_b$. Using a formula expressing the correlative relationship, the ratio $\Delta R_{ib}/\Delta A_b$ can be calculated from the first difference $\Delta Z_a$ and the second difference $\Delta Z_b$. In addition, from the calculated ratio $\Delta R_{ib}/\Delta A_b$, it is possible to determine the type of respiration of the human subject (costal respiration or abdominal respiration). The centering value $Z_{a0}$ is a standard level of change over time in the bioelectrical impedance $Z_a$ used for extracting information on respiration of the human subject, and is the average of the bioelectrical impedances at the upper body trunk within a unit time. The centering value $Z_{b0}$ is a standard level of change over time in the bioelectrical impedance $Z_b$ used for extracting information on respiration of the human subject, and is the average of the bioelectrical impedances at the middle body trunk within a unit time.

Fig. 30 is a graph showing relationships between $\Delta R_{ib}/\Delta A_b$ and $\Delta Z_b/\Delta Z_a$ obtained by measurement data on a plurality of human subjects. As will be understood from Fig. 30, there is a close correlative relationship between the ratio $\Delta R_{ib}/\Delta A_b$ and the $\Delta Z_b/\Delta Z_a$, in which the coefficient of correlation $R = 0.651$, and the probability $P < 0.01$. Regression formula (1) below is derived from the correlative relationship.

$$\Delta R_{ib}/\Delta A_b = a_0 * \Delta Z_b/\Delta Z_a + b_0 \quad ... (1)$$

where $a_0$ is a coefficient of regression, and $b_0$ is a constant. According to the inventor's analysis, $a_0$ is 2.7882 whereas $b_0$ is 0.2015.

Regression formula (1) can be rewritten as follows:

$$\Delta R_{ib}/\Delta A_b = (a_0 * \Delta Z_b - \Delta Z_a)/\Delta Z_a + b_1 \quad ... (2)$$

where $b_1$ is a constant and is equal to $a_0$ plus $b_0$.

In this embodiment, using regression formula (2), the ratio $\Delta R_{ib}/\Delta A_b$ corresponding to the aforementioned first difference $\Delta Z_{aR}$ and the third difference $\Delta Z_{bR}$ is calculated. The ratio $\Delta R_{ib}/\Delta A_b$ can be considered as indicative information that is used for identifying whether respiration of the human subject at the right body trunk is abdominal or costal. In addition, in this embodiment, using regression formula (2), the ratio $\Delta R_{ib}/\Delta A_b$ corresponding to the aforementioned second difference $\Delta Z_{aL}$ and the fourth difference $\Delta Z_{bL}$ is calculated. The ratio $\Delta R_{ib}/\Delta A_b$ can be considered as indicative information that is used for identifying whether respiration of the human subject at the left body trunk is abdominal or costal.

Next, the respiration depth analysis process in the third embodiment will be described in detail. Figs. 31 and 32 form a flow chart showing an example of the respiration depth analysis process in the third embodiment. As shown in Fig. 31, in the respiration depth analysis process of this embodiment, after the second right-lung-respiration-depth calculating process (step S810), the CPU 170 executes a right $\Delta R_{ib}/\Delta A_b$ assumption process for calculating the ratio $\Delta R_{ib}/\Delta A_b(n)$ about the right body trunk corresponding to the first difference $\Delta Z_{aR}(n)$ and the third difference $\Delta Z_{bR}(n)$ (step S815). As shown in Fig. 32, after the second left-lung-respiration-depth calculating process (step S860), the CPU 170 executes a left $\Delta R_{ib}/\Delta A_b$ assumption process for calculating the ratio $\Delta R_{ib}/\Delta A_b$ about the left body trunk corresponding to the second difference $\Delta Z_{aL}(n)$ and the fourth difference $\Delta Z_{bL}(n)$ (step S865). Steps S860 and S865 are additional features in comparison with the respiration depth analysis process of the above-described second embodiment. Other steps are the same as those in the respiration depth analysis process of the above-described second embodiment, and will not be described in detail.

Figs. 33 and 34 form a flow chart showing an example of a right $AR_{ib}/\Delta A_b$, assumption process executed by the CPU 170 at step S815. As shown in Fig. 33, the CPU 170 first decides whether or not the first difference $AZ_{aR}(n)$ at the n-th sampling time is equal to or greater than zero (step S1000).

If the decision at step S1000 is negative, the CPU 170 decides that respiration of the human subject is exhalation, and assumes or calculates the right $\Delta Rib/\Delta A_b(n)$ at the n-th sampling time (step S1010). More specifically, the CPU 170 executes an arithmetic process in accordance with the above-described regression formula (2) to calculate the ratio $AR_{ib}/\Delta A_b(n)$ corresponding to the first difference $AZ_{aR}(n)$ and the third difference $AZ_{bR}(n)$.

If the decision at step S1000 is affirmative, the CPU 170 decides that respiration of the human subject is inhalation, and sets the right ratio $\Delta R_{ib}/\Delta A_b(n)$ at the n-th sampling time to an initial value. In this embodiment, if the decision at step S1000 is affirmative, the CPU 170 sets the right ratio $\Delta R_{ib}/\Delta Ab(n)$ at the n-th sampling time to the initial value, 1.0.

Next, the CPU 170 decides whether or not the right ratio $\Delta R_{ib}/\Delta A_b(n)$ is equal to or greater than -2.5 and is equal to or less than 4.5 (step S1020). If the decision at step S1020 is negative, the CPU 170 sets the right ratio $\Delta R_{ib}/\Delta A_b$, to the initial value 1.0, and the process proceeds to step S1030. If the decision at step S1020 is affirmative, the CPU 170 proceeds the process directly to step S1030.

At step S1030, the CPU 170 decides whether or not the difference $(|\Delta R_{ib}/\Delta A_b(n)| - |\Delta R_{ib}/\Delta A_b(n-1)|)$ between the absolute value of the right ratio $\Delta R_{ib}/\Delta A_b(n)$ and the absolute value of the right ratio $\Delta R_{ib}/\Delta A_b(n-1)$ at the n-lth sampling time is greater than 0.3. If the decision at step S 1030 is negative, the CPU 170 calculates the average of the right ratio $\Delta R_{ib}/\Delta A_b(n-1)$ at the n-1th sampling time and the right ratio $\Delta R_{ib}/\Delta A_b(n)$, and decides the resulting average as the $\Delta R_{ib}/\Delta A_b(n)$ at the n-th sampling time, and the process proceeds to step S1040 (see Fig. 34). The calculation is expressed as:

$$[\Delta R_{ib}/\Delta A_b(n-1) + \Delta R_{ib}/\Delta A_b(n)]/2 = \Delta R_{ib}/\Delta A_b(n). \quad \text{If the decision at step}$$

If the decision at step S1030 is affirmative, the CPU 170 sets the right $\Delta R_{ib}/\Delta A_b(n)$ at the n-th sampling time to the initial value 1.0, and the process proceeds to step S1040.

With reference to Fig. 34, description of the right $\Delta R_{ib}/\Delta A_b$ assumption process will be continued. As shown in Fig. 34, at step S1040, the CPU 170 decides whether or not the first difference $\Delta Z_{aR}(n)$ at the n-th sampling time is less than zero. If the decision at step S1040 is affirmative, the CPU 170 decides that respiration of the human subject is exhalation, and it adds one to the last count value $N_i$ of the number of integrations. More specifically, the CPU 170 adds one to the last count value $N_i(n-1)$ of the number of integrations at the n-1th sampling time, and decides the resulting sum as the current count value $N_i(n)$ of the number of integrations at the n-th sampling time.

If the decision at step S1040 is negative, the CPU 170 decides that respiration of the human subject is inhalation, and sets the count value $N_i$ of the number of integrations to an initial value zero. That is to say, the count value $N_i(n)$ of the number of integrations at the n-th sampling time is set to zero.

Then, as shown in Fig. 34, the CPU 170 decides whether or not the first difference $\Delta Z_{aR}(n)$ is less than zero, again (step S1050). If the decision at step S1050 is affirmative, the CPU 170 calculates the sum of the integral value $\Sigma\Delta R_{ib}/\Delta A_b(n-1))$ of the right ratio $\Delta R_{ib}/\Delta A_b$ at the n-1th sampling time and the right ratio $\Delta R_{ib}/\Delta A_b(n)$, and decides the resulting sum as the integral value $(\Sigma\Delta R_{ib}/\Delta A_b(n))$ of the right ratio $\Delta R_{ib}/\Delta A_b$ at the n-th sampling time. Then, the CPU 170 proceeds the process to step S1060. If the decision at step S1050 is negative (i.e., respiration of the human subject is inhalation), the CPU 170 sets the peak-or-bottom indication flag $F_1(n)$ to plus one, and the process proceeds to step S1060.

Next, the CPU 170 decides whether or not the count value $N_i(n)$ of the number of integrations at the n-th sampling time is zero (step S1060). If the decision at step S1060 is negative, the CPU 170 divides the integral value $[\Sigma\Delta R_{ib}/\Delta A_b(n)]$ at the n-th sampling time by the count value $N_i(n)$ of the number of integrations at the n-th sampling time, so as to calculate the current average of the right ratio $\Delta R_{ib}/\Delta A_b$. If the decision at step S1060 is affirmative, the CPU 170 decides the last average $([\Sigma\Delta R_{ib}/\Delta A_b(n-1)]/N_i(n-1))$ of the right ratio $\Delta R_{ib}/\Delta A_b$ at the n-1th sampling time as the current average of the right ratio $\Delta R_{ib}/\Delta A_b$ at the n-th sampling time. The right $\Delta R_{ib}/\Delta A_b$ assumption process at step S815 in Fig. 31 is thus completed.

When respiration of the human subject is abdominal respiration, the above-described average $([\Sigma\Delta R_{ib}/\Delta A_b]/N_i)$ of the ratio $\Delta R_{ib}/\Delta A_b$ varies as shown in Fig. 35. In Fig. 35, if the average $([\Sigma\Delta R_{ib}/\Delta A_b]/N_i)$ of the ratio $\Delta R_{ib}/\Delta A_b$ at exhalations is equal to or less than 1.0, it is assumed that respiration of the human subject is abdominal respiration. If the average is greater than 1.0, it is assumed that respiration of the human subject is costal respiration. The left $\Delta R_{ib}/\Delta A_b$ assumption process for calculating the ratio $\Delta R_{ib}/\Delta A_b$ about the left body trunk at step S865 in Fig. 32 is similar to the above-described right $\Delta R_{ib}/\Delta A_b$ assumption process, and it will not be described in detail.

Next, a respiration depth displaying process executed by the CPU 170 will be described. In this embodiment, the CPU 170 executes the respiration depth displaying process for causing the display device 160 to show the first right lung respiration depth BR1, the first left lung respiration depth BL1, the second right lung respiration depth BR2, and the second left lung respiration depth BL2 at every respiration of the human subject (in a manner similar to the above-described second embodiment). Furthermore, as shown in Fig. 36, the CPU 170 controls the display device 160 to display the magnitude of each of abdominal respiration and costal respiration of the right body trunk of the human subject in a fifth bar graph BG5, and to display the magnitude of each of abdominal respiration and costal respiration of the left body trunk in a sixth bar graph BG6.

What is shown by the fifth bar graph BG5 will be described. In this embodiment, the CPU 170 decides the number of degree indicators to be colored in the fifth bar graph BG5 on the basis of the first right lung respiration depth BR1 at the last single respiration determined by the above-described respiration depth analysis process. Then, on the basis of the average of the ratio $\Delta R_{ib}/\Delta A_b$ with respect to the right body trunk at the last single respiration, the CPU 170 distributes the number of degree indicators to be colored in the fifth bar graph BG5 to abdominal respiration and costal respiration.

Next, indication by the sixth bar graph BG6 will be described. In this embodiment, the CPU 170 decides the number of degree indicators to be colored in the sixth bar graph BG6 on the basis of the first left lung respiration depth BL1 at the last single respiration determined by the above-described respiration depth analysis process. Then, on the basis of the average of the ratio $\Delta R_{ib}/\Delta A_b$ with respect to the left body trunk at the last single respiration, the CPU 170 distributes the number of degree indicators to be colored in the sixth bar graph BG6 to abdominal respiration and costal respiration.

As has been described above, in this embodiment, at every respiration of the human subject, the CPU 170 controls the display device 160 to show not only the right lung respiration depth (the first right lung respiration depth BR1 and the first left lung respiration depth BL1) related to the respiration capability of the right lung, and the left lung respiration depth (the second right lung respiration depth BR2 and the second left lung respiration depth BL2) related to the respiration capability of the left lung, but also the magnitude of each of abdominal respiration and costal respiration at the right body trunk of the human subject and the magnitude of each of abdominal respiration and costal respiration at the left body trunk. Accordingly, the human subject or another person can easily understand not only the difference between the right lung respiration depth and the left lung respiration depth, but also the difference between the degrees of abdominal respiration at the right and left body trunks of the human subject. In summary, the difference between the left and right respiration capabilities of the human subject can be clearly understood.

In an alternative embodiment, the first right lung respiration depth BR1, the first left lung respiration depth BL1, and the degrees of abdominal respiration at the right and left body trunks may be displayed at every respiration, but the second right lung respiration depth BR2 and the second left lung respiration depth BL2 need not be displayed. Accordingly, the second right lung respiration depth BR2 and the second left lung respiration depth BL2 need not be determined, whereas the first right lung respiration depth BR1, the first left lung respiration depth BL1, and the degrees of abdominal respiration at the right and left body trunks may be determined.

In another alternative embodiment, the second right lung respiration depth BR2, the second left lung respiration depth BL2, and the degrees of abdominal respiration at the right and left body trunks may be displayed at every respiration, but the first right lung respiration depth BR1 and the first left lung respiration depth BL1 need not be displayed. Accordingly, the first right lung respiration depth BR1 and the first left lung respiration depth BL1 need not be determined, whereas the second right lung respiration depth BR2, the second left lung respiration depth BL2, and the degrees of abdominal respiration at the right and left body trunks may be determined.

## 4. FOURTH EMBODIMENT

According to the body condition determination apparatus of a fourth embodiment, it is possible to determine whether respiration of the human subject is costal respiration or abdominal respiration. The structure of the body condition determination apparatus in the fourth embodiment is the same as that in the first embodiment, and so the same elements as in the first embodiment will not be described in detail and the same reference symbols for such elements will be used in the following description.

### 4.1. OPERATION

Fig. 37 is a flow chart showing an operation of the body condition determination apparatus 1 of this embodiment. Once the power switch (not shown) on the human interface 150 is turned on, the power source circuit (not shown) provides power to electrical elements. Then, the CPU 170 causes the display device 160 to show a screen for facilitating the user to enter personal or individual body information (including the height, age, and sex) at step T1.

After the human interface 150 inputs the personal information into the CPU 170, the CPU 170 causes the weighing scale 110 to measure the weight, and obtains the weight from the weighing scale 110 at step T2.

The CPU 170 causes the bioelectrical impedance determination part 200 to measure the voltages and the currents, each of which is influenced by bioelectrical impedances at desired regions (for example, the extremities and the body trunk), and determines the impedances at the desired body region. Thus, the CPU 170 and the bioelectrical impedance determination part 200 serve as a bioelectrical impedance determiner for determining bioelectrical impedance at the desired body regions. The CPU 170 executes a respiration analysis process at step T3. As will be described later in detail, in the respiration analysis process, the CPU 170 obtains indicative information that is used for identifying whether respiration of the human subject is abdominal or costal.

After step T3, the CPU 170 executes a respiration depth displaying process (step T4). As will be described later in detail, in the respiration depth displaying process, the CPU 170 causes the display device 160 to show the magnitude of each of abdominal respiration and costal respiration at every respiration and to show the margin level beyond the essential respiration depth for each of abdominal respiration and costal respiration at every respiration. Although not shown, the operation may be ended by the user's instruction.

4.2. PRINCIPLES OF RESPIRATION ANALYSIS

The principles of respiration analysis will be described. Fig. 6 is a schematic view showing tissues in the body trunk of humans. As shown in Fig. 6, tissues in the body trunk are separated by the diaphragm into the upper part and the lower part. The upper part includes the lungs and the chest skeletal muscle including the internal and external intercostal muscles. On the other hand, the lower part includes the visceral tissue and the abdominal skeletal muscle including the internal and external abdominal oblique muscles, the transverse abdominal muscle, and the abdominal rectus muscle.

In both of abdominal respiration and costal respiration, the diaphragm moves up to compress the lungs at exhalation and moves down to expand the lungs at inhalation. The characteristic of abdominal respiration that does not appear in costal respiration is that the visceral tissue expands and contracts in the perpendicular direction so as to move the diaphragm up and down due to ventrodorsal contraction and expansion of the abdominal respiratory muscles including the abdominal rectus muscle, the internal and external abdominal oblique muscles, and the transverse abdominal muscle.

As will be understood from Fig. 7B, the bioelectrical impedance $Z_a$ at the upper body trunk is influenced by impedances $Z_1$ and $Z_2$ in parallel with each other and by impedance $Z_3$. The synthetic impedance of $Z_1$ and $Z_2$ corresponds to the bioelectrical impedance at the upper lobes of lungs.

As will be understood from Fig. 7A, the bioelectrical impedance $Z_b$ at the middle body trunk is influenced by impedances $Z_4$ and $Z_5$ in parallel with each other and by impedances $Z_6$ and $Z_7$ in parallel with each other. The synthetic impedance of $Z_4$ and $Z_5$ corresponds to the bioelectrical impedance at the median and lower lobes of lungs. The bioelectrical impedance $Z_7$ at the visceral tissue includes the bioelectrical impedance of the diaphragm.

In the specification, the bioelectrical impedance $Z_a$ at the upper body trunk will be referred to as the fifth bioelectrical impedance $Z_a$, whereas the bioelectrical impedance $Z_b$ at the middle body trunk will be referred to as the sixth bioelectrical impedance $Z_b$.

With reference to Fig. 8, the relationship between respiration and change of bioelectrical impedance will be described.

Change in the fifth bioelectrical impedance $Z_a$ at the upper body trunk in respiration is mainly caused by air entering and leaving the lungs, which has a high electrical insulation property and causes change in the electrical characteristics (i.e., the electrical conductivity or the inverse of the volume resistivity) at the upper body trunk. During exhalations (expirations), the bioelectrical impedance $Z_2$ at the lungs decreases due to reduction in the volume of air inside tissues in the lungs ($\Delta Z_{1u} < 0$). During inhalations (inspirations), the bioelectrical impedance $Z_2$ at the lungs increases due to increase in the volume of air inside tissues in the lungs ($\Delta Z_{lu} > 0$).

In costal respiration, which expands and contracts the thoracic cage, the chest skeletal muscle that contributes to respiration (e.g., internal and external intercostal muscles) expands and contracts in a similar way to the expansion and contraction of the lungs. Therefore, in costal respiration, when the lungs expand so that the bioelectrical impedance $Z_2$ at the lungs increases, the chest skeletal muscle also expands, and the bioelectrical impedance $Z_1$ at the chest skeletal muscle also increases. Similarly, when the lungs contract so that the bioelectrical impedance $Z_2$ at the lungs decreases, the chest skeletal muscle also contracts and the bioelectrical impedance $Z_1$ at the chest skeletal muscle also decreases.

However, in abdominal respiration in which the thoracic cage does not change in volume significantly, although the bioelectrical impedance $Z_2$ at the lungs changes significantly due to respiration, the bioelectrical impedance $Z_1$ at the chest skeletal muscle does not change significantly. The fifth bioelectrical impedance $Z_a$ includes the bioelectrical impedance $Z_3$ at the upper extremity skeletal muscle, but the upper extremity skeletal muscle does not directly contribute to respiration. In this embodiment, the human subject stands on the platform 20 shown in Fig. 2 and grips the electrode handles 30L and 30R with the left and right hands, respectively, with the arms extending downward, so that the upper extremity skeletal muscle does not move during the measurement of bioelectrical impedance, and the bioelectrical

impedance $Z_3$ thereat does not change significantly. As shown in Figs. 9 and 10, in both of costal respiration and abdominal respiration, the fifth bioelectrical impedance $Z_a$ increases at inhalations and decreases at exhalations.

Change in the sixth bioelectrical impedance $Z_b$ at the middle body trunk in respiration relates to movement of the diaphragm. As described above, in both abdominal respiration and costal respiration, the diaphragm moves up to compress the lungs at exhalation and moves down to expand the lungs at inhalation. The characteristic of abdominal respiration is that the visceral tissue expands and contracts in the perpendicular direction for moving the diaphragm up and down due to ventrodorsal contraction and expansion of the abdominal respiratory muscles (abdominal skeletal muscles).

More specifically, in abdominal respiration, during exhalations, the human subject contracts the abdominal muscle ventrodorsally so as to move up the diaphragm together. As a result, the visceral tissue and the abdominal skeletal muscle expand in the perpendicular direction, thereby increasing the impedance $Z_6$ at the abdominal skeletal muscle and the impedance $Z_7$ at the visceral tissue ($\Delta Z_{st} > 0$). During exhalations, the bioelectrical impedance at the lungs decreases due to reduction in the volume of air inside tissues in the lungs as described above ($\Delta Z_{1u} < 0$). Therefore, in abdominal respiration, when the bioelectrical impedance at the chest region above the diaphragm decreases, the bioelectrical impedance at the abdominal region beneath the diaphragm increases. In other words, increase in the bioelectrical impedance of the abdominal region beneath the diaphragm cancels decrease in the bioelectrical impedance at the chest region above the diaphragm when the human subject performs exhalation in abdominal respiration. As will be understood from the above description, the movement of the abdominal region (including abdominal skeletal muscle and the visceral tissue) beneath the diaphragm varies depending on the type of respiration (costal and abdominal respiration).

As shown in Fig. 9, when respiration of the human subject is abdominal respiration, the sixth bioelectrical impedance $Z_b$ increases at inhalations, and also increases at exhalations since the bioelectrical impedance of the abdominal region beneath the diaphragm cancels decrease in the bioelectrical impendance at the chest region above the diaphragm. As shown in Fig. 10, when respiration of the human subject is costal respiration, the sixth bioelectrical impedance $Z_b$ increases at inhalations and decreases at exhalation, in a manner similar to the above-described change of the fifth bioelectrical impedance $Z_a$.

As will be understood from Fig. 28, when respiration of the human subject is abdominal respiration, the abdominal circumference $A_b$ significantly changes due to respirations, whereas the costal circumference $R_{ib}$ does not significantly change. Let us define that variation in the costal circumference $R_{ib}$ of a human subject is $\Delta R_{ib}$. More specifically, the difference between the measurement value of $R_{ib}$ and the standard level, i.e., standard value of measurement values of $R_{ib}$ is $\Delta R_{ib}$. Let us define that variation in the abdominal circumference $A_b$ of the human subject is $\Delta A_b$. More specifically, the difference between the measurement value of $A_b$ and the standard level, i.e., standard value of measurement values of $A_b$ is $\Delta A_b$. In abdominal respiration, the ratio $\Delta R_{ib}/\Delta A_b$ is equal to or less than one. In contrast, as shown in Fig. 29, when respiration of the human subject is costal respiration, variation in the costal circumference $R_{ib}$ due to respirations is significantly large and greater than variation in the abdominal circumference $A_b$. Therefore, the above-described ratio $\Delta R_{ib}/\Delta A_b$ is greater than one.

The present inventor found that there was a close correlative relationship among the ratio $\Delta R_{ib}/\Delta A_b$, of the variation in the costal circumference $\Delta R_{ib}$ and the variation in the abdominal circumference $\Delta A_b$, a fifth difference $\Delta Z_a$, and a sixth difference $\Delta Z_b$. The fifth difference $\Delta Z_a$ is a difference between an instantaneous measurement value of the fifth bioelectrical impedance $Z_a$ and a fifth centering value $Z_{a0}$ of measurement values of the fifth bioelectrical impedance $Z_a$, whereas the sixth difference $\Delta Z_b$ is a difference between an instantaneous measurement value of the sixth bioelectrical impedance $Z_b$ and a sixth centering value $Z_{b0}$ of measurement values of the sixth bioelectrical impedance $Z_b$. Using a formula expressing the correlative relationship, the ratio $\Delta R_{ib}/\Delta A_b$ can be calculated from the fifth difference $\Delta Z_a$ and the sixth difference $\Delta Z_b$. In addition, from the calculated ratio $\Delta R_{ib}/\Delta A_B$, it is possible to determine the type of respiration of the human subject (costal respiration or abdominal respiration). As will be described later in detail, the fifth centering value $Z_{a0}$ is a standard level of change over time in the fifth bioelectrical impedance $Z_a$ used for extracting information on respiration of the human subject, and is the average of the fifth bioelectrical impedances within a unit time. As will be described later in detail, the sixth centering value $Z_{b0}$ is a standard level of change over time in the sixth bioelectrical impedance $Z_b$ used for extracting information on respiration of the human subject, and is the average of the sixth bioelectrical impedances within a unit time.

As has been described in conjunction with Fig. 30, there is a close correlative relationship between the ratio $\Delta R_{ib}/\Delta A_b$ and the $\Delta Z_b/\Delta Z_a$, in which the coefficient of correlation R = 0.651, and the probability P < 0.01. Regression formulae (1) and (2) are found as has been described in conjunction with the third embodiment.

Change in the fifth bioelectrical impedance $Z_a$ at the upper body trunk in respiration can be considered as change in the bioelectrical impedance at the upper lobes of lungs (the synthetic impedance of $Z_1$ and $Z_2$ in parallel with each other). Change in the sixth bioelectrical impedance $Z_b$ at the middle body trunk in respiration can be considered as the sum of change in the bioelectrical impedance at the median and lower lobes of lungs (the synthetic impedance of $Z_4$ and $Z_5$ in parallel each other) and change in the abdominal bioelectrical impedance (the synthetic impedance of

$Z_6$ and $Z_7$ in parallel with each other).

Change in the bioelectrical impedance at the upper lobes of lungs can be equivalent to change in the bioelectrical impedance at the median and lower lobes of lungs since the locations are near the chest. Thus, the difference between change in the sixth bioelectrical impedance $Z_b$ and change in the fifth bioelectrical impedance $Z_a$ is equivalent to change in the abdominal bioelectrical impedance. Accordingly, formula (2) can be considered to be a formula expressing a relationship between the ratio of the abdominal bioelectrical impedance to change in the costal bioelectrical impedance and the ratio $\Delta R_{ib}/\Delta A_b$. Constant $a_0$ in formula (2) can be a compensation coefficient for compensating the difference between sensitivities for measurements on the upper lobes of the lungs and the median and lower lobes of the lungs.

## 4.3. RESPIRATION ANALYSIS PROCESS

Next, a respiration analysis process executed by the CPU 170 will be described. Fig. 38 is a flow chart showing an example of the respiration analysis process. In this embodiment, the CPU 170 executes the respiration analysis process ten times within a single respiration, i.e., single respiratory action (consisting of a single inhalation and a single exhalation) at a normal speed. In this embodiment, since a normal single respiration takes four seconds, the CPU 170 executes the respiration analysis process every 0.4 seconds. In the following, the start time for each respiration analysis process that occurs every 0.4 seconds will be referred to as a sampling time. However, this is only an example, and the cycle of the respiration analysis process may be decided in a different manner.

As shown in Fig. 38, the CPU 170 first decides whether or not the current time reaches a sampling time (step T10), and the process proceeds to step T20 if the decision at step T10 is affirmative. Description of subsequent steps will be continued on the assumption that the current time reaches the n-th sampling time where n is a natural number that is equal to or greater than one. Prior to detailed description of subsequent steps, brief description will be made for each of the subsequent steps.

At step T20, the CPU 170 bioelectrical impedance determiner) determines the fifth bioelectrical impedance $Z_a$ at the upper body trunk. Next, at step T30, the CPU 170 (bioelectrical impedance determiner) determines the sixth bioelectrical impedance $Z_b$ at the middle body trunk. Next, at step T40, the CPU 170 executes a smoothing process for each of the fifth bioelectrical impedance $Z_a$ determined at step T20 and the sixth bioelectrical impedance $Z_b$ determined at step T30. Next, at step T50, the CPU 170 (third centering value generator) generates the fifth centering value $Z_{a0}$ that is a standard level of change over time in the fifth bioelectrical impedance $Z_a$. Next, at step T60, the CPU 170 (fifth difference calculator) calculates the fifth difference $\Delta Z_a$ that is the difference between the instantaneous measurement value of the fifth bioelectrical impedance $Z_a$ and the fifth centering value $Z_{a0}$. Next, at step T70, the CPU 170 (third centering value generator) generates the sixth centering value $Z_{b0}$ that is a standard level of change over time in the sixth bioelectrical impedance $Z_b$, and the CPU 170 (sixth difference calculator) calculates the sixth difference $\Delta Z_b$ that is the difference between the instantaneous measurement value of the sixth bioelectrical impedance $Z_b$ and the sixth centering value $Z_{b0}$. Next, at step T80, the CPU 170 (respiration depth calculator) executes an arithmetic process in accordance with regression formula (2) described above so as to calculates the ratio $\Delta R_{ib}/\Delta A_b$ corresponding to the fifth difference $\Delta Z_a$ and the sixth difference $\Delta Z_b$. In the following, these steps will be described in detail.

At step T20, the CPU 170 determines the fifth bioelectrical impedance $Z_a$ at the upper body trunk. For example, the CPU 170 controls the electrode switching circuit 252 to select the left-hand current electrode X3 and the right-hand current electrode X4, and controls the electrode switching circuit 251 to select the right-foot voltage electrode Y2 and the right-hand voltage electrode Y4. Then, the CPU 170 determines the fifth bioelectrical impedance $Z_a$ at the right upper extremity and the upper body trunk on the basis of the current data $D_i$ indicating the reference current $I_{ref}$ flowing between the right and left hands and the voltage data $D_v$ indicating the potential difference between the right-foot voltage electrode Y2 and the right-hand voltage electrode Y4. Hereinafter, the actual measured value of the fifth bioelectrical impedance at the n-th sampling time (n is a natural number that is equal to or greater than one) will be referred to as $Z_a(n)'$.

Next, the CPU 170 determines the sixth bioelectrical impedance $Z_b$ at the middle body trunk (step T30). For example, the CPU 170 controls the electrode switching circuit 252 to select the left-foot current electrode X1 and the right-hand current electrode X4, and controls the electrode switching circuit 251 to select the left-hand voltage electrode Y3 and the right-foot voltage electrode Y2. Then, the CPU 170 determines the sixth bioelectrical impedance $Z_b$ at the middle body trunk on the basis of the current data $D_i$ indicating the reference current $I_{rcf}$ flowing between the left foot and the right hand and the voltage data $D_v$ indicating the potential difference between the left-hand voltage electrode Y3 and the right-foot voltage electrode Y2. Hereinafter, the actual measured value of the sixth bioelectrical impedance at the n-th sampling time (n is a natural number that is equal to or greater than one) will be referred to as $Z_b(n)'$.

Next, at step T40, the CPU 170 executes a smoothing process for each of the fifth bioelectrical impedance $Z_a(n)'$ determined at step T20 and the sixth bioelectrical impedance $Z_b(n)'$ determined at step T30. First, the smoothing process for the fifth bioelectrical impedance $Z_a(n)'$ will be described in detail. The CPU 170 calculates the moving average of the actual measured value $Z_a(n-2)'$ of the fifth bioelectrical impedance at the n-2th sampling time, the actual measured value $Z_a(n-1)'$ of the fifth bioelectrical impedance at the n-1th sampling time, and the actual measured value $Z_a(n)'$ of

the fifth bioelectrical impedance at the n-th sampling time. Then, the CPU 170 decides the calculated moving average as the measurement value of the fifth bioelectrical impedance at the n-th sampling time (this is called the smoothing process). The measurement value of the fifth bioelectrical impedance resulting from the smoothing process will be referred to as $Z_a(n)$.

Next, the smoothing process for the sixth bioelectrical impedance $Z_b(n)'$ will be described in detail. The CPU 170 calculates the moving average of the actual measured value $Z_b(n-2)'$ of the sixth bioelectrical impedance at the n-2th sampling time, the actual measured value $Z_b(n-1)'$ of the sixth bioelectrical impedance at the n-1th sampling time, and the actual measured value $Z_b(n)'$ of the sixth bioelectrical impedance at the n-th sampling time. Then, the CPU 170 decides the calculated moving average as the measurement value of the sixth bioelectrical impedance at the n-th sampling time (this is called the smoothing process). The measurement value of the sixth bioelectrical impedance resulting from the smoothing process will be referred to as $Z_b(n)$.

Next, the CPU 170 executes a fifth centering process for generating a fifth centering value $Z_{a0}$ that is a standard level of change over time in the fifth bioelectrical impedance $Z_a$ (step T50). Hereinafter, the fifth centering value at the n-th sampling time will be referred to as $Z_{a0}(n)$. In this embodiment, the CPU 170 generates or calculates the fifth centering value $Z_{a0}(n)$ on the basis of the measurement values of the fifth bioelectrical impedance $Z_a$ at multiple sampling times within a centering period starting from a time point that is a predetermined time length before the n-th sampling time and ending at the n-th sampling time. The time length of the centering period is variable and is set depending on the respiration speed of the human subject at the n-th sampling time. The fifth centering process will be described in detail.

Fig. 39 is a flow chart showing an example of the fifth centering process. As shown in Fig. 39, the CPU 170 first executes a MA10 calculating process for calculating the moving average (that is, MA10) of the measurement values of the fifth bioelectrical impedance $Z_a$ at the last ten sampling times (step T51). More specifically, the CPU 170 calculates the moving average of the measurement values ($Z_a(n-9)$ through $Z_a(n)$) of the fifth bioelectrical impedance $Z_a$ at the n-9th through n-th (ten) sampling times. The resulting moving average is referred to as the moving average MA10(n) at the n-th sampling time. The calculation is expressed as:

$$[Z_a(n\text{-}9) + Z_a(n\text{-}8) + ... + Z_a(n)]/10 = MA10(n).$$

Next, the CPU 170 executes a MA20 calculating process for calculating the moving average (that is, MA20) of the measurement values of the fifth bioelectrical impedance $Z_a$ at the last 20 sampling times (step T52). More specifically, the CPU 170 calculates the moving average of the measurement values ($Z_a(n-19)$ through $Z_a(n)$) of the fifth bioelectrical impedance $Z_a$ at the n-19th through n-th (twenty) sampling times. The resulting moving average is referred to as the moving average MA20(n) at the n-th sampling time. The calculation is expressed as:

$$[Z_a(n\text{-}19) + Z_a(n\text{-}18) + ... + Z_a(n)]/20 = MA20(n).$$

Next, the CPU 170 executes a MAX10 extraction process for extracting the maximum (referred to as MAX10) among the measurement values of the fifth bioelectrical impedance $Z_a$ at the last ten sampling times (step T53). More specifically, the CPU 170 selects the maximum among the measurement values of the fifth bioelectrical impedance $Z_a$ at the n-9th through n-th (ten) sampling times, and determines it to be the maximum MAX10(n) at the n-th sampling time.

Next, the CPU 170 executes a MIN10 extraction process for extracting the minimum (referred to as MIN10) among the measurement values of the fifth bioelectrical impedance $Z_a$ at the last ten sampling times (step T54). More specifically, the CPU 170 selects the minimum among the measurement values of the fifth bioelectrical impedance $Z_a$ at the n-9th through n-th (ten) sampling times, and determines it to be the minimum MIN10(n) at the n-th sampling time.

Next, at step T55, the CPU 170 executes a median value calculating process for calculating the moving average of mean values AV10 at the last 20 sampling times, in which AV10 is the mean value of the maximum MAX10 and the minimum MIN10 at a sampling time. For example, AV10(n) is the mean value of the maximum MAX10(n) and the minimum MIN10(n) at the n-th sampling time. More specifically, the CPU 170 calculates the moving average of the mean values AV10(n-19) through AV10(n) at the n-19th through n-th (twenty) sampling times, and decides the resulting moving average as a median value CNT20(n) at the n-th sampling time. The calculation is expressed as:

$$[AV10(n\text{-}19) + AV10(n\text{-}18) + ... + AV10(n)]/20 = CNT20(n).$$

Although not described in detail, the median value CNT20(n) is calculated in order to exclude artifacts (measurement errors that are unsuitable for processing) caused by body motion or other reasons.

Next, the CPU 170 executes a respiration timing extraction process for deciding respiration timing of the human subject at the n-th sampling time (step T56). In the following, with reference to Figs. 40 and 41, respiration timing extraction process will be described in detail. Figs. 40 and 41 form a flow chart showing an example of the respiration timing extraction process. As shown in Fig. 40, the CPU 170 first executes a differential coefficient calculating process for calculating the differential coefficient $dZ_a(n)$ of the fifth bioelectrical impedance $Z_a$ at the n-th sampling time (step T201). More specifically, the CPU 170 executes an arithmetic process in accordance with formula (3) below for calculating the differential coefficient $dZ_a(n)$.

$$[Z_a(n) - Z_a(n-2)]/1.2 = dZ_a(n) \quad ... \quad (3)$$

Next, the CPU 170 decides whether or not the absolute value of the differential coefficient $dZ_a(n)$ calculated at step T201 is less than 0.1 (step T202). If the decision at step T202 is affirmative, the CPU 170 sets the tendency indication flag $F_0(n)$ to zero, and the process proceeds to step T204. The tendency indication flag $F_0(n)$ indicates tendency (increase, decrease or no trend) of the differential coefficient $dZ_a(n)$. The fact that the tendency indication flag $F_0(n)$ is zero indicates that the measurement value $Z_a(n)$ of the fifth bioelectrical impedance $Z_a$ at the n-th sampling time has no trend, that is to say, it is at a local maximum (peak value) or at a local minimum (bottom value).

If the decision at step T202 is negative, the CPU 170 decides whether or not the differential coefficient $dZ_a(n)$ is greater than zero (step T203). If the decision at step T203 is affirmative, the CPU 170 sets the tendency indication flag $F_0(n)$ to plus one, and the process proceeds to step T204. The fact that the tendency indication flag $F_0(n)$ is plus one indicates that the measurement value $Z_a(n)$ of the fifth bioelectrical impedance $Z_a$ at the n-th sampling time has a positive (increasing) trend. If the decision at step T203 is negative, the CPU 170 sets the tendency indication flag $F_0(n)$ to minus one, and the process proceeds to step T204. The fact that the tendency indication flag $F_0(n)$ is minus one indicates that the measurement value $Z_a(n)$ of the fifth bioelectrical impedance $Z_a$ at the n-th sampling time has a negative (decreasing) trend.

At step T204, the CPU 170 decides whether or not the absolute value of the tendency indication flag $F_0(n)$ at the n-th sampling time is equal to the absolute value of the tendency indication flag $F_0(n-1)$ at the n-1th sampling time. In addition, the CPU 170 decides whether or not the value of $F_0(n-1)$ is unequal to $F_0(n)$. If the composite decision at step T204 is affirmative, the CPU 170 sets the tendency indication flag $F_0(n)$ to zero, and the process proceeds to step T206 (see Fig. 41). If the decision at step T204 is negative, the CPU 170 holds $F_0(n)$ set before step T204, and the process proceeds to step T206.

With reference to Fig. 41, description of the respiration timing extraction process will be continued. The CPU 170 decides whether or not the tendency indication flag $F_0(n)$ at the n-th sampling time is zero (step T206). If the decision at step T206 is negative, the CPU 170 sets a peak-or-bottom indication flag $F_1(n)$ to zero, and the process proceeds to step T209. The fact that the peak-or-bottom indication flag $F_1(n)$ is zero indicates that the measurement values $Z_a(n)$ of the fifth bioelectrical impedance at the n-th sampling time is not the peak value or the bottom value.

If the decision at step T206 is affirmative, the CPU 170 decides whether or not the sum of the tendency indication flags $F_0$ at the last three sampling times is greater than plus one (step T207). More specifically, the CPU 170 calculates the sum of the tendency indication flags $F_0(n-2)$ through $F_0(n)$ at the n-2th through the n-th sampling times, and makes the decision.

If the decision at step T207 is affirmative, the CPU 170 sets the peak-or-bottom indication flag $F_1(n)$ to plus one, and the process proceeds to step T209. The fact that the peak-or-bottom indication flag $F_1(n)$ is plus one indicates that the measurement value $Z_a(n)$ of the fifth bioelectrical impedance at the n-th sampling time is at a local maximum (peak value).

If the decision at step T207 is negative, the CPU 170 decides whether or not the sum of the tendency indication flags $F_0$ at the last three sampling times is less than minus one (step T208). In other words, the CPU 170 decides whether or not the sum of the tendency indication flags $F_0(n-2)$ through $F_0(n)$ at the n-2th through the n-th sampling times is less than minus one.

If the decision at step T208 is affirmative, the CPU 170 sets the peak-or-bottom indication flag $F_1(n)$ to minus one, and the process proceeds to step T209. The fact that the peak-or-bottom indication flag $F_1(n)$ is minus one indicates the measurement value $Z_a(n)$ of the fifth bioelectrical impedance at the n-th sampling time is at a local minimum (bottom value). If the decision at step T208 is negative, the CPU 170 sets the peak-or-bottom indication flag $F_1(n)$ to zero, and the process proceeds to step T209.

At step T209, the CPU 170 decides whether or not the peak-or-bottom indication flag $F_1(n)$ is plus one. If the decision at step T209 is negative, the CPU 170 adds one to a sampling counter value N(n-1) at the n-1th sampling time (step T210). If the decision at step T209 is affirmative, the CPU 170 initializes the sampling counter value N (step T211). As will be understood from Figs. 9 and 10, irrespective of whether respiration of the human subject is costal respiration or abdominal respiration, the waveform of change in the fifth bioelectrical impedance $Z_a$ in respiration is nearly sinusoidal. The sampling counter value N is initialized to be zero whenever the measurement value of the fifth bioelectrical impedance $Z_a$ reaches a peak value. The sampling counter value N is incremented at each new sampling time, so that the number of sampling times is counted by the sampling counter until the next peak value of the measurement value of the fifth bioelectrical impedance $Z_o$. Thus, the respiration timing extraction process at step T56 in Fig. 39 is completed.

Returning to Fig. 39, description will be continued. After the above-described respiration timing extraction process is completed, the CPU 170 sets a respiration speed indication flag that indicates whether respiration of the human subject is fast or slow (step T57). In the following, with reference to Fig. 42, the respiration speed indication flag setting process executed by the CPU 170 at step T57 will be described in detail. Fig. 42 is a flow chart showing an example of the respiration speed indication flag setting process. As shown in Fig. 42, the CPU 170 first decides whether or not the tendency indication flag $F_0(n)$ is zero (step T301). If the decision at step T301 is negative, the CPU 170 considers that the respiration speed indication flag $F_{ma}(n)$ at the n-th sampling time as being equal to the respiration speed indication flag $F_{ma}(n-1)$ at the n-1th sampling time, and the process ends.

If the decision at step T301 is affirmative, the CPU 170 decides whether or not the peak-or-bottom indication flag $F_1(n)$ is plus one (step T302). If the decision at step T302 is affirmative, the CPU 170 decides whether or not the sampling counter value N(n-1) at the n-1th sampling time is greater than ten (step T303). If the respiration speed of the human subject is slower, the time length between the time point at which the measurement value of the fifth bioelectrical impedance $Z_a$ is at a peak value and the time point at which the measurement value of the fifth bioelectrical impedance $Z_a$ arrives at the next peak value is longer, so that the sampling counter value N directly before the next peak value is larger. In this embodiment, when the CPU 170 decides that the measurement value of the fifth bioelectrical impedance $Z_a$ at n-th sampling time has reached a peak value, the CPU 170 decides whether or not the sampling counter value at the n-1th sampling time is greater than ten. If the CPU 170 has decided that the sampling counter value is greater than ten, the CPU 170 determines that the respiration of the human subject is slow. Specifically, if the decision at step T303 is affirmative, the CPU 170 sets the respiration speed indication flag $F_{ma}(n)$ to 20, and the process ends. The fact that the respiration speed indication flag $F_{ma}(n)$ is 20 indicates that the respiration of the human subject is slow. If the decision at step T303 is negative, the CPU 170 sets the respiration speed indication flag $F_{ma}(n)$ to ten, and the process ends. The fact that the respiration speed indication flag $F_{ma}(n)$ is ten indicates that respiration of the human subject is fast.

If the decision at step T302 is negative, the CPU 170 decides whether or not the peak-or-bottom indication flag $F_1(n)$ is minus one (step T304). If the decision at step T304 is negative, the CPU 170 determines that the respiration speed indication flag $F_{ma}(n)$ at the n-th sampling time is equal to respiration speed indication flag $F_{ma}(n-1)$ at the n-1th sampling time, and the process ends. If the decision at step T304 is affirmative, the CPU 170 decides whether or not the sampling counter value N(n-1) at the n-1th sampling time is greater than five (step T305). In this embodiment, when the CPU 170 decides that the sampling counter value N(n-1) directly before its arrival at the bottom value is greater than five, the CPU 170 determines that respiration of the human subject is slow. When the CPU 170 decides that the sampling counter value N(n-1) directly before its arrival at the bottom value is less than five, the CPU 170 determines that respiration of the human subject is fast. Specifically, if the decision at step T305 is affirmative, the CPU 170 sets the respiration speed indication flag $F_{ma}(n)$ to 20, and the process ends. If the decision at step T305 is negative, the CPU 170 sets the respiration speed indication flag $F_{ma}(n)$ to ten, and the process ends. The respiration speed indication flag setting process at step T57 in Fig. 39 is thus completed.

Returning to Fig. 39, description will be continued. Once the above-described respiration speed indication flag setting process is completed, the CPU 170 calculates the fifth centering value $Z_{a0}(n)$ at the n-th sampling time (step T58). In the following, with reference to Fig. 43, the fifth centering value calculating process executed by the CPU 170 at step T58 will be described in detail. Fig. 43 is a flow chart showing an example of the fifth centering value calculating process. As shown in Fig. 43, the CPU 170 first decides whether or not the respiration speed indication flag $F_{ma}(n)$ is ten (step T401). In other words, the CPU 170 decides whether or not respiration of the human subject at the n-th sampling time is fast.

In this embodiment, on the basis of the measurement values of the fifth bioelectrical impedance $Z_a$ at multiple sampling times within a centering period that is variable and is set depending on the respiration speed of the human subject, the fifth centering value $Z_{a0}(n)$ at the n-th sampling time is calculated or generated. If the decision at step T401 is affirmative (i.e., the respiration speed of the human subject at the n-th sampling time is fast), a time length (e.g., about four seconds) taken for a faster single respiration (single respiratory action) is set as the centering period. Specifically, if respiration of the human subject is fast, a period starting from the n-9th sampling time and ending at the n-th sampling time is set as the centering period, and the fifth centering value $Z_{a0}(n)$ is generated or calculated on the basis of the moving average MA10(n) of the measurement values of the fifth bioelectrical impedance at the n-9 th through n-th sampling times. In other words, if the decision at step T401 is affirmative, the CPU 170 generates or calculates the fifth centering value $Z_{a0}(n)$ at the n-th sampling time on the basis of the moving average MA10(n) calculated at step T51 in Fig. 39 (step T402). More specifically, the CPU 170 calculates the moving average of the fifth centering value $Z_{a0}(n-2)$ at the n-2th sampling time, the fifth centering value $Z_{a0}(n-1)$ at the n-1th sampling time, and the moving average MA10(n), and decides the resulting moving average as the fifth centering value $Z_{a0}(n)$ at the n-th sampling time. The calculation is expressed as:

$$[Z_{a0}(n\text{-}2) + Z_{a0}(n\text{-}1) + MA10(n)]/3 = Z_{a0}(n).$$

If the decision at step T401 is negative (i.e., the respiration speed of the human subject at the n-th sampling time is slow), a time length (e.g., about eight seconds) taken for a slower single respiration (single respiratory action) is set as the centering period. Specifically, if respiration of the human subject is slow, a period starting from the n-19th sampling time and ending at the n-th sampling time is set as the centering period, and the fifth centering value $Z_{a0}(n)$ is generated or calculated on the basis of the moving average MA20(n) of the measurement values of the fifth bioelectrical impedance at the n-19th through n-th sampling times. In other words, if the decision at step T401 is negative, the CPU 170 generates or calculates the fifth centering value $Z_{a0}(n)$ at the n-th sampling time on the basis of the moving average MA20(n) calculated at step T52 in Fig. 39 (step T403). More specifically, the CPU 170 calculates the moving average of the fifth centering value $Z_{a0}(n-2)$ at the n-2th sampling time, the fifth centering value $Z_{a0}(n-1)$ at the n-1th sampling time, and the moving average MA20(n), and decides the resulting moving average as the fifth centering value $Z_{a0}(n)$ at the n-th sampling time. The calculation is expressed as:

$$[Z_{a0}(n\text{-}2) + Z_{a0}(n\text{-}1) + MA20(n)]/3 = Z_{a0}(n).$$

The fifth centering process at step T50 in Fig. 38 is thus completed.

As described above, irrespective of whether respiration of the human subject is costal respiration or abdominal respiration, the waveform of change in the fifth bioelectrical impedance $Z_{aR}$ in respiration is nearly sinusoidal. The CPU 170 (third centering value generator) generates or calculates the fifth centering value $Z_{a0}$ on the basis of measurement values of the fifth bioelectrical impedance $Z_a$ at the sampling times of which the number is predetermined in order to obtain a suitable fifth centering value $Z_{a0}$ even if one or more instantaneous values of the fifth bioelectrical impedance $Z_a$ are disturbed by body motion or for other reasons. More specifically, at each sampling time, the CPU 170 generates or calculates a moving average MA10(n) or MA20(n) of measurement values of the fifth bioelectrical impedance at multiple sampling times within a centering period starting from a time point that is a predetermined time length before the n-th sampling time and ending at the n-th sampling time, and calculates the fifth centering value $Z_{a0}$ at the sampling time. Accordingly, even if one or more instantaneous values of the fifth bioelectrical impedance $Z_a$ are disturbed, a suitable fifth centering value $Z_{a0}$ can be generated. In addition, the time length of the centering period is variable and is set depending on the respiration speed of the human subject at the sampling time.

Returning to Fig. 38, description will be continued. As shown in Fig. 38, after the fifth centering process of step T50 is completed, the CPU 170 executes a fifth difference calculating process for calculating a fifth difference $\Delta Z_a(n)$ that is the difference between the instantaneous measurement value $Z_a(n)$ of the fifth bioelectrical impedance and the fifth centering value $Z_{a0}(n)$ (step T60). More specifically, the CPU 170 calculates the difference between the measurement value $Z_a(n)$ of the fifth bioelectrical impedance calculated at step T40 and the fifth centering value $Z_{a0}(n)$ calculated at step T50, and decides the difference as the fifth difference $\Delta Z_a(n)$ at the n-th sampling time.

After step T60, the CPU 170 generates or calculates a sixth centering value $Z_{b0}$ that is a standard level of change over time in the sixth bioelectrical impedance $Z_b$, and calculates a sixth difference $\Delta Z_b$ that is the difference between the instantaneous measurement value of the sixth bioelectrical impedance $Z_b$ and the sixth centering value $Z_{b0}$ (step T70).

As described above, change in the sixth bioelectrical impedance $Z_b$ during exhalations of abdominal respiration is completely different from that of the fifth bioelectrical impedance $Z_a$. Accordingly, in contrast to the calculation of the fifth centering value $Z_{a0}$, if a moving average is calculated on the basis of measurement values of the sixth bioelectrical impedance $Z_b$ at the sampling times of which the number is predetermined, the standard level (sixth centering value $Z_{b0}$) of change over time in the sixth bioelectrical impedance $Z_b$ cannot be calculated accurately.

Accordingly, in this embodiment, as shown in Fig. 44, zero-cross times are decided in which the instantaneous measurement value of the fifth bioelectrical impedance $Z_a$ is equal to the fifth centering value $Z_{a0}$. In addition, the sixth centering value $Z_{b0}$ is generated or calculated on the basis of measurement values of the sixth bioelectrical impedance $Z_b$ at the zero-cross times, so that the standard level (sixth centering value $Z_{b0}$) of change over time in the sixth bioelectrical impedance $Z_b$ can be calculated accurately. Fig. 44 is a graph for explaining a scheme for generating the sixth centering value $Z_{b0}$. In the following, with reference to Fig. 45, the sixth difference calculating process executed at step T70 by the CPU 170 will be described in detail.

Fig. 45 is a flow chart showing an example of the sixth difference calculating process. As shown in Fig. 45, the CPU 170 extracts the minimum among the fifth difference $\Delta Z_a$ at the last five sampling times (step T71). More exactly, the CPU 170 selects the minimum among the absolute value $|\Delta Z_a|$ ($|\Delta Z_a(n-4)|$ through $|\Delta Z_a(n)|$) of the fifth difference $\Delta Z_a$ at the n-4th through the n-th sampling times, and decides the minimum as a zero-cross-time reference value $\Delta MIN5(n)$ at the n-th sampling time.

Next, at step T72, the CPU 170 (zero-cross time decider) decides whether or not the zero-cross-time reference value at the n-1th sampling time is equal to the current zero-cross-time reference value decided at step T71. In addition, the CPU 170 decides whether or not the n-1th sampling time is a zero-cross time (third zero-cross time). More specifically, the CPU 170 decides whether or not the zero-cross-time reference value $\Delta MIN5(n-1)$ at the n-1th sampling time is equal to the current $\Delta MIN5(n)$. In addition, the CPU 170 decides whether or not a zero-cross-time reference flag $F_2(n-1)$ at the n-1th sampling time is set to plus one. When the zero-cross-time reference flag $F_2(n-1)$ is set to plus one, the n-1th sampling time is a zero-cross time. The initial value (default value) of the zero-cross-time reference flag $F_2$, i.e., the zero-cross-time reference flag $F_2(1)$ at the first sampling time, is zero.

If the decision at step T72 is affirmative, the CPU 170 sets the zero-cross-time reference flag $F_2(n)$ to zero, which means that the n-th sampling time is not a zero-cross time, and the process proceeds to step T74. If the decision at step T72 is negative, the CPU 170 decides whether or not the absolute value of the fifth difference $\Delta Z_a(n)$ at the n-th sampling time is equal to or less than 0.3 (step T73). If the decision at step T73 is negative, the CPU 170 sets the zero-cross-time reference flag $F_2(n)$ to zero, which means that the n-th sampling time is not a zero-cross time, and the process proceeds to step T74. If the decision at step T73 is affirmative, the CPU 170 sets the zero-cross-time reference flag $F_2(n)$ to plus one, which means that the n-th sampling time is a zero-cross time, and the process proceeds to step T74.

Next, the CPU 170 decides whether or not the zero-cross-time reference flag $F_2(n)$ at the n-th sampling time is plus one (step T74). If the decision at step T74 is affirmative, the CPU 170 (third centering value generator) calculates the sixth centering value $Z_{b0}$ (step T75). More specifically, the CPU 170 calculates the average of the sixth centering value $Z_{b0}(n-2)$ at the n-2th sampling time, the sixth centering value $Z_{b0}(n-1)$ at the n-1th sampling time, and the measurement value $Z_b(n)$ of the sixth bioelectrical impedance at the n-th sampling time, and decides the resulting average as the sixth centering value $Z_{b0}(n)$ at the n-th sampling time. The calculation is expressed as:

$$[Z_{b0}(n-2) + Z_{b0}(n-1) + Z_b(n)]/3 = Z_{b0}(n).$$

If the decision at step T74 is negative, the CPU 170 decides the sixth centering value $Z_{b0}(n-1)$ at the n-1th sampling time as the sixth centering value $Z_{b0}(n)$ at the n-th sampling time. This is expressed as:

$$Z_{b0}(n-1) = Z_{b0}(n).$$

Then, the CPU 170 calculates the sixth difference $\Delta Z_b(n)$ at the n-th sampling time (step T76). More specifically, the CPU 170 calculates the difference between the instantaneous measurement values $Z_b(n)$ of the sixth bioelectrical impedance and the sixth centering value $Z_{b0}(n)$, and decides the difference as the sixth difference $\Delta Z_b(n)$ at the n-th sampling time. The sixth difference calculating process at step T70 in Fig. 38 is thus completed.

When respiration of the human subject is abdominal respiration and the fifth bioelectrical impedance $Z_a$ and the sixth bioelectrical impedance $Z_b$ vary as shown in Fig. 9, the waveforms of the fifth difference $\Delta Z_a$ and the sixth difference $\Delta Z_b$ are as shown in Fig. 46. In Fig. 46, the waveform of change over time in the fifth difference $\Delta Z_a$ is described in such a manner that the fifth centering value $Z_{a0}$ that is the standard level of measurement values of the fifth bioelectrical impedance $Z_a$ is placed at zero on the Y axis, whereas the waveform of change over time in the sixth difference $\Delta Z_b$ is described in such a manner that the sixth centering value $Z_{b0}$ that is the standard level of measurement values of the sixth bioelectrical impedance $Z_b$ is placed at zero on the Y axis. When both waveforms are overlapped as in Fig. 22, the fifth difference $\Delta Z_a$ and the sixth difference $\Delta Z_b$ at inhalations can be distinguished from each other, whereas the fifth difference $\Delta Z_a$ and the sixth difference $\Delta Z_b$ at exhalations can be distinguished from each other. In this embodiment, the CPU 170 decides whether the respiratory action of the human subject is inhalation or exhalation on the basis of the fifth difference $\Delta Z_a$. More specifically, if the fifth difference $\Delta Z_a$ is positive, the CPU 170 decides that the respiratory action is inhalation. If the fifth difference $\Delta Z_a$ is negative, the CPU 170 decides that the respiratory action is exhalation. The coefficient in the above-mentioned regression formula (2) may be amended on the basis of the difference between the amplitudes of the fifth difference $\Delta Z_a$ and the sixth difference $\Delta Z_b$ and the difference between integral values of the fifth difference $\Delta Z_a$ and the sixth difference $\Delta Z_b$, so as to improve the accuracy of determination.

Returning to Fig. 38, description will be continued. As shown in Fig. 38, after the sixth difference calculating process at step T70 is completed, the CPU 170 executes a $\Delta R_{ib}/\Delta A_b$ assumption process for calculating the ratio $\Delta R_{ib}/\Delta A_b$ corresponding to the fifth difference $\Delta Z_a(n)$ and the sixth difference $\Delta Z_b(n)$ (step T80). In the following, with reference to Figs. 47 and 48, the $\Delta R_{ib}/\Delta A_b$ assumption process executed at step T80 by the CPU 170 will be described in detail. Figs. 47 and 48 form a flow chart showing an example of the $\Delta R_{ib}/\Delta A_b$ assumption process. As shown in Fig. 47, the CPU 170 first decides whether or not the fifth difference $\Delta Z_a(n)$ at the n-th sampling time is equal to or greater than zero (step T81).

If the decision at step T81 is negative, the CPU 170 decides that respiration of the human subject is exhalation, and assumes or calculates the $\Delta R_{ib}/\Delta A_b(n)$ at the n-th sampling time (step T82). More specifically, the CPU 170 executes an arithmetic process in accordance with the above-described regression formula (2) to calculate the ratio $\Delta R_{ib}/\Delta A_b(n)$ corresponding to the fifth difference $\Delta Z_a(n)$ and the sixth difference $\Delta Z_b(n)$.

If the decision at step T81 is affirmative, the CPU 170 decides that respiration of the human subject is inhalation, and sets the ratio $\Delta R_{ib}/\Delta A_b(n)$ at the n-th sampling time to an initial value. In this embodiment, if the decision at step T81 is affirmative, the CPU 170 sets the ratio $\Delta R_{ib}/\Delta A_b(n)$ at the n-th sampling time to the initial value, 1.0.

Next, the CPU 170 decides whether or not the ratio $\Delta R_{ib}/\Delta A_b(n)$ is equal to or greater than -2.5 and is equal to or less than 4.5 (step T83). If the decision at step T83 is negative, the CPU 170 sets the value of $\Delta R_{ib}/\Delta A_b$ to the initial value 1.0, and the process proceeds to step T84. If the decision at step T83 is affirmative, the CPU 170 proceeds the process directly to step T84.

At step T84, the CPU 170 decides whether or not the difference ($|\Delta R_{ib}/\Delta A_b(n)|$ - $|\Delta R_{ib}/\Delta A_b(n-1)|$) between the absolute value of the ratio $\Delta R_{ib}/\Delta A_b(n)$ and the absolute value of the ratio $\Delta R_{ib}/\Delta A_b(n-1)$ at the n-1th sampling time is greater than 0.3. If the decision at step T84 is negative, the CPU 170 calculates the average of the ratio $\Delta R_{ib}/\Delta A_b(n-1)$ at the n-1th sampling time and the ratio $\Delta R_{ib}/\Delta A_b(n)$, and decides the resulting average as the $\Delta R_{ib}/\Delta A_b(n)$ at the n-th sampling time, and the process proceeds to step T85 (see Fig. 48). The calculation is expressed as:

$$[\Delta R_{ib}/\Delta A_b(n-1) + \Delta R_{ib}/\Delta A_b(n)]/2 = \Delta R_{ib}/\Delta A_b(n). \quad \text{If the decision at step}$$

If the decision at step T84 is affirmative, the CPU 170 sets the $\Delta R_{ib}/\Delta A_b(n)$ at the n-th sampling time to the initial value 1.0, and the process proceeds to step T85.

With reference to Fig. 48, description of the $\Delta R_{ib}/\Delta A_b$ assumption process will be continued. As shown in Fig. 48, at step T85, the CPU 170 decides whether or not the fifth difference $\Delta Z_a(n)$ at the n-th sampling time is less than zero. If the decision at step T85 is affirmative, the CPU 170 decides that respiration of the human subject is exhalation, and it adds one to the last count value $N_i$ of the number of integrations. More specifically, the CPU 170 adds one to the last count value $N_i(n-1)$ of the number of integrations at the n-1th sampling time, and decides the resulting sum as the current count value $N_i(n)$ of the number of integrations at the n-th sampling time.

If the decision at step T85 is negative, the CPU 170 decides that respiration of the human subject is inhalation, and sets the count value $N_i$ of the number of integrations to an initial value of zero. That is to say, the count value $N_i(n)$ of the number of integrations at the n-th sampling time is set to zero.

Then, as shown in Fig. 48, the CPU 170 decides whether or not the fifth difference $\Delta Z_a(n)$ is less than zero, again (step T86). If the decision at step T86 is affirmative, the CPU 170 calculates the sum of the integral value ($\sum\Delta R_{ib}/\Delta A_b(n-1)$) of the ratio $\Delta R_{ib}/\Delta A_b$ at the n-1th sampling time and the ratio $\Delta R_{ib}/\Delta A_b(n)$, and decides the resulting sum as the integral value ($\sum\Delta R_{ib}/\Delta A_b(n)$) of the ratio $\Delta R_{ib}/\Delta A_b$ at the n-th sampling time. Then, the CPU 170 proceeds the process to step T87. If the decision at step T86 is negative (i.e., respiration of the human subject is inhalation), the CPU 170 sets the peak-or-bottom indication flag $F_1(n)$ to plus one, and the process proceeds to step T87.

Next, the CPU 170 decides whether or not the count value $N_i(n)$ of the number of integrations at the n-th sampling time is zero (step T87). If the decision at step T87 is negative, the CPU 170 divides the integral value $[\sum\Delta R_{ib}/\Delta A_b(n)]$ at the n-th sampling time by the count value $N_i(n)$ of the number of integrations at the n-th sampling time, so as to calculate the current average of the ratio $\Delta R_{ib}/\Delta A_b$. If the decision at step T87 is affirmative, the CPU 170 decides the last average ($[\sum\Delta R_{ib}/\Delta A_b(n-1)]/N_i(n-1)$) of the ratio $\Delta R_{ib}/\Delta A_b$ at the n-1th sampling time as the current average of the ratio $\Delta R_{ib}/\Delta A_b$ at the n-th sampling time. The $\Delta R_{ib}/\Delta A_b$ assumption process is thus completed, whereby the respiration analysis process at the n-th sampling time is thus completed.

When respiration of the human subject is abdominal respiration and the fifth difference $\Delta Z_a$ and the sixth difference $\Delta Z_b$ vary as shown in Fig. 46, the above-described average ($[\sum\Delta R_{ib}/\Delta A_b]N_i$) of the ratio $\Delta R_{ib}/\Delta A_b$ varies as shown in Fig. 35. In Fig. 35, if the average ($[\sum\Delta R_{ib}/\Delta A_b]/N_i$) of the ratio $\Delta R_{ib}/\Delta A_b$ at exhalations is equal to or less than 1.0, it is assumed that respiration of the human subject is abdominal respiration. If the average is greater than 1.0, it is assumed that respiration of the human subject is costal respiration.

As has been described above, in this embodiment, the CPU 170 executes the respiration analysis process at every sampling time, so as to calculate the ratio $\Delta R_{ib}/\Delta A_b$ corresponding to the fifth difference $\Delta Z_a$ and the sixth difference $\Delta Z_b$ at every sampling time. Accordingly, it is possible to accurately decide the type of respiration of the human subject (abdominal respiration or costal respiration) in real time.

## 4.4. RESPIRATION DEPTH DISPLAYING PROCESS

Next, a respiration depth displaying process executed by the CPU 170 will be described. In this embodiment, the CPU 170 executes the respiration depth displaying process at every respiration of the human subject for displaying (reporting) the magnitude of each of abdominal respiration and costal respiration and a margin level beyond the essential respiration depth with respect to each of abdominal respiration and costal respiration in the single respiration. More specifically, at every respiration of the human subject, on the basis of the respiration depth at the respiration and the results of the respiration analysis process at the respiration, the CPU 170 controls the display device 160 (reporter) to show the magnitude of each of costal respiration and abdominal respiration and the margin level with respect to costal respiration and abdominal respiration at the single respiration. As shown in Fig. 49, in this embodiment, in addition to the magnitude of each of costal respiration and abdominal respiration and the margin level with respect to costal respiration and abdominal respiration at the single respiration, the CPU 170 controls the display device 160 to display the abdominal respiration percentage level that is a ratio of the abdominal respiration in the single respiration. A seventh bar graph BG7 shown in Fig. 49 indicates the magnitude of each of costal respiration and abdominal respiration and the margin level with respect to costal respiration and abdominal respiration. An eighth bar graph BG8 shown in Fig. 49 indicates the abdominal respiration percentage level of the human subject. The bar graphs will be described later in more detail.

Prior to detailed description of the respiration depth displaying process, with reference to Fig. 50, a respiration depth calculating process executed by the CPU 170 will be described. Fig. 50 is a flow chart showing an example of the respiration depth calculating process executed by the CPU 170 (respiration depth calculator). The respiration depth of the human subject calculated at the respiration depth calculating process is used in the respiration depth displaying process, as will be described later.

As shown in Fig. 50, the CPU 170 first decides whether or not the current time reaches a sampling time (step T501). If the decision at step T501 is affirmative, the process proceeds to step T502. At step T502, the CPU 170 decides whether or not the respiratory action of the human subject is inhalation. Specifically, if the fifth difference $\Delta Z_a$ is positive, the CPU 170 decides that the respiratory action is inhalation. If the fifth difference $\Delta Z_a$ is negative, the CPU 170 decides that the respiratory action is exhalation. When the respiratory action is decided as inhalation, the CPU 170 sets the inhalation-or-exhalation flag $F_3$ to plus one. When the respiratory action is decided as exhalation, the CPU 170 sets the inhalation-or-exhalation flag $F_3$ to zero. In the initial status, the inhalation-or-exhalation flag $F_3$ is one as the default value.

If the decision at step T502 is affirmative (inhalation), the CPU 170 executes a peak-hold process (step T503). More specifically, the CPU 170 holds the maximum among the fifth differences $\Delta Z_a$ at multiple sampling times as a peak value $\Delta Z_a(MAX)$. If the decision at step T502 is negative (exhalation), the CPU 170 executes a bottom-hold process (step T504). More specifically, the CPU 170 holds the minimum among the fifth differences $\Delta Z_a$ at multiple sampling times as a bottom value $\Delta Z_a(MIN)$.

Next, at step T505, the CPU 170 decides whether or not the n-th sampling time is a zero-cross time. More specifically, the CPU 170 decides whether or not zero-cross-time reference flag $F_2$ at the sampling time is plus one, whereby deciding whether or not the sampling time is a zero-cross time. If the decision at step T505 is negative, the respiration depth calculating process at the sampling time ends. If the decision at step T505 is affirmative, at step T506, the CPU 170 decides whether or not the differential coefficient $dZ_a$ of the fifth bioelectrical impedance $Z_a$ at the sampling time is positive (greater than zero). In other words, the CPU 170 decides whether or not the respiratory action is changing from exhalation to inhalation at the sampling time.

If the decision at step T506 is affirmative, on the assumption that the respiratory action is changing from exhalation to inhalation at the n-th sampling time, the CPU 170 calculates the sum of the absolute values of the peak value $\Delta Z_a(MAX)$ and the bottom value $\Delta Z_a(MIN)$ held at that time, and decides the resulting sum as the both-lungs-respiration depth $Z_{ap-p}$ related to both lungs at the last respiratory action (step T507). Then, the CPU 170 executes an inhalation flag setting process (step T508). More specifically, the CPU 170 sets the inhalation-or-exhalation flag $F_3$ to plus one. Then, the CPU 170 initializes the peak-hold process (step T509). More specifically, the CPU 170 sets the peak value $\Delta Z_a(MAX)$ set at step T503 to the initial value zero, and the respiration depth calculating process ends at the sampling time.

If the decision at step T506 is negative, on the assumption that the respiratory action is changing from inhalation to exhalation at the sampling time, the CPU 170 executes an exhalation flag setting process (step T510). More specifically, the CPU 170 sets the inhalation-or-exhalation flag $F_3$ to zero. Then, the CPU 170 initializes the bottom-hold process (step T511). More specifically, the CPU 170 sets the bottom value $\Delta Z_n(MIN)$ set at step T504 to the initial value zero, and ends the respiration depth calculating process at the sampling time.

Next, with reference to Fig. 51, the respiration depth displaying process executed by the CPU 170 will be described in detail. Fig. 51 is a flow chart showing an example of the respiration depth displaying process. As shown in Fig. 51, the CPU 170 (normalizer) normalizes the respiration depth at the last single respiration (step T601). In this specification, "normalize ... the respiration depth" is meant to adjust the respiration depth at the last single respiration calculated by the respiration depth calculating process in order to exclude individual variation of physical constitutions of human subjects. Specifically, a normalized respiration depth value $\%\Delta Z_a$ is calculated as the respiration depth $\Delta Z_{ap-p}$ at the last respiration divided by the sixth centering value $Z_{b0}$ at the last respiration (more exactly, the average of the sixth centering values $Z_{b0}$ at the multiple sampling times within the last respiration) multiplied by 100. The calculation is expressed as:

$$\%\Delta Z_a = (\Delta Z_{ap-p}/Z_{b0}) * 100$$

Next, at step T602, the CPU 170 decides the number of degree indicators to be colored in the seventh bar graph BG7 on the basis of the normalized respiration depth value $\%\Delta Z_a$ calculated at step T601. The number of colored degree indicators will be referred to as a "first colored-degree-indicator number". More specifically, the CPU 170 calculates a normalized value $\%\Delta T_v$ of a one-time ventilation volume corresponding to the normalized respiration depth value $\%\Delta Z_a$ calculated at step T601, and decides the first colored-degree-indicator number on the basis of the normalized one-time ventilation volume $\%\Delta T_v$. The term "one-time ventilation volume" is meant to be a volume of air entering and leaving the lungs of the human subject in a single respiratory action, and will be referred to as $\Delta T_v$. The term "normalize a (the) one-time ventilation volume" is meant to amend a one-time ventilation volume $\Delta T_v$ in order to exclude individual variation of physical constitutions of human subjects. In this embodiment, the normalized one-time ventilation volume $\%\Delta T_v$ is calculated using a coefficient $\%V_c$ that was calculated as the breathing capacity $V_{CA}$ of the human subject measured actually by a spirometer or other suitable device divided by the normal breathing capacity $V_{CN}$. This calculation is expressed as $\%V_C = V_{CA}/V_{CN}$). As is well known, the normal breathing capacity $V_{CN}$ is (27.63 - 0.112 * age) * height (cm) for males and is (21.78 - 0.101 * age) * height (cm) for females.

Fig. 52 is a diagram showing relationships between the normalized one-time ventilation volume $\%\Delta T_V$ and the normalized respiration depth value $\%\Delta Z_a$. Fig. 52 shows results of experiments in which a one-time ventilation volume $\Delta T_V$ is measured three times (small ventilation once, middle ventilation once, and large ventilation once) for each of 20 human subjects. As shown in Fig. 29, there is a close correlative relationship between the normalized one-time ventilation volume $\%\Delta T_V$ and the normalized respiration depth value $\%\Delta Z_a$, in which the coefficient of correlation R = 0.75, and the probability P < 0.01. Regression formula (4) below is derived from the correlative relationship.

$$\%\Delta Z_a = c_0 * \%\Delta T_V \quad ... \quad (4)$$

where $c_0$ is a coefficient of regression. According to the inventor's analysis, $c_0$ is 50.954.

In accordance with the above-described regression formula (4), the CPU 170 (reporter) calculates the normalized one-time ventilation volume $\%\Delta T_V$ corresponding to the normalized respiration depth value $\%\Delta Z_a$ calculated at step T601. Then, on the basis of the calculated the normalized one-time ventilation volume $\%\Delta T_V$, the CPU 170 decides the first colored-degree-indicator number. In this embodiment, the maximum of the first colored-degree-indicator number is ten, which includes five degrees for costal respiration and five degrees for abdominal respiration (see Fig. 49).

As shown in Fig. 52, in this embodiment, when the normalized one-time ventilation volume $\%\Delta T_V$ is equal to or greater than a first predetermined value $\alpha_1$, the respiration depth of the human subject is considered to be the maximum. In this case, the CPU 170 decides the first colored-degree-indicator number as ten, which is the maximum. When the normalized one-time ventilation volume $\%\Delta T_V$ is equal to or greater than a second predetermined value $\alpha_2$, which is less than $\alpha_1$, and is less than the first predetermined value $\alpha_1$, the respiration depth of the human subject is considered to be large (see Fig. 52). In this case, the CPU 170 decides the first colored-degree-indicator number as eight. When the normalized one-time ventilation volume $\%\Delta T_V$ is equal to or greater than a third predetermined value $\alpha_3$, which is less than $\alpha_2$, and is less than the second predetermined value $\alpha_2$, the respiration depth of the human subject is considered to be in the middle (see Fig. 52). In this case, the CPU 170 decides the first colored-degree-indicator number as six. When the normalized one-time ventilation volume $\%\Delta T_V$ is equal to or greater than a fourth predetermined value $\alpha_4$, which is less than $\alpha_3$, and is less than the third predetermined value $\alpha_3$, the respiration depth of the human subject is considered to be small (see Fig. 52). In this case, the CPU 170 decides the first colored-degree-indicator number as four. When the normalized one-time ventilation volume $\%\Delta T_V$ is less than the fourth predetermined value $\alpha_4$, the respi-

ration depth of the human subject is considered to be an essential respiration depth at rest (see Fig. 52). In this case, the CPU 170 decides the first colored-degree-indicator number as two.

Returning to Fig. 51, description will be continued. As shown in Fig. 51, after the decision of the number of degree indicators to be colored (step T602), the CPU 170 decides the abdominal respiration percentage level at the last single respiration (step T603). Specifically, the CPU 170 decides a percentage value of the abdominal respiration percentage level on the basis of the ratio $\Delta R_{ib}/\Delta A_b$, at the last single respiration (more exactly, the average of the ratios $\Delta R_{ib}/\Delta A_b$ at multiple sampling times within the last single respiration). The abdominal respiration percentage level is represented as a number within a range from zero to 100. The greater the abdominal respiration percentage level, the greater the degree of abdominal respiration (the greater the ratio of abdominal respiration in respiration of the human subject).

As shown in Fig. 51, after step T603, the CPU 170 executes a degree-indicator-number distribution process for distributing the first colored-degree-indicator number decided at step T602 to abdominal respiration and costal respiration (step T604). More specifically, the CPU 170 executes the degree-indicator-number distribution process on the basis of the first colored-degree-indicator number decided at step T602 and the abdominal respiration percentage level decided at step T602. As an example, let us assume that the first colored-degree-indicator number indicating the whole respiration depth is six, whereas the abdominal respiration percentage level is 70. The abdominal respiration percentage level "70" indicates that the ratio of abdominal respiration to costal respiration is 7:3. The first colored-degree-indicator number "6" is divided by the ratio indicated by the abdominal respiration percentage level, so that four degree-indicators are allocated to abdominal respiration and two degree-indicators are allocated to costal respiration. Consequently, as shown in Fig. 26, the colored-degree-indicator number in the seventh bar graph BG7 that indicates the magnitude of costal respiration is set to two, whereas the colored-degree-indicator number in the seventh bar graph BG7 that indicates the magnitude of abdominal respiration is set to four.

As shown in Fig. 51, after step T604, in accordance with the respiration speed of the human subject, the CPU 170 (reporter) decides a margin level with respect to each of abdominal respiration and costal respiration in the last single respiration (step T605). The margin level is a respiration depth beyond the essential respiration depth at rest with respect to each of abdominal respiration and costal respiration. The essential respiration depth at rest corresponds to the respiration speed of the human subject. In this embodiment, the relationship between respiration speed and the degree of essential respiration depth at rest is described in the program for executing the respiration depth displaying process. In accordance with the relationship, on the basis of the respiration speed, the CPU 170 controls the display device 160 to indicate the degree of essential respiration depth in the seventh bar graph BG7 shown in Fig. 49.

Let us assume that the last single respiration took four seconds or more. The degree of essential respiration depth at rest is two in accordance with the relationship. The CPU 170 distributes the number two equally to abdominal respiration and costal respiration, so that one degree is allocated to abdominal respiration, whereas one degree is allocated to costal respiration, and causes the display device 160 to show the degree of essential respiration depth (one) for each of abdominal respiration and costal respiration in the seventh bar graph BG7 (as depicted by a faint color in Fig. 49). As described in conjunction with the above-described step T604, the colored-degree-indicator number in the seventh bar graph BG7 that indicates the magnitude of costal respiration is set to two, whereas the colored-degree-indicator number in the seventh bar graph BG7 that indicates the magnitude of abdominal respiration is set to four. The margin level is the respiration depth beyond the essential respiration depth. Therefore, the margin level for costal respiration is degree one, whereas that for abdominal respiration is degree three (as depicted by a deep color in Fig. 49). As a result, the display device 160 displays the margin level for each of abdominal respiration and costal respiration in the seventh bar graph BG7.

In this case, if the colored-degree-indicator number in the seventh bar graph BG7 that indicates the magnitude of abdominal respiration is one or more, the magnitude of abdominal respiration of the human subject satisfies the essential level. If the colored-degree-indicator number is two, the magnitude of abdominal respiration of the human subject has a small margin level beyond the essential level. If the colored-degree-indicator number is three, the magnitude of abdominal respiration of the human subject has a middle margin level beyond the essential level. If the colored-degree-indicator number is four, the magnitude of abdominal respiration of the human subject has a large margin level beyond the essential level. If the colored-degree-indicator number is five, the magnitude of abdominal respiration of the human subject has the maximum margin level beyond the essential level. As described above, in the assumed example, since the colored-degree-indicator number in the seventh bar graph BG7 that indicates the magnitude of abdominal respiration is four, the magnitude of abdominal respiration of the human subject has a large margin level beyond the essential level (faint colored-degree-indicator number one). The same is true for costal respiration. In the assumed example, since the colored-degree-indicator number in the seventh bar graph BG7 that indicates the magnitude of costal respiration is two, the magnitude of costal respiration of the human subject has a small margin level beyond the essential level (faint colored-degree-indicator number one).

The higher the respiration speed, the greater the essential respiration depth at rest. This means that when the respiration speed or rate is higher, the degree of essential respiration depth for each of abdominal respiration and costal respiration depicted by a faint color in the seventh bar graph BG7 is greater. For example, if the cycle of respiration is equal to or greater than three seconds and is less than four seconds, the degree of essential respiration depth for each of abdominal respiration and costal respiration depicted by a faint color in the seventh bar graph BG7 is four. The CPU 170 distributes the number four equally to abdominal respiration and costal respiration, so that two degree-indicators are allocated to abdominal respiration, whereas two degree-indicators are allocated to costal respiration, and causes the display device 160 to show the degree of essential respiration depth (two) in a faint color for each of abdominal respiration and costal respiration in the seventh bar graph BG7. The margin level for each of costal respiration and abdominal respiration is based on the thus allocated degree of essential respiration depth.

After step T605, the CPU 170 causes the display device 160 to show the magnitude of each of costal respiration and abdominal respiration and the margin level with respect to costal respiration and abdominal respiration in the seventh bar graph BG7, and to show the abdominal respiration percentage level in the second bar graph BG2 (step T606). As shown in Fig. 49, since the degree of essential respiration depth and the margin level are depicted in different colors, the human subject or other person can easily understand the magnitude of each of abdominal respiration and costal respiration and the margin level beyond the essential respiration depth of the human subject.

As shown in Fig. 49, the abdominal respiration percentage level depicted in the second bar graph BG2 is classified into five degrees corresponding to five sections. According to the abdominal respiration percentage level calculated at the above-described step T603, one of the five sections in the second bar graph BG2 is colored. Specifically, if the abdominal respiration percentage level is from zero to 20, the CPU 170 causes the display device 160 to color the uppermost section. If the abdominal respiration percentage level is from 21 to 40, the CPU 170 causes the display device 160 to color the second top section. If the abdominal respiration percentage level is from 41 to 60, the CPU 170 causes the display device 160 to color the middle section. If the abdominal respiration percentage level is from 61 to 80, the CPU 170 causes the display device 160 to color the second bottom section. If the abdominal respiration percentage level is from 81 to 100, the CPU 170 causes the display device 160 to color the lowermost section. As described above, in the illustrated example, the abdominal respiration percentage level is 70, and the second bottom section in the second bar graph BG2 is colored as shown in Fig. 49.

As has been described above, in this embodiment, the CPU 170 causes the display device 160 to show the magnitude of each of abdominal respiration and costal respiration and a margin level beyond the essential respiration depth with respect to each of abdominal respiration and costal respiration at every respiration of the human subject. Accordingly, the human subject or another person can understand strengths and weaknesses of activity of the costal respiratory muscles and abdominal respiratory muscles of the human subject. Whereas the human subject is made aware of the strength of the human subject, the human subject may be motivated to train the respiratory muscles by, for example, voluntary abdominal respiration, in order to overcome a weakness. In accordance with this embodiment, the margin level of the respiration capability of the human subject can be known even if the human subject does not breathe at the maximum respiration depth, in contrast to use of spirometers. Therefore, the use of the body condition determination apparatus is safer for human subjects than the use of spirometers.

5. FIFTH EMBODIMENT

In addition to costal respiration and abdominal respiration, respiration of human beings includes a draw-in respiration in which inhalation and exhalation are repeated with the abdomen held in a constricted position. By draw-in respiration, inner muscles at the body trunk (e.g., the transverse abdominal muscle and the erector muscle of the spine) that are not frequently used in normal daily activity can be toned up effectively. Strengthening the inner muscles improves the function of respiration, and strengthening muscles at the body trunk supporting the backbone improves the motor function. Accordingly, draw-in respiration has been incorporated into training of athletes in order to improve motion function. In addition, draw-in respiration has been recommended for improvement or prevention of backaches in the fields of physical therapy and rehabilitation. Draw-in respiration is also effective for dieting.

In accordance with a modification of the body condition determination apparatus 1 of the fourth embodiment, it is possible to determine that the type of respiration of the human subject is costal respiration, abdominal respiration, or draw-in respiration. This modification will be described in a fifth embodiment. The structure of the body condition determination apparatus in the fifth embodiment is the same as that in the first embodiment, and therefore the same elements as in the first embodiment will not be described in detail and the same reference symbols for such elements will be used in the following description.

Fig. 53 is a graph showing change over time of each of the fifth bioelectrical impedance $Z_a$ at the upper body trunk and the sixth bioelectrical impedance $Z_b$ at the middle body trunk when respiration is draw-in respiration. As shown in Fig. 53, in draw-in respiration, both of the fifth bioelectrical impedance $Z_a$ and the sixth bioelectrical impedance $Z_b$ increase at inhalations, whereas both of the fifth bioelectrical impedance $Z_a$ and the sixth bioelectrical impedance $Z_b$ decrease at exhalations. The manner of change is the same as that in costal respiration since human beings expand and contract the thoracic cage in both of costal respiration and draw-in respiration. However, in draw-in respiration, the abdomen is held in a constricted position continually so as to be stressed continually. Therefore, the standard level of the sixth bioelectrical impedance $Z_b$ at the middle body trunk in draw-in respiration is higher than that in costal respiration as shown in Fig. 53.

Thus, it is possible to determine whether or not respiration of the human subject is draw-in respiration on the basis of the difference in the standard level of the sixth bioelectrical impedance $Z_b$ between draw-in respiration and costal respiration. In the first embodiment, by the respiration analysis process (Fig. 38) described in conjunction with the first embodiment, the type of respiration of the human subject is determined as costal or abdominal on the basis of the average of the ratio $\Delta R_{ib}/\Delta A_b$. If respiration is determined as costal as the average of the ratio $\Delta R_{ib}/\Delta A_b$ is greater than one, determination is again made as to whether the type of respiration is actually costal respiration or is draw-in respiration. More specifically, if the standard level (second centering value $Z_{b0}$) of the sixth bioelectrical impedance $Z_b$ is higher than the standard level in costal respiration by a predetermined value or more, respiration of the human subject is draw-in respiration.

Each human subject may have a different standard level (second centering value $Z_{b0}$) of the sixth bioelectrical impedance $Z_b$ in costal respiration. Accordingly, it is preferable that, in order to distinguish draw-in respiration, the standard level (second centering value $Z_{b0}$) of the sixth bioelectrical impedance $Z_b$ in costal respiration be determined in advance for the human subject. More preferably the standard level in costal respiration may be stored in the second memory 130. In addition, the above-described predetermined value should be stored in the first memory 120 in advance.

Accordingly, the CPU 170 of the body condition determination apparatus 1 in this embodiment reports a message for instructing the human subject to repeat costal respiration, and calculates the average of multiple sixth centering values $Z_{b0}$ during the repetition of costal respiration. The CPU 170 stores the average as the standard level of the sixth bioelectrical impedance $Z_b$ in the second memory 130. Hereinafter, the standard level of the sixth bioelectrical impedance $Z_b$ stored in the second memory 130 for costal respiration will be referred to as $Z_{bl}$.

On the other hand, the above-described predetermined value to be stored in the first memory 120 can be determined in accordance with experiments in which the standard level of the sixth bioelectrical impedance $Z_b$ for each of many human subjects in costal respiration is determined, and the standard level of the sixth bioelectrical impedance $Z_b$ for each of many human subjects in draw-in respiration is also determined. The predetermined value corresponds to the difference between the standard level in costal respiration and the standard level in draw-in respiration. Hereinafter, the predetermined value stored in the first memory 120 will be referred to as $\Delta Z_{b1}$.

Fig. 54 is a flow chart showing an example of a respiration type determination process. This process is executed after completion of the respiration analysis process described in conjunction with the fourth embodiment. In a manner similar to the fourth embodiment, in this embodiment, the CPU 170 also executes the respiration analysis process shown in Fig. 38 at each sampling time.

Then, the CPU 170 starts the respiration type determination process. The CPU 170 first decides whether or not the average of $\Delta R_{ib}/\Delta A_b$ ($[\Sigma \Delta R_{ib}/\Delta A_b]/N_i$) is equal to or less than 1.0 (step T701). If the decision at step T701 is affirmative, the CPU 170 determines that respiration of the human subject is abdominal respiration (step T702).

If the decision at step T701 is negative, the CPU 170 retrieves the standard level $Z_{b1}$ of the sixth bioelectrical impedance $Z_b$ in costal respiration from the second memory 130, and retrieves the predetermined value $\Delta Z_{b1}$ from the first memory 120 (step T703). At this step, the predetermined value $\Delta Z_{bl}$ retrieved from the first memory 120 may be amended on the basis of the personal or individual body information (including the height, age, and sex) entered at step T1 (Fig. 37) or the weight measured at step T2 (Fig. 37).

Then, the CPU 170 decides whether or not the sixth centering value $Z_{b0}$ generated at step T70 in the sixth difference calculating process for calculating the sixth difference $\Delta Z_b$ is equal to or greater than the sum of the standard level $Z_{b1}$ and the predetermined value $\Delta Z_{b1}$ (step T704). The sixth centering value $Z_{b0}$ used in the comparison at step T704 may be, for example, the average of the sixth centering values $Z_{b0}$ within the last single respiration or within the last multiple respirations. If the decision at step T704 is affirmative, the CPU 170 determines that respiration of the human subject is draw-in respiration (step T705). If the decision at step T704 is negative, the CPU 170 determines that respiration of the human subject is costal respiration (step T706).

As has been described above, according to this embodiment, it is possible to determine accurately that the type of respiration of the human subject is abdominal respiration, costal respiration, or draw-in respiration in real time. In an alternative embodiment, the body condition determination apparatus 1 may determine whether or not respiration of the human subject is draw-in respiration (and need not determine that the type of respiration of the human subject is abdominal or costal).

In this embodiment, the standard level $Z_{b1}$ of the sixth bioelectrical impedance $Z_b$ in costal respiration stored in the second memory 130 is the average of a plurality of sixth centering value $Z_{b0}$ determined during costal respiration. However, the present invention should not be limited to the disclosure. For example, the standard level $Z_{b1}$ may be a sixth bioelectrical impedance $Z_b$ determined during costal respiration.

At step T703, the CPU 170 compares the sixth centering value $Z_{b0}$ with the sum of the standard level $Z_{b1}$ and the predetermined value $\Delta Z_{b1}$. However, the present invention should not be limited to the disclosure. For example, the CPU 170 may multiply the standard level $Z_{b1}$ by a predetermined coefficient (e.g., 1.035) greater than 1.0, and then may determine whether or not the sixth centering value $Z_{b0}$ is equal to or greater than the standard level $Z_{b1}$ multiplied by the coefficient. The coefficient can also be determined in accordance with experiments in which the standard level of the sixth bioelectrical impedance $Z_b$ for each of many human subjects in costal respiration is determined, and the standard level of the sixth bioelectrical impedance $Z_b$ for each of many human subjects in draw-in respiration is also determined. The coefficient corresponds to the ratio between the standard level in costal respiration and the standard level in draw-in respiration. The value obtained as the standard level $Z_{b1}$ multiplied by the coefficient may be stored in the second memory 130 instead of the standard level $Z_{b1}$ per se, and at step T703 the CPU 170 may compare the sixth centering value $Z_{b0}$ with the value retrieved from the second memory 130.

The respiration depth calculating process and the respiration depth displaying process described in conjunction with the fourth embodiment are executed irrespective to the type of respiration of the human subject. That is to say, they are executed even if the human subject performs draw-in respiration. As a result, even in draw-in respiration, the seventh bar graph BG7 and the eighth bar graph BG8 shown in Fig. 49 are shown on the display device 160. The human subject or another person can know the magnitude of draw-in respiration of the human subject by observing the degree indicators indicative of magnitude of costal respiration in the seventh bar graph BG7.

The fifth embodiment may be modified as follows.

5.1. FIRST MODIFICATION OF FIFTH EMBODIMENT

As shown in Fig. 53, in draw-in respiration, only the standard level of the sixth bioelectrical impedance $Z_b$ at the middle body trunk increases in comparison with that in costal respiration and abdominal respiration. Accordingly, the CPU 170 may continually monitor the fifth centering value $Z_{a0}$ that is the standard level of the fifth bioelectrical impedance $Z_a$ at the upper body trunk and the sixth centering value $Z_{b0}$ that is the standard level of the sixth bioelectrical impedance $Z_b$ at the middle body trunk, and it may determine that the last respiration is draw-in respiration when only the sixth centering value $Z_{b0}$ increases by a predetermined value (e.g., 0.5 ohms).

5.2. SECOND MODIFICATION OF FIFTH EMBODIMENT

As shown in Fig. 53, in draw-in respiration, both of the amplitudes (local maximum-local minimum) of the fifth bioelectrical impedance $Z_a$ and the sixth bioelectrical impedance $Z_b$ are less than those in costal respiration and abdominal respiration. In order to identify draw-in respiration on the basis of the amplitudes, thresholds for both amplitudes may be determined and stored in the first memory 120. The CPU 170 may continually monitor the amplitudes of the fifth bioelectrical impedance $Z_a$ and the sixth bioelectrical impedance $Z_b$, and may determine that the last respiration is draw-in respiration when both amplitudes are less than the thresholds (e.g., 1.8 ohms) and when only the sixth centering value $Z_{b0}$ increases by a predetermined value (in a manner similar to the first modification). The thresholds may be different from each other, or they may be the same as each other, so that a single common threshold may be stored in the first memory 120.

5.3. THIRD MODIFICATION OF FIFTH EMBODIMENT

In draw-in respiration, both of the fifth bioelectrical impedance $Z_a$ and the sixth bioelectrical impedance $Z_b$ increase at inhalations, whereas both of the fifth bioelectrical impedance $Z_a$ and the sixth bioelectrical impedance $Z_b$ decrease at exhalations, in a manner similar to that in costal respiration. Accordingly, the CPU 170 may continually monitor the fifth bioelectrical impedance $Z_a$ and the sixth bioelectrical impedance $Z_b$, and may determine that the last respiration is draw-in respiration when both of the fifth bioelectrical impedance $Z_a$ and the sixth bioelectrical impedance $Z_b$ increase at inhalations, whereas both of the fifth bioelectrical impedance $Z_a$ and the sixth bioelectrical impedance $Z_b$ decrease at exhalations and when only the sixth centering value $Z_{b0}$ increases by a predetermined value (in a manner similar to the first modification).

6. SIXTH EMBODIMENT

A Lissajous figure showing the status of breathing of the human subject, e.g., change over time in each of the fifth bioelectrical impedance $Z_a$ and the sixth bioelectrical impedance $Z_b$ is a convenient expedient for reporting whether respiration of the human is mainly dependent on costal respiration or abdominal respiration. When respiration of the human subject is costal respiration, as shown in Fig. 10, both the fifth bioelectrical impedance $Z_a$ and the sixth bioelectrical impedance $Z_b$ increase at inhalations, whereas both the fifth bioelectrical impedance $Z_a$ and the sixth bioelectrical impedance $Z_b$ decrease at exhalations. Therefore, the Lissajous figure showing this case within a single respiration is, for example, as shown in Fig. 55. That is to say, when the ratio of costal respiration in a single respiration is extremely high, the Lissajous figure indicates a substantially straight track.

When respiration of the human subject is abdominal respiration, as shown in Fig. 9, both of the fifth bioelectrical impedance $Z_a$ and the sixth bioelectrical impedance $Z_b$ increase at inhalations, but the fifth bioelectrical impedance $Z_a$ decreases, whereas the sixth bioelectrical impedance $Z_b$ increases at exhalations. Therefore, the Lissajous figure showing this case within a single respiration is, for example, as shown in Fig. 56. That is to say, when a single respiration includes abdominal respiration, the Lissajous figure indicates a track of a bent shape, e.g., a boomerang shape.

Accordingly, by presenting a Lissajous figure showing the status of the last respiration of the human subject, the human subject or another person can easily understand whether respiration of the human is costal respiration or abdominal respiration. In this case, it is not necessary to execute the above-described respiration analysis process, and the type of respiration of the human subject can be assumed in a simple fashion. In addition, the human subject can cause the human subject's respiration to resemble costal respiration by breathing so that the human subject's Lissajous figure indicates a track resembling a straight line. Accordingly, presenting a Lissajous figure is used as biofeedback information for training for appropriate breathing.

The Lissajous figures of Figs. 55 and 56 show only the status of the last single respiration of the human subject. However, it is possible to generate Lissajous figures showing the status of multiple respirations on the basis of data on fifth bioelectrical impedances $Z_a$ and the sixth bioelectrical impedances $Z_b$ in the multiple respirations as shown in Figs. 57 and 58. When the ratio of costal respiration in multiple respirations is high (respiration of the human is mainly dependent on costal respiration), the Lissajous figure is, for example, as shown in Fig. 57. When multiple respirations include abdominal respiration, the Lissajous figure is, for example, as shown in Fig. 58. In addition, it is possible to change to display the Lissajous figure at every respiration of the human subject in order to show the instantaneous status cyclically.

A sixth embodiment for displaying a Lissajous figure will be described. The structure of the body condition determination apparatus in the sixth embodiment is the same as that in the first embodiment, and therefore the same elements as in the first embodiment will not be described in detail and the same reference symbols for such elements will be used in the following description.

6.1. DISPLAYING NORMAL LISSAJOUS FIGURE

Fig. 59 is a flow chart showing an example of a Lissajous figure displaying process. In the flow chart, the processes of steps S801 through S804 are the same as those in the above-described fourth embodiment (steps T10 through T40 in Fig. 38), and will not be described in detail.

After step T804, the CPU 170 (display data generator) generates display data for displaying a Lissajous figure (step T805). In the Lissajous figure, for example, the X axis is the sixth bioelectrical impedance $Z_b$, whereas the Y axis is the fifth bioelectrical impedance $Z_a$. The second memory 130 has a Lissajous figure memory area for temporarily storing the display data for displaying a Lissajous figure in the display device 160.

The display data represents coordinates of dots to be shown in a plane of the Lissajous figure, in which each dot has an x-coordinate indicating a measurement value of the sixth bioelectrical impedance $Z_b$ at one time and a y-coordinate indicating a measurement value of the fifth bioelectrical impedance $Z_a$ at that time. Whenever a new dot position is determined, the CPU 170 updates the display data in order to update the track in the Lissajous figure.

The CPU 170 retrieves the display data from the Lissajous figure memory area, and causes the display device 160 to show the Lissajous figure indicated by the display data (step T806). The Lissajous figure displaying process is executed at every sampling time, so that the display data is updated at every sampling time and the Lissajous figure displayed on the display device 160 is also updated at every sampling time.

The display device 160 thus shows the Lissajous figure in real time insofar as the human subject performs respiration. When the ratio of costal respiration in respiration is extremely high, the display device 160 will show a Lissajous figure similar to that in Fig. 55 or 57. When respiration includes abdominal respiration, the display device 160 will show a Lissajous figure similar to that in Fig. 56 or 58. When respiration of the human subject is changing from costal to abdominal, the display device 160 will show a Lissajous figure similar to that in Fig. 60, in which the track of the Lissajous figure is changing from a straight shape rising from bottom left to top right to a bent shape of which the lower portion is downward-sloping.

As shown in Figs. 55 and 57, when the ratio of costal respiration in respiration is extremely high, the track of the Lissajous figure is of a straight shape rising from bottom left to top right. When costal respiration is shallow, the track of the Lissajous figure is small. When costal respiration is deep, the track of the Lissajous figure is large.

As shown in Figs. 56 and 58, when respiration includes abdominal respiration, the track of the Lissajous figure is of a bent shape. The Lissajous figure shown in Fig. 56 indicates a case in which 50% of a single respiration is costal and 50% of the single respiration is abdominal. In this case, the track of the Lissajous figure is of a boomerang shape (an L-shape) that is symmetric with respect to a horizontal line. However, if the percentage of abdominal respiration is less than that of costal respiration, the upper upward-sloping portion of the track of the Lissajous figure corresponding to costal respiration is larger than that in Fig. 56, whereas the lower downward-sloping portion of the track of the Lissajous figure corresponding to abdominal respiration is smaller than that in Fig. 56. If the percentage of abdominal respiration is greater than that of costal respiration, the upper upward-sloping portion of the track of the Lissajous figure corresponding to costal respiration is smaller than that in Fig. 56, whereas the lower downward-sloping portion of the track of the Lissajous figure corresponding to abdominal respiration is larger than that in Fig. 56. Thus, depending on the percentage of abdominal respiration, the track of the Lissajous figure describes various tracks.

Theoretically, when abdominal respiration occupies 100% of respiration, the track of the Lissajous figure is of an inclined straight shape in which the inclination is opposite to that in costal respiration (downward sloping in the coordinate system in Fig. 56). However, respiration of human beings must include costal respiration except for persons for whom the diaphragms do not work at all due to a disorder (e.g., a disease). This can be confirmed by observing that even if a human stops breathing, when the human expands and contracts the abdomen, the diaphragm moves up and down so as to expand and contract the lungs. Accordingly, even if the human subject performs abdominal respiration as much as possible, the track of the Lissajous figure is of a bent shape having a downward-sloping straight portion corresponding to costal respiration.

The bend angle AG formed between the downward-sloping straight portion (approximate straight line LN1) corresponding to costal respiration and the upward-sloping straight portion (approximate straight line LN2) corresponding to abdominal respiration shown in Fig. 56 is small when abdominal respiration is shallow. When abdominal respiration is deep, the bend angle AG is large. In addition, the shallower the abdominal respiration, the smaller the track of the Lissajous figure.

Thus, the track of the Lissajous figure varies depending on whether or not respiration is costal or abdominal. The size and the shape of the track of the Lissajous figure vary depending on the magnitude (depth) of each of the magnitude (depth) of each of costal respiration and abdominal respiration. By observing the Lissajous figure, the human subject or another person can understand whether current respiration of the human subject is costal or abdominal, or can understand whether respiration of the human subject is mainly dependent on costal respiration or abdominal respiration. The human subject or another person can also understand the magnitude of each of costal respiration and abdominal respiration by the Lissajous figure. Accordingly, the body condition determination apparatus 1 can be used as a breathing training apparatus.

When the human subject trains for costal breathing, the human subject may pay attention to the Lissajous figure so that the track of the Lissajous figure becomes a straight shape rising from bottom left to top right and the size of the track becomes large. When the human subject trains for abdominal breathing, the human subject may pay attention to the Lissajous figure so that the track of the Lissajous figure is of a bent shape, and the size and the bend angle AG become large. Thus, by observing the Lissajous figure and confirming the type and the magnitude of breathing at any time, the human subject can train for appropriate costal or abdominal breathing.

When respiration of the human subject is draw-in respiration, as described above with reference to Fig. 53 in conjunction with the fifth embodiment, both the fifth bioelectrical impedance $Z_a$ and the sixth bioelectrical impedance $Z_b$ increase at inhalations, whereas both the fifth bioelectrical impedance $Z_a$ and the sixth bioelectrical impedance $Z_b$ at the middle body trunk decrease at exhalations. The manner of change is the same as that in costal respiration since human beings expand and contract the thoracic cage in both of costal respiration and draw-in respiration. However, in draw-in respiration, the abdomen is held in a constricted position continually so as to be stressed continually. Therefore, the standard level of the sixth bioelectrical impedance $Z_b$ at the middle body trunk in draw-in respiration is higher than that in costal respiration as shown in Fig. 53. For this reason, as in Fig. 68 which shows Lissajous figures in draw-in respiration and costal respiration, the two tracks of the Lissajous figures are of a straight shape rising from bottom left to top right, but locations of both tracks are different in the axis indicating the sixth bioelectrical impedance $Z_b$ at the middle body trunk (the X axis in Fig. 68).

Thus, by observing the Lissajous figure, the human subject or another person can understand whether or not respiration of the human subject is draw-in respiration. The shallower the draw-in respiration, the smaller is the track of the Lissajous figure, so that the magnitude of draw-in respiration can be understood from the Lissajous figure. By observing the Lissajous figure and confirming the type and the magnitude of respiration at any time, the human subject can train for appropriate draw-in breathing.

As has been described above, by virtue of displaying the Lissajous figure, the human subject or another person can understand the type and magnitude of respiration of the human subject, and can understand whether or not the human subject is performing appropriately the target type of breathing. Accordingly, the human subject can train for breathing effectively.

By an effective training for breathing, respiratory muscles (for example, the transverse abdominal muscle, the diaphragm, the internal and external intercostal muscles, the sternomastoid muscle, the anterior scalene muscle, the middle scalene muscle, the posterior scalene muscle, the abdominal rectus muscle, the internal and external abdominal oblique muscles, etc.) can be toned up effectively. In particular, the transverse abdominal muscle is a body trunk muscle that significantly influences not only respiration, but also motion functions. By draw-in respiration, not only respiratory muscles, but also inner muscles at the body trunk (e.g., the erector muscle of the spine) can be strengthened effectively.

For this reason, training for breathing enhances not only respiratory functions, but also motion functions, and is effective for improvement or prevention of backache and for dieting.

In addition, training for breathing improves mental health. For example, deep breathing (deep abdominal respiration) or respiration in which time of exhalation is longer than that of inhalation improves the parasympathetic action and promotes relaxation.

The body condition determination apparatus 1 of this embodiment determines the fifth bioelectrical impedance $Z_a$ and the sixth bioelectrical impedance $Z_b$, and displays a Lissajous figure indicating change over time of each of the impedances. Accordingly, among the respiration analysis process (Fig. 38) described in conjunction with the fourth embodiment, it is not necessary to execute the fifth centering process (step T50), the fifth difference calculating process (step T60), the sixth difference calculating process (step T70), and the $\Delta R_{ib}/\Delta A_b$ assumption process (step T80). The entire process in the body condition determination apparatus 1 can thus be simplified. However, it is possible to execute these steps even in this embodiment.

Training for breathing includes, for example, rehabilitation of respiratory function for patients with respiratory disease or for human subjects with exacerbated respiratory function, training of athletes for improving motion function, and training of physically unimpaired persons for strengthening respiratory functions or mental health and for improving respiratory functions that are deteriorated by smoking, lifestyle, lack of activity, or aging.

In the Lissajous figures in Figs. 55 through 58 and 60, the X axis is the sixth bioelectrical impedance $Z_b$ whereas the Y axis is the fifth bioelectrical impedance $Z_a$, but the X axis and the Y axis may be replaced with each other. For example, Fig. 61 is a Lissajous figure for abdominal respiration corresponding to Fig. 59, in which the X axis is the fifth bioelectrical impedance $Z_a$, whereas the Y axis is the sixth bioelectrical impedance Z. Even if the X axis and the Y axis are replaced, the track of the Lissajous figure is of a bent shape. In a Lissajous figure in which the X axis and the Y axis are thus replaced (e.g., Fig. 62 corresponding to Fig. 60), when respiration of the human subject is changing from costal to abdominal, the track of the Lissajous figure is changing from a straight shape rising from bottom left to top right to a bent shape of which the left portion is upward-sloping. In addition, the X axis and the Y axis of a Lissajous figure may be inclined at 45 degrees as shown in Fig. 73. In summary, the Lissajous figure may be represented in any type of orthogonal coordinate system having two orthogonal coordinate axes in which one axis is the fifth bioelectrical impedance $Z_a$ and the other axis is the sixth bioelectrical impedance $Z_b$.

6.2. DISPLAYING LISSAJOUS FIGURES FOR THE RIGHT LUNG AND THE LEFT LUNG

In order to recognize the difference between the respiration capabilities of the right lung and the left lung, the body condition determination apparatus 1 may display the Lissajous figures for the right lung and the left lung. Fig. 59A is a flow chart showing a Lissajous figure displaying process in this case, the Lissajous figure displaying process being a variation shown in Fig. 59. In order to generate the display data for the Lissajous figure for the right lung, the CPU 170 (bioelectrical impedance determiner) may determine the first bioelectrical impedance $Z_{aR}$ at the right upper body trunk and the third bioelectrical impedance $Z_{bR}$ at the right middle body trunk (steps T802A and T803A). In order to generate the display data for the Lissajous figure for the left lung, the CPU 170 may determine the second bioelectrical impedance $Z_{aL}$ at the left upper body trunk and the fourth bioelectrical impedance $Z_{bL}$ at the left middle body trunk. Consequently, in order to generate the display data for two Lissajous figures for both of the right lung and the left lung, it is preferable to determine the first bioelectrical impedance $Z_{aR}$, the second bioelectrical impedance $Z_{aL}$, the third bioelectrical impedance $Z_{bR}$, and the fourth bioelectrical impedance $Z_{bL}$.

For displaying two Lissajous figures for the right lung and the left lung, the CPU 170 (bioelectrical impedance determiner) controls switching selection of the current electrodes X1 through X4 and the voltage electrodes Y1 through Y4, and determines the first bioelectrical impedance $Z_{aR}$, the second bioelectrical impedance $Z_{aL}$, the third bioelectrical impedance $Z_{bR}$, and the fourth bioelectrical impedance $Z_{bL}$ at steps T802 and T803 of the Lissajous figure displaying process (Fig. 59).

The CPU 170 executes a smoothing process for measurement values of the first bioelectrical impedance $Z_{aR}$ and for measurement values of the third bioelectrical impedance $Z_{bR}$ (step T804A), and executes a smoothing process for measurement values of the second bioelectrical impedance $Z_{aL}$ and for measurement values of the fourth bioelectrical impedance $Z_{bL}$ (step T804B). At step T805A, the CPU 170 generates display data for displaying a Lissajous figure for the right lung, in which, for example, the X axis is the third bioelectrical impedance $Z_{bR}$, whereas the Y axis is the first bioelectrical impedance $Z_{aR}$. At step T805B, the CPU 170 generates display data for displaying a Lissajous figure for the left lung, in which, for example, the X axis is the fourth bioelectrical impedance $Z_{bL}$, whereas the Y axis is the second bioelectrical impedance $Z_{aL}$. Then, at step T806, the CPU 170 supplies the display data to the display device 160 for causing the display device 160 to show the two Lissajous figures for the right lung and the left lung.

In this case, since two Lissajous figures for the right lung and the left lung are displayed, the type and the magnitude of respiration with respect to the right lung and the left lung can be understood. By comparing two Lissajous figures, it is possible to easily understand the difference between the respiration capabilities of the right lung and the left lung. In addition, it is possible to train for breathing of the right lung and the left lung, respectively. There is no significant difference between the respiration capabilities of the right lung and the left lung of a physically unimpaired person. However, if one of the right lung and the left lung has been diseased, there is a significant difference between the respiration capabilities of the right lung and the left lung. If one of the right lung and the left lung was previously diseased, there may be a difference between the respiration capabilities of the right lung and the left lung. A method for improving the respiration capability of only the left lung is in which the human subject repeats respiration while a load is applied to the left lung by positioning the left arm behind the right shoulder and pushing the left elbow backward with the right hand. This method is suitable for, for example, a person whose respiration capability of the left lung is lower than the respiration capability of the right lung.

Two Lissajous figures for the right lung and the left lung may be arranged next to each other on the display device 160. However, in order to facilitate understanding of the differences between the respiration capabilities of the right lung and the left lung, it is preferable to overlay the Lissajous figures one on the other as shown in Fig. 63. The CPU 170 may cause the Lissajous figures for the right lung and the left lung to be overlaid one on the other on a single screen.

When two Lissajous figures for the right lung and the left lung are overlaid, in order to distinguish the Lissajous figures, it is preferable that the manner for displaying the Lissajous figure for the right lung be different from that for the left lung. For example, the CPU 170 may indicate the color of the Lissajous figure for the right lung is blue and the color of the Lissajous figure for the left lung is red. Alternatively, the CPU 170 may allocate different line thicknesses or line types (e.g., solid line or dotted line) to two Lissajous figures for the right lung and the left lung. Even if two Lissajous figures for the right lung and the left lung may be arranged next to each other, it is possible that the manner for displaying the Lissajous figure for the right lung will be different from that for the left lung.

In addition, as shown in Fig. 64, the difference between two Lissajous figures for the right lung and the left lung may be highlighted. At each sampling time, the CPU 170 compares the coordinates $(X_R, Y_R)$ of the instantaneous dot of the Lissajous figure for the right lung, in which the x-coordinate indicates the instantaneous measurement value of the third bioelectrical impedance $Z_{bR}$ and the y-coordinate indicates the instantaneous measurement value of the first bioelectrical impedance $Z_{aR}$ with the coordinates $(X_L, Y_L)$ of the instantaneous dot of the Lissajous figure for the left lung, in which the x-coordinate indicates the instantaneous measurement value of the fourth bioelectrical impedance $Z_{bL}$ and the y-coordinate indicates the instantaneous measurement value of the second bioelectrical impedance $Z_{aL}$. If the dot positions represented by the coordinates $(X_R, Y_R)$ and the coordinates $(X_L, Y_L)$ are different, the CPU 170 generates additional display data representing a bar (straight line) connecting the positions. The difference between two Lissajous figures for the right lung and the left lung can be highlighted by the bars, so that it is possible to recognize the difference between the respiration capabilities of the right lung and the left lung.

The CPU 170 may allocate different colors to the bars at exhalation and the bars at inhalation. As shown in Fig. 24, the first bioelectrical impedance $Z_{aR}$ is higher than the first centering value $Z_{aR0}$ at inhalations, and is lower than that at exhalations. Accordingly, when the average of the first centering value $Z_{aR0}$ at the last respiration is as indicated by the horizontal straight line (two-dot chain line) in Fig. 64, it is possible to use different colors for above and below the straight line. However, in order to allocate different colors to the bars at exhalation and the bars at inhalation, the first centering process described in conjunction with the first embodiment should be executed for the first bioelectrical impedance $Z_{aR}$. Alternatively, instead of the right first bioelectrical impedance $Z_{aR}$, the second centering process described in conjunction with the first embodiment should be executed for the second bioelectrical impedance $Z_{aL}$.

The CPU 170 may also allocate different colors to the bars on the basis of whether x-coordinate $X_R$ minus x-coordinate $X_L$ is positive or negative at each sampling time. The CPU 170 may also allocate different colors to the bars on the basis of whether y-coordinate $Y_R$ minus y-coordinate $Y_L$ is positive or negative at each sampling time.

The CPU 170 may change the tone of the color of the bars depending on the distance between the position $(X_R, Y_R)$ and the position $(X_L, Y_L)$. For example, if the distance is greater, a deeper color may be used.

Instead of displaying the bars, the area defined between the two Lissajous figures may be painted by a faint color.

The CPU 170 may calculate the differential area between two Lissajous figures for the right lung and the left lung. The differential area indicates the difference between the respiration capabilities of the right lung and the left lung. On the basis of the magnitude of the differential area, the CPU 170 may classify the difference between the respiration capabilities into multiple rankings. The CPU 170 may use the sum of the lengths of the bars indicating the difference instead of the differential area for classifying the difference between the respiration capabilities into multiple rankings.

As shown in Fig. 65, the CPU 170 may calculate the coordinates of the median position between the position $(X_R, Y_R)$ and the position $(X_L, Y_L)$, and may plot the dot of the median position at each sampling time, thereby displaying the median track of two Lissajous figures for the right lung and the left lung.

The CPU 170 may highlight the difference between two Lissajous figures by indicating the different part in the Lissajous figure for the right lung in a thick line and by indicating the different part in the Lissajous figure for the left lung in a thick line.

The X axis and the Y axis may be replaced with each other in the Lissajous figures for the right lung and the left lung. In summary, the Lissajous figure may be represented in any type of orthogonal coordinate system having two orthogonal coordinate axes in which one axis is the first bioelectrical impedance $Z_{aR}$ or the second bioelectrical impedance $Z_{aL}$ and the other axis is the third bioelectrical impedance $Z_{bR}$ or the fourth bioelectrical impedance $Z_{bL}$.

Although Fig. 63 through Fig. 65 show Lissajous figures representing abdominal respiration, Lissajous figures may be displayed representing costal respiration or draw-in respiration.

Although two Lissajous figures are displayed in this embodiment, either one of the two Lissajous figures for the right lung and the left lung may be displayed. For example, if the human subject would like to train for breathing of the right lung, the human subject or another person can manipulate the human interface 150 in order to instruct displaying only the Lissajous figure for the right lung. In this case, the CPU 170 may determine only the first bioelectrical impedance $Z_{aR}$ and the third bioelectrical impedance $Z_{bR}$ in the Lissajous figure displaying process and generate the display data for the Lissajous figure for the right lung to cause the display device 160 to show only the Lissajous figure for the right lung.

## 6.3. CENTERING THE LOCATION OF THE LISSAJOUS FIGURE

The displayed location of a Lissajous figure can be centered using the fifth centering value $Z_{a0}$ and the sixth centering value $Z_{b0}$ that are described in conjunction with the fourth embodiment. Description will be made taking as an example a Lissajous figure, in which the X axis is the sixth bioelectrical impedance $Z_b$, whereas the Y axis is the fifth bioelectrical impedance $Z_a$.

After completion of the smoothing process (step T804) in the Lissajous figure displaying process shown in Fig. 59, the CPU 170 executes steps S50 through T70 in the respiration analysis process (Fig. 38) described in conjunction with the fourth embodiment, so as to calculate the fifth centering value $Z_{a0}$ and the sixth centering value $Z_{b0}$. As can be clearly understood from Fig. 44, the fifth centering value $Z_{a0}$ is the standard level of the standard level of the fifth bioelectrical impedance $Z_a$, whereas the sixth centering value $Z_{b0}$ is the standard level of the sixth bioelectrical impedance $Z_b$.

For generating the display data for displaying a Lissajous figure at step T805 of the Lissajous figure displaying process, the CPU 170 adjusts the displayed location of the Lissajous figure such that the center position C having coordinates $(Z_{b0}, Z_{a0})$ defined by the fifth centering value $Z_{a0}$ and the sixth centering value $Z_{bo}$ becomes located at the center of the screen 160A for the Lissajous figure of the display device 160, as shown in Fig. 66. Since the location of the Lissajous figure is centered with respect to the screen 160A, visualization of the Lissajous figure can be facilitated.

Since the fifth centering value $Z_{a0}$ and the sixth centering value $Z_{b0}$ is obtained by a moving average process, even if centering of the displayed location of the Lissajous figure is repeated at small intervals (i.e., at each sampling time), the displayed location of the Lissajous figure is not changed remarkably at small intervals. However, repetition of centering of the displayed location of the Lissajous figure results in increase of processing load. Accordingly, it is possible to reduce the frequency of repetition. For example, the x-coordinate of the center position C of the Lissajous figure may be the average of the fifth centering values $Z_{a0}$ within the last single respiration and the y-coordinate of the center position C of the Lissajous figure may be the average of the sixth centering values $Z_{b0}$ within the last single respiration, so that the coordinates of the center position C of Lissajous figure are updated at each respiration (not at each sampling time). Alternatively, the x-coordinate of the center position C of Lissajous figure may be the average of the fifth centering values $Z_{a0}$ within a longer window (e.g., 20 seconds) and the y-coordinate of the center position C of Lissajous figure may be the average of the sixth centering values $Z_{b0}$ within the window, so that the coordinates of the center position C of the Lissajous figure is updated whenever a window passes over. Accordingly, intervals for updating the displayed location of the Lissajous figure may be determined freely.

In addition to centering the displayed location of the Lissajous figure, it is possible to adjust the display range of the Lissajous figure on the screen in each of the X axis and the Y axis. For example, the CPU 170 decides the local maximum (fifth local maximum) and the local minimum (fifth local minimum) of measurement values of the fifth bioelectrical impedance $Z_n$ within every respiration, and decides the local maximum (sixth local maximum) and the local minimum (sixth local minimum) of measurement values of the sixth bioelectrical impedance $Z_b$ within every respiration.

Then, the CPU 170 adjusts the display range of the Lissajous figure on the screen in the X axis on the basis of the sixth local maximum and the sixth local minimum, and adjusts the display range of the Lissajous figure on the screen in the Y axis on the basis of the fifth local maximum and the fifth local minimum.

More specifically, the CPU 170 adjusts the size and location of the Lissajous figure on the X axis on the screen 160A, so that the displayed range of the Lissajous figure on the X axis corresponding to the difference between the sixth local maximum and the sixth local minimum is about 80 to 90% of the width of the screen 160A in the X axis, whereas both of the sixth local maximum and the sixth local minimum are displayed within the screen 160A. Similarly, the CPU 170 adjusts the size and location of the Lissajous figure in the Y axis on the screen 160A, so that the displayed range of the Lissajous figure in the Y axis corresponding to the difference between the fifth local maximum and the fifth local minimum is about 80 to 90% of the width of the screen 160A in the Y axis, whereas both of the fifth local maximum and the fifth local minimum are displayed within the screen 160A.

Thus, for example, as shown in Fig. 67, the Lissajous figure can be displayed and centered at a suitable size with respect to the screen 160A, so that visualization of the Lissajous figure can be facilitated. Intervals for updating the size and location of the Lissajous figure on the X axis and the Y axis may be determined freely. However, since it is preferable that the entire Lissajous figure for at least a single respiration be located within the screen 160A, the CPU 170 preferably decides the fifth local maximum, the fifth local minimum, the sixth local maximum, and the sixth local minimum from measurement values within a period longer than the period of respiration.

As in Fig. 68 which shows Lissajous figures in draw-in respiration and costal respiration, both tracks of the Lissajous figures are of a straight shape rising from bottom left to top right, but locations of both tracks are different in the axis indicating the sixth bioelectrical impedance $Z_b$ (the X axis in Fig. 68). If centering of the displayed location of the Lissajous figure is repeated at small intervals, the Lissajous figure will always be displayed at the center of the screen 160A, and it will be difficult to understand whether respiration of the human subject is draw-in respiration or costal respiration from looking at the Lissajous figures. In order to prevent this disadvantageous effect, it is preferable to reduce the frequency of centering of the displayed location of the Lissajous figure on the X axis.

Accordingly, when the displayed location of the Lissajous figure is centered on the basis of the fifth centering value $Z_{a0}$ and the sixth centering value $Z_{b0}$, the CPU 170 may execute a second location centering process less frequently than that for a first location centering process, in which the first location centering process is for centering the Lissajous figure in the Y axis on the basis of the fifth centering value $Z_{a0}$, whereas the second centering process is for centering the Lissajous figure on the X axis on the basis of the sixth centering value $Z_{b0}$, In addition, when the displayed location of the Lissajous figure is centered on the basis of the fifth local maximum, the fifth local minimum, the sixth local maximum, and the sixth local minimum, the CPU 170 may execute a second range adjustment process less frequently than that for a first range adjustment process, in which the first range adjustment process is for adjusting the displayed range of the Lissajous figure in the Y axis on the basis of the fifth local maximum and the fifth local minimum whereas the second range adjustment process is for adjusting the displayed range of the Lissajous figure in the X axis on the basis of the sixth local maximum and the sixth local minimum.

For example, the first location centering process or the first range adjustment process may be executed at every respiration, whereas the second location centering process or the second range adjustment process may be executed only once (for example, at an initial stage of the process). Alternatively, the first location centering process or the first range adjustment process may be executed at every respiration, whereas the second location centering process or the second range adjustment process may be executed at every 30 respirations. Alternatively, the first location centering process or the first range adjustment process may be executed at every eight seconds, whereas the second location centering process or the second range adjustment process may be executed at every five minutes.

If the second location centering process or the second range adjustment process for centering the Lissajous figure on the X axis corresponding to the sixth centering value $Z_{b0}$ is executed less frequently, it will be easy to understand whether respiration of the human subject is draw-in respiration or costal respiration from looking at the Lissajous figure. This is because the locations of tracks of the Lissajous figures for draw-in respiration and costal respiration will become different in the X axis for a certain period, even though the shapes of the tracks are similar. In addition, it is possible to reduce power consumption at the body condition determination apparatus 1 by reducing the frequency of the second location centering process or the second range adjustment process. Although the frequency is less, by executing the second location centering process or the second range adjustment process, the Lissajous figure can be displayed on the center of the screen 160A and at a suitable size with respect to the screen 160A.

The sixth bioelectrical impedance $Z_b$ at the middle body trunk is less likely to be affected by the disturbance resulting from motion of extremities, such as arms, in comparison with the fifth bioelectrical impedance $Z_a$ at the upper body trunk, and therefore measurement values of the sixth bioelectrical impedance $Z_b$ are stable. Accordingly, even if centering the Lissajous figure on the X axis corresponding to the sixth centering value $Z_{b0}$ is executed less frequently, there will be few problems in that the Lissajous figure is not located within the screen 160A.

When the Lissajous figure is displayed in which the X axis is the fifth bioelectrical impedance $Z_a$, whereas the Y axis is the sixth bioelectrical impedance $Z_b$, centering the Lissajous figure in the Y axis may be executed less frequently. In summary, it is preferable that centering the Lissajous figure on the axis, corresponding to the sixth bioelectrical impedance $Z_b$ among the two orthogonal coordinate axes, be executed less frequently than that for the other axis corresponding to the fifth bioelectrical impedance $Z_a$

Although Figs. 66 and 67 show Lissajous figures representing abdominal respiration, Lissajous figures may be displayed for representing costal respiration or draw-in respiration.

The fifth bioelectrical impedance $Z_a$ at the upper body trunk has a larger fluctuation range than that of the sixth bioelectrical impedance $Z_b$ at the middle body trunk. This is because the fifth bioelectrical impedance $Z_a$ is more likely to be affected by the disturbance resulting from motion of extremities, such as arms. If the frequency of centering the location of the Lissajous figure is reduced and such fluctuation is omitted, there will not be a significant problem. Accordingly, it is possible to execute centering the displayed location of the Lissajous figure on the axis corresponding to the fifth bioelectrical impedance $Z_a$ at the same frequency (e.g., at every 30 respirations or every five minutes) as that for centering the displayed location of the Lissajous figure on the axis corresponding to the sixth bioelectrical impedance $Z_b$. That is to say, the cycle for centering in the axis corresponding to the fifth bioelectrical impedance $Z_a$ may be the same as that for centering in the axis corresponding to the sixth bioelectrical impedance $Z_b$, and it is possible to prolong the cycle for centering to 30 respirations or five minutes.

6.4. TRACK DISPLAYING PROCESS

In a Lissajous figure that continually shows status of multiple respirations as shown in Fig. 57 or 58, if the manner for displaying the track within a single respiration is the same as that for the track within another single respiration, it is difficult to identify the track for the latest single respiration. Accordingly, it is preferable to use different displaying manners for the track of the latest single respiration and the tracks for past respirations in the Lissajous figure.

For example, the CPU 170 may generate the display data for displaying a Lissajous figure in such a manner that a deep color is allocated to the track for the latest single respiration and a faint color is allocated to the tracks for past respirations, as shown in Fig. 69. Alternatively, the CPU 170 may allocate a solid line to the track for the latest single respiration and may allocate a dotted line to the tracks for past respiration. Alternatively, the CPU 170 may allocate different tones of colors to the tracks for the latest single respiration and for past respirations. For example, the CPU 170 may decide whether or not a new respiration starts on the basis of change in each of the fifth bioelectrical impedance $Z_a$ and the sixth bioelectrical impedance $Z_b$, and may allocate red to the track for the new single respiration and may change the color for tracks for previous respirations from red to blue.

The CPU 170 may change the displaying manner for the tracks of the Lissajous figure depending on the elapsed time. For example, the CPU 170 may lighten the color as the elapsed time increases. In this case, the newer the track, the fainter the color of the track. It is easy to identify the tracks for newer respirations (e.g., the track for the latest respiration).

The Lissajous figure may be represented in any type of orthogonal coordinate system having two orthogonal coordinate axes in which one axis is the fifth bioelectrical impedance $Z_a$ and the other axis is the sixth bioelectrical impedance $Z_b$. The X axis and the Y axis may be replaced with each other in the Lissajous figures for the right lung and the left lung. In addition, the X axis and the Y axis of a Lissajous figure may be inclined at any angle, whereas the angle formed between the X axis and the Y axis is also orthogonal.

Although Fig. 69 shows a Lissajous figure representing abdominal respiration, Lissajous figures may be displayed for representing costal respiration or draw-in respiration.

6.5. ASSISTANCE DISPLAY

As shown in Fig. 70, it is preferable to display a target Lissajous figure TL showing a target model of breathing to be performed by the human subject in such a manner that the target Lissajous figure TL is overlaid on an actually measured Lissajous figure ML showing the status of breathing of the human subject.

For example, the target Lissajous figure TL may be generated by processing an actually measured Lissajous figure ML of the human subject measured in past times (e.g., an actually measured Lissajous figure ML showing the status of the last respiration of the human subject). When the human subject trains for costal breathing or draw-in breathing, the CPU 170 may generate display data indicating the target Lissajous figure TL that is the actually measured Lissajous figure ML showing the status of the last respiration of the human subject enlarged at a predetermined magnification (e.g., 1.1 -fold magnification). The track of actually measured Lissajous figure ML may be of a straight shape rising from bottom left to top right. When the human subject trains for abdominal breathing, the CPU 170 may generate display data indicating the target Lissajous figure TL that is the actually measured Lissajous figure ML showing the status of the last respiration of the human subject which is enlarged at a predetermined magnification or of which the bend angle AG is adjusted. The track of actually measured Lissajous figure ML may be of a bent shape.

The CPU 170 may cause the display device 160 to show the target Lissajous figure TL on the screen 160A, whereas the CPU 170 generates the data for displaying the actually measured current Lissajous figure ML on the basis of measurement values of the fifth bioelectrical impedance $Z_a$ and measurement values of the sixth bioelectrical impedance $Z_b$, and may cause the display device 160 to show the actually measured current Lissajous figure ML in such a manner that the actually measured current Lissajous figure ML is overlaid on the target Lissajous figure TL on the screen 160A. Thus, the human subject can train for breathing comparing the actually measured Lissajous figure ML showing the status of breathing with the target Lissajous figure ML showing the current status of breathing with the target of the Lissajous figure TL. The human subject may focus on making the track of the actually measured Lissajous figure ML coincide with the track of the target Lissajous figure TL, so as to learn the target breathing.

Instead of generating a target Lissajous figure TL by processing the actually measured Lissajous figure ML measured in past times, the CPU 170 may generate a target Lissajous figure TL that indicates a respiration having a type (costal, abdominal, or draw-in) and a magnitude (depth) to be performed by the human subject. For example, when a training menu for guiding the human subject to train for breathing indicates a step for performing a single costal respiration of which the magnitude is small and a next step for performing a single abdominal respiration of which the magnitude is in the middle, the CPU 170 may generate and display a target Lissajous figure TL corresponding to the single costal respiration of which the magnitude is small, and then may generate and display another target Lissajous figure TL corresponding to the single abdominal respiration of which the magnitude is in the middle in the interval between the last and current respirations. In addition, the CPU 170 may control the cycle for displaying the target Lissajous figure TL in order to guide the rhythm of respiration of the human subject. Thus, by the assistance display for guiding the human subject to perform breathing in which the target Lissajous figure TL is used, an affective guidance is given to the human subject as to the type, magnitude, or rhythm of breathing.

The CPU 170 may allocate different displaying manners (e.g., different colors and the different line types) to the actually measured Lissajous figure ML and the target Lissajous figure TL in order to easily distinguish the actually measured Lissajous figure ML from the target Lissajous figure TL. The CPU 170 may highlight the difference between the two Lissajous figures by showing bars between the tracks of the two Lissajous figures, as shown in Fig. 71.

The CPU 170 may calculate the differential area between the actually measured Lissajous figure ML and the target Lissajous figure TL, and on the basis of the magnitude of the differential area, the CPU 170 may classify the difference into multiple rankings. The CPU 170 may use the sum of the lengths of the bars indicating the difference instead of the differential area for classifying the difference into multiple rankings.

Although the actually measured Lissajous figure ML and the target Lissajous figure TL are overlaid in Figs. 70 and 71, it is not necessary to overlay them. The two Lissajous figures may be arranged next to each other.

The Lissajous figure may be represented in any type of orthogonal coordinate system having two orthogonal coordinate axes in which one axis is the fifth bioelectrical impedance $Z_a$ and the other axis is the sixth bioelectrical impedance $Z_b$ The X axis and the Y axis may be replaced with each other in the Lissajous figures for the right lung and the left lung. In addition, the X axis and the Y axis of a Lissajous figure may be inclined at any angle, whereas the angle formed between the X axis and the Y axis is also orthogonal.

6.6. DETERMINATION AS TO WHETHER LUNG VENTILATION CAPABILITY IS GOOD OR BAD

It is possible to determine whether the lung ventilation capability (respiration capability) is good or bad on the basis of the inclination angle of the track of the Lissajous figure. For example, when the human subject performs costal respiration, the CPU 170 may obtain an approximately straight line LN of the track of a Lissajous figure for a single respiration as shown in Fig. 72, in accordance with a suitable algorithm, for example, the least-square method, on the basis of the x-y coordinates of the track corresponding to the measurement values of the fifth bioelectrical impedance $Z_a$ and the sixth bioelectrical impedance $Z_b$ obtained at each sampling time within the single respiration. Next, the CPU 170 (inclination angle calculator) may calculate an angle $\alpha$ formed between the approximate straight line LN and the X axis, and decides the angle $\alpha$ as an inclination angle of the track of the Lissajous figure.

As shown in Fig. 72, when the X axis is the sixth bioelectrical impedance $Z_b$, whereas the Y axis is the fifth bioelectrical impedance $Z_a$, the higher the lung ventilation capability, the greater the inclination angle a of the approximate straight line LN (of the track for a single respiration). In other words, when the lung ventilation capability is greater, the inclination angle $\alpha$ is nearer to 90 degrees. Therefore, the CPU 170 (ventilation capability determiner) may compare the inclination angle $\alpha$ with a predetermined reference inclination angle $\beta$, and may determine that the lung ventilation capability is good when the inclination angle $\alpha$ s equal to or greater than the reference inclination angle $\beta$. When the inclination angle $\alpha$ s less than the reference inclination angle $\beta$, the CPU 170 may determine that the lung ventilation capability is bad. The reference inclination angle $\beta$ is a threshold for facilitating the determination as to whether the lung ventilation capability is good or bad, and may be defined on the basis of experiments from which the inclination angle of the track of a Lissajous figure for a single respiration is determined for a number of human subjects. The reference inclination angle $\beta$ is stored in the first memory 120.

As has been described above, it is possible to easily determine whether the lung ventilation capability is good or bad on the basis of the inclination angle of the track of the Lissajous figure.

Depending on the posture of a human subject (standing, sitting, or supine), the inclination angle may be varied. Accordingly, multiple reference inclination angles $\beta$ may be defined depending on the posture and be stored in the first memory 120. In this case, for example, the human interface 150 may be used to input the posture of the human subject, and the reference inclination angle $\beta$ corresponding to the posture may be retrieved from the first memory 120. The reference inclination angle $\beta$ retrieved from the first memory 120 may be amended on the basis of the personal or individual body information (including the height, age, and sex) entered at step T1 (Fig. 37) or the weight measured at step T2 (Fig. 37).

In an alternative embodiment, as shown in Fig. 73, the Lissajous figure may be rotated by coordinate conversion so that the straight line inclined at the reference inclination angle $\beta$ from the X axis is oriented vertically. If the approximate straight line LN of the track of the resulting Lissajous figure is vertical or sloping from top left to bottom right, the lung ventilation capability may be determined as good. If the approximate straight line LN is sloping from bottom left to top right, the lung ventilation capability may be determined as bad.

When the human subject performs abdominal respiration, an approximate straight line LN may be obtained for a part of the track encompassed by the oval shown in Fig. 74, and then it is possible to determine whether the lung ventilation capability is good or bad in a manner similar to that in costal respiration. When the human subject performs draw-in respiration, it is possible to determine whether the lung ventilation capability is good or bad in a manner similar to that in costal respiration.

The track of a Lissajous figure showing status within two or more respirations or a half of a respiration may be used for determining whether respiration capability is good or bad, instead of the track of a Lissajous figure showing status within a single respiration.

In determination as to whether the lung ventilation capability is good or bad, the Lissajous figure may be represented in any type of orthogonal coordinate system having two orthogonal coordinate axes in which one axis is the fifth bioelectrical impedance $Z_a$ and the other axis is the sixth bioelectrical impedance $Z_b$. The X axis and the Y axis may be replaced with each other in the Lissajous figures for the right lung and the left lung. In addition, the X axis and the Y axis of a Lissajous figure may be inclined at any angle, whereas the angle formed between the X axis and the Y axis is also orthogonal.

In an alternative embodiment, a reference inclination angle $\beta$ stored in the first memory 120 may be defined freely, and it is possible to determine whether or not the lung ventilation capability of the human subject is higher than a predetermined reference capability,

6.7. TIME COMPRESSION DISPLAYING OF GRAPH SHOWING RESPIRATION DEPTH

The CPU 170 may calculate the respiration depth at every respiration by executing the respiration depth calculating process (Fig. 50) described in conjunction with the fourth embodiment. That is to say, on the basis of measurement values of the fifth bioelectrical impedance $Z_a$ and the sixth bioelectrical impedance $Z_b$, the CPU 170 may calculate the respiration depth at every respiration. The CPU 170 (graph generator) may execute the normalization process (step T601 in Fig. 51 described in conjunction with the fourth embodiment) for the calculated respiration depths, and may generate display data for indicating a graph (respiration depth graph) showing change over time of respiration depth for displaying it on the display device 160. For example, the respiration depth graph is as shown in Fig. 75A.

The human subject or another person may refer to the respiration depth graph in order to understand how the magnitude (depth) of respiration is changed due to training for breathing. For this purpose, it is important to know the magnitude of latest respirations. The time for breathing training may be frequently long, e.g., ten minutes or more. In order to display the entire graph from the start of measurement to the current time on the display device 160, it is preferable that the graph be compressed in the direction of the time axis. If the entire graph is uniformly compressed, the time resolution will be reduced uniformly in the graph. This results in it being difficult to recognize details of the magnitude of the latest respirations.

Accordingly, the CPU 170 generates the display data for the graph in such a manner that the graph is nonlinearly compressed in the direction of the time axis and earlier time intervals (nearer to the start of measurement) are more compressed than later time intervals, so that the time resolution for later time intervals is higher than that for earlier time intervals. For example, the CPU 170 may generate the graph as a single logarithmic chart in which the time axis is represented on a logarithmic scale, as shown in Fig. 75B, so that the time resolution for later time intervals is higher than that for earlier time intervals. Alternatively, as shown in Fig. 75C, the CPU 170 may divide the entire elapsed time (from the start of measurement to the present) into three time intervals, and the earliest interval is most compressed, whereas the latest interval is least compressed, so that time resolution for the latest time interval is higher than that for earlier time intervals. In either of the cases in Figs 75B and 75C, although the graph is compressed in the direction of the time axis, it is easy to recognize details of the magnitude of the latest respirations.

It is possible to use the teachings in the above-described sixth embodiment when only one of two Lissajous figures for the right lung and the left lung is displayed. More specifically, it is possible to use the above-described track displaying process for the displayed Lissajous figure. It is possible to use the above-described assistance display for the displayed Lissajous figure. It is possible to determine whether the lung ventilation capability is good or bad for either of the right lung and the left lung. Hereinafter, displaying only a Lissajous figure for the right lung will be described.

In the track displaying process, the CPU 170 may generate the display data for displaying a Lissajous figure for the right lung, in such a manner that a deep color is allocated to the track for the latest single respiration and a faint color is allocated to the tracks for past respirations. Alternatively, the CPU 170 may allocate a solid line to the track for the latest single respiration and may allocate a dotted line to the tracks for past respirations. Alternatively, the CPU 170 may allocate different tones of colors to the tracks for the latest single respiration and for past respirations.

In the assistance display, the CPU 170 may generate a target Lissajous figure for the right lung showing a target model of breathing to be performed by the right lung of the human subject on the basis of an actually measured Lissajous figure ML of the human subject measured in past times or on the basis of data indicating a type and a magnitude (depth) of respiration to be performed by the human subject. The CPU 170 may cause the display device 160 to show the target Lissajous figure for the right lung together with the actually measured current Lissajous figure for the right lung.

In the determination as to whether the lung ventilation capability is good or bad, the CPU 170 may calculate an inclination angle $\alpha$ of the track of the Lissajous figure for the right lung on the basis of measurement values of impedances for describing the Lissajous figure for the right lung. Then, the CPU 170 may compare the inclination angle $\alpha$ with a reference inclination angle $\beta$, so as to determine whether the ventilation capability of the right lung is good or bad. For both of the Lissajous figures for the right lung and the left lung, a single reference inclination angle $\beta$ can be used.

In the sixth embodiment, the Lissajous figure is represented in such a manner that one axis is the fifth bioelectrical impedance $Z_a$ and the other axis is the sixth bioelectrical impedance $Z_b$. However, the CPU 170 may execute steps T10 through step T70 in the respiration analysis process (Fig. 38) described in conjunction with the fourth embodiment, so as to calculate the fifth difference $\Delta Z_a$ and the sixth difference $\Delta Z_b$. Then, the CPU 170 may generate display data for displaying a Lissajous figure for both lungs in an orthogonal coordinate system having two orthogonal coordinate axes in which one axis is the fifth difference $\Delta Z_a$ and the other axis is the sixth difference $\Delta Z_b$.

Alternatively, the CPU 170 may execute the respiration depth analysis process (Figs. 22 and 23) described in conjunction with the second embodiment, except for steps S790, S810, S840, and S860. Thus, the CPU 170 may calculate the first difference $\Delta Z_{aR}$, the second difference $\Delta Z_{aL}$, the third difference $\Delta Z_{bR}$, and the fourth difference $\Delta Z_{bL}$. Then, the CPU 170 may generate display data for displaying a Lissajous figure for the right lung in an orthogonal coordinate system having two orthogonal coordinate axes in which one axis is the first difference $\Delta Z_{aR}$ and the other axis is the third difference $\Delta Z_{bR}$. The CPU 170 may also generate display data for displaying a Lissajous figure for the left lung in an orthogonal coordinate system having two orthogonal coordinate axes in which one axis is the second difference $\Delta Z_{aL}$ and the other axis is the fourth difference $\Delta Z_{bL}$. The CPU 170 may cause the display device 160 to show only one of the two Lissajous figures for the right lung and the left lung.

Teachings in this embodiment can be combined with teachings in any one of the first through fifth embodiments. For example, the body condition determination apparatus 1 may display at least one Lissajous figure and may display the right lung respiration depth (first right lung respiration depth BR1 and the second right lung respiration depth BR2) related to the respiration capability of the right lung and the left lung respiration depth (first left lung respiration depth BL1 and the second left lung respiration depth BL2) related to the respiration. The body condition determination apparatus 1 may display at least one Lissajous figure and may display the bar graphs BG5 and BG6 shown in Fig. 36. The body condition determination apparatus 1 may display at least one Lissajous figure and may determine and report whether respiration of the human subject is costal respiration or abdominal respiration. The body condition determination apparatus 1 may display at least one Lissajous figure and may display the bar graphs BG7 and BG8 shown in Fig. 49. The body condition determination apparatus 1 may display at least one Lissajous figure and may determine and report that respiration of the human subject is costal respiration, abdominal respiration, or draw-in respiration.

## 7. VARIATIONS

The present invention is not limited to the above-described embodiments. For example, variations described below may be made without departing from the scope of the present invention. Any combination of the variations below may also be made without departing from the scope of the present invention.

### 7.1. FIRST VARIATION

In the first through the third embodiments, the CPU 170 calculates the sum of the absolute values of the peak value $\Delta Z_{aR}(MAX)$ and the bottom value $\Delta Z_{aR}(MIN)$ among the first differences $\Delta Z_{aR}$ within a single respiration, and decides the resulting sum as the first right lung respiration depth BR1 at every respiration of the human subject. However, for example, the CPU 170 may calculate the sum of the absolute value of the average of the first differences $\Delta Z_{aR}$ at inhalations within a single respiration and the absolute value of the average of the first differences $\Delta Z_{aR}$ at exhalations within the single respiration, and decides the resulting sum as the first right lung respiration depth BR1 at every respiration of the human subject. The same is true for the first left lung respiration depth BL1, the second right lung respiration depth BR2, and the second left lung respiration depth BL2.

### 7.2. SECOND VARIATION

The CPU 170 may calculate the difference between the first right lung respiration depth BR1 and the first left lung respiration depth BL1 at every respiration of the human subject, and may display the resulting difference. Similarly, the CPU 170 may calculate the difference between the second right lung respiration depth BR2 and the second left lung respiration depth BL2 at every respiration of the human subject, and may display the resulting difference.

## 7.3. THIRD VARIATION

In the first embodiment, the first right lung respiration depth BR1 is indicated by the first bar graph BG1, whereas the first left lung respiration depth BL1 is indicated by the second bar graph BG2 (see Fig. 21). However, for example, as shown in Fig. 76, the first right lung respiration depth BR1 and the first left lung respiration depth BL1 may be indicated in a single bar graph BG0. In the bar graph BG0, the number of degree indicators to be colored at the right-lung side is determined depending on the first right lung respiration depth BR1, whereas the number of degree indicators to be colored at the left-lung side is determined depending on the left right lung respiration depth BR2. Similarly, the second right lung respiration depth BR2 and the second left lung respiration depth BL2 may be indicated in a single bar graph.

## 7.4. FOURTH VARIATION

In the above-described third difference calculating process (step S800 in Figs. 22 and 31), the third centering value $Z_{bR0}$ is generated or calculated on the basis of measurement values of the third bioelectrical impedance $Z_{bR}$ at the first zero-cross times. However, for example, in a manner similar to the calculation of the first centering value $Z_{aR0}$ or the second centering value $Z_{aL0}$, the third centering value $Z_{bR0}$ may be calculated on the basis of a moving average of measurement values of the third bioelectrical impedance $Z_{bR}$ at the sampling times of which the number is predetermined. In other words, the third centering value $Z_{bR0}$ may be calculated independently of the first bioelectrical impedance $Z_{aR}$. The same is true for calculation of the fourth centering value $Z_{bL0.}$

## 7.5. FIFTH VARIATION

In the fourth embodiment, the CPU 170 may execute an assistance report for guiding the human subject to perform appropriate breathing. For example, the CPU 170 may cause the display device 160 to show and change a ninth bar graph BG9 for instructing appropriate rhythm and pattern of inhalations and exhalations and appropriate respiration depth. As shown in Fig. 77, the total number of degrees capable of being depicted by the ninth bar graph BG9 is six, in which three degrees are allocated to the inhalation side, whereas three degrees are allocated to the exhalation side. The greater the number of degree indicators colored, the greater the respiration depth to be performed. The number of colored degree indicators will be referred to as a "colored-degree-indicator number".

If the colored-degree-indicator number at the inhalation side is one (only one degree indicator at the inhalation side is colored), the ninth bar graph BG9 instructs the human subject to perform a small-depth inhalation. If the colored-degree-indicator number at the inhalation side is two (two degree indicators at the inhalation side are colored), the ninth bar graph BG9 instructs the human subject to perform a middle-depth inhalation. If the colored-degree-indicator number at the inhalation side is three (all degree indicators at the inhalation side are colored), the ninth bar graph BG9 instructs the human subject to perform a large-depth inhalation.

If the colored-degree-indicator number at the exhalation side is one (only one degree indicator at the exhalation side is colored), the ninth bar graph BG9 instructs the human subject to perform a small-depth exhalation. If the colored-degree-indicator number at the exhalation side is two (two degree indicators at the exhalation side are colored), the ninth bar graph BG9 instructs the human subject to perform a middle-depth exhalation. If the colored-degree-indicator number at the exhalation side is three (all degree indicators at the exhalation side are colored), the ninth bar graph BG9 instructs the human subject to perform a large-depth exhalation.

Let us assume that the body condition determination apparatus instructs the human subject to perform middle-depth inhalations twice and then to perform middle-depth exhalations twice. In this case, the indication on the ninth bar graph BG9 is changed as shown in Fig. 78. When a single breathing takes two seconds, the change in the indication is meant to guide two middle-depth exhalations and two middle-depth inhalation in four seconds. This is only an example, and various patterns of change may be used for instructing appropriate rhythm and pattern of inhalations and exhalations and appropriate respiration depth. For example, it is possible to report assistance information for guiding the human subject to perform abdominal breathing. Instead of, or in addition to, displaying the ninth bar graph BG9, assistance information may be announced by speech. By the above-described assistance report, the human subject is guided to perform appropriate breathing paying attention to the rhythm, pattern, and depth.

## 7.6. SIXTH VARIATION

In the fourth embodiment, as shown in Fig. 79, the CPU 170 may cause the display device 160 to display a schematic view for showing the magnitude of respiration. Fig. 79 includes four schematic views. In the schematic views, the greater the magnitude of respiration, the greater the area colored in the illustration of the lungs. Such a schematic view can be displayed as a measurement result or as guidance information for guiding the human subject to perform breathing.

Alternatively, the type and magnitude of respiration can be reported by an animation that shows a human model or an abstract animal model. When respiration of the human subject is small-depth costal respiration, an animation is displayed in which the model's chest repeats contraction and expansion in a small range. When respiration of the human subject is large-depth costal respiration, another animation is displayed in which the model's chest repeats contraction and expansion in a large range. When respiration of the human subject is small-depth abdominal respiration, an animation is displayed in which the model's abdomen repeats contraction and expansion in a small range. When respiration of the human subject is large-depth abdominal respiration, another animation is displayed in which the model's abdomen repeats contraction and expansion in a large range. When respiration of the human subject is small-depth draw-in respiration, an animation is displayed in which the model's chest repeats contraction and expansion in a small range, whereas the model's abdomen is held in a constricted position. When respiration of the human subject is large-depth draw-in respiration, another animation is displayed in which the model's chest repeats contraction and expansion in a large range, whereas the model's abdomen is held in a constricted position. Such an animation can be displayed as a measurement result or as guidance information for guiding the human subject to perform breathing.

Thus, measurement results or guidance information can be reported in an easily understandable manner.

## 7.7. SEVENTH VARIATION

In the first embodiment, the CPU 170 may use bar graphs that are similar to the bar graphs BG1 and BG2 shown in Fig. 21 in the execution of the assistance display for guiding the human subject to perform breathing. Such bar graphs indicate target magnitudes of breathing at the right lung and the left lung of the human subject. For example, when a training menu for guiding the human subject to train for breathing indicates a step for performing a single coastal breathing of which the magnitude is small and a next step for performing a single abdominal breathing of which the magnitude is in the middle, the CPU 170 may cause the display device 160 to display the target bar graph for the right lung (similar to the bar graph BG1) and the target bar graph for the left lung (similar to the bar graph BG2) that show a small-depth breathing. The CPU 170 may cause the display device 160 to display the target bar graph for the right lung and the target bar graph for the left lung that show a middle-depth breathing in the interval between the last and current breathings. In addition, the CPU 170 may control the cycle for displaying the target bar graphs in order to guide the rhythm of breathing of the human subject.

The CPU 170 may display only one target bar graph among the two bar graphs for the right lung and the left lung. For example, if the right lung is trained for breathing, only the target bar graph for the right lung may be displayed.

It is possible to display the bar graphs BG1 and BG2 that show the magnitude of breathing of the human subject and to display the target bar graphs at the same time, so that the human subject can compare the status of breathing and the target model of breathing during training.

It is also possible to display target bar graphs resembling the bar graphs BG1 through BG4 shown in Fig. 27, or the bar graphs BG5 and BG6 shown in Fig. 36 in order to guide the human subject to train for breathing.

As shown in Fig. 80, the CPU 170 may also cause the display device 160 to display a schematic view of lungs for showing the magnitude of respiration. Fig. 80 includes four schematic views. In the schematic views, the greater the magnitude of respiration, the greater the area colored in the illustration of the lungs. If there is a significant difference between the respiration capabilities of the right lung and the left lung, the colored areas in the right lung and the left lung are different from each other, as shown in Fig. 80. Such a schematic view can be displayed as a measurement result or as guidance information for guiding the human subject to perform breathing.

Alternatively, the type and magnitude of respiration can be reported by an animation that shows contraction and expansion of lungs.

## 7.8. EIGHTH VARIATION

In the respiration depth displaying process described in conjunction with the fourth embodiment, the CPU 170 calculates the normalized respiration depth value $\%\Delta Z_a$ as the respiration depth $\Delta Z_{ap-p}$ at the last respiration divided by the sixth centering value $Z_{b0}$ at the last respiration multiplied by 100. The calculation is expressed as:

$$\%\Delta Z_a = (\Delta Z_{ap-p}/Z_{b0}) * 100$$

However, another scheme may be used for normalizing the respiration depth $\Delta Z_{ap-p}$. In summary, any type of normalization scheme for amending the respiration depth $\Delta Z_{ap-p}$ in order to exclude individual variation of physical constitutions of human subjects. For example, when an index indicative of the skeletal muscle mass (degree of development of skeletal muscle) is MV, an index indicative of the height of the human subject and the length of part in which the bioelectrical impedance is measured is H, bioelectrical impedance at a part which is important for development of the skeletal muscle is $Z_x$, MV is proportional to $H^2/Z_x$. Instead of the sixth centering value $Z_{b0}$, using $H^2/Z_x$" the respiration depth $\Delta Z_{ap-p}$ may be normalized. In order to assume the index MV, a multiple linear regression analysis may be used, and sex, age, and weight may be used as variables in the multiple linear regression analysis in order to enhance accuracy.

7.9. NINTH VARIATION

In the above-described embodiments, four current electrodes and four voltage electrodes are arranged at both hands and both feet in accordance with the limb-lead eight-electrode method. The present invention is not limited to the embodiment. For example, current electrodes and voltage electrodes may be adhered directly to the body trunk of the human subject, so as to determine the first bioelectrical impedance $Z_{aR}$ through the sixth bioelectrical impedance $Z_b$. The bioelectrical impedance at the upper body trunk can be measured with the use of a combination of the limb-lead method and ear electrodes. By using ear electrodes, the bioelectrical impedance at the upper body trunk is influenced by only one upper extremity rather than both upper extremities. If ear electrodes are incorporated in earphones or headphones, further advantages are obtained since sound information and relaxing effects are given to the human subject.

In the above-described embodiments, impedances are determined when the human subject is standing. However, impedances may be determined when the human subject is at a sitting or a relaxed position on a sofa, a seat, e.g., a lavatory seat, or a chair, e.g., a massage chair, with electrodes being arranged at parts of the seat, armrests, footrests, or a combination thereof.

Furthermore, impedances are determined when the human subject is bathing with electrodes being arranged at armrests and the bottom surface of the bathtub at which the buttocks and soles of the human subject are in contact. The body trunk includes physiological saline and is electrically more conductive than the hot water in a bathtub. Accordingly, when the human subject is relaxed while bathing, impedances are determined and breathing training may be performed.

The body condition determination apparatus 1 of the above-described embodiments may include a blood-pressure meter with a cuff. Change of the status of breathing or the tension on the arms is determined on the basis of the determined impedances, and obtained information may be used for correcting or compensating the measured blood pressure.

It is preferable to relax the human subject when the analysis of respiration or training for breathing is executed. Accordingly, it is desirable to display a picture of peaceful scene, to turn on relaxing music or sounds of birds or a waterfall, and to adjust the temperature and humidity when the analysis of respiration or training for breathing is executed.

It is also preferable to display a video about training for appropriate breathing in order to enhance the efficiency of training.

7.10. TENTH VARIATION

The present invention may be applied in a system including a game machine. Fig. 81 is an overall view showing a body condition determination system 5 including a household game machine 300. As shown in Fig. 81, the body condition determination system 5 includes a biological information input apparatus 200', a game machine 300 (respiration characteristic analysis apparatus), a controller 350, and a monitor 400. The game machine 300 includes a disk slot into which an optical disk 500 is inserted.

The biological information input apparatus 200' includes a platform 20' on which the human subject stands, a left-hand left electrode handle 30L, and a right-hand right electrode handle 30R. The biological information input apparatus 200' basically has the same structure as that of the bioelectrical impedance determination part 200 shown in Fig. 1, and additionally includes a weighing scale. The biological information input apparatus 200' is connected with the controller 350 via a communication cable, and biological information measured at the biological information input apparatus 200', e.g., bioelectrical impedances at various parts of the human body and the human body weight, is supplied from the biological information input apparatus 200' via the controller 350 and the communication cable to the game machine 300.

The controller 350 is a human interface. The human subject or another person may manipulate the controller 350 in order to input various instructions and personal information on the human subject, for example, the height, age, and sex into the controller 350. The controller 350 is communicable with the game machine 300 by radiowaves, e.g., Bluetooth (registered trademark), and transmits to the game machine 300 instructions input to the controller 350 and the biological information supplied from the biological information input apparatus 200'.

The monitor 400 may be, for example, a television receiver connected with the game machine 300 via a communication cable.

The optical disk 500 stores a program and data for executing various processes that have been described in conjunction with the above-described first through sixth embodiments and the first through ninth variations.

In this embodiment, biological information measured at the biological information input apparatus 200' is supplied via the controller 350 to the game machine 300. However, the biological information input apparatus 200' may supply the biological information to the game machine 300 by radiowaves. In this case, the biological information input apparatus 200' may include a radio module for radio communication with the game machine 300. The biological information input apparatus 200' may supply the biological information to the game machine 300 by cable. In this case, the biological information input apparatus 200' may be connected with the game machine 300 via a communication cable. Instead of the left-hand left electrode handle 30L, the current electrode X3 and the voltage electrodes Y3 may be provided in a left-hand controller having at least part of the function of the controller 350. Instead of the right-hand right electrode handle 30R, the current electrode X4 and the voltage electrodes Y4 may be provided in a right-hand controller having at least part of the function of the controller 350.

Fig. 82 is a block diagram showing a structure of the game machine 300. As shown in Fig. 82, the game machine 300 includes a ROM 301, a RAM 302, a hard disk 303, a disk drive 310, a radio communication module 320, an image processor 330, a sound processor 340, and a CPU 360. The ROM 301 stores a program or data for controlling the whole game machine 300. The RAM 302 is used as a work area for the CPU 360.

The hard disk 303 stores a program or data read from the optical disk 500. Such data include data describing a training menu management table TBL that will be described with reference to Fig. 83. The disk drive 310 reads the program or data from the optical disk 500. The program or data may be supplied via another information storage medium instead of the optical disk 500, or may be downloaded from another apparatus, such as a server apparatus via a communication network. For downloading, the game machine 300 may include a network communication module.

The radio communication module 320 controls radiowave communication with the controller 350. The radio communication module 320 serves as an input part for inputting biological information measured at the biological information input apparatus 200' to the game machine 300 (respiration characteristic analysis apparatus).

The image processor 330 generates image data and supplies the image data to the monitor 400. The sound processor 340 generates audio data indicating sound effects and speech and supplies the audio data to the monitor 400 in order that speakers of the monitor 400 can emit sounds.

The CPU 360 serves as a main controller for controlling the entire game machine 300 by executing various programs stored in the ROM 301 and the hard disk 303. For example the CPU 360 controls the radio communication module 320, thereby communicating with the biological information input apparatus 200' via the controller 350. The CPU 360 instructs the biological information input apparatus 200' to select appropriate electrodes among the current electrodes X1 through X4 and the voltage electrodes Y1 through Y4, to determine bioelectrical impedances, and to measure body weight.

The CPU 360 of the game machine 300 executes the respiration depth analysis process and the respiration depth displaying process described in conjunction with the first embodiment, so as to cause the monitor 400 to display the first bar graph BG1 related to the respiration capability of the upper lobe of the right lung, and to display the second bar graph BG2 related to the respiration capability of the upper lobe of the left lung (see Fin. 21). The CPU 360 may execute the respiration depth analysis process and the respiration depth displaying process described in conjunction with the second embodiment, so as to cause the monitor 400 to display the third bar graph BG3 related to the respiration capability of the median and lower lobes of the right lung and the fourth bar graph BG4 related to the respiration capability of the median and lower lobes of the left lung, in addition to the first bar graph BG1 and the second bar graph BG2 (see Fig. 27). The CPU 360 may execute the respiration depth analysis process described in conjunction with the third embodiment, so as to cause the monitor 400 to display the fifth bar graph BG5 indicative af the magnitude of each of costal respiration and abdominal respiration at the right body trunk and the sixth bar graph BG6 indicative of the magnitude of each of costal respiration and abdominal respiration at the left body trunk (see Fig. 36).

The CPU 360 may execute the respiration analysis process described in conjunction with the fourth embodiment, so as to decide respiration of the human subject is costal respiration or abdominal respiration. The CPU 360 also executes the respiration depth calculating process and the respiration depth displaying process described in conjunction with the fourth embodiment, so as to cause the monitor 400 to display the seventh bar graph BG7 indicative of the magnitude of each of costal respiration and abdominal respiration, and to display the eighth bar graph BG8 indicative of the abdominal respiration percentage level (see Fig. 49).

The CPU 360 may execute the respiration type determination process described in conjunction with the fifth embodiment, so as to decide that respiration of the human subject is costal respiration, abdominal respiration, or draw-in respiration. The CPU 360 may execute the Lissajous figure displaying process described in conjunction with the sixth embodiment, so as to display at least one Lissajous figure on the monitor 400. The CPU 360 may execute the process for training the human subject for appropriate breathing using the bar graph BG0 through BG9, the Lissajous figure, the schematic view of lungs, or the like. Thus, the CPU 360 may execute various processes described in conjunction with the first through (sixth embodiments and the first through ninth variations.

Fig. 83 is a diagram showing the data format of a training menu management table TBL. The training menu management table TBL is used for training the human subject for breathing in accordance with training menus that match respiration capability of the human subject. The training menu management table TBL records multiple groups each consisting of multiple menus of training for breathing, each group corresponding to a ranking of respiration capability, and requirements for advancing through the rankings and for advancing to the next rankings. In the illustrated example, there are five rankings (rankings 1 to 5). For each group at a ranking, 20 training menus are prepared.

Examples of the training menus include a training for learning costal breathing and abdominal breathing, a training for learning a complete breathing in which costal breathing and abdominal breathing is combined, a training for learning draw-in breathing, a training for improving lung ventilation capability by guiding into abdominal breathing and complete breathing, a training for improving respiratory functions and motion functions by guiding into draw-in breathing, and a training for strengthening respiratory functions and motion functions by guiding into various types of breathing with load on respiratory muscles by holding various poses used in *yoga* or *Qigong.*

In the specification, a complete breathing means a type of respiration in which lung respiration capability is used to the maximum with movement of the abdomen, chest, and scapular region. At inhalation in complete breathing, the human expands the abdomen at the start of aspiration, expands the thoracic cage with moving the chest forward while holding aspiration, and then moves the shoulders upward and aspires a little more, whereby the lung volume is broadened to the maximum. Exhalation in complete breathing is opposite to inhalation. At exhalation in complete breathing, the human moves the shoulders downward at the start of expiration, constricts the thoracic cage while holding expiration, and constricts the abdomen and expires more.

Examples of the training menus also include a training menu in which a Lissajous figure, bar graphs, or a schematic view of lungs showing the status of breathing of the human subject is displayed, so that the human subject can confirm the status of breathing during training. Examples of the training menus also include a training menu in which a target Lissajous figure, a bar graph, or a schematic view of lungs showing a target model of breathing to be performed by the human subject is displayed, so that the human subject can confirm the target model of breathing during training. Examples of the training menus also include a training menu in which both of a Lissajous figure, bar graphs, or a schematic view of lungs showing the status of breathing of the human subject and a target Lissajous figure, bar graphs, or a schematic view of lungs showing a target model of breathing to be performed by the human subject are displayed, so that the human subject can compare the status of breathing and the target model of breathing during training.

The requirement for advancing through each ranking may be, for example, that each of the right lung respiration depth (the first right lung respiration depth BR1 and the second right lung respiration depth BR2) and the left lung respiration depth (the first left lung respiration depth BL1 and the second left lung respiration depth BL2) is equal to or greater than a predetermined standard level; the magnitude of costal respiration or abdominal respiration at each of the right lung and the left lung is equal to or greater than a predetermined standard level; the magnitude of costal respiration or abdominal respiration by both lungs is equal to or greater than a predetermined standard level; that the ventilation capability of both lungs is equal to or greater than a predetermined standard level; that the human subject can perform draw-in respiration; that the abdominal respiration percentage level is equal to or greater than a predetermined standard level; or all of 20 training menus for the ranking have been completed. The number of rankings described in the training menu management table TBL is not limited as long as it is at least two. The number of the training menus in each group at a ranking is also not limited as long as it is at least one.

More specific examples of training menus in each ranking will be described next.

7.10.1. RANKING 1 (TRAINING OF PATIENTS WITH SEVERE RESPIRATORY DISEASE)

The training menus include training plans for strengthening the costal respiratory muscles that contribute to fundamental respiratory motion by guiding into costal breathing, thereby healing respiratory functions deteriorated by respiratory disease.

7.10.2. RANKING 2 (TRAINING OF PATIENTS WITH MILD RESPIRATORY DISEASE)

The training menus include training plans for strengthening the abdominal respiratory muscles including the diaphragm by guiding into abdominal breathing, thereby healing respiratory functions deteriorated by respiratory disease.

7.10.3. RANKING 3 (STANDARD TRAINING OF PHYSICALLY UNIMPAIRED PERSONS)

The training menus include training plans for enhancing costal respiratory muscles and abdominal respiratory muscles by guiding into the complete breathing into which costal breathing and abdominal breathing are combined, thereby improving healthy respiratory functions or healthy respiratory functions deteriorated by smoking, lifestyle, lack of activity, or aging.

7.10.4. RANKING 4 (LIGHTLY-LOADED TRAINING)

The training menus include training plans for strengthening inner muscles at the body trunk (e.g., the transverse abdominal muscle and the erector muscle of the spine) by guiding into draw-in breathing, thereby improving respiratory functions, preventing backache, or enhancing motion functions.

7.10.5. RANKING 5 (HIGHLY-LOADED TRAINING OF ATHLETES)

The training menus include training plans for strengthening motion functions by guiding into draw-in breathing with load on respiratory muscles by holding various poses used in *yoga* or *Qigong*.

Training for patients with respiratory disease (rankings 1 and 2) will be conducted under medical guidance by therapy specialists. Training for patients with respiratory disease will be conducted by human subjects whose diaphragm can function to some extent and whose respiratory functions are expected to be improved by training of breathing.

For all of rankings 1 to 5, a load may be applied in such a manner that the human subject breathes through pursed lips. It is possible to freely determine combination of type of respiration and poses, and allocation of time in training.

Fig. 84 is a flow chart showing an example of a breathing training management process. The breathing training management process is started being executed when the human subject starts training for breathing using the system 5. In the breathing training management process, the CPU 360 first executes a process for determining the respiration capability of the human subject (step T1201). For example, the CPU 360 reports a message for instructing the human subject to perform a type of respiration, and then executes the respiration depth analysis process and respiration depth displaying process described in conjunction with any one of the first through third embodiments, the respiration analysis process and the respiration depth calculating process described in conjunction with the fourth embodiment, or the Lissajous figure displaying process and the process for deciding whether the lung ventilation capability is good or bad described in conjunction with the sixth embodiment. Therefore, the CPU 360 determines the respiration capability of the human subject that may include, for example, the right lung's respiration capability (respiration capability at the upper lobe of the right lung or at the median and lower lobes of the right lung), the left lung's respiration capability (respiration capability at the upper lobe of the left lung or at the median and lower lobes of the left lung), the magnitude of each of costal respiration and abdominal respiration at each of the right lung and the left lung, the magnitude of each of costal respiration and abdominal respiration by both lungs, the ventilation capability of both lungs, and the abdominal respiration percentage level.

In order to determine the normal respiration capability of the human subject at normal status, step T1201 may be executed without informing that the respiration parameters are being determined. At step T1201, the respiration capability of the human subject may be determined on the basis of the first bioelectrical impedance $Z_{aR}$ and the second bioelectrical impedance $Z_{aL}$, on the basis of the third bioelectrical impedance $Z_{bR}$ and the fourth bioelectrical impedance $Z_{bL}$, on the basis of the first through fourth bioelectrical impedances $Z_{aR}$, $Z_{aL}$ $Z_{bR}$, and $Z_{bL}$, or on the basis of the fifth bioelectrical impedance $Z_a$ and the sixth bioelectrical impedance $Z_b$.

Next, at step T1202, the CPU 360 refers to the training menu management table TBL, and it identifies the ranking in the table corresponding to the respiration capability of the human subject determined at step T1201. Next, the CPU 360 selects the group of training menus corresponding to the ranking from among the training menu management table TBL (step T1203). For example, if the ranking of the human subject is ranking 3, the CPU 360 selects the group consisting of menus 41 through 60 from among the training menu management table TBL shown in Fig. 83. If there is a significant difference between the respiration capabilities of the right lung and the left lung, the CPU 360 may select different groups of training menus for the right lung and the left lung.

Then, at step T1204, the CPU 360 executes a breathing training process for training the human subject for breathing using the training menus selected at step T1203. For example, if the ranking of the human subject is ranking 3, the CPU 360 executes a process for prompting the human subject to train for breathing using menus 41 through 60. If the ranking of the human subject is ranking 3 for standard training of physically unimpaired persons, in accordance with the training menus, the CPU 360 guides the human subject into the complete breathing into which costal breathing and abdominal breathing are combined, so that the human subject learns the complete breathing or improves lung ventilation capability. The CPU 360 may cause the monitor 400 to display a Lissajous figure, bar graphs, or a schematic view of lungs showing the status of breathing of the human subject, so that the human subject can confirm the status of breathing during training. The CPU 360 may cause the monitor 400 to display a target Lissajous figure, a bar graph, or a schematic view of lungs showing a target model of breathing to be performed by the human subject, so that the human subject can confirm the target model of breathing during training. The CPU 360 may cause the monitor 400 to display a Lissajous figure, bar graphs, or a schematic view of lungs showing the status of breathing of the human subject and a target Lissajous figure, a bar graph, or a schematic view of lungs showing a target model of breathing to be performed by the human subject, so that the human subject can compare the status of breathing, and the target model of breathing during training.

Then, the CPU 360 retrieves the requirement for advancing through the ranking in which the human subject is being placed from the training menu management table TBL, and decides whether or not the requirement for advancing through the ranking is satisfied (step T1205). For example, if the ranking on which the human subject is placed is ranking 3, the CPU 360 reads requirement C from the training menu management table TBL shown in Fig. 83, and decides whether or not requirement C is satisfied.

For example, if requirement C is that the ventilation capability of both lungs is equal to or greater than a predetermined standard level, the CPU 360 decides whether or not the ventilation capability of both lungs is equal to or greater than the predetermined standard level, on the basis of the result of the respiration analysis process and the respiration depth displaying process described in conjunction with the any one of the first through third embodiments. Alternatively, if requirement C is that the magnitude of abdominal respiration by both lungs is equal to or greater than a predetermined standard value, the CPU 360 decides whether or not the magnitude of abdominal respiration of the human subject is equal to or greater than the predetermined standard value, on the basis of the result of the respiration analysis process and the respiration depth calculating process described in conjunction with the fourth embodiment. If requirement C is that the human subject can perform draw-in respiration, the CPU 360 decides whether or not the human subject can perform draw-in respiration, on the basis of the respiration type determination process described in conjunction with the fifth embodiment. If requirement C is that all of 20 training menus for the ranking have been completed, the CPU 360 decides whether or not the menus 41 to 60 have been completed.

If the decision at step T1205 is negative, the process returns to step T1204 for continuing the breathing training at the current ranking. If the decision at step T1205 is affirmative, the CPU 360 advances the ranking of the human subject to the next ranking (step T1206), and returns to step T1203. For example, if the current ranking of the human subject is ranking 3, the CPU 360 changes the ranking of the human subject to ranking 4 at step T1206, and returns to step T1203, and starts a new training in accordance with menus 61 to 80 corresponding to ranking 4 from the training menu management table TBL.

If the human subject or another person manipulates the controller 350 to instruct the end of training, the breathing training management process is thus ended for the human subject. At the ending thereof, the CPU 360 stores information in the ranking of the human subject is written on the hard disk 303. When the human subject next starts training for breathing, the CPU 360 reads the information on the ranking from the hard disk 303, and then starts with step T1203 in the next breathing training management process.

As has been described above, according to this variation, respiration of the human subject can be determined easily at home in a manner similar to the measurements of the body weight and the body fat. In addition, training for breathing can be conducted easily at home by the game machine 300. In this variation, since the respiration capability of the human subject is determined on the basis of the determinations of bioelectrical impedance at the body trunk, it is unnecessary to deploy a respiration sensor in the mouth or under the nose of the human subject, and the human subject may use either or both of the mouth and nose for respiration, so that training for breathing can be conducted easily. The human subject can effectively train for breathing in accordance with the training menus that match the respiration capability of the human subject. The training menus are prepared at each ranking of respiration capability, and if the requirement defined at each ranking is satisfied, the human subject can advance to the next ranking, Accordingly, the training process has a game element by which the human subject is amused, and the human subject is motivated to train for breathing.

Instead of the game machine 300, the body condition determination apparatus 1 in any one of the first through sixth embodiments may execute the breathing training management process (Fig. 84). In this case, the computer program and data, such as the training menu management table TBL (Fig. 83) to execute the breathing training management process may be stored in the first memory 120 of the body condition determination apparatus 1. Instead of the game machine 300, a personal computer or a portable electrical device (e.g., a cell phone or a tablet computer) may be used for executing the breathing training management process, and a head-mounted display may be used as a display device, especially in the use of a portable electrical device.

### 7.11. ELEVENTH VARIATION

When two Lissajous figures for the right lung and the left lung are overlaid on a screen, for each of the Lissajous figures, the displayed location may be centered and the displayed range may be adjusted in the X axis and the Y axis. Description will be made taking as an example a Lissajous figure for the right lung, in which the X axis is the third bioelectrical impedance $Z_{bR}$, whereas the Y axis is the first bioelectrical impedance $Z_{aR}$, and taking as an example a Lissajous figure for the left lung, in which the X axis is the fourth bioelectrical impedance $Z_{bL}$ whereas the Y axis is the second bioelectrical impedance $Z_{aL}$.

The CPU 170 decides the local maximum (first local maximum) and the local minimum (first local minimum) of measurement values of the first bioelectrical impedance $Z_{aR}$ within every respiration, and decides the local maximum (second local maximum) and the local minimum (second local minimum) of measurement values of the second bioelectrical impedance $Z_{aL}$ within every respiration. Similarly, the CPU 170 decides the local maximum (third local maximum) and the local minimum (third local minimum) of measurement values of the third bioelectrical impedance $Z_{bR}$ within every respiration, and decides the local maximum (fourth local maximum) and the local minimum (fourth local minimum) of measurement values of the fourth bioelectrical impedance $Z_{bL}$ within every respiration.

Then, the CPU 170 adjusts the display range of the Lissajous figure on the screen in the X axis on the basis of the third local maximum, the third local minimum, the fourth local maximum, and the fourth local minimum, and adjusts the display range of the Lissajous figure on the screen in the Y axis on the basis of the first local maximum, the first local minimum, the second local maximum, and the second local minimum.

More specifically, the CPU 170 adjusts the size and location of the Lissajous figure for the right lung in the X axis on the screen 160A, so that the displayed range of the Lissajous figure on the X axis corresponding to the difference between the third local maximum and the third local minimum is about 80 to 90% of the width of the screen 160A in the X axis, whereas both of the third local maximum and the third local minimum are displayed within the screen 160A. Similarly, the CPU 170 adjusts the size and location of the Lissajous figure for the left lung in the X axis on the screen 160A, so that the displayed range of the Lissajous figure on the X axis corresponding to the difference between the fourth local maximum and the fourth local minimum is about 80 to 90% of the width of the screen 160A in the X axis, whereas both of the fourth local maximum and the fourth local minimum are displayed within the screen 160A.

In addition, the CPU 170 adjusts the size and location of the Lissajous figure for the right lung in the Y axis on the screen 160A, so that the displayed range of the Lissajous figure in the Y axis corresponding to the difference between the first local maximum and the first local minimum is about 80 to 90% of the width of the screen 160A in the Y axis, whereas both of the first local maximum and the first local minimum are displayed within the screen 160A. Similarly, the CPU 170 adjusts the size and location of the Lissajous figure for the left lung in the Y axis on the screen 160A, so that the displayed range of the Lissajous figure in the Y axis corresponding to the difference between the second local maximum and the second local minimum is about 80 to 90% of the width of the screen 160A in the Y axis, whereas both of the second local maximum and the second local minimum are displayed within the screen 160A.

Thus, for example, as shown in Fig. 85, each of two Lissajous figures for the right lung and the left lung can be displayed and centered at a suitable size with respect to the screen 160A, so that visualization of the Lissajous figure can be facilitated. Intervals for updating the size and location of the Lissajous figures in X and Y axes may be determined freely, However, since it is preferable that the entire Lissajous figures for at least a single respiration be located within the screen 160A, the CPU 170 preferably decides the first through the fourth local maximums and the first through fourth local minimums from measurement values within a period longer than the period of respiration.

If centering of the displayed locations of the Lissajous figures is repeated at small intervals, the two Lissajous figures will be always displayed at the center of the screen 160A and it will be difficult to understand whether respiration of the human subject is draw-in respiration or costal respiration from looking at the Lissajous figures. In order to prevent this disadvantageous effect, it is preferable to reduce the frequency of centering of the displayed locations of the Lissajous figures on the X axis.

Accordingly, the CPU 170 may execute a second location centering process less frequently than that for a first location centering process, in which the first location centering process is for centering the Lissajous figures in the Y axis on the basis of the first local maximum, the first local minimum, the second local maximum, and the second local minimum, whereas the second centering process is for centering the Lissajous figures in the X axis on the basis of the third local maximum, the third local minimum, the fourth local maximum, and the fourth local minimum.

For example, the first location centering process or the first range adjustment process may be executed at every respiration, whereas the second location centering process or the second range adjustment process may be executed only once (for example, at an initial stage of the process). Alternatively, the first location centering process or the first range adjustment process may be executed at every respiration, whereas the second location centering process or the second range adjustment process may be executed at every 30 respirations. Alternatively, the first location centering process or the first range adjustment process may be executed at every eight seconds, whereas the second location centering process or the second range adjustment process may be executed at every five minutes.

If the second location centering process or the second range adjustment process for centering the Lissajous figure on the X axis is executed less frequently, it will be easy to understand whether respiration of the human subject is draw-in respiration or costal respiration from looking at the Lissajous figure. This is because the locations of tracks of the Lissajous figures for draw-in respiration and costal respiration will become different in the X axis for a certain period, even though the shapes of the tracks are similar. In addition, it is possible to reduce power consumption at the body condition determination apparatus 1 by reducing the frequency of the second location centering process or the second range adjustment process. Although the frequency is less, by executing the second location centering process or the second range adjustment process, each of the Lissajous figures can be displayed on the center of the screen 160A and at a suitable size with respect to the screen 160A.

When the Lissajous figure for the right lung is displayed in which the X axis is the first bioelectrical impedance $Z_{aR}$, whereas the Y axis is the third bioelectrical impedance $Z_{bR}$ and when the Lissajous figure for the left lung is displayed in which the X axis is the second bioelectrical impedance $Z_{aL}$, whereas the Y axis is the fourth bioelectrical impedance $Z_{bL}$, centering the Lissajous figures in the Y axis may be executed less frequently. In summary, it is preferable that centering the Lissajous figures in the axis corresponding to the third bioelectrical impedance $Z_{bR}$ or the fourth bioelectrical impedance $Z_{bL}$ among the two orthogonal coordinate axes be executed less frequently than that for the other axis corresponding to the first bioelectrical impedance $Z_{aR}$ or the second bioelectrical impedance $Z_{aL}$.

7.12. TWELFTH VARIATION

The body condition determination apparatus 1 need not include the display device 160, and may instead cause an external display device to display Lissajous figures, bar graphs, and so on. The body condition determination apparatus 1 need not include the bioelectrical impedance determination part 200, and may include an input part for inputting to the body condition determination apparatus 1 information on the bioelectrical impedances of the human body that is determined at an external bioelectrical impedance determination apparatus. The external bioelectrical impedance determination apparatus may send the information to the input device by radiowaves or by cable. The input part may be a communication interface, for example, a radiowave communication module, a network communication module, or a USB (Universal Serial Bus) interface.

7.13. THIRTEENTH VARIATION

The present invention is not limited to an apparatus or system that is adapted for only prompting training for breathing. For example, teachings of the present invention can be incorporated into various strength-training machines or systems, or in fitness training machines or systems.

7.14. FOURTEENTH VARIATION

Instead of the body condition determination apparatus 1 or the biological information input apparatus 200', an apparatus 620 shown in Figs. 86 and 87 may be used. The apparatus 620 includes a platform 610 on which the human subject stands and a handle unit 620. The platform 610 is provided with a left-foot current electrode X1 and a left-foot voltage electrode Y1 on which the left foot of the human subject will be placed, and a right-foot current electrode X2 and a right-foot voltage electrode Y2 on which the right foot of the human subject will be placed.

The handle unit 620 is provided with a human interface 622 and a display device 626. The human interface 622 includes touch buttons 624, and the human subject or another person may manipulate the touch buttons 624 in order to input personal information on the human subject, for example, the height, age, and sex into the apparatus 620. The display device 626 shows the measurement results, such as the weight or the type of respiration. The display device 626 also shows instructions (of rhythm and pattern of exhalation and inhalation) to exhale and inhale in order to lead the human subject to perform abdominal breathing. The display device 626 shows messages for leading the human subject to input various information into the human interface 622. The handle unit 620 further includes a right electrode handle 630R and a left electrodes handle 630L. The right electrode handle 630R includes a right-hand current electrode X4 and a right-hand voltage electrode Y4, whereas the left electrode handle 30L includes a left-hand current electrode X3 and a left-hand voltage electrode Y3,

The handle unit 620 is mechanically connected with a housing (not shown) beneath the platform 610 via a cable 640, and is electrically connected with electric circuits beneath the platform 610. The cable 640 is extendable, i.e., capable of being pull out from the housing (not shown) beneath the platform 610, so that the handle unit 620 can be brought into a position shown in Fig. 87 in which the apparatus 620 is away from the platform 610, from another position shown in Fig. 86 in which the apparatus 620 is close to the platform 610. The cable 640 is retractable, i.e., wound by a take-up unit (not shown) beneath the platform 610, so that handle unit 620 can be brought into the position shown in Fig, 86 in which the apparatus 620 is close to the platform 610, from the position shown in Fig. 87 in which the apparatus 620 is away from the platform 610.

In the use of the apparatus 620, the human subject stands on the platform 610, grips the electrode handles 630R and 630L, extends the cable 640, and holds the arms at a predetermined able, e.g., horizontally. Then, the bioelectrical impedances are measured.

**Claims**

1. A respiration characteristic analysis apparatus (1, 620) comprising:

   a bioelectrical impedance determiner (170, 200) adapted for determining a bioelectrical impedance ($Z_{aR}$) at a part including the right lung of a human subject and for determining a bioelectrical impedance ($Z_{aL}$) at a part including the left lung of the human subject; and
   a respiration depth calculator (170) adapted for calculating a right lung respiration depth (BR1, BR2) related to a respiration capability of the right lung of the human subject on the basis of change over time in the bioelectrical impedance ($Z_{aR}$, $Z_{bR}$) at the part including the right lung determined by the bioelectrical impedance determiner (170, 200), and for calculating a left lung respiration depth (BL1, BL2) related to a respiration capability of the left lung of the human subject on the basis of change over time in the bioelectrical impedance ($Z_{aL}$, $Z_{bL}$) at the part including the left lung determined by the bioelectrical impedance determiner (170, 200).

2. The respiration characteristic analysis apparatus (1, 620) according to claim 1, wherein the bioelectrical impedance determiner (170, 200) is adapted for determining a first bioelectrical impedance ($Z_{aR}$) at the right upper body trunk of the human subject including the upper lobe of the right lung of the human subject and excluding the abdomen of the human subject, and for determining a second bioelectrical impedance ($Z_{aL}$) at the left upper body trunk of the human subject including the upper lobe of the left lung of the human subject and excluding the abdomen of the human subject, and

wherein the respiration depth calculator (170) is adapted for calculating a first right lung respiration depth (BR1) related to a respiration capability of the upper lobe of the right lung of the human subject on the basis of change over time in the first bioelectrical impedance ($Z_{aR}$), and for calculating a first left lung respiration depth (BL1) related to a respiration capability of the upper lobe of the left lung of the human subject on the basis of change over time in the second bioelectrical impedance ($Z_{aL}$).

3. The respiration characteristic analysis apparatus (1, 620) according to claim 2, further comprising:

a first centering value generator (170) adapted for generating a first centering value ($Z_{aR0}$) that is an average of the first bioelectrical impedances ($Z_{aR}$) within a past unit time on the basis of change over time in the first bioelectrical impedance ($Z_{aR}$), and for generating a second centering value ($Z_{aL0}$) that is an average of the second bioelectrical impedances ($Z_{aL}$) within a past unit time on the basis of change over time in the second bioelectrical impedance ($Z_{aL}$), the first centering value ($Z_{aR0}$) being a standard level of change over time in the first bioelectrical impedance ($Z_{aR}$), the second centering value ($Z_{aL0}$) being a standard level of change over time in the second bioelectrical impedance ($Z_{aL}$);

a first difference calculator (170) adapted for calculating a first difference ($\Delta Z_{aR}$) between the first bioelectrical impedance ($Z_{aR}$) and the first centering value ($Z_{aR0}$); and

a second difference calculator (170) adapted for calculating a second difference ($\Delta Z_{aL}$) between the second bioelectrical impedance ($Z_{aL}$) and the second centering value ($Z_{aL0}$),

wherein the respiration depth calculator (170) is adapted for calculating the first right lung respiration depth (BR1) on the basis of the first difference ($\Delta Z_{aR}$), and for calculating the first left lung respiration depth (BL1) on the basis of the second difference ($\Delta Z_{aL}$).

4. The respiration characteristic analysis apparatus (1, 620) according to claim 3, wherein the respiration depth calculator (170) is adapted for calculating, at every of the human subject, the first right lung respiration depth (BR1) that is a sum of absolute values of a local maximum ($\Delta Z_{aR}$(MAX)) and a local minimum ($\Delta Z_{aR}$(MIM)) of the first differences ($\Delta Z_{aR}$) within a single respiration, and wherein the respiration depth calculator (170) is adapted for calculating, at every respiration of the human subject, the first left lung respiration depth (BL1) that is a sum of absolute values of a local maximum ($\Delta Z_{aL}$(MAX)) and a local minimum ($\Delta Z_{aL}$(MIM)) of the second differences ($\Delta Z_{aL}$) within a single respiration.

5. The respiration characteristic analysis apparatus (1, 620) according to claim 3 or 4, wherein the bioelectrical impedance determiner (170, 200) is adapted for determining the first bioelectrical impedance ($Z_{aR}$) and the second bioelectrical impedance ($Z_{aL}$) at each sampling time occurring at a predetermined cycle,

wherein the first centering value generator (170) is adapted for generating the first centering value ($Z_{aR0}$) on the basis of the first bioelectrical impedance ($Z_{aR}$) at each of sampling times, a number of the sampling times being predetermined, and

wherein the first centering value generator (170) is adapted for generating the second centering value ($Z_{aL0}$) on the basis of the second bioelectrical impedance ($Z_{aL}$) at each of sampling times, a number of the sampling times being predetermined.

6. The respiration characteristic analysis apparatus (1, 620) according to claim 5, wherein the first centering value generator (170) is adapted for calculating a first moving average (MA10) at each sampling time, the first moving average (MA10) being a moving average of the first bioelectrical impedances ($Z_{aR}$) at multiple sampling times within a centering period starting from a time point that is a predetermined time length before a current sampling time and ending at the current sampling time, wherein the first centering value generator (170) is adapted for generating the first centering value ($Z_{aR0}$) at the current sampling time on the basis of the first moving averages (MA10) at multiple sampling times,

wherein the first centering value generator (170) is adapted for calculating a second moving average (MA10) at each sampling time, the second moving average (MA10) being a moving average of the second bioelectrical impedances ($Z_{aL}$) at multiple sampling times within the centering period, and wherein the first centering value generator (170) is adapted for generating the second centering value ($Z_{aL0}$) at the current sampling time on the basis of the second moving averages (MA10) at multiple sampling times.

7. The respiration characteristic analysis apparatus (1, 620) according to claim 6, wherein a time length of the centering period is variable and is set depending on the respiration speed of the human subject at the current sampling time.

8. The respiration characteristic analysis apparatus (1, 620) according to any one of claims 5 through 7, wherein the bioelectrical impedance determiner (170, 200) is adapted for determining a third bioelectrical impedance ($Z_{bR}$) at the right middle body trunk of the human subject including the median and lower lobes of the right lung of the human subject and the abdomen of the human subject at each sampling time, and is adapted for determining a fourth bioelectrical impedance ($Z_{bL}$) at the left middle body trunk of the human subject including the median and lower lobes of the left lung of the human subject and the abdomen of the human subject at each sampling time,
the respiration characteristic analysis apparatus (1, 620) further comprising:

   a second centering value generator (170) adapted for generating a third centering value ($Z_{bR0}$) that is an average of the third bioelectrical impedances ($Z_{bR}$) within a past unit time on the basis of change over time in the third bioelectrical impedance ($Z_{bR}$), and for generating a fourth centering value ($Z_{bL0}$) that is an average of the fourth bioelectrical impedances ($Z_{bL}$) within a past unit time on the basis of change over time in the fourth bioelectrical impedance ($Z_{bL}$), the third centering value ($Z_{bR0}$) being a standard level of change over time in the third bioelectrical impedance ($Z_{bR}$), the fourth centering value ($Z_{bL0}$) being a standard level of change over time in the fourth bioelectrical impedance ($Z_{bL}$);
   a third difference calculator (170) adapted for calculating a third difference ($\Delta Z_{bR}$) between the third bioelectrical impedance ($Z_{bR}$) and the third centering value ($Z_{bR0}$);
   a fourth difference calculator (170) adapted for calculating a fourth difference ($\Delta Z_{BL}$) between the fourth bioelectrical impedance ($Z_{bL}$) and the fourth centering value ($Z_{bL0}$); and
   a zero-cross time decider (170) adapted for deciding first zero-cross times in which the first bioelectrical impedance ($Z_{aR}$) is equal to the first centering value ($Z_{aR0}$), and for deciding second zero-cross times in which the second bioelectrical impedance ($Z_{aL}$) is equal to the second centering value ($Z_{aL0}$),
   wherein the second centering value generator (170) is adapted for generating a third centering value ($Z_{bR0}$) on the basis of the third bioelectrical impedances ($Z_{bR}$) at the first zero-cross times decided by the zero-cross time decider (170), and for generating a fourth centering value ($Z_{BL0}$) on the basis of the fourth bioelectrical impedances ($Z_{bL}$) at the second zero-cross times decided by the zero-cross time decider (170), and
   wherein the respiration depth calculator (170) is adapted for calculating a second right lung respiration depth (BR2) related to a respiration capability of the median and lower lobes of the right lung of the human subject on the basis of the third difference ($\Delta Z_{bR}$), and for calculating a second left lung respiration depth (BL2) related to a respiration capability of the median and lower lobes of the left lung of the human subject on the basis of the fourth difference ($\Delta Z_{bL}$),

9. The respiration characteristic analysis apparatus (1, 620) according to claim 8, wherein the second centering value generator (170) is adapted for deciding whether or not each sampling time is a first zero-cross time, and for generating the third centering value ($Z_{bR0}$) at the current sampling time on the basis of the third bioelectrical impedances ($Z_{bR}$) including the third bioelectrical impedance ($Z_{bR}$) at the current sampling time if the current sampling time is a first zero-cross time, and wherein the centering value generator (170) is adapted for deciding the third centering value ($Z_{bR0}$) generated at a last sampling time as the third centering value ($Z_{bR0}$) at the current sampling time if the current sampling time is not a first zero-cross time.

10. The respiration characteristic analysis apparatus (1, 620) according to claim 8 or 9, wherein the second centering value generator (170) is adapted for deciding whether or not each sampling time is a second zero-cross time, and for generating the fourth centering value ($Z_{bL0}$) at the current sampling time on the basis of the fourth bioelectrical impedances ($Z_{bL}$) including the fourth bioelectrical impedance ($Z_{bL}$) at the current sampling time if the current sampling time is a second zero-cross time, and wherein the centering value generator (170) is adapted for deciding the fourth centering value ($Z_{bL0}$) generated at a last sampling time as the fourth centering value ($Z_{bL0}$) at the current sampling time if the current sampling time is not a second zero-cross time.

11. The respiration characteristic analysis apparatus (1, 620) according to any one of claims 8 through 10, wherein the respiration depth calculator (170) is adapted for calculating, at every respiration of the human subject, the second right lung respiration depth (BR2) that is a sum of absolute values of a local maximum and a local minimum of the third differences ($\Delta Z_{bR}$) within a single respiration, and wherein the respiration depth calculator (170) is adapted for calculating, at every respiration of the human subject, the second left lung respiration depth (BL2) that is a sum of absolute values of a local maximum and a local minimum of the fourth differences ($\Delta Z_{bL}$) within a single respiration.

12. The respiration characteristic analysis apparatus (1, 620) according to claim 1, wherein the bioelectrical impedance determiner (170, 200) is adapted for determining a third bioelectrical impedance ($Z_{bR}$) at the right middle body trunk of the human subject including the median and lower lobes of the right lung of the human subject and the abdomen of the human subject, and for determining a fourth bioelectrical impedance ($Z_{bL}$) at the left middle body trunk of the human subject including the median and lower lobes of the left lung of the human subject and the abdomen of the human subject,
wherein the respiration depth calculator (170) is adapted for calculating a second right lung respiration depth (BR2) related to a respiration capability of the median and lower lobes of the right lung of the human subject on the basis of change over time in the third bioelectrical impedance ($Z_{bR}$), and is adapted for calculating a second left lung respiration depth (BL2) related to a respiration capability of the median and lower lobes of the left lung of the human subject on the basis of change over time in the fourth bioelectrical impedance ($Z_{bL}$).

13. The respiration characteristic analysis apparatus (1, 620) according to claim 1, wherein the bioelectrical impedance determiner (170, 200) is adapted for determining a first bioelectrical impedance ($Z_{aR}$) at the right upper body trunk of the human subject including the upper lobe of the right lung of the human subject and excluding the abdomen of the human subject, for determining a second bioelectrical impedance ($Z_{aL}$) at the left upper body trunk of the human subject including the upper lobe of the left lung of the human subject and excluding the abdomen of the human subject, for determining a third bioelectrical impedance ($Z_{bR}$) at the right middle body trunk of the human subject including the median and lower lobes of the right lung of the human subject and the abdomen of the human subject, and for determining a fourth bioelectrical impedance ($Z_{bL}$) at the left middle body trunk of the human subject including the median and lower lobes of the left lung of the human subject and the abdomen of the human subject, and
wherein the respiration depth calculator (170) is adapted for calculating the right lung respiration depth (BR1, Bur2) related to the respiration capability of the right lung of the human subject on the basis of change over time in each of the first bioelectrical impedance ($Z_{aR}$) and the third bioelectrical impedance ($Z_{bR}$), and for calculating the left lung respiration depth (BL1, BL2) related to the respiration capability of the left lung of the human subject on the basis of change over time in each of the second bioelectrical impedance ($Z_{aL}$) and the fourth bioelectrical impedance ($Z_{bL}$).

14. The respiration characteristic analysis apparatus (1, 620) according to claim 13, further comprising a display data generator (170) adapted for generating first display data for displaying a first Lissajous figure showing change over time in the first bioelectrical impedance ($Z_{aR}$) and change over time in the third bioelectrical impedance ($Z_{bR}$) in an orthogonal coordinate system having two orthogonal coordinate axes in which a first axis is the first bioelectrical impedance ($Z_{aR}$) and a second axis is the third bioelectrical impedance ($Z_{bR}$), and for generating second display data for displaying a second Lissajous figure showing change over time in the second bioelectrical impedance ($Z_{aL}$) and change over time in the fourth bioelectrical impedance ($Z_{bL}$) in an orthogonal coordinate system having two orthogonal coordinate axes in which a first axis is the second bioelectrical impedance ($Z_{aL}$) and a second axis is the fourth bioelectrical impedance ($Z_{bL}$).

15. The respiration characteristic analysis apparatus (1, 620) according to claim 14, wherein the display data generator (170) is adapted for generating the first display data for displaying the first Lissajous figure and the second display data for displaying the second Lissajous figure so that the first Lissajous figure and the second Lissajous figure are overlaid on a screen (160A).

16. The respiration characteristic analysis apparatus (1, 620) according to claim 14 or 15, wherein the display data generator (170) is adapted for generating the first display data for displaying the first Lissajous figure and the second display data for displaying the second Lissajous figure so that a displaying manner for the first Lissajous figure is different from a displaying manner for the second Lissajous figure.

17. The respiration characteristic analysis apparatus (1, 620) according to any one of claims 14 through 16, further comprising a track analyzer (170) adapted for detecting differences between a track of the first Lissajous figure and a track of the second Lissajous figure,
wherein the display data generator (170) is adapted for generating the first display data for displaying the first Lissajous figure and the second display data for displaying the second Lissajous figure so that the differences are highlighted on a screen (160A).

**18.** The respiration characteristic analysis apparatus (1, 620) according to any one of claims 14 through 17, further comprising a local-maximum-and-minimum decider (170) adapted for deciding a first local maximum that is a local maximum of change in the first bioelectrical impedance ($Z_{aR}$), for deciding a first local minimum that is a local minimum of change in the first bioelectrical impedance ($Z_{aR}$), for deciding a second local maximum that is a local maximum of change in the second bioelectrical impedance ($Z_{aL}$), for deciding a second local minimum that is a local minimum of change in the second bioelectrical impedance ($Z_{aL}$), for deciding a third local maximum that is a local maximum of change in the third bioelectrical impedance ($Z_{bR}$), for deciding a third local minimum that is a local minimum of change in the third bioelectrical impedance ($Z_{bR}$), for deciding a fourth local maximum that is a local maximum of change in the fourth bioelectrical impedance ($Z_{bL}$), and for deciding a fourth local minimum that is a local minimum of change in the fourth bioelectrical impedance ($Z_{bL}$),

wherein the display data generator (170) is adapted for generating the first display data for displaying the first Lissajous figure so that a range of the first Lissajous figure on a screen (160A) on which the first and second Lissajous figures are displayed in the first axis is adjusted on the basis of the first local maximum and the first local minimum, and a range of the first Lissajous figure on the screen (160A) in the second axis is adjusted on the basis of the third loyal maximum and the third local minimum, and

wherein the display data generator (170) is adapted for generating the second display data for displaying the second Lissajous figure so that a range of the second Lissajous figure on the screen (160A) in the first axis is adjusted on the basis of the second local maximum and the second local minimum, and a range of the second Lissajous figure on the screen (160A) in the second axis is adjusted on the basis of the fourth local maximum and the fourth local minimum.

**19.** The respiration characteristic analysis apparatus (1, 620) according to any one of claims 14 through 17, further comprising a local-maximum-and-minimum decider (170) adapted for deciding a first local maximum that is a local maximum of change in the first bioelectrical impedance ($Z_{aR}$), for deciding a first local minimum that is a local minimum of change in the first bioelectrical impedance ($Z_{aR}$), for deciding a second local maximum that is a local maximum of change in the second bioelectrical impedance ($Z_{aL}$), for deciding a second local minimum that is a local minimum of change in the second bioelectrical impedance ($Z_{aL}$), for deciding a third local maximum that is a local maximum of change in the third bioelectrical impedance ($Z_{bR}$), for deciding a third local minimum that is a local minimum of change in the third bioelectrical impedance ($Z_{bR}$), for deciding a fourth local maximum that is a local maximum of change in the fourth bioelectrical impedance($Z_{bL}$), and for deciding a fourth local minimum that is a local minimum of change in the fourth bioelectrical impedance ($Z_{bL}$),

wherein when the display data generator (170) generates the first display data for displaying the first Lissajous figure and the second display data for displaying the second Lissajous figure, the display data generator (170) is adapted for executing a first range adjustment process in which a range of the first Lissajous figure on a screen (160A) in which the first and second Lissajous figures are displayed in the first axis is adjusted on the basis of the first local maximum and the first local minimum, whereas a range of the second Lissajous figure on the screen (160A) in the first axis is adjusted on the basis of the second local maximum and the second local minimum, and is adapted for executing a second range adjustment process in which a range of the first Lissajous figure on the screen (160A) in the second axis is adjusted on the basis of the third local maximum and the third local minimum, whereas a range of the second Lissajous figure on the screen (160A) in the second axis is adjusted on the basis of the fourth local maximum and the fourth local minimum, and wherein the display data generator (170) is adapted for executing the second range adjustment process less frequently than that for the first range adjustment process.

**20.** The respiration characteristic analysis apparatus (1, 620) according to claim 13, further comprising a display data generator (170) adapted for generating display data for displaying a Lissajous figure showing change over time in the first bioelectrical impedance ($Z_{aR}$) and change over time in the third bioelectrical impedance ($Z_{bR}$) in an orthogonal coordinate system having two orthogonal coordinate axes in which a first axis is the first bioelectrical impedance ($Z_{aR}$) and a second axis is the third bioelectrical impedance ($Z_{bR}$), or for generating display data for displaying a Lissajous figure showing change over time in the second bioelectrical impedance ($Z_{aL}$) and change over time in the fourth bioelectrical impedance ($Z_{bL}$) in an orthogonal coordinate system having two orthogonal coordinate axes in which a first axis is the second bioelectrical impedance ($Z_{aL}$) and a second axis is the fourth bioelectrical impedance ($Z_{bL}$).

**21.** The respiration characteristic analysis apparatus (1, 620) according to claim 20, wherein the display data generator (170) is adapted for generating the display data for displaying the Lissajous figure so that a displaying manner for a track of the Lissajous figure for a latest single respiration is different from a displaying manner for a track of the Lissajous figure for past respirations.

**22.** The respiration characteristic analysis apparatus (1, 620) according to claim 20, wherein the display data generator (170) is adapted for generating the display data for displaying the Lissajous figure so that a displaying manner for tracks of the Lissajous figure is changed depending on an elapsed time.

**23.** The respiration characteristic analysis apparatus (1, 620) according to any one of claims 20 through 22, wherein the display data generator (170) is adapted for further generating target display data for displaying a target Lissajous figure showing a target model of breathing having a type and a magnitude of respiration to be performed by the human subject for guiding the human subject to perform breathing.

**24.** The respiration characteristic analysis apparatus (1, 620) according to any one of claims 20 through 23, further comprising:

an inclination angle calculator (170) adapted for calculating an inclination angle ($\alpha$) of a track of the Lissajous figure; and

a ventilation capability determiner (170) adapted for comparing the inclination angle ($\alpha$) calculated by the inclination angle calculator (170) with a predetermined reference inclination angle ($\beta$), so as to decide whether or not a lung ventilation capability of the human subject is good or bad.

**25.** The respiration characteristic analysis apparatus (1, 620) according to any one of claims 1 through 24, further comprising:

a memory (120) adapted for storing training menus that are used for training the human subject for breathing, the training menus being classified into rankings of respiration capability, the memory (120) storing requirements for advancing through the rankings;

a respiration capability determiner (170) adapted for determining a respiration capability of the human subject on the basis of determination by the bioelectrical impedance determiner (170, 200) or calculation by the respiration depth calculator (170); and

a training manager (170) adapted for referring to the memory (120) for identifying a ranking corresponding to the respiration capability determined by the respiration capability determiner (170), and for executing a process for training the human subject for breathing using the training menus corresponding to the ranking,

wherein the training manager (170) is adapted for advancing the ranking to a next ranking if the requirement for advancing through the ranking is satisfied.

**26.** The respiration characteristic analysis apparatus (1, 620) according to claim 25, wherein the bioelectrical impedance determiner (170, 200) is adapted for determining a fifth bioelectrical impedance ($Z_a$) at the upper body trunk of the human subject including the upper lobes of the lungs of the human subject and excluding the abdomen of the human subject, and for determining a sixth bioelectrical impedance ($Z_b$) at the middle body trunk of the human subject including the median and lower lobes of lungs of the human subject and the abdomen of the human subject,

wherein the respiration capability determiner (170) is adapted for determining a type and a magnitude of respiration of the human subject as the respiration capability of the human subject on the basis of determination by the bioelectrical impedance determiner (170, 200).

**27.** The respiration characteristic analysis apparatus (1, 620) according to claim 25, further comprising:

a third centering value generator (170) adapted for generating a fifth centering value ($Z_{a0}$) that is an average of the fifth bioelectrical impedances ($Z_a$) within a past unit time on the basis of change over time in the fifth bioelectrical impedance ($Z_a$), and for generating a sixth centering value ($Z_{b0}$) that is an average of the sixth bioelectrical impedances ($Z_b$) within a past unit time on the basis of change over time in the sixth bioelectrical impedance ($Z_b$), the fifth centering value ($Z_{a0}$) being a standard level of change over time in the fifth bioelectrical impedance ($Z_a$), the sixth centering value ($Z_{b0}$) being a standard level of change over time in the sixth bioelectrical impedance ($Z_b$);

a fifth difference calculator (170) adapted for calculating a fifth difference ($\Delta Z_a$) between the fifth bioelectrical impedance ($Z_a$) and the fifth centering value ($Z_{a0}$); and

a sixth difference calculator (170) adapted for calculating a sixth difference ($\Delta Z_b$) between the sixth bioelectrical impedance ($Z_b$) and the sixth centering value ($Z_{b0}$),

wherein the respiration capability determiner (170) is adapted for determining a type and a magnitude of respiration of the human subject on the basis of the fifth difference ($\Delta Z_a$) and the sixth difference ($\Delta Z_b$).

**28.** The respiration characteristic analysis apparatus (1, 620) according to claim 27, further comprising a second zero-cross time decider (170) adapted for deciding third zero-cross times in which the fifth bioelectrical impedance ($Z_a$) is equal to the fifth centering value ($Z_{a0}$), and for deciding fourth zero-cross times in which the sixth bioelectrical impedance ($Z_b$) is equal to the sixth centering value ($Z_{b0}$),

wherein the bioelectrical impedance determiner (170, 200) is adapted for determining the fifth bioelectrical impedance ($Z_a$) and the sixth bioelectrical impedance ($Z_b$) at each sampling time occurring at a predetermined cycle,

wherein the third centering value generator (170) is adapted for generating the fifth centering value ($Z_{a0}$) on the basis of the fifth bioelectrical impedance ($Z_a$) at each of sampling times, a number of the sampling times being predetermined, and

wherein the third centering value generator (170) is adapted for generating the sixth centering value ($Z_{b0}$) on the basis of the sixth bioelectrical impedance ($Z_b$) at each of third zero-cross times decided by the second zero-cross time decider (170), a number of the third zero-cross times being predetermined.

**29.** The respiration characteristic analysis apparatus (1, 620) according to claim 28, wherein
the third centering value generator (170) is adapted for calculating a moving average (MA10) at each sampling time, the moving average (MA10) being a moving average of the fifth bioelectrical impedances ($Z_a$) at multiple sampling times within a centering period starting from a time point that is a predetermined time length before a current sampling time and ending at the current sampling time, and wherein the third centering value generator (170) is adapted for generating the fifth centering value ($Z_{a0}$) at the current sampling time on the basis of the moving averages (MA10) at multiple sampling times.

**30.** The respiration characteristic analysis apparatus (1, 620) according to claim 29, wherein a time length of the centering period is variable and is set depending on the respiration speed of the human subject at the current sampling time.

**31.** The respiration characteristic analysis apparatus (1, 620) according to any one of claims 28 through 30, wherein the third centering value generator (170) is adapted for deciding whether or not each sampling time is a third zero-cross time, and for generating the sixth centering value ($Z_{b0}$) at the current sampling time on the basis ofthe sixth bioelectrical impedances ($Z_b$) including the sixth bioelectrical impedance($Z_b$) at the current sampling time if the current sampling time is a third zero-cross time, and wherein the third centering value generator (170) is adapted for deciding the sixth centering value ($Z_{b0}$) generated at a last sampling time as the sixth centering value ($Z_{b0}$) at the current sampling time if the current sampling time is not a third zero-cross time.

**32.** The respiration characteristic analysis apparatus (1, 620) according to any one of claims 26 through 31, further comprising a display data generator (170) adapted for generating display data for displaying a Lissajous figure showing change over time in the fifth bioelectrical impedance ($Z_a$) and change over time in the sixth bioelectrical impedance ($Z_b$) in an orthogonal coordinate system having two orthogonal coordinate axes in which a first axis is the fifth bioelectrical impedance ($Z_a$) and a second axis is the sixth bioelectrical impedance ($Z_b$).

**33.** The respiration characteristic analysis apparatus (1, 620) according to claim 26, further comprising:

a display data generator (170) adapted for generating display data for displaying a Lissajous figure showing change over time in the fifth bioelectrical impedance ($Z_a$) and change over time in the sixth bioelectrical impedance ($Z_b$) in an orthogonal coordinate system having two orthogonal coordinate axes in which a first axis is the fifth bioelectrical impedance ($Z_a$) and a second axis is the sixth bioelectrical impedance ($Z_b$); and

a third centering value generator (170) adapted for generating a fifth centering value ($Z_{a0}$) that is an average of the fifth bioelectrical impedances ($Z_a$) within a past unit time on the basis of change over time in the fifth bioelectrical impedance ($Z_a$), and for generating a sixth centering value ($Z_{b0}$) that is an average of the sixth bioelectrical impedances ($Z_b$) within a past unit time on the basis of change over time in the sixth bioelectrical impedance ($Z_b$), the fifth centering value ($Z_{a0}$) being a standard level of change over time in the fifth bioelectrical impedance ($Z_a$), the sixth centering value ($Z_{b0}$) being a standard level of change over time in the sixth bioelectrical impedance ($Z_b$),

wherein the display data generator (170) is adapted for generating display data for displaying the Lissajous figure so that a position (C) on the Lissajous figure defined by the fifth centering value ($Z_{a0}$) and the sixth centering value ($Z_{b0}$) is located at a center of a screen (160A) in which the Lissajous figure is displayed.

**34.** The respiration characteristic analysis apparatus (1, 620) according to claim 26, further comprising a display data generator (170) adapted for generating display data for displaying a Lissajous figure showing change over time in the fifth bioelectrical impedance ($Z_a$) and change over time in the sixth bioelectrical impedance ($Z_b$) in an orthogonal coordinate system having two orthogonal coordinate axes in which a first axis is the fifth bioelectrical impedance ($Z_a$) and a second axis is the sixth bioelectrical impedance ($Z_b$); and

a third centering value generator (170) adapted for generating a fifth centering value ($Z_{a0}$) that is an average of the fifth bioelectrical impedances ($Z_a$) within a past unit time on the basis of change over time in the fifth bioelectrical impedance ($Z_a$), and for generating a sixth centering value ($Z_{b0}$) that is an average of the sixth bioelectrical impedances ($Z_b$) within a past unit time on the basis of change over time in the sixth bioelectrical impedance ($Z_b$), the fifth centering value ($Z_{a0}$) being a standard level of change over time in the fifth bioelectrical impedance ($Z_a$), the sixth centering value ($Z_{b0}$) being a standard level of change over time in the sixth bioelectrical impedance ($Z_b$), wherein when the display data generator (170) generates the display data for displaying the Lissajous figure, the display data generator (170) is adapted for executing a first location centering process in which the Lissajous figure is centered in the first axis with respect to a screen (160A) in which the Lissajous figure is displayed on the basis of the fifth centering value ($Z_{a0}$), and is adapted for executing a second location centering process in which the Lissajous figure is centered in the second axis with respect to the screen (160A) on the basis of the sixth centering value ($Z_{b0}$), wherein the display data generator (170) is adapted for executing the second location centering process at less frequently than that for the first location centering process.

35. The respiration characteristic analysis apparatus (1, 620) according to any one of claims 32 through 34, further comprising a local-maximum-and-minimum decider (170) adapted for deciding a fifth local maximum that is a local maximum of change in the fifth bioelectrical impedance ($Z_a$), for deciding a fifth local minimum that is a local minimum of change in the fifth bioelectrical impedance ($Z_a$), for deciding a sixth local maximum that is a local maximum of change in the sixth bioelectrical impedance ($Z_b$), and for deciding a sixth local minimum that is a local minimum of change in the sixth bioelectrical impedance ($Z_b$), wherein the display data generator (170) is adapted for generating the display data for displaying the Lissajous figure so that a range of the Lissajous figure on a screen (160A) in which the Lissajous figure is displayed in the first and second axes is adjusted on the basis of the fifth local maximum, the fifth local minimum, the sixth local maximum, and the sixth local minimum.

36. The respiration characteristic analysis apparatus (1, 620) according to any one of claims 32 through 34, further comprising a local-maximum-and-minimum decider (170) adapted for deciding a fifth local maximum that is a local maximum of change in the fifth bioelectrical impedance ($Z_a$), for deciding a fifth local minimum that is a local minimum of change in the fifth bioelectrical impedance ($Z_a$), for deciding a sixth local maximum that is a local maximum of change in the sixth bioelectrical impedance ($Z_b$), and for deciding a sixth local minimum that is a local minimum of change in the sixth bioelectrical impedance ($Z_b$), wherein when the display data generator (170) generates the display data for displaying the Lissajous figure, the display data generator (170) is adapted for executing a first range adjustment process in which a range of the Lissajous figure on a screen (160A) in which the Lissajous figure is displayed in the first axis is adjusted on the basis of the fifth local maximum and the fifth local minimum, and is adapted for executing a second range adjustment process in which a range of the Lissajous figure on the screen (160A) in the second axis is adjusted on the basis of the sixth local maximum and the sixth local minimum, and wherein the display data generator (170) is adapted for executing the second range adjustment process less frequently than that for the first range adjustment process.

37. The respiration characteristic analysis apparatus (1, 620) according to any one of claims 32 through 36, wherein the display data generator (170) is adapted for generating the display data for displaying the Lissajous figure so that a displaying manner for a track of the Lissajous figure for a latest single respiration is different from a displaying manner for a track of the Lissajous figure for past respirations.

38. The respiration characteristic analysis apparatus (1, 620) according to any one of claims 32 through 36, wherein the display data generator (170) is adapted for generating the display data for displaying the Lissajous figure so that a displaying manner for tracks of the Lissajous figure is changed depending on an elapsed time.

39. The respiration characteristic analysis apparatus (1, 620) according to any one of claims 32 through 38, wherein the display data generator (170) is adapted for further generating target display data for displaying a target Lissajous figure showing a target model of breathing having a type and a magnitude of respiration to be performed by the human subject for guiding the human subject to perform breathing.

40. The respiration characteristic analysis apparatus (1, 620) according to any one of claims 32 through 39, further comprising:

an inclination angle calculator (170) adapted for calculating an inclination angle ($\alpha$) of a track of the Lissajous figure; and

a ventilation capability determiner (170) adapted for comparing the inclination angle ($\alpha$) calculated by the inclination angle calculator (170) with a predetermined reference inclination angle ($\beta$), so as to decide whether or not a lung ventilation capability of the human subject is good or bad.

41. The respiration characteristic analysis apparatus (1, 620) according to any one of claims 27 through 40, further comprising:

a respiration depth calculator (170) adapted for calculating a respiration depth of the human subject at every respiration of the human subject; and

a graph generator (170) adapted for generating display data for indicating a graph showing change over time of respiration depth calculated by the respiration depth calculator (170), in such a manner that the graph is nonlinearly compressed in a direction of time axis and earlier time intervals are more compressed than later time intervals, so that a time resolution for later time intervals is higher than that for earlier time intervals.

42. A respiration characteristic analysis apparatus (300) comprising:

an input part (320) for inputting to the respiration characteristic analysis apparatus (300) a bioelectrical impedance at a part including the right lung of a human subject and for determining a bioelectrical impedance at a part including the left lung of the human subject, the bioelectrical impedances being determined at a bioelectrical impedance determination apparatus (200', 620); and

a respiration depth calculator (360) adapted for calculating a right lung respiration depth (BR1, BR2) related to a respiration capability of the right lung of the human subject on the basis of change over time in the bioelectrical impedance ($Z_{aR}$, $Z_{bR}$) at the part including the right lung, and for calculating a left lung respiration depth (BL I, BL2) related to a respiration capability of the left lung of the human subject on the basis of change over time in the bioelectrical impedance ($Z_{aL}$, $Z_{bL}$) at the part including the left lung.

43. The respiration characteristic analysis apparatus (300) according to claim 42, further comprising:

a memory (303) adapted for storing training menus that are used for training the human subject for breathing, the training menus being classified into rankings of respiration capability, the memory (303) storing requirements for advancing through the rankings;

a respiration capability determiner (360) adapted for determining a respiration capability of the human subject on the basis of change over time in each of the bioelectrical impedances input by the input part (320); and

a training manager (360) adapted for referring to the memory (303) for identifying a ranking corresponding to the respiration capability determined by the respiration capability determiner (360), and for executing a process for training the human subject for breathing using the training menus corresponding to the ranking,

wherein the training manager (360) is adapted for advancing the ranking to a next ranking if the requirement for advancing through the ranking is satisfied.

44. A respiration characteristic analysis system (5) comprising:

a bioelectrical impedance determiner (360) adapted for determining a bioelectrical impedance ($Z_{aR}$, $Z_{bR}$) at a part including the right lung of a human subject and for determining a bioelectrical impedance ($Z_{aL}$, $Z_{bL}$) at a part including the left lung of the human subject; and

a respiration depth calculator (360) adapted for calculating a right lung respiration depth (BR1, BR2) related to a respiration capability of the right lung of the human subject on the basis of change over time in the bioelectrical impedance ($Z_{aR}$, $Z_{bR}$) at the part including the right lung determined by the bioelectrical impedance determiner (360), and for calculating a left lung respiration depth (BL1, BL2) related to a respiration capability of the left lung of the human subject on the basis of change over time in the bioelectrical impedance ($Z_{aL}$, $Z_{bL}$) at the part including the left lung determined by the bioelectrical impedance determiner (360).

45. The respiration characteristic analysis system (5) according to claim 44, further comprising:

a memory (303) adapted for storing training menus that are used for training the human subject for breathing, the training menus being classified into rankings of respiration capability, the memory (303) storing requirements for advancing through the rankings;

a respiration capability determiner (360) adapted for determining a respiration capability of the human subject on the basis of the bioelectrical impedances determined by the bioelectrical impedance determiner (360); and

a training manager (360) adapted for referring to the memory (303) for identifying a ranking corresponding to the respiration capability determined by the respiration capability) determiner (360), and for executing a process for training the human subject for breathing using the training menus corresponding to the ranking,

wherein the training manager (360) is adapted for advancing the ranking to a next ranking if the requirement for advancing through the ranking is satisfied.

## Fig. 1

Fig. 2

1  160 (150)  30R

30L

30

20  X1  Y1  X2  Y2

Fig. 3

160 (150)  X4  Y4

X3

Y3

Fig. 4

(A) SYSTEMIC BODY

(B) RIGHT LOWER EXTREMITY

(C) LEFT LOWER EXTREMITY

(D) RIGHT UPPER EXTREMITY AND UPPER BODY TRUNK

(E) LEFT UPPER EXTREMITY AND UPPER BODY TRUNK

◄- - -► CURRENT
——— VOLTAGE

(F) UPPER BODY TRUNK (BOTH HANDS)

(G) MIDDLE BODY TRUNK

(H) MIDDLE BODY TRUNK

(I) MIDDLE BODY TRUNK

(J) MIDDLE BODY TRUNK

(K) SYSTEMIC BODY (BOTH HANDS-BOTH FEET)

Fig. 5

```
                    ┌──────────────┐
                    │    START     │
                    └──────┬───────┘
                           │
                           ▼
        ┌────────────────────────────────┐
  S1 ───│     ENTER PERSONAL             │
        │     INFORMATION               │
        └───────────────┬────────────────┘
                        │
                        ▼
           ┌───────────────────────┐
     S2 ───│      MEASURE          │
           │      WEIGHT           │
           └───────────┬───────────┘
                       │
                       ▼◄──────────────────────┐
        ┌────────────────────────────────┐     │
  S3 ───│  RESPIRATION DEPTH             │     │
        │  ANALYSIS PROCESS             │     │
        └───────────────┬────────────────┘     │
                        │                       │
                        ▼                       │
        ┌────────────────────────────────┐     │
  S4 ───│  RESPIRATION DEPTH             │     │
        │  DISPLAYING PROCESS           │─────┘
        └────────────────────────────────┘
```

Fig. 6

CHEST SKELETAL MUSCLE

ABDOMINAL SKELETAL MUSCLE

LUNG

DIAPHRAGM

VISCERAL TISSUE

Fig. 7A

$Z_b$: BIOELECTRICAL IMPEDANCE AT MIDDLE BODY TRUNK

$Z_4$ CHEST SKELETAL MUSCLE

$Z_5$ LUNGS

$Z_6$ ABDOMINAL SKELETAL MUSCLE

$Z_7$ VISCERAL TISSUE

Fig. 7B

$Z_a$: BIOELECTRICAL IMPEDANCE AT UPPER BODY TRUNK

$Z_3$ UPPER EXTREMITY SKELETAL MUSCLE

$Z_1$ CHEST SKELETAL MUSCLE

$Z_2$ LUNGS

## Fig. 8

# Fig. 9

ABDOMINAL RESPIRATION

INHALATION

EXHALATION

$Z_a$

$Z_b$

IMPEDANCE

TIME

# Fig. 10

COSTAL RESPIRATION

INHALATION

EXHALATION

$Z_a$

$Z_b$

IMPEDANCE

TIME

Fig. 11

$Z_{aR}$: FIRST BIOELECTRICAL IMPEDANCE    $Z_{aL}$: SECOND BIOELECTRICAL IMPEDANCE

$Z_1R$

RIGHT CHEST
SKELETAL
MUSCLE

$Z_3R$

RIGHT UPPER
EXTREMITY
SKELETAL MUSCLE

$Z_2R$

RIGHT LUNG

$Z_1L$

LEFT CHEST
SKELETAL
MUSCLE

$Z_2L$

LEFT LUNG

$Z_3L$

LEFT UPPER
EXTREMITY
SKELETAL MUSCLE

Fig. 12

RESPIRATION DEPTH
ANALYSIS PROCESS

S10 — SAMPLING TIME? — NO

YES

S20 — DETERMINE FIRST
BIOELECTRICAL IMPEDANCE

S30 — DETERMINE SECOND
BIOELECTRICAL IMPEDANCE

S40 — SMOOTHING PROCESS

S50 — FIRST CENTERING PROCESS

S60 — SECOND CENTERING PROCESS

S70 — FIRST DIFFERENCE
CALCULATION PROCESS

S80 — SECOND DIFFERENCE
CALCULATION PROCESS

S90 — FIRST RIGHT-LUNG-RESPIRATION-
DEPTH CALCULATING PROCESS

S100 — FIRST LEFT-LUNG-RESPIRATION-
DEPTH CALCULATING PROCESS

RETURN

Fig. 13

```
        ┌─────────────────────────────┐
        │  FIRST CENTERING PROCESS    │
        └─────────────────────────────┘
                     │
                     ▼
  S51   ┌─────────────────────────────────┐
        │    MA10 CALCULATION PROCESS     │
        └─────────────────────────────────┘
                     │
                     ▼
  S52   ┌─────────────────────────────────┐
        │    MA20 CALCULATION PROCESS     │
        └─────────────────────────────────┘
                     │
                     ▼
  S53   ┌─────────────────────────────────┐
        │    MAX10 EXTRACTION PROCESS     │
        └─────────────────────────────────┘
                     │
                     ▼
  S54   ┌─────────────────────────────────┐
        │    MIN10 EXTRACTION PROCESS     │
        └─────────────────────────────────┘
                     │
                     ▼
  S55   ┌─────────────────────────────────┐
        │   MEDIAN VALUE CALCULATION      │
        │          PROCESS                │
        └─────────────────────────────────┘
                     │
                     ▼
  S56   ┌─────────────────────────────────┐
        │     RESPIRATION TIMING          │
        │    EXTRACTION PROCESS           │
        └─────────────────────────────────┘
                     │
                     ▼
  S57   ┌─────────────────────────────────┐
        │ RESPIRATION SPEED INDICATION    │
        │    FLAG SETTING PROCESS         │
        └─────────────────────────────────┘
                     │
                     ▼
  S58   ┌─────────────────────────────────┐
        │   FIRST CENTERING VALUE         │
        │    CALCULATION PROCESS          │
        └─────────────────────────────────┘
                     │
                     ▼
              ┌──────────┐
              │   END    │
              └──────────┘
```

# Fig. 14

```
        ┌─────────────────────────┐
        │   RESPIRATION TIMING     │
        │   EXTRACTION PROCESS     │
        └─────────────────────────┘
                    │
                    ▼
S201  ┌──────────────────────────────┐
      │  DIFFERENTIAL COEFFICIENT    │
      │    CALCULATION PROCESS       │
      └──────────────────────────────┘
                    │
                    ▼
S202   ⟨ |dZₐR(n)| < 0.1? ⟩ ──YES──┐
                    │                │
                   NO                │
                    ▼                │
S203   ⟨ dZₐR(n) > 0? ⟩ ──NO──┐     │
                    │           │     ▼
                  YES          │  ┌─────────┐
                    ▼          │  │ F₀(n) = 0│
        ┌──────────────┐       ▼  └─────────┘
        │  F₀(n) = +1  │  ┌──────────────┐
        └──────────────┘  │  F₀(n) = −1  │
                    │      └──────────────┘
                    ▼
S204 ⟨ |F₀(n)| = |F₀(n−1)|? AND F₀(n−1) ≠ F₀(n)? ⟩ ──NO──┐
                    │                                       │
                  YES                                       │
                    ▼                                       │
S205   ┌──────────────┐                                     │
       │  F₀(n) = 0   │                                     │
       └──────────────┘                                     │
                    │◄──────────────────────────────────────┘
                    ▼
                  ( A )
```

$S202: |dZ_aR(n)| < 0.1?$ — YES

$S203: dZ_aR(n) > 0?$ — NO

$F_0(n) = 0$

$F_0(n) = +1$

$F_0(n) = -1$

$S204: |F_0(n)| = |F_0(n-1)|?$ AND $F_0(n-1) \neq F_0(n)?$

$S205: F_0(n) = 0$

## Fig. 15

A

$F_0(n) = 0$? — S206

NO

YES

S207 — $\sum_{i=n-2}^{n} F_0(i) > +1$? — YES

NO

$\sum_{i=n-2}^{n} F_0(i) < -1$? — S208

NO

YES

$F_1(n) = 0$   $F_1(n) = -1$   $F_1(n) = +1$

S209 — $F_1(n) = +1$? — YES

NO

S210 — ADD ONE TO SAMPLING COUNTER VALUE

INITIALIZE SAMPLING COUNTER VALUE — S211

END

Fig. 16

RESPIRATION SPEED INDICATION
FLAG SETTING PROCESS

NO $F_0(n) = 0$? ~S301

YES

S302 $F_1(n) = +1$? YES

NO

NO $F_1(n) = -1$? ~S304 YES $N(n-1) > 10$? ~S303 (B)

YES NO

S305 $N(n-1) > 5$? YES

NO

$F_{ma}(n) = F_{ma}(n-1)$ (B) $F_{ma}(n) = 20$ $F_{ma}(n) = 10$

END

Fig. 17

FIRST CENTERING VALUE
CALCULATION PROCESS

S401 $F_{ma}(n) = 10?$  NO

YES

S402 $[Z_{aR0}(n-2) + Z_{aR0}(n-1) + MA10(n)]/3 = Z_{aR0}(n)$

S403 $[Z_{aR0}(n-2) + Z_{aR0}(n-1) + MA20(n)]/3 = Z_{aR0}(n)$

END

Fig. 18

# Fig. 19

```
        ┌──────────────────────────────┐
        │  FIRST RIGHT-LUNG-RESPIRATION- │
        │  DEPTH CALCULATING PROCESS    │
        └──────────────────────────────┘
                      │
                      ▼
  S501          ╱‾‾‾‾‾‾‾‾‾‾‾‾‾‾‾╲   NO
          ╱  INHALATION?   ╲──────────────────┐
                ╲‾‾‾‾‾‾‾‾‾‾‾‾‾╱                 │
                      │ YES                      │
                      ▼                          ▼
  S502    ┌──────────────────┐      S503  ┌──────────────────┐
          │   PEAK-HOLD      │            │  BOTTOM-HOLD     │
          │   PROCESS        │            │  PROCESS         │
          └──────────────────┘            └──────────────────┘
                      │                          │
                      ▼◄─────────────────────────┘
  NO    ╱‾‾‾‾‾‾‾‾‾‾‾‾‾‾‾‾‾‾‾‾‾‾‾╲
  ┌─────  FIRST ZERO-CROSS TIME?  ╲── S504
  │       ╲‾‾‾‾‾‾‾‾‾‾‾‾‾‾‾‾‾‾‾‾‾‾╱
  │                 │ YES
  │                 ▼
  │  S505    ╱‾‾‾‾‾‾‾‾‾‾‾‾‾‾╲   NO
  │     ╱  DIFFERENTIAL      ╲──────────────────┐
  │        ╲ COEFFICIENT > 0? ╱                  │
  │              ╲‾‾‾‾‾‾‾‾‾‾‾╱                   │
  │                 │ YES                         │
  │                 ▼                             │
  │    ┌────────────────────────────┐            │
  │    │ CALCULATE FIRST RESPIRATION │─ S506      │
  │    │ DEPTH AT LAST RESPIRATORY   │            │
  │    │ ACTION                      │            │
  │    └────────────────────────────┘            │
  │                 │                             ▼
  │    ┌────────────────────┐      S509 ┌────────────────────┐
  │    │  INHALATION FLAG   │           │  EXHALATION FLAG   │
  │ S507│ SETTING PROCESS   │           │  SETTING PROCESS   │
  │    └────────────────────┘           └────────────────────┘
  │                 │                             │
  │    ┌────────────────────┐           ┌────────────────────┐
  │    │ INITIALIZE PEAK-HOLD│           │ INITIALIZE BOTTOM- │
  │    │ PROCESS            │           │ HOLD PROCESS       │
  │    └────────────────────┘           └────────────────────┘
  │  S508        │                             │  S510
  └──────────────▼◄─────────────────────────────┘
               ┌──────┐
               │ END  │
               └──────┘
```

Fig. 20

```
        ┌─────────────────────────────┐
        │   FIRST ZERO-CROSS TIME      │
        │   DETERMINATION PROCESS      │
        └─────────────────────────────┘
                      │
                      ▼
        ┌─────────────────────────────┐
S601 ───│   Δ MIN5(n) EXTRACTION       │
        │        PROCESS               │
        └─────────────────────────────┘
                      │
                      ▼
S602 ───┌─────────────────────────────┐
        │  Δ MIN5(n) = Δ MIN5(n−1)?    │──── YES
        │  AND F₂(n−1) = +1?           │       │
        └─────────────────────────────┘       │
                      │ NO                     │
                      ▼                        │
S603 ───┌─────────────────────────────┐       │
        │   |ΔZₐR(n)| ≤ 0.3?           │── NO ─│
        └─────────────────────────────┘       │
                      │ YES                    │
                      ▼                        ▼
              ┌─────────────┐          ┌─────────────┐
              │  F₂(n) = +1 │          │  F₂(n) = 0  │
              └─────────────┘          └─────────────┘
                      │                        │
                      └───────────┬────────────┘
                                  ▼
                              ┌───────┐
                              │  END  │
                              └───────┘
```

$\Delta MIN5(n)$ EXTRACTION PROCESS — S601

$\Delta MIN5(n) = \Delta MIN5(n-1)?$ AND $F_2(n-1) = +1?$ — S602

$|\Delta Z_{aR}(n)| \leq 0.3?$ — S603

$F_2(n) = +1$

$F_2(n) = 0$

Fig. 21

Fig. 22

RESPIRATION DEPTH ANALYSIS PROCESS

S700 — SAMPLING TIME? — NO

YES

DETERMINATION OF IMPEDANCES AT RIGHT BODY TRUNK

DETERMINE FIRST BIOELECTRICAL IMPEDANCE — S710

DETERMINE THIRD BIOELECTRICAL IMPEDANCE — S720

SMOOTHING PROCESS — S730

DETERMINATION OF IMPEDANCES AT LEFT BODY TRUNK

DETERMINE SECOND BIOELECTRICAL IMPEDANCE — S740

DETERMINE FOURTH BIOELECTRICAL IMPEDANCE — S750

SMOOTHING PROCESS — S760

CALCULATION OF RESPIRATION DEPTH AT RIGHT LUNG

FIRST CENTERING PROCESS — S770

FIRST DIFFERENCE CALCULATING PROCESS — S780

FIRST RIGHT-LUNG-RESPIRATION-DEPTH CALCULATING PROCESS — S790

THIRD DIFFERENCE CALCULATING PROCESS — S800

SECOND RIGHT-LUNG-RESPIRATION-DEPTH CALCULATING PROCESS — S810

A

Fig. 23

A

| S820 | SECOND CENTERING PROCESS |
| S830 | SECOND DIFFERENCE CALCULATING PROCESS |
| S840 | FIRST LEFT-LUNG-RESPIRATION-DEPTH CALCULATING PROCESS |
| S850 | FOURTH DIFFERENCE CALCULATING PROCESS |
| S860 | SECOND LEFT-LUNG-RESPIRATION-DEPTH CALCULATING PROCESS |

CALCULATION OF RESPIRATION DEPTH AT LEFT LUNG

RETURN

Fig. 24

# Fig. 25

THIRD DIFFERENCE
CALCULATING PROCESS

S801 — $F_2(n) = +1?$ — NO

YES

S802 — CALCULATE THIRD
CENTERING VALUE $Z_{bR0}(n)$

S803 — $Z_{bR0}(n-1) = Z_{bR0}(n)$

S804 — CALCULATE THIRD
DIFFERENCE $\Delta Z_{bR}(n)$

END

Fig. 26

```
        ╭─────────────────────────────────────╮
        │ SECOND RIGHT-LUNG-RESPIRATION-      │
        │ DEPTH CALCULATING PROCESS           │
        ╰─────────────────────────────────────╯
                        │
                        ▼ ◄──────────────────────┐
                 ╱─────────────╲                 │
        S501 ───┤   SAMPLING    │────────────────┘
                 ╲    TIME?     ╱
                        │
                        ▼
                 ╱─────────────╲           NO
        S900 ───┤  INHALATION?  │──────────────────────────┐
                 ╲─────────────╱                           │
                        │ YES                              │ S902
                        ▼                                   ▼
        S901 ┌──────────────────┐          ┌──────────────────┐
             │   PEAK-HOLD      │          │  BOTTOM-HOLD     │
             │    PROCESS       │          │    PROCESS       │
             └──────────────────┘          └──────────────────┘
                        │                           │
                        ▼ ◄─────────────────────────┘
         NO      ╱─────────────╲
       ◄────────┤  F₂(n) = +1?  │── S903
       │         ╲─────────────╱
       │                │ YES
       │                ▼
       │         ╱─────────────╲        NO
       │ S904 ──┤ DIFFERENTIAL  │───────────────────────────┐
       │         ╲COEFFICIENT>0?╱                           │
       │                │ YES                               │
       │                ▼                                   │
       │  ┌──────────────────────────┐                      │
       │  │ CALCULATE SECOND RIGHT   │── S905               │
       │  │ LUNG RESPIRATION DEPTH AT│                      │
       │  │ LAST RESPIRATORY ACTION  │                      │
       │  └──────────────────────────┘                      │
       │                │                      S908         │
       │                ▼                        ▼          ▼
       │  ┌──────────────────┐        ┌──────────────────┐
       │  │ INHALATION FLAG  │        │ EXHALATION FLAG  │
       │  │ SETTING PROCESS  │        │ SETTING PROCESS  │
       │  └──────────────────┘        └──────────────────┘
       │  S906          │                      │
       │                ▼                        ▼
       │  ┌──────────────────┐        ┌──────────────────┐
       │  │INITIALIZE PEAK-  │        │ INITIALIZE BOTTOM-│
       │  │HOLD PROCESS      │        │ HOLD PROCESS     │
       │  └──────────────────┘        └──────────────────┘
       │  S907          │                      │    S909
       │                ▼                        ▼
       └──────────────►╭─────╮◄─────────────────┘
                       │ END │
                       ╰─────╯
```

Fig. 27

EP 2 407 102 A1

Fig. 28

ABDOMINAL RESPIRATION

MEASUREMENTS BY RESPITRACE

$A_b$

$R_{ib}$

TIME

Fig. 29

COSTAL RESPIRATION

MEASUREMENTS BY RESPITRACE

$R_{ib}$

$A_b$

TIME

104

Fig. 30

Fig. 31

RESPIRATION DEPTH ANALYSIS
PROCESS

S700 — SAMPLING TIME? — NO

YES

DETERMINATION
OF IMPEDANCES
AT RIGHT BODY
TRUNK

DETERMINE FIRST
BIOELECTRICAL IMPEDANCE — S710

DETERMINE THIRD
BIOELECTRICAL IMPEDANCE — S720

SMOOTHING PROCESS — S730

DETERMINATION
OF IMPEDANCES
AT LEFT BODY
TRUNK

DETERMINE SECOND
BIOELECTRICAL IMPEDANCE — S740

DETERMINE FOURTH
BIOELECTRICAL IMPEDANCE — S750

SMOOTHING PROCESS — S760

CALCULATION OF
RESPIRATION
DEPTH AT RIGHT
LUNG

FIRST CENTERING PROCESS — S770

FIRST DIFFERENCE CALCULATING
PROCESS — S780

FIRST RIGHT-LUNG-RESPIRATION-
DEPTH CALCULATING PROCESS — S790

THIRD DIFFERENCE
CALCULATING PROCESS — S800

SECOND RIGHT-LUNG-RESPIRATION-
DEPTH CALCULATING PROCESS — S810

RIGHT $\Delta R_{ib} / \Delta A_b$ ASSUMPTION PROCESS — S815

A

Fig. 32

A

S820 ── SECOND CENTERING PROCESS

S830 ── SECOND DIFFERENCE
CALCULATING PROCESS

S840 ── FIRST LEFT-LUNG-RESPIRATION-
DEPTH CALCULATING PROCESS

S850 ── FOURTH DIFFERENCE
CALCULATING PROCESS

S860 ── SECOND LEFT-LUNG-RESPIRATION-
DEPTH CALCULATING PROCESS

CALCULATION OF
RESPIRATION
DEPTH AT LEFT
LUNG

LEFT $\Delta R_{ib}/\Delta A_b$ ASSUMPTION PROCESS ── S865

RETURN

Fig. 33

```
            ┌─────────────────────────┐
            │   RIGHT ΔR_ib/ΔA_b      │
            │  ASSUMPTION PROCESS     │
            └─────────────────────────┘
                         │
                         ▼
   S1000 ──⟨ ΔZ_aR(n) ≥ 0? ⟩────────YES────────────┐
                         │                           │
                        NO                           ▼
                         ▼                    ┌──────────────────┐
   S1010 ──┌──────────────────────┐          │ ΔR_ib/ΔA_b(n) = 1.0│
           │ ASSUME ΔR_ib/ΔA_b    │          └──────────────────┘
           └──────────────────────┘                  │
                         │◄─────────────────────────┘
                         ▼
   S1020 ──⟨ −2.5 ≤ ΔR_ib/ΔA_b(n) ≤ 4.5? ⟩───NO────┐
                         │                           │
                        YES                          ▼
                         │                    ┌──────────────────┐
                         │                    │ ΔR_ib/ΔA_b(n) = 1.0│
                         │                    └──────────────────┘
   S1030                 │                           │
                         ▼◄─────────────────────────┘
    ⟨ |ΔR_ib/ΔA_b(n)| − |ΔR_ib/ΔA_b(n−1)| > 0.3? ⟩──YES──┐
                         │                                 │
                        NO                                 ▼
                         ▼                          ┌──────────────────┐
   ┌──────────────────────────────────────┐        │ ΔR_ib/ΔA_b(n) = 1.0│
   │ [ΔR_ib/ΔA_b(n−1) + ΔR_ib/ΔA_b(n)]/2 =│        └──────────────────┘
   │        ΔR_ib/ΔA_b(n)                 │                 │
   └──────────────────────────────────────┘                 │
                         │◄─────────────────────────────────┘
                         ▼
                       ( B )
```

EP 2 407 102 A1

Fig. 34

$S1040$ — $\Delta Z_{aR}(n) < 0?$ — NO

YES

ADD ONE TO LAST COUNT VALUE $N_i(n-1)$ OF THE NUMBER OF INTEGRATIONS

INITIALIZE COUNT VALUE $N_i$ OF THE NUMBER OF INTEGRATIONS

$S1050$ — $\Delta Z_{aR}(n) < 0?$ — NO

YES

$\Sigma \Delta R_{ib}/\Delta A_b(n-1) + \Delta R_{ib}/\Delta A_b(n) = \Sigma \Delta R_{ib}/\Delta A_b(n)$

$F_1(n) = +1$

$S1060$ — $N_i(n) = 0?$ — NO

YES

$[\Sigma \Delta R_{ib}/\Delta A_b(n)]/N_i(n) = \Sigma \Delta R_{ib}/\Delta A_b/N_i(n)$

$[\Sigma \Delta R_{ib}/\Delta A_b(n-1)]/N_i(n-1) = \Sigma \Delta R_{ib}/\Delta A_b(n)/N_i(n)$

END

109

Fig. 35

Fig. 36

# Fig. 37

```
        ┌─────────────┐
        │    START    │
        └──────┬──────┘
               │
               ▼
T1 ─┤ ENTER PERSONAL │
    │  INFORMATION   │
    └───────┬────────┘
            │
            ▼
T2 ─┤   MEASURE   │
    │   WEIGHT    │
    └──────┬──────┘
           │
           ▼
T3 ─┤   RESPIRATION        │◄───┐
    │ ANALYSIS PROCESS     │    │
    └──────────┬───────────┘    │
               │                │
               ▼                │
T4 ─┤ RESPIRATION DEPTH    │    │
    │ DISPLAYING PROCESS   │────┘
    └──────────────────────┘
```

Fig. 38

```
                    ┌─────────────────────────┐
                    │  RESPIRATION ANALYSIS   │
                    │        PROCESS          │
                    └─────────────────────────┘
                                │
                                ▼ ◄──────────────┐
        T10 ⌇  ◄─────────────────────────►        │
              ⟨    SAMPLING TIME?    ⟩ NO ────────┘
                                │
                              YES
                                │
                                ▼
                    ┌─────────────────────────┐
        T20 ⌇      │    DETERMINE FIFTH      │
                    │  BIOELECTRICAL IMPEDANCE│
                    └─────────────────────────┘
                                │
                                ▼
                    ┌─────────────────────────┐
        T30 ⌇      │    DETERMINE SIXTH      │
                    │  BIOELECTRICAL IMPEDANCE│
                    └─────────────────────────┘
                                │
                                ▼
                    ┌─────────────────────────┐
        T40 ⌇      │    SMOOTHING PROCESS    │
                    └─────────────────────────┘
                                │
                                ▼
                    ┌─────────────────────────┐
        T50 ⌇      │  FIRST CENTERING PROCESS│
                    └─────────────────────────┘
                                │
                                ▼
                    ┌─────────────────────────┐
        T60 ⌇      │    FIFTH DIFFERENCE     │
                    │   CALCULATION PROCESS   │
                    └─────────────────────────┘
                                │
                                ▼
                    ┌─────────────────────────┐
        T70 ⌇      │    SIXTH DIFFERENCE     │
                    │   CALCULATION PROCESS   │
                    └─────────────────────────┘
                                │
                                ▼
                    ┌─────────────────────────┐
        T80 ⌇      │ ΔRib/ΔAb ASSUMPTION     │
                    │        PROCESS          │
                    └─────────────────────────┘
                                │
                                ▼
                    ┌─────────────────────────┐
                    │         RETURN          │
                    └─────────────────────────┘
```

At step T80: $\Delta R_{ib} / \Delta A_b$ ASSUMPTION PROCESS

# Fig. 39

$$\text{FIFTH CENTERING PROCESS}$$

T51 — MA10 CALCULATION PROCESS

T52 — MA20 CALCULATION PROCESS

T53 — MAX10 EXTRACTION PROCESS

T54 — MIN10 EXTRACTION PROCESS

T55 — MEDIAN VALUE CALCULATION PROCESS

T56 — RESPIRATION TIMING EXTRACTION PROCESS

T57 — RESPIRATION SPEED INDICATION FLAG SETTING PROCESS

T58 — FIFTH CENTERING VALUE CALCULATION PROCESS

END

Fig. 40

$$\boxed{\begin{array}{c}\text{RESPIRATION TIMING}\\\text{EXTRACTION PROCESS}\end{array}}$$

T201 — $\boxed{\begin{array}{c}\text{DIFFERENTIAL COEFFICIENT}\\\text{CALCULATION PROCESS}\end{array}}$

T202 — $|dZ_a(n)| < 0.1?$ → YES

NO

T203 — $dZ_a(n) > 0?$ → NO

YES

$\boxed{F_0(n) = +1}$    $\boxed{F_0(n) = -1}$    $\boxed{F_0(n) = 0}$

T204

$|F_0(n)| = |F_0(n-1)|?$ AND $F_0(n-1) \neq F_0(n)?$ → NO

YES

T205 — $\boxed{F_0(n) = 0}$

$\boxed{A}$

114

## Fig. 41

A

$F_0(n) = 0?$ — T206

NO → (to $F_1(n) = 0$)

YES

T207 — $\sum_{i=n-2}^{n} F_0(i) > +1?$ — YES → $F_1(n) = +1$

NO

$\sum_{i=n-2}^{n} F_0(i) < -1?$ — T208

NO → (to $F_1(n) = 0$)

YES

$F_1(n) = 0$

$F_1(n) = -1$

$F_1(n) = +1$

T209 — $F_1(n) = +1?$ — YES → T211 INITIALIZE SAMPLING COUNTER VALUE

NO

T210 — ADD ONE TO SAMPLING COUNTER VALUE

END

Fig. 42

RESPIRATION SPEED INDICATION
FLAG SETTING PROCESS

$F_0(n) = 0?$ ~T301 — NO

YES

T302 — $F_1(n) = +1?$ — YES

NO

$F_1(n) = -1?$ ~T304 — NO

YES — $N(n-1) > 10?$ ~T303

YES

NO

B

T305 — $N(n-1) > 5?$ — YES

NO

$F_{ma}(n) = F_{ma}(n-1)$

B

$F_{ma}(n) = 20$

$F_{ma}(n) = 10$

END

Fig. 43

FIFTH CENTERING VALUE
CALCULATION PROCESS

T401 — $F_{ma}(n) = 10?$  —NO

YES

T402 — $[Z_{a0}(n-2) + Z_{a0}(n-1) + MA10(n)]/3 = Z_{a0}(n)$

$[Z_{a0}(n-2) + Z_{a0}(n-1) + MA20(n)]/3 = Z_{a0}(n)$ — T403

END

Fig. 44

## Fig. 45

$$\text{SIXTH DIFFERENCE CALCULATION PROCESS}$$

T71 — $\Delta \text{MIN5}(n)$ EXTRACTION PROCESS

T72 — $\Delta \text{MIN5}(n) = \Delta \text{MIN5}(n-1)$? AND $F_2(n-1) = +1$? — YES

NO

T73 — $|\Delta Z_a(n)| \leq 0.3$? — NO

YES

$F_2(n) = +1$

$F_2(n) = 0$

T74 — $F_2(n) = +1$? — NO

YES

T75 — CALCULATE SIXTH CENTERING VALUE $Z_{b0}(n)$

$Z_{b0}(n-1) = Z_{b0}(n)$

T76 — CALCULATE SIXTH DIFFERENCE $\Delta Z_b(n)$

END

Fig. 46

Fig. 47

Fig. 48

$(C)$

T85 — $\Delta Z_a(n) < 0?$ —NO→

↓YES

ADD ONE TO LAST COUNT VALUE $N_i(n-1)$ OF THE NUMBER OF INTEGRATIONS

INITIALIZE COUNT VALUE $N_i$ OF THE NUMBER OF INTEGRATIONS

T86 — $\Delta Z_a(n) < 0?$ —NO→

↓YES

$\sum \Delta R_{ib} / \Delta A_b(n-1) + \Delta R_{ib} / \Delta A_b(n) = \sum \Delta R_{ib} / \Delta A_b(n)$

$F_1(n) = +1$

T87 — $N_i(n) = 0?$ —NO→

↓YES

$[\sum \Delta R_{ib} / \Delta A_b(n)]/N_i(n) = \sum \Delta R_{ib} / \Delta A_b / N_i(n)$

$[\sum \Delta R_{ib} / \Delta A_b(n-1)]/N_i(n-1) = \sum \Delta R_{ib} / \Delta A_b(n)/N_i(n)$

$(\text{END})$

## Fig. 49

RESPIRATION DEPTH

COSTAL

ABDOMINAL

FIVE DEGREES

FIVE DEGREES

BG7

ABDOMINAL
RESPIRATION
PERCENTAGE
LEVEL

0 - 20

21 - 40

41 - 60

61 - 80

81 - 100

BG8

## Fig. 50

```
                    ┌─────────────────────────┐
                    │   RESPIRATION DEPTH      │
                    │  CALCULATION PROCESS     │
                    └─────────────────────────┘
                              │        ◄─────────────────────┐
                              ▼                               │
              T501      ╱───────────────╲                     │
                       ⟨    SAMPLING     ⟩                    │
                       ⟨     TIME?       ⟩                    │
                        ╲───────────────╱                     │
                              │                               │
                              ▼                               │
              T502      ╱───────────────╲      NO             │
                       ⟨   INHALATION?   ⟩──────────────┐     │
                        ╲───────────────╱               │     │
                              │ YES                      │     │
                              ▼                          ▼          T504
              T503   ┌─────────────────┐         ┌─────────────────┐
                     │   PEAK-HOLD     │         │  BOTTOM-HOLD    │
                     │   PROCESS       │         │   PROCESS       │
                     └─────────────────┘         └─────────────────┘
                              │                          │
                              ▼   ◄──────────────────────┘
              NO       ╱───────────────────╲
             ┌────────⟨  ZERO-CROSS TIME?   ⟩  T505
             │         ╲───────────────────╱
             │                │ YES
             │                ▼
             │   T506   ╱───────────────────╲    NO
             │         ⟨   DIFFERENTIAL      ⟩────────────┐
             │         ⟨  COEFFICIENT > 0?   ⟩            │
             │          ╲───────────────────╱            │
             │                │ YES                       │
             │                ▼                           │
             │   ┌────────────────────────────┐           │
             │   │ CALCULATE RESPIRATION DEPTH │  T507     │
             │   │ AT LAST RESPIRATORY ACTION  │           │
             │   └────────────────────────────┘           │
             │                │                            │
             │                ▼                            ▼              T510
             │   ┌─────────────────────┐      ┌─────────────────────┐
             │   │  INHALATION FLAG    │      │   EXHALATION FLAG   │
             │   │  SETTING PROCESS    │      │   SETTING PROCESS   │
             │   └─────────────────────┘      └─────────────────────┘
             │  T508         │                         │
             │                ▼                         ▼
             │   ┌─────────────────────┐      ┌─────────────────────┐
             │   │ INITIALIZE PEAK-HOLD│      │ INITIALIZE BOTTOM-  │
             │   │      PROCESS        │      │   HOLD PROCESS      │
             │   └─────────────────────┘      └─────────────────────┘
             │  T509         │                         │      T511
             │                ▼                         │
             └───────────────►●◄────────────────────────┘
                              │
                              ▼
                          ┌───────┐
                          │  END  │
                          └───────┘
```

Fig. 51

```
          ⎛ RESPIRATION DEPTH  ⎞
          ⎝ DISPLAYING PROCESS ⎠
                    │
                    ▼
T601 ─┤ NORMALIZE RESPIRATION DEPTH AT
       │ LAST SINGLE RESPIRATION
                    │
                    ▼
T602 ─┤ DECIDE THE NUMBER OF DEGREE
       │ INDICATORS TO BE COLORED
                    │
                    ▼
T603 ─┤ DECIDE ABDOMINAL RESPIRATION
       │ PERCENTAGE LEVEL
                    │
                    ▼
T604 ─┤ DEGREE-NUMBER DISTRIBUTION
       │ PROCESS
                    │
                    ▼
T605 ─┤ DECIDE MARGIN LEVEL
       │ CORRESPONDING TO RESPIRATION
       │ SPEED
                    │
                    ▼
T606 ─┤ DISPLAY BAR GRAPHS
```

Fig. 52

Fig. 53

## Fig. 54

```
        ╭─────────────────────────╮
        │   RESPIRATION TYPE      │
        │ DETERMINATION PROCESS   │
        ╰─────────────────────────╯
                    │
                    ▼
   T701
        ╱───────────────────────╲        NO
       ⟨  [Σ ΔR_ib / ΔA_b]/N_i ≤ 1.0?  ⟩ ─────┐
        ╲───────────────────────╱             │
                    │ YES                      │
                    ▼                          ▼
   T702 ┌──────────────┐      T703 ┌────────────────────────┐
        │  ABDOMINAL   │           │ RETRIEVE Z_b1 AND ΔZ_b1│
        │ RESPIRATION  │           └────────────────────────┘
        └──────────────┘                      │
                    │         T704            ▼
                    │        ╱──────────────────╲      NO
                    │       ⟨ Z_b0 ≥ Z_b1 + ΔZ_b1? ⟩ ─────┐
                    │        ╲──────────────────╱         │
                    │                  │ YES              │
                    │                  ▼            T706  ▼
                    │     T705 ┌──────────────┐   ┌──────────────┐
                    │          │   DRAW-IN    │   │   COSTAL     │
                    │          │ RESPIRATION  │   │ RESPIRATION  │
                    │          └──────────────┘   └──────────────┘
                    │                  │                  │
                    ▼◄─────────────────┴──────────────────┘
              ╭─────────╮
              │   END   │
              ╰─────────╯
```

$$[\Sigma \Delta R_{ib} / \Delta A_b]/N_i \leq 1.0?$$

$$Z_{b0} \geq Z_{b1} + \Delta Z_{b1}?$$

RETRIEVE $Z_{b1}$ AND $\Delta Z_{b1}$

Fig. 55

Fig. 56

Fig. 57

Fig. 58

## Fig. 59

LISSAJOUS FIGURE DISPLAYING PROCESS

T801 — SAMPLING TIME? — NO

YES

T802 — DETERMINE FIFTH BIOELECTRICAL IMPEDANCE

T803 — DETERMINE SIXTH BIOELECTRICAL IMPEDANCE

T804 — SMOOTHING PROCESS

T805 — GENERATE DISPLAY DATA FOR DISPLAYING LISSAJOUS FIGURE

T806 — DISPLAY LISSAJOUS FIGURE

RETURN

## Fig. 59A

```
          ┌─────────────────────────────┐
          │  LISSAJOUS FIGURE DISPLAYING │
          │           PROCESS            │
          └─────────────────────────────┘
                        │
                        ▼
  T801 ⌇    ⟨   SAMPLING TIME?   ⟩────NO──┐
                        │                  │
                       YES                 │
                        ▼                  │
  T802A ⌇  ┌─────────────────────────────┐│  ┐
           │      DETERMINE FIRST         ││  │ DETERMINATION
           │  BIOELECTRICAL IMPEDANCE     ││  │ OF IMPEDANCES
           └─────────────────────────────┘│  │ AT RIGHT BODY
                        ▼                  │  │ TRUNK
  T803A ⌇  ┌─────────────────────────────┐│  │
           │      DETERMINE THIRD         ││  │
           │  BIOELECTRICAL IMPEDANCE     ││  │
           └─────────────────────────────┘│  │
                        ▼                  │  │
  T804A ⌇  ┌─────────────────────────────┐│  │
           │      SMOOTHING PROCESS       ││  ┘
           └─────────────────────────────┘│
                        ▼                  │
  T802B ⌇  ┌─────────────────────────────┐│  ┐
           │     DETERMINE SECOND         ││  │ DETERMINATION
           │  BIOELECTRICAL IMPEDANCE     ││  │ OF IMPEDANCES
           └─────────────────────────────┘│  │ AT LEFT BODY
                        ▼                  │  │ TRUNK
  T803B ⌇  ┌─────────────────────────────┐│  │
           │     DETERMINE FOURTH         ││  │
           │  BIOELECTRICAL IMPEDANCE     ││  │
           └─────────────────────────────┘│  │
                        ▼                  │  │
  T804B ⌇  ┌─────────────────────────────┐│  │
           │      SMOOTHING PROCESS       ││  ┘
           └─────────────────────────────┘│
                        ▼                  │
  T805A ⌇  ┌─────────────────────────────┐│
           │   GENERATE DISPLAY DATA      ││
           │ FOR DISPLAYING LISSAJOUS     ││
           │   FIGURE FOR RIGHT LUNG      ││
           └─────────────────────────────┘│
                        ▼                  │
  T805B ⌇  ┌─────────────────────────────┐│
           │   GENERATE DISPLAY DATA      ││
           │ FOR DISPLAYING LISSAJOUS     ││
           │   FIGURE FOR LEFT LUNG       ││
           └─────────────────────────────┘│
                        ▼                  │
  T806 ⌇   ┌─────────────────────────────┐│
           │  DISPLAY LISSAJOUS FIGURES   │┘
           └─────────────────────────────┘
                        ▼
                 (   RETURN   )
```

Fig. 60

COSTAL
RESPIRATION

$Z_a$ [Ω]

ABDOMINAL
RESPIRATION

$Z_b$ [Ω]

Fig. 61

$Z_b$ [Ω]

$Z_a$ [Ω]

Fig. 62

ABDOMINAL
RESPIRATION

$Z_b$ [Ω]

COSTAL
RESPIRATION

$Z_a$ [Ω]

Fig. 63

RIGHT LUNG

LEFT LUNG

$Z_{aR}, Z_{aL}$ [Ω]

$Z_{bR}, Z_{bL}$ [Ω]

Fig. 64

RIGHT LUNG

LEFT LUNG

$Z_{aR}, Z_{aL}$ [Ω]

$Z_{bR}, Z_{bL}$ [Ω]

Fig. 65

RIGHT LUNG

LEFT LUNG

$Z_{aR}, Z_{aL}$ [Ω]

AVERAGE

$Z_{bR}, Z_{bL}$ [Ω]

Fig. 66

$C(Z_{b0}, Z_{a0})$

160A

Fig. 67

160A

Fig. 68

Fig. 69

Fig. 70

Fig. 71

TL: TARGET

ML: ACTUALLY MEASURED

$Z_a$ [Ω]

$Z_b$ [Ω]

Fig. 72

LN

α

β

$Z_a$ [Ω]

$Z_b$ [Ω]

Fig. 73

α

β

LN

$Z_a$ [Ω]

$Z_b$ [Ω]

## Fig. 74

## Fig. 75A

PRESENT

TIME ELAPSED [sec]

MEASUREMENT START

## Fig. 75B

PRESENT

LOG OF TIME ELAPSED [sec]

MEASUREMENT START

## Fig. 75C

## Fig. 76

# Fig. 77

BG9

INHALATION

EXHALATION

THREE DEGREES

THREE DEGREES

Fig. 78

| MIDDLE DEPTH INHALATION | MIDDLE DEPTH INHALATION | MIDDLE DEPTH EXHALATION | MIDDLE DEPTH EXHALATION |

## Fig. 79

SMALL ←————————————————→ LARGE

## Fig. 80

SMALL ←————————————————→ LARGE

Fig. 81

Fig. 82

## Fig. 83

TBL: TRAINING MENU
MANAGEMENT TABLE

| RANKING | BREATHING TRAINING MENUS | REQUIREMENT FOR ADVANCING THROUGH RANKING |
|---|---|---|
| 5 | MENU 81 → MENU 82 → MENU 83 → ⋯⋯ → MENU 100 | REQUIREMENT E |
| 4 | MENU 61 → MENU 62 → MENU 63 → ⋯⋯ → MENU 80 | REQUIREMENT D |
| 3 | MENU 41 → MENU 42 → MENU 43 → ⋯⋯ → MENU 60 | REQUIREMENT C |
| 2 | MENU 21 → MENU 22 → MENU 23 → ⋯⋯ → MENU 40 | REQUIREMENT B |
| 1 | MENU 01 → MENU 02 → MENU 03 → ⋯⋯ → MENU 20 | REQUIREMENT A |

RESPIRATION CAPABILITY — HIGH / LOW

Fig. 84

```
                    ┌─────────────────────────────┐
                    │   BREATHING TRAINING        │
                    │   MANAGEMENT PROCESS        │
                    └─────────────────────────────┘
                                 │
                                 ▼
                    ┌─────────────────────────────┐
         T901 ──────│   DETERMINE RESPIRATION     │
                    │   CAPABILITY                │
                    └─────────────────────────────┘
                                 │
                                 ▼
                    ┌─────────────────────────────┐
         T902 ──────│   IDENTIFY RANKING          │
                    └─────────────────────────────┘
                                 │
                                 ▼
                    ┌─────────────────────────────┐
         T903 ──────│   SELECT TRAINING MENUS     │
                    └─────────────────────────────┘
                                 │
                                 ▼
                    ┌─────────────────────────────┐
         T904 ──────│ BREATHING TRAINING PROCESS  │
                    └─────────────────────────────┘
                                 │
                                 ▼
                    ╱─────────────────────────────╲
                   ╱    REQUIREMENT FOR            ╲   NO
         T905 ────〈    ADVANCING THROUGH           〉──────
                   ╲    RANKING IS SATISFIED?      ╱
                    ╲─────────────────────────────╱
                                 │ YES
                                 ▼
                    ┌─────────────────────────────┐
         T906 ──────│   ADVANCE TO NEXT RANKING   │
                    └─────────────────────────────┘
```

Fig. 85

160A

## Fig. 86

## Fig. 87

Europäisches
Patentamt

European
Patent Office

Office européen
des brevets

**EUROPEAN SEARCH REPORT**

Application Number

EP 11 17 3346

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/043302 A1 (MASUO YOSHIHISA [JP]) 22 February 2007 (2007-02-22) * paragraphs [0397] - [0458]; figures 24-25 * | 1,42,44 | INV. A61B5/08 A61B5/053 |
| A | WO 2009/035965 A1 (CARDINAL HEALTH 207 INC [US]; WEILER NORBERT [DE]) 19 March 2009 (2009-03-19) * paragraphs [0023] - [0030]; figures 1,3,4 * | 1,42,44 | |
| A | FR 2 020 493 A5 (ANVAR) 10 July 1970 (1970-07-10) * page 1, paragraph 15-25 * * figures * | 1,42,44 | |

TECHNICAL FIELDS
SEARCHED (IPC)

A61B

The present search report has been drawn up for all claims

| Place of search | Date of completion of the search | Examiner |
|---|---|---|
| Munich | 21 October 2011 | Rosenblatt, Thomas |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

& : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 11 17 3346

This annex lists the patent family members relating to the patent documents cited in the above-mentioned European search report.
The members are as contained in the European Patent Office EDP file on
The European Patent Office is in no way liable for these particulars which are merely given for the purpose of information.

21-10-2011

| Patent document cited in search report | | Publication date | Patent family member(s) | | Publication date |
|---|---|---|---|---|---|
| US 2007043302 | A1 | 22-02-2007 | EP 1754442 A2 | | 21-02-2007 |
| | | | JP 2007050127 A | | 01-03-2007 |
| WO 2009035965 | A1 | 19-03-2009 | AU 2008299098 A1 | | 19-03-2009 |
| | | | CA 2699281 A1 | | 19-03-2009 |
| | | | CN 101801265 A | | 11-08-2010 |
| | | | EP 2194863 A1 | | 16-06-2010 |
| | | | JP 2010538748 A | | 16-12-2010 |
| | | | US 2009118634 A1 | | 07-05-2009 |
| FR 2020493 | A5 | 10-07-1970 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007043302 A1